# EUROPÄISCHE PATENTSCHRIFT

(11) **EP 1 693 380 B1**
(45) Veröffentlichungstag und Bekanntmachung des Hinweises auf die Patenterteilung: **22.04.2009**
(21) Anmeldenummer: 06110205.9
(22) Anmeldetag: 31.10.2002
(51) Int. Cl.: C07K 14/34, C12P 13/08, C12Q 1/68, C12N 15/63, C12N 15/77, C12N 1/21, C12P 1/04, C12R 1/15

(54) **Nukleinsäuresequenz, die für das OPCA Gen kodiert**
Nucleic acid sequence encoding the OPCA gene
Séquence d'acide nucléique codante pour le gène OPCA

(30) Priorität: 05.11.2001 DE 10154180
(43) Veröffentlichungstag der Anmeldung: 23.08.2006
(62) Teilanmeldung aus: 02796537.5
(73) Patentinhaber: Paik Kwang Industrial Co., Ltd., Jeollabuk-do (KR)
(72) Erfinder: Zelder, Oskar, 67346 Speyer (DE); Pompejus, Markus, Yongsan-gu, Seoul 140-210 (KR); Schröder, Hartwig, 69226 Nußloch (DE); Kröger, Burkhard, 67117 Limburgerhof (DE); Klopprogge, Corinna, 68239 Mannheim (DE); Haberhauer, Gregor, 67117 Limburgerhof (DE)
(74) Vertreter: Maiwald Patentanwalts GmbH

(56) Entgegenhaltungen:
- WO-A-01/00804
- WO-A-01/04322

## Beschreibung

### Hintergrund der Erfindung

Bestimmte Produkte und Nebenprodukte von natürlich vorkommenden Stoffwechselprozessen in Zellen werden in vielen Industriezweigen verwendet, einschließlich der Nahrungsmittel-, Futtermittel-, Kosmetik- und pharmazeutischen Industrie. Diese Moleküle, die gemeinsam als "Feinchemikalien" bezeichnet werden, umfassen organische Säuren, sowohl proteinogene als auch nicht-proteinogene Aminosäuren, Nukleotide und Nukleoside, Lipide und Fettsäuren, Diole, Kohlehydrate, aromatische Verbindungen, Vitamine und Cofaktoren sowie Enzyme. Ihre Produktion erfolgt am besten mittels Anzucht von Bakterien im Großmaßstab, die entwickelt wurden, um große Mengen des jeweils gewünschten Moleküls zu produzieren und sezernieren. Ein für diesen Zweck besonders geeigneter Organismus ist *Corynebacterium glutamicum*, ein gram-positives, nichtpathogenes Bakterium. Über Stammselektion ist eine Reihe von Mutantenstämmen entwickelt worden, die ein Sortiment wünschenswerter Verbindungen produzieren. Die Auswahl von Stämmen, die hinsichtlich der Produktion eines bestimmten Moleküls verbessert sind, ist jedoch ein zeitaufwendiges und schwieriges Verfahren.

Aus WO 01/00804 sind eine Reihe von Genen aus *C. glutamicum* bekannt, sie Stress-, Resiztenz- und Toleranzproteine kodieren. Dort wird auch die Sequenz für das opca-Gen offenbart.

WO 01/09322 offenbart ebenfalls das opca-Gen aus *C. glutamicum*.

### Zusammenfassung der Erfindung

Diese Erfindung stellt neuartige Nukleinsäuremoleküle bereit, die sich zur Identifizierung oder Klassifizierung von *Corynebacterium glutamicum* oder verwandten Bakterienarten verwenden lassen. *C. glutamicum* ist ein gram-positives, aerobes Bakterium, das gewöhnlich in der Industrie für die Produktion im Großmaßstab einer Reihe von Feinchemikalien und auch zum Abbau von Kohlenwasserstoffen (bspw. beim Überlaufen von Rohöl) und zur Oxidation von Terpenoiden gemeinhin verwendet wird. Die Nukleinsäuremoleküle können daher zum Identifizieren von Mikroorganismen eingesetzt werden, die sich zur Produktion von Feinchemikalien, bspw. durch Fermentationsverfahren, verwenden lassen. *C. glutamicum* selbst ist zwar nicht-pathogen, jedoch ist es mit anderen Corynebacterium-Arten, wie *Corynebacterium diphtheriae* (dem Erreger der Diphtherie) verwandt, die bedeutende Pathogene beim Menschen sind. Die Fähigkeit, das Vorhandensein von Corynebacterium-Arten zu identifizieren, kann daher auch eine signifikante klinische Bedeutung haben, z.B. bei diagnostischen Anwendungen. Diese Nukleinsäuremoleküle können zudem als Bezugspunkte zur Kartierung des *C. glutamicum*-Genoms oder von Genomen verwandter Organismen dienen.

Diese neuen Nukleinsäuremoleküle codieren Proteine, die hier als Genstabilitäts-, Genexpressions- oder Proteinsekretions-/Faltungs- (SES-) Proteine bezeichnet werden. Diese SES-Proteine können bspw. eine an der Reparatur oder Rekombination von DNA, Transposition von genetischem Material, Expression von Genen (d. h. die an der Transkription oder Translation beteiligt sind), Proteinfaltung oder Proteinsekretion in *C. glutamicum* beteiligte Funktion ausüben. Aufgrund der Verfügbarkeit von in *Corynebacterium glutamicum* verwendbaren Klonierungsvektoren, wie bspw. offenbart in Sinskey et al., US-Patent Nr. 4 649 119, und von Techniken zur genetischen Manipulation von *C. glutamicum* und den verwandten *Brevibacterium*-Arten (z.B. *lactofermentum*) (Yoshihama et al., J. Bacteriol. 162:591-597 (1985); Katsumata et al., J. Bacteriol. 159:306-311 (1984); und Santamaria et al. J. Gen. Microbiol. 130:2237-2246 (1984)), lassen sich die erfindungsgemäßen Nukleinsäuremoleküle zur genetischen Manipulation dieses Organismus verwenden, damit er ein effizienterer Produzent einer oder mehrerer Feinchemikalien wird. Diese verbesserte Produktion oder Effizienz der Produktion einer Feinchemikalie kann aufgrund einer direkten Auswirkung der Manipulation eines erfindungsgemäßen Gens oder aufgrund einer indirekten Auswirkung einer solchen Manipulation erfolgen.

Es gibt eine Reihe von Mechanismen, durch die die Veränderung eines erfindungsgemäßen SES-Proteins die Ausbeute, Produktion und/oder Effizienz der Produktion einer Feinchemikalie aus einem *C. glutamicum*-Stamm, der dieses veränderte Protein enthält, direkt beeinflussen kann. Zum Beispiel sollte die Modulation von Proteinen, die direkt an der Transkription oder Translation beteiligt sind (z.B. Polymerasen oder Ribosomen), so daß ihre Anzahl oder Aktivität gesteigert wird, die zelluläre Transkription oder Translation (oder die Geschwindigkeiten dieser Prozesse) insgesamt steigern. Diese erhöhte zelluläre Genexpression sollte solche Proteine umfassen, die an der Feinchemikalienbiosynthese beteiligt sind, so daß eine Steigerung der Ausbeute, Produktion oder Effizienz der Produktion einer oder mehrerer gewünschten Verbindungen erfolgen kann. Modifikationen der Transkriptions-/Translations-Proteinmaschinerie von *C. glutamicum*, so daß die Regulation dieser Proteine verändert wird, kann auch die erhöhte Expression von Genen, die an der Produktion von Feinchemikalien beteiligt sind, ermöglichen. Die Modulation der Aktivität einer Reihe von Proteinen, die an der Peptidfaltung beteiligt sind, kann eine Erhöhung der Gesamtproduktion korrekt gefalteter Moleküle in der Zelle ermöglichen, wodurch die Möglichkeit erhöht wird, daß gewünschte Proteine (z.B. Feinchemikalienbiosynthese-Proteine) richtig funktionieren. Ferner kann es durch Mutation von an der Sekretion aus *C. glutamicum* beteiligten Proteinen, so daß ihre Anzahl oder Aktivität erhöht ist, möglich sein, die Sekretion einer Feinchemikalie (z.B. eines Enzyms) aus Zellen in der Fermentationskultur zu erhöhen, aus der sie leicht gewonnen werden kann.

Die genetische Modifikation der erfindungsgemäßen SES-Moleküle kann auch zu einer indirekten Modulation der Produktion einer oder mehrerer Feinchemikalien führen. Beispielsweise kann man durch Erhöhen der Anzahl oder Aktivität eines erfindungsgemäßen DNA-Reparatur- oder -Rekombinationsproteins die Fähigkeit der Zelle, eine DNA-Schädigung zu entdecken und zu reparieren, erhöhen. Dies sollte die Fähigkeit der Zelle, ein mutiertes Gen in ihrem Genom zu halten, wirksam erhöhen und dadurch die Wahrscheinlichkeit erhöhen, daß ein gentechnologisch in *C. glutami*cum eingebrachtes Transgen (das z.B. ein Protein codiert, das die Biosynthese einer Feinchemikalie steigert) nicht während der Züchtung des Mikroorganismus verloren geht. Dagegen kann es durch Verringern der Anzahl oder Aktivität eines oder mehrerer DNA-Reparatur- oder -Rekombinationsproteine möglich sein, die genetische Instabilität des Organismus zu steigern. Diese Manipulationen sollten die Fähigkeit des Organismus, durch Mutagenese modifiziert zu werden, verbessern, ohne daß die eingebrachte Mutation berichtigt wird. Das gleiche gilt für Proteine, die an der Transposition oder Umlagerung genetischer Elemente in *C. glutamicum* beteiligt sind (z.B. Transposons). Durch Mutagenese dieser Proteine, so daß ihre Anzahl oder Aktivität entweder gesteigert oder verringert wird, ist es möglich, gleichzeitig die genetische Stabilität des Mikroorganismus zu steigern oder zu verringern. Dies hat eine bedeutende Auswirkung darauf, daß eine andere Mutation in *C. glutamicum* eingebracht und die eingebrachte Mutation beibehalten werden kann. Transposons bieten ebenfalls einen geeigneten Mechanismus, durch den die Mutagenese von *C. glutamicum* durchgeführt werden kann; die Duplikation gewünschter Gene (z.B. von Feinchemikalienbiosynthese-Genen) läßt sich leicht mittels Transposonmutagenese durchführen, wie auch die Disruption ungewünschter Gene (z.B. Gene, die am Abbau gewünschter Feinchemikalien beteiligt sind).

Durch die Modulation eines oder mehrerer Proteine (z.B. Sigma-Faktoren), die an der Regulation der Transkription oder Translation in Reaktion auf besondere Umweltbedingungen beteiligt sind, kann es möglich sein, die Zelle daran zu hindern, daß sie die Proteinsynthese unter ungünstigen Umweltbedingungen, wie man sie in einer Fermenterkultur im Großmaßstab antrifft, verlangsamt oder beendet. Dies sollte zu erhöhter Genexpression führen, was wiederum die gesteigerte Biosynthese gewünschter Feinchemikalien unter diesen Bedingungen ermöglichen kann. Die Mutagenese von an Sekretionssystemen beteiligten Proteinen kann zu modulierten Sekretionsraten führen. Viele dieser sezernierten Proteine haben Funktionen, die für die Zellebensfähigkeit wichtig sind (z.B. Zelloberflächenproteasen oder -Rezeptoren). Eine Änderung des Sekretionswegs, so daß diese Proteine leichter an ihren extrazellulären Ort transportiert werden, kann die Gesamtlebensfähigkeit der Zelle erhöhen und somit zu höheren Zahlen an *C. glutamicum*-Zellen führen, die Feinchemikalien während eine Züchtung im Großmaßstab produzieren können. Ferner ist der Sekretionsapparat (z.B. das sec-System) bekanntlich auch an der Insertion von integralen Membranproteinen (z.B. Poren, Kanälen oder Transportern) in die Membran beteiligt. So kann die Modulation der Aktivität von Proteinen, die an der Proteinsekretion aus *C. glu*tamicum beteiligt sind, die Fähigkeit der Zelle zur Ausscheidung von Abfallprodukten oder zum Import notwendiger Metabolite beeinflussen. Ist die Aktivität dieser sekretorischen Proteine erhöht, kann ebenfalls die Fähgikeit der Zelle zur Produktion von Feinchemikalien erhöht sein. Ist die Aktivität dieser sekretorischen Proteine verringert, können nicht genügend Nährstoffe zur Unterstützung der Überproduktion gewünschter Verbindungen vorhanden sein, oder Abfallprodukte können diese Biosynthese stören.

Die Erfindung beschreibt neue Nukleinsäuremoleküle, die Proteine codieren, die hier als SES-Proteine bezeichnet werden und bspw an der Reparatur oder Rekombination von DNA, Transposition von genetischem Material, Expression von Genen (d. h. den Transkriptions- oder Translationsprozessen), Proteinfaltung oder Proteinsekretion in *Corynebacterium glutamicum* beteiligt sein können. Nukleinsäuremoleküle, die ein SES-Protein codieren, werden hier als SES-Nukleinsäuremoleküle bezeichnet. Bei einer bevorzugten Ausführungsform ist ein SES-Protein an der Verbesserung oder Verringerung der genetischen Stabilität in *C. glutami*cum, der Expression von Genen (z.B. bei der Transkription oder Translation) oder der Proteinfaltung in diesem Organismus oder an der Proteinsekretion aus *C. glutamicum* beteiligt. Beispiele für solche Proteine sind diejenigen, die von den in Tabelle 1 angegebenen Genen codiert werden.

Ein Aspekt der Erfindung beschreibt folglich isolierte Nukleinsäuremoleküle (bspw. cDNAs), umfassend eine Nukleotidsequenz, die ein SES-Protein oder biologisch aktive Abschnitte davon codiert, sowie Nukleinsäurefragmente, die sich als Primer oder Hybridisierungssonden zum Nachweis oder zur Amplifikation von SEScodierender Nukleinsäure (bspw. DNA oder mRNA) eignen. Bei besonders bevorzugten Ausführungsformen umfaßt das isolierte Nukleinsäuremolekül eine der in Anhang A aufgeführten Nukleotidsequenzen oder den codierenden Bereich einer dieser Nukleotidsequenzen oder ein Komplement davon. In anderen bevorzugten Ausführungsformen codiert das isolierte Nukleinsäuremolekül eine der in Anhang B aufgeführten Aminosäuresequenzen. Die bevorzugten erfindungsgemäßen SES-Proteine besitzen ebenfalls vorzugsweise mindestens eine der hier beschriebenen SES-Aktivitäten.

Als Anhang A werden im folgenden die Nukleinsäuresequenzen des Sequenzprotokolls zusammen mit den in Tabelle 1 beschriebenen Sequenzveränderungen an der jeweiligen Position definiert.

Als Anhang B werden im folgenden die Polypeptidsequenzen des Sequenzprotokolls zusammen mit den in Tabelle 1 beschriebenen Sequenzveränderungen an der jeweiligen Position definiert.

Bei einer weiteren Ausführungsform ist ein isolierte Nukleinsäuremolekül beschrieben, das mindestens 15 Nukleotide lang ist und hybridisiert unter stringenten Bedingungen an ein Nukleinsäuremolekül, das eine Nukleotidsequenz aus Anhang A umfaßt. Das isolierte Nukleinsäuremolekül entspricht vorzugsweise einem natürlich vorkommenden Nukleinsäuremolekül. Die isolierte Nukleinsäure codiert stärker bevorzugt ein natürlich vorkommendes *C. glutamicum*-SES-Protein oder einen biologisch aktiven Abschnitt davon.

Ein weiterer Aspekt der Erfindung beschreibt Vektoren, bspw. rekombinante Expressionsvektoren, die die erfindungsgemäßen Nukleinsäuremoleküle enthalten, und Wirtszellen, in die diese Vektoren eingebracht worden sind. Bei einer Ausführungsform wird diese Wirtszelle zur Herstellung eines SES-Proteins verwendet, indem die Wirtszelle in einem geeigneten Medium gezüchtet wird. Das SES-Protein kann dann aus dem Medium oder der Wirtszelle isoliert werden.

Ein weiterer Aspekt der Erfindung beschreibt einen genetisch veränderten Mikroorganismus, bei dem ein SES-Gen eingebracht oder verändert worden ist. Das Genom des Mikroorganismus ist bei einer Ausführungsform durch Einbringen mindestens eines erfindungsgemäßen Nukleinsäuremoleküls verändert worden, das die mutierte SES-Sequenz als Transgen codiert. Bei einer anderen Ausführungsform wird ein endogenes SES-Gen beschrieben, das im Genom des Mikroorganismus durch homologe Rekombination mit einem veränderten SES-Gen verändert, z.B. funktionell disruptiert worden ist. Der Mikroorganismus gehört bei einer bevorzugten Ausführungsform zur Gattung *Corynebacterium* oder *Brevibacterium*, wobei *Corynebacterium glutamicum* besonders bevorzugt ist. Der Mikroorganismus wird in einer bevorzugten Ausführungsform auch zur Herstellung einer gewünschten Verbindung, wie einer Aminosäure, verwendet, wobei Lysin besonders bevorzugt ist.

Ein weiterer Aspekt der Erfindung beschreibt ein isoliertes SES-Protein oder einen Abschnitt, bspw. einen biologisch aktiven Abschnitt, davon. Das isolierte SES-Protein oder sein Abschnitt kann in einer bevorzugten Ausführungsform an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teilnehmen. Bei einer weiteren bevorzugten Ausführungsform ist das isolierte SES-Protein oder ein Abschnitt davon hinreichend homolog zu einer Aminosäuresequenz von Anhang B, so daß das Protein oder sein Abschnitt die Fähigkeit behält, bspw. an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teilzunehmen.

Eine weitere beschriebene Ausführungsform sind Wirtszellen, die mehr als eine der in Anhang A beschriebenen Nukleinsäuremoleküle besitzen. Solche Wirtszellen lassen sich auf verschiedene dem Fachmann bekannte Wege herstellen. Beispielsweise können sie durch Vektoren, die mehrere der erfindungsgemäßen Nukleinsäuremoleküle tragen, transfiziert werden. Es ist aber auch möglich mit einem Vektor jeweils ein erfindungsgemäßes Nukleinsäuremolekül in die Wirtszelle einzubringen und deshalb mehrere Vektoren entweder gleichzeitig oder zeitlich abgestuft einzusetzen. Es können somit Wirtszellen konstruiert werden, die zahlreiche, bis zu mehreren Hundert der erfindungsgemäßen Nukleinsäuresequenzen tragen. Durch eine solche Akkumulation lassen sich häufig überadditive Effekte auf die Wirtszelle hinsichtlich der Feinchemikalien-Produktivität erzielen.

Die Erfindung beschreibt zudem ein isoliertes SES-Proteinpräparat bereit. Das SES-Protein umfaßt bei bevorzugten Ausführungsformen eine Aminosäuresequenz aus Anhang B. Bei einer weiteren bevorzugten Ausführungsform betrifft die Erfindung ein isoliertes vollängenprotein, das zu einer vollständigen Aminosäuresequenz aus Anhang B (welche von einem offenen Leseraster in Anhang A codiert wird) im wesentlichen homolog ist.

Das SES-Polypeptid oder ein biologisch aktiver Abschnitt davon kann mit einem Nicht-SES-Polypeptid funktionsfähig verbunden werden, damit ein Fusionsprotein entsteht. Dieses Fusionsprotein hat bei bevorzugten Ausführungsformen eine andere Aktivität als das SES-Protein allein. Bei anderen bevorzugten Ausführungsformen nimmt dieses Fusionsprotein an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teil. Die Integration dieses Fusionsproteins in eine Wirtszelle moduliert bei besonders bevorzugten Ausführungsformen die Produktion einer gewünschten Verbindung von der Zelle.

Ein weiterer Aspekt der Erfindung beschreibt ein Verfahren zur Herstellung einer Feinchemikalie. Das Verfahren sieht die Anzucht einer Zelle vor, die einen Vektor enthält, der die Expression eines erfindungsgemäßen SES-Nukleinsäuremoleküls bewirkt, so daß eine Feinchemikalie produziert wird. Dieses Verfahren umfaßt bei einer bevorzugten Ausführungsform zudem den Schritt der Gewinnung einer Zelle, die einen solchen Vektor enthält, wobei die Zelle mit einem Vektor transfiziert ist, der die Expression einer SES-Nukleinsäure bewirkt. Dieses Verfahren umfaßt bei einer weiteren bevorzugten Ausführungsform zudem den Schritt, bei dem die Feinchemikalie aus der Kultur gewonnen wird. Die Zelle gehört bei einer besonders bevorzugten Ausführungsform zur Gattung *Corynebacterium* oder *Brevibacterium*.

Ein weiterer Aspekt der Erfindung beschreibt Verfahren zur Modulation der Produktion eines Moleküls von einem Mikroorganismus. Diese Verfahren umfassen das Zusammenbringen der Zelle mit einer Substanz, die die SES-Proteinaktivität oder die SES-Nukleinsäure-Expression moduliert, so daß eine zellassoziierte Aktivität verglichen mit der gleichen Aktivität bei Fehlen der Substanz verändert wird. Die Zelle wird bei einer bevorzugten Ausführungsform hinsichtlich einer oder mehrerer *C. glutamicum-*Prozesse moduliert, die an der genetischen Stabilität, Genexpression, Proteinfaltung oder Proteinsekretion beteiligt sind, so daß die Ausbeute, Produktion oder Effizienz der Produktion einer gewünschten Feinchemikalien durch diesen Mikroorganismus verbessert wird. Die Substanz, die die SES-Proteinaktivität moduliert, kann eine Substanz sein, die die SES-Proteinaktivität oder die SES-Nukleinsäure-Expression stimuliert. Beispiele für Substanzen, die die SES-Proteinaktivität oder SES-Nukleinsäureexpression stimulieren, umfassen kleine Moleküle, aktive SES-Proteine und Nukleinsäuren, die SES-Proteine codieren und in die Zelle eingebracht worden sind. Beispiele für Substanzen, die die SES-Aktivität oder -Expression hemmen, umfassen kleine Moleküle und SES-Antisense-Nukleinsäuremoleküle.

Ein weiterer Aspekt der Erfindung beschreibt Verfahren zur Modulation der Ausbeuten einer gewünschten Verbindung von einer Zelle, umfassend das Einbringen eines SES-Wildtyp- oder -Mutantengens in eine Zelle, das entweder auf einem gesonderten Plasmid bleibt oder in das Genom der Wirtszelle integriert wird. Die Integration in das Genom kann zufallsgemäß oder durch homologe Rekombination erfolgen, so daß das native Gen durch die eingebrachte Kopie ersetzt wird, was die Produktion der gewünschten Verbindung von der zu modulierenden Zelle hervorruft. Diese Ausbeuten sind bei einer bevorzugten Ausführungsform erhöht. Bei einer weiteren bevorzugten Ausführungsform ist die Chemikalie eine Feinchemikalie, die in einer besonders bevorzugten Ausführungsform eine Aminosäure ist. Diese Aminosäure ist in einer besonders bevorzugten Ausführungsform L-Lysin.

Der Gegenstand der Erfindung sind die in den Anspruchen definierten Nukleinsäuremoleküle, Vektoren, Wirtszellen und Verfahren.

### Eingehende Beschreibung der Erfindung

Die vorliegende Erfindung SES-Nukleinsäure- und -Proteinmoleküle bereit, die an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* beteiligt sind. Die erfindungsgemäßen Moleküle könne zur Modulation der Produktion von Feinchemikalien von Mikroorganismen, wie *C. glutamicum*, entweder direkt (z.B. wenn die Überexpression oder Optimierung der Aktivität eines an der Sekretion einer Feinchemikalie beteiligten Proteins (z.B. eines Enzyms) eine direkte Auswirkung auf die Ausbeute, Produktion und/oder Effizienz der Produktion einer Feinchemikalie von den modifizierten *C. glutamicum* hat) oder durch indirekte Auswirkung verwendet werden, die dennoch zu einer Erhöhung der Ausbeute, Produktion und/oder Effizienz der gewünschten Verbindung führt, (z.B. wenn die Modulation der Aktivität oder Kopienzahl eines *C. glutamicum*-DNA-Reparaturproteins zu Änderungen in der Fähigkeit des Mikroorganismus, die eingebrachte Mutation aufrechtzuerhalten, was wiederum die Produktion einer oder mehrerer Feinchemikalien von diesem Stamm beeinflussen kann). Die Aspekte der Erfindung sind nachstehend weiter erläutert.

### I. Feinchemikalien

Der Begriff "Feinchemikalie" ist im Fachgebiet bekannt und beinhaltet Moleküle, die von einem Organismus produziert werden und in verschiedenen Industriezweigen Anwendungen finden, wie bspw., jedoch nicht beschränkt auf die pharmazeutische Industrie, die Landwirtschafts- und Kosmetikindustrie. Diese Verbindungen umfassen organische Säuren, wie Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlehydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malaysia, AOCS Press (1995)), Enzyme und sämtliche anderen von Gutcho (1983) in Chemicals by Fermentation, Noyes Data Corporation, ISBN: 0818805086 und den darin angegebenen Literaturstellen beschriebenen Chemikalien. Der Metabolismus und die Verwendungen bestimmter Feinchemikalien sind nachstehend weiter erläutert.

### A. Metabolismus und Verwendungen von Aminosäuren

Die Aminosäuren umfassen die grundlegenden Struktureinheiten sämtlicher Proteine und sind somit für die normalen Zellfunktionen in allen Organismen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nicht-proteinogenen Aminosäuren (von denen Hunderte bekannt sind) gewöhnlich nicht in Proteinen vorkommen (siehe Ulmann's Encyclopedia of Industrial Chemistry, Bd. A2, S. 57-97 VCH: Weinheim (1985)). Die Aminosäuren können in der optischen D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L., Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, weil sie aufgrund der Komplexität ihrer Biosynthese gewöhnlich mit der Ernährung aufgenommen werden müssen, werden durch einfache Biosynthesewege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihrer Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine wichtige Aminosäure, sondern auch für monogastrische Tiere, wie Geflügel und Schweine. Glutamat wird am häufigsten als Geschmacksadditiv (Mononatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen in Ulmann's Encyclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, bspw. Bakterien, ist gut charakterisiert worden (für einen Überblick der bakteriellen Aminosäure-Biosynthese und ihrer Regulation s. Umbarger, H.E. (1978) Ann. Rev. Biochem. 47:533-606). Glutamat wird durch reduktive Aminierung von α-Ketoglutarat, einem Zwischenprodukt im CitronensäureZyklus, synthetisiert. Glutamin, Prolin und Arginin werden jeweils nacheinander aus Glutamat erzeugt. Die Biosynthese von Serin erfolgt in einem Dreischritt-Verfahren, beginnt mit 3-Phosphoglycerat (einem Zwischenprodukt der Glykolyse) und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten-β-Kohlenstoffatoms auf Tetrahydrofolat in einer durch Serin-Transhydroxymethylase katalysierten Reaktion. Phenylalanin und Tyrosin werden aus den Vorstufen des Glykolyse- und Pentosephosphatweges, Erythrose-4-phosphat und Phosphoenolpyruvat, in einem 9-Schritt-Biosyntheseweg synthetisiert, der sich nur in den letzten beiden Schritten nach der Synthese von Präphenat unterscheidet. Tryptophan wird ebenfalls aus diesen beiden Ausgangsmolekülen produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-1-pyrophosphat, einem aktivierten Zucker.

Aminosäuren, deren Menge den Proteinbiosynthesebedarf übersteigt, können nicht gespeichert werden, und werden statt dessen abgebaut, so daß Zwischenprodukte für die Haupt-Stoffwechselwege der Zelle bereitgestellt werden (für einen Überblick siehe Stryer, L., Biochemistry, 3. Aufl. Kap. 21 "Amino Acid Degradation and the Urea Cycle"; S 495-516 (1988)). Die Zelle ist zwar in der Lage, ungewünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet (für einen Überblick über Rückkopplungs-Mechanismen bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### B. Metabolismus und Verwendungen von Vitaminen, Cofaktoren und Nutrazeutika

Vitamine, Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenüberträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfsstoffe. (Für einen Überblick über die Struktur, Aktivität und die industriellen Anwendungen dieser Verbindungen siehe bspw. Ullman's Encyclopedia of Industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nichtproteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine, Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullman's Encyclopedia of Industrial Chemistry, "Vitamins", Bd. A27, S. 443-613, VCH: Weinheim, 1996, Michal, G. (1999) Biochemical Pathways: An Atlas of Biochemistry and Molecular Biology, John Wiley & Sons; Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X, 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B₆" bezeichnet werden (bspw. Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)-β-alanin) kann entweder durch chemische Synthese oder durch Fermentation hergestellt werden. Die letzten Schritte bei der Pantothenat-Biosynthese bestehen aus der ATP-getriebenen Kondensation von β-Alanin und Pantoinsäure. Die für die Biosyntheseschritte für die Umwandlung in Pantoinsäure, in β-Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des α-Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukleotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien, wie Riboflavin, Vitamin B₆, Pantothenat und Biotin, auch durch großangelegte Anzucht von Mikroorganismen produziert worden sind. Nur Vitamin B₁₂ wird aufgrund der Komplexität seiner Synthese lediglich durch Fermentation produziert. In-vitro-Verfahren erfordern einen erheblichen Aufwand an Materialien und Zeit und häufig an hohen Kosten.

### C. Purin-, Pyrimidin-, Nukleosid- und Nukleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteile der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisierung zur Bildung von Nukleinsäuremolekülen ist es möglich, die RNA- und DNA-Synthese zu hemmen; wird diese Aktivität zielgerichtet bei Krebszellen gehemmt, läßt sich die Teilungs- und Replikationsfähigkeit von Tumorzellen hemmen. Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalien für diese medizinischen Indikationen beschrieben, wobei der Purin- und/oder Pyrimidin-Metabolismus beeinflußt wird (bspw. Christopherson, R.I. und Lyons, S.D. (1990) "Potent inhibitors of de novo pyrimidine and purine biosynthesis as chemotherapeutic agents", Med. Res. Reviews 10:505-548). Untersuchungen an Enzymen, die am Purin- und Pyrimidin-Metabolismus beteiligt sind, haben sich auf die Entwicklung neuer Medikamente konzentriert, die bspw. als Immunsuppressiva oder Antiproliferantien verwendet werden können (Smith, J.L. (1995) "Enzymes in Nucleotide Synthesis" Curr. Opin. Struct. Biol. 5:752-757; (1995) Biochem. Soc. Transact. 23:877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (bspw. ATP oder GTP) und für Chemikalien selbst, die gewöhnlich als Geschmacksverstärker (bspw. IMP oder GMP) oder für viele medizinische Anwendungen verwendet werden (siehe bspw. Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden, Herbiziden und Insektiziden, entwickelt werden.

Der Metabolismus dieser Verbindungen in Bakterien ist charakterisiert worden (für Übersichten siehe bspw. Zalkin, H. und Dixon, J.E. (1992) "De novo purin nucleotide biosynthesis" in Progress in Nucleic Acids Research and Molecular Biology, Bd. 42, Academic Press, S. 259-287; und Michal, G. (1999) "Nucleotides and Nucleosides"; Kap. 8 in: Biochemical Pathways: An Atlas of Biochemistry and Molecular Biology, Wiley, New York). Der Purin-Metabolismus, das Objekt intensiver Forschung, ist für das normale Funktionieren der Zelle essentiell. Ein gestörter Purin-Metabolismus in höheren Tieren kann schwere Erkrankungen verursachen, bspw. Gicht. Die Purinnukleotide werden aus Ribose-5-phosphat über eine Reihe von Schritten über die Zwischenverbindung Inosin-5'-phosphat (IMP) synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridi-n-5'-monophosphat (UMP) aus Ribose-5-phosphat. UMP wiederum wird in Cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt. Nach der Phosphorylierung können diese Moleküle an der DNA-Synthese teilnehmen.

### D. Trehalose-Metabolismus und Verwendungen

Trehalose besteht aus zwei Glucosemolekülen, die über eine α,α-1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. bspw. Nishimoto et al., (1998) US-Patent Nr. 5 759 610; Singer, M.A. und Lindquist, S. (1998) Trends Biotech. 16:460-467; Paiva, C.L.A. und Panek, A.D. (1996) Biotech Ann. Rev. 2:293-314; und Shiosaka, M. (1997) J. Japan 172:97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

### II. Genetische Stabilität, Proteinsynthese und Proteinsekretion in C. glutamicum

Die Produktion einer gewünschten Verbindung von einer Zelle, wie *C. glutamicum,* ist die Kulmination einer großen Zahl an getrennten und trotzdem miteinander verknüpften Prozessen, von denen jeder für die Gesamtproduktion und die Freisetzung der Verbindung aus der Zelle entscheidend ist. Bei der Veränderung einer Zelle, so daß sie eine oder mehrere Chemikalien überproduziert, muß jeder dieser Prozesse berücksichtigt werden, um zu gewährleisten, daß die biochemische Maschinerie der Zelle mit dieser genetischen Manipulation kompatibel ist. Besonders bedeutende zelluläre Mechanismen umfassen die Stabilität das/der veränderten Gen(s/e) beim Einbringen in die Zelle, die Fähigkeit der mutierten Gens, richtig transkribiert und translatiert zu werden (einschließlich der Codonverwendung) und die Fähigkeit des mutierten Proteinproduktes, richtig gefaltet und/oder sezerniert zu werden.

### A. Bakterielle Reparatur- und Rekombinationssysteme

Zellen sind ständig nukleinsäureschädigenden Agenzien, wie UV-Bestrahlung, Sauerstoffradikale und Alkylierung, ausgesetzt. Ferner ist sogar die Wirkung von DNA-Polymerasen nicht fehlerfrei. Die Zellen müssen ein Gleichgewicht zwischen der genetischen Stabilität (die gewährleistet, daß Gene, die für zelluläre Funktionen notwendig sind, nicht während des normalen Wachstums und Stoffwechsels beschädigt werden) und der genetische Variabilität(die es den Zellen ermöglicht, sich an eine sich ändernde Umwelt anzupassen) aufrechterhalten. Daher gibt es in den meisten Zellen getrennte, aber miteinander zusammenhängende Wege für die DNA-Reparatur und DNA-Rekombination. Ersterer dient der strikten Korrektur von Fehlern in DNA-Molekülen durch entweder das direkte Rückgängigmachen der Schädigung oder durch Ausschneiden des geschädigten Bereichs und Ersetzen durch die korrekte Sequenz. Das letztere Rekombinationssystem repariert auch Nukleinsäuremoleküle, aber nur solche Schäden, die zu einer Schädigung in beiden DNA-Strängen führen, so daß kein Strang als Matrize zur Korrektur des anderen verwendet werden kann. Die Rekombinationsreparatur und die SOS-Reaktion können leicht zu Inversionen, Deletionen oder anderen genetischen Umlagerungen innerhalb des oder um den beschädigten Bereich führen, was wiederum einen bestimmten Grad an genomischer Instabilität fördert, der zur Fähigkeit der Zelle, sich an ändernde Umgebungen oder Streß anzupassen, beitragen kann.

High-fidelity-Reparaturmechanismen beinhalten das direkte Rückgängigmachen des DNA-Schadens und das Ausschneiden des Schadens und die Resynthese unter Verwendung der im Gegenstrang codierten Information. Das direkte Rückgängigmachen des Schadens erfordert ein Enzym mit einer Aktivität, die das Gegenteil desjenigen bewirkt, was ursprünglich die DNA beschädigt hat. Beispielsweise kann eine unrichtige Methylierung von DNA durch die Wirkung von DNA-Reparatur-Methyltransferasen korrigiert werden, und durch UV-Bestrahlung erzeugte Nukleotiddimere können durch die Aktivität der Desoxyribodipyrimidinphotolyase repariert werden, die in Gegenwart von Licht das Dimer wieder in die entsprechenden Nukleotide spaltet (s. Michal, G. (1999) Biochemical Pathways: An Atlas of Biochemistry and Molecular Biology, Wiley: New York, und die darin zitierten Literaturstellen).

Die genaue Reparatur größerer Schäden erfordert spezialisierte Reparaturmechanismen. Dazu gehören die Mismatch-Reparatur- und die Ausschneide-Reparatursysteme. Die Beschädigung einer einzelnen Base kann durch eine Reihe von Spaltungsreaktionen korrigiert werden, wobei zuerst die Zuckerbindung gespalten wird, gefolgt von Spaltung des DNA-Rückgrats an der beschädigten Stelle und Entfernen der beschädigten Base selbst. Schließlich bewirken DNA-Polymerase und DNA-Ligase das Auffüllen und Versiegeln der Lücke unter Verwendung des zweiten DNA-Strangs als Matrize. Ein erheblicherer DNA-Schaden, der zu einer veränderten Konformation der Doppelhelix führt, wird durch das ABC-System korrigiert, bei dem Helicase II, DNA-Polymerase I, die UvrA-, UvrB- und UvrC-Proteine zusammen die Doppelhelix an der beschädigten Stelle einzelsträngig spalten, den beschädigten Bereich auf ATP-abhängige Weise aufwinden, den beschädigten Bereich ausschneiden und den fehlenden Bereich mit dem anderen Strang als Matrize auffüllen. Schließlich versiegelt die DNA-Ligase den Einzelstrangbruch. Spezifische Reparatursysteme gibt es auch für G-T-Mismatches (an denen das Vsr-Protein beteiligt ist) und für kleine Deletions-/Insertionsfehler aufgrund der falschen Reparatur der beiden Stränge (an denen der methylierungsgesteuerte Weg beteiligt ist).

Es gibt auch Low-fidelity-Reparatursysteme, die gewöhnlich zur Korrektur sehr ausgedehnter DNA-Schäden bei Bakterien verwendet werden. Doppelstrangreparatur und Rekombination erfolgen bei Vorliegen einer Schädigung, die beide DNA-Stränge betrifft. In dieser Situation ist es unmöglich, den Schaden unter Verwendung des anderen Strangs als Matrize zu reparieren. Somit beinhaltet das Reparatursystem ein Doppel-Crossover-Ereignis zwischen dem beschädigten Bereich und einer anderen Kopie des Bereichs auf einem homologen DNA-Molekül. Dies ist möglich, da sich Bakterien so schnell teilen, daß eine zweite Kopie der genomischen DNA gewöhnlich verfügbar ist, bevor die Zellteilung tatsächlich stattfindet. Dieses Crossover-Ereignis kann leicht zu Inversionen, Duplikationen, Deletionen, Insertionen und anderen genetischen Umlagerungen führen und erhöht so insgesamt die genetische Instabilität des Organismus.

Die SOS-Reaktion wird aktiviert, wenn eine ausreichende Schädigung in der DNA vorliegt, daß die DNA-Polymerase III anhält und nicht mit der Replikation fortfahren kann. Unter diesen Umständen ist einzelsträngige DNA zugegen. Das RecA-Protein wird durch Bindung an einzelsträngige DNA aktiviert, und diese aktivierte Form führt zur Aktivierung des LexA-Repressors, wodurch der Transkriptionsblock von mehr als 20 Genen aufgehoben wird, einschließlich UvrA, UvrB, UvrC, Helicase II, DNA pol III, UmuC und UmuD. Die kombinierten Aktivitäten dieser Enzyme bewirken ein ausreichendes Auffüllen des Lückenbereichs, daß DNA pol III die Replikation wieder aufnehmen kann. Diese Lücken werden jedoch mit Basen aufgefüllt, die nicht vorliegen sollten; somit führt dieser Reparaturtyp zu einer fehleranfälligen Reparatur, was insgesamt zur genetischen Instabilität in der Zelle beiträgt.

### B. Transposons

Die oben genannten Systeme mit High- oder Low-fidelity sollen DNA-Schäden reparieren. Unter bestimmten Umständen kann diese Reparatur zusätzliche Genumlagerungen umfassen. Viele Bakterienzellen haben außerdem Mechanismen, die spezifisch solche Genumlagerungen verursachen sollen. Besonders gut bekannte Beispiele für solche Mechanismen sind die Transposons.

Transposons sind genetische Elemente, die von einer Stelle zu einer anderen entweder innerhalb eines Chromosoms oder zwischen einem Stück extrachromosomaler DNA (z.B. einem Plasmid) und einem Chromosom wandern können. Die Transposition auf auf mehrere Weisen erfolgen; beispielsweise kann das transponierbare Element aus der Donorstelle ausgeschnitten und in die Zielstelle integriert werden (nicht-replikative Transposition), oder das transponierbare Element kann alternativ von der Donorstelle zur Zielstelle dupliziert werden, was zwei Kopien des Elements ergibt (replikative Transposition). Gewöhnlich gibt es keine Sequenzverwandtschaft zwischen der Donor- und der Zielstelle.

Dieses Transpositionsereignis hat eine Vielzahl möglicher Ergebnisse. Die Integration eines transposablen Elementes in ein Gen disrumpiert das Gen, was dessen Funktion gewöhnlich völlig ausschaltet. Ein Integrationsereignis, das in der das Gen umgebenden DNA stattfindet, kann nicht die codierende Sequenz selbst stören, aber eine grundlegende Auswirkung auf die Regulation des Gens und somit auf seine Expression haben. Rekombinationsereignisse zwischen zwei Kopien eines transposablen Elementes, das sich in verschiedenen Abschnitten des Genoms befindet, können zu Deletionen, Duplikationen, Inversionen, Transpositionen oder Amplifikationen von Segmenten des genoms führen. Es ist auch möglich, daß verschiedene Replikons fusionieren.

Die einfachsten transposonartigen genetischen Elemente werden als Insertions- (IS-) Elemente bezeichnet. IS-Elemente enthalten einen Nukleotidbereich variabler Länge (aber gewöhnlich weniger als 1500 Basen), der keine codierenden Bereiche enthält und an jedem ende von Inverted Repeats umgeben ist. Da das IS-Element keine Proteine codiert, deren Aktivität nachgewiesen werden kann, wird das Vorliegen eines IS-Elementes gewöhnlich nur aufgrund eines Funktionsverlustes von einem oder mehreren Genen, in die das IS-Element inseriert ist, beobachtet.

Transposons sind mobile genetische Elemente, die im Gegensatz zu IS-Elementen von Repeats begrenzte Nukleinsäuresequenzen enthalten, die ein oder mehrere Proteine codieren können. Es ist nicht ungewöhnlich, daß diese Repeatbereiche aus IS-Elementen bestehen. Die vom Transposon codierten Proteine sind gewöhnlich Transposasen (Proteine, die die Wanderung des Transposons von einer Stelle zur anderen katalysieren) und Antibiotika-Resistenzgene. Die Mechanismen und die Regulation der transposablen Elemente sind im Fachgebiet bekannt und wurden zumindest bspw. beschrieben in: Lengeler et al. (1999) Biology of Prokaryotes, Thieme Verlag: Stuttgart, S. 375-361; Neidhardt et al. (1996) Escherichia coli and Salmonella, ASM Press: Washington, D.C.; Sonenshein, Al.L., et al., Hrsg. (1993) Bacillus subtilis, ASM Press, Washington, D.C.; Voet, D., und Voet, J.G. (1992) Biochemie, VCH: Weinheim, S. 985-990; Brock, T.D., und Madigan, M.T. (1991) Biology of Mocroorganisms, 6. Aufl. Prentice Hall: New York, S. 267-269; und Kleckner, N. (1990) "Regulation of transposition in bacteria", Annu. Rev. Biochem. 61:297-327.

### C. Transkription

Die Genexpression in Bakterien wird hauptsächlich auf der Ebene der Transkription reguliert. Der Transkriptionsapparat besteht aus einer Reihe von Proteinen, die man in zwei Gruppen einteilen kann: RNA-Polymerase (das operierende DNA-transkribierende Enzym) und Sigma-Faktoren (die die Gentranskription regulieren, indem sie die RNA-Polymerase zu spezifischer Promotor-DNA-Sequenzen lenken, die diese Faktoren erkennen). Die Kombination von RNA-Polymerase und Sigma-Faktoren erzeugt das RNA-Polymerase-Holoenzym, einen aktivierten Komplex. Gram-positive Bakterien, wie Corynebakterien, enthalten nur einen Typ der RNA-Polymerase, aber eine Anzahl verschiedener Sigma-Faktoren, die für verschiedene Promotoren, Wachstumsphasen, Umweltbedingungen, Substrate, Sauerstoffspiegel, Transportprozesse und dgl. Spezifisch sind, wodurch sich der Organismus an verschiedene Umwelt- und Stoffwechselbedingungen anpassen kann.

Promotoren sind spezifische DNA-Sequenzen, die als Andockstellen für das RNA-Polymerase-Holoenzym dienen. Viele Promotorelemente besitzen konservierte Sequenzelemente, die durch Homologiesuchen erkannt werden können; alternativ können Promotorbereiche für ein bestimmtes Gen unter Verwendung von Standard-Techniken, wie Primerextension, identifiziert werden. Viele Promotorbereiche von gram-positiven Bakterien sind bekannt (s. z.B. Sonenshein, A.L., Hoch, J.A., und Losick, R., Hrsg. (1993) Bacillus subtilis, ASM Press: Washington, D.C.).

Die Promotor-Transkriptionskontrolle wird durch mehrere Repressions- oder Aktivierungsmechanismen beeinflußt. Spezifische regulatorische Protein, die an Promotoren binden, haben die Fähigkeit, die Bindung des RNA-Holoenzyms zu blockieren (Repressoren) oder diese zu unterstützen (Aktivatoren) und so die Transkription zu regulieren. Die Bindung dieser Repressor- und Aktivatormoleküle wird wiederum durch ihre Wechselwirkungen mit anderen Molekülen, wie Proteinen oder anderen Stoffwechselverbindungen, reguliert. Die Transkription kann alternativ durch Faktoren reguliert werden, die Prozesse, wie die Elongation oder Termination beeinflussen (s. z.B. Sonenshein, A.L., Hoch, J.A., und Losick, R., Hrsg. (1993) Bacillus subtilis, ASM Press: Washington, D.C.). Durch die Fähigkeit, die Transkription von Genen als Reaktion auf eine Vielzahl von Umwelt- oder Stoffwechselzeichen zu regulieren, können die Zellen genau steuern, wann ein Gen exprimiert werden kann und wieviel eines Genproduktes in der Zelle zu einem Zeitpunkt vorliegen kann. Dies verhindert wiederum die unnötige Verschwendung von Energie oder die unnötige Verwendung möglicherweise rarer Zwischenverbindungen oder Cofaktoren.

### D. Translation und Aminoacyl-tRNA-Synthetasen

Die Translation ist der Prozeß, durch den ein Polypeptid aus Aminosäuren gemäß der in einem RNA-Molekül enthaltenen Information synthetisiert wird. Die Hauptkomponenten dieses Prozesses sind Ribosomen und spezifische Initiations- oder Elongationsfaktoren, wie IFl-3, ERFINDUNGSGEMÄSS-G und EFTu (s. z.B. Sonenshein, A.L., Hoch, J.A., und Losick, R., Hrsg. (1993) Bacillus subtilis, ASM Press: Washington, D.C.).

Jedes Codon des mRNA-Moleküls codiert eine bestimmte Aminosäure. Die Umwandlung von mRNA in Aminosäure wird durch Transfer-RNA-(tRNA-) Moleküle durchgeführt. Diese Moleküle bestehen aus einem RNA-Einzelstrang (zwischen 60 und 100 Basen), der in einer L-förmigen dreidimensionalen Struktur mit hinausragenden Bereichen oder "Armen" vorliegt. Einer dieser Arme bildet Basenpaare mit einer bestimmten Codonsequenz auf dem mRNA-Molekül. Ein zweiter Arm interagiert spezifisch mit einer bestimmten Aminosäure (die vom Codon codiert wird). Andere tRNA-Arme umfassen den variablen Arm, den TΨC-Arm (der Thymidylat- und Pseudouridylatmodifikationen trägt) und den D-Arm (der eine Dihydrouridinmodifikation trägt). Die Funktion dieser letzteren Strukturen ist immer noch unbekannt, aber ihre Konservierung zwischen den tRNA-Molekülen legt eine Rolle bei der Proteinsynthese nahe.

Damit das auf Nukleinsäure basierende tRNA-Molekül sich mit der korrekten Aminosäure paart, muß eine Familie von Enzymen, die als Aminoacyl-tRNA-Synthetasen bezeichnet werden, wirken. Es gibt viele verschiedene dieser Enzyme, und jedes ist spezifisch für eine bestimmte tRNA und eine bestimmte Aminosäure. Diese Enzyme binden das 3'-Hydroxyl der endständigen tRNA-Adenosin-Ribose-Einheit in einer Zwei-Schritt-Reaktion an die Aminosäure. Zuerst wird das Enzym durch Reaktion mit ATP und der Aminosäure aktiviert, woraus ein Aminoacyl-tRNA-Synthetase-Aminoacyl-Adenylat-Komplex resultiert. Zweitens wird die Aminoacylgruppe vom Enzym auf die Ziel-tRNA übertragen, an der sie in einem energiereichen Zustand bleibt. Die Bindung des tRNA-Moleküls an sein Erkennungscodon auf dem mRNA-Molekül bringt dann die an die tRNA gebundene energiereiche Aminosäure in Kontakt mit dem Ribosom. Innerhalb des Ribosoms besetzt die Aminosäure-beladene tRNA (Aminoacyl-tRNA) eine Bindungsstelle (die A-Stelle) neben einer zweiten Stelle (der P-Stelle), die ein tRNA-Molekül trägt, dessen Aminosäure an die naszierende Polypeptidkette gebunden ist (Peptidyl-tRNA). Die aktivierte Aminosäure an der Aminoacyl-tRNA ist ausreichend reaktiv, daß sich spontan eine Peptidbindung zwischen dieser Aminosäure und der nächsten Aminosäure an der naszierenden Polypeptidkette bildet. Die GTP-Hydrolyse liefert die Energie zum Transfer der jetzt mit der Polypeptidkette beladenen tRNA von der A-Stelle zur P-Stelle des Ribosoms, und der Prozeß wiederholt sich, bis ein Stopcodon erreicht wird.

Es gibt eine Reihe verschiedener Schritte, an denen die Translation reguliert werden kann. Dazu gehören die Bindung des Ribosoms an mRNA, das Vorliegen von mRNA-Sekundärstruktur, die Codonverwendung oder die Häufigkeit bestimmter tRNAs. Auch spezielle Regulationsmechanismen, wie Attenuation, können auf der Translationsebene wirken. Eine tiefgreifende Übersicht über viele dieser Mechanismen s. z.B. in Vellanoweth, R.L. (1993) "Translation and its Regulation", in: Bacillus subtilis and o-ther Gram Positive Bacteria, Sonenshein, A.L., et al., Hrsg., ASM Press: Washington, D.C., S. 699-711 und die darin zitierten Literaturstellen.

### E. Proteinfaltung und -Sekretion

Die Synthese von Proteinen durch das Ribosom führt zu Polypeptidketten, die eine dreidimensionale Form annehmen müssen, bevor das Protein normal funktionieren kann. Die dreidimensionale Struktur wird durch einen Faltungsprozeß erzielt. Polypeptidketten sind flexibel und bewegen sich (im Prinzip) leicht und frei in Lösung, bis sie eine Konformation annehmen, die zu einer stabilen dreidimensionalen Struktur führt. Manchmal ist es jedoch für Proteine schwierig, sich richtig zu falten, entweder aufgrund der Umweltbedingungen (z.B. hohe Temperatur, bei der die im System vorhandene kinetische Energie es dem Protein schwieriger macht, in das Energieloch einer stabilen Struktur zu fallen) oder aufgrund der Art des Proteins selbst (z.B. neigen hydrophobe Bereiche in nahe beieinander befindlichen Proteinen zur Aggregation, wodurch sie sich selbst aus wäßrigen Lösungen ausfällen).

Proteinartige Faktoren sind identifiziert worden, die die Faltung von Proteinen katalysieren, begleiten oder anderweitig unterstützen können und co- oder posttranslational synthetisiert werden. Zu diesen Proteinfaltungsmolekülen gehören die Prolyl-Peptidyl-Isomerasen (z.B. Trigger-Faktor, Cyclophilin und FKBP-Homologa) sowie Proteine der Hitzeschockprotein-Gruppe (z.B. DnaK, DnaJ, GroEL, kleine Hitzeschockproteine, HtpG und Mitglieder der Clp-Familie (z.B. ClpA, ClpB, ClpW, ClpP und ClpX). Viele dieser Proteine sind für die Lebensfähigkeit von Zellen wichtig: zusätzlich zu ihrer Funktion bei der Proteinfaltung, - translokation und -prozessierung dienen sie häufig als Ziele für die Gesamtregulation der Proteinsynthese (s. z.B. Bukau, B. (1993) Molecular Microbiology 9(4):671-680; Bukau, B., und Horwich, A.L. (1998) Cell 92(3):351-366; Hesterkamp, T., Bukau, C. (1996) FEBS Lett. 389(1):32-34; Yaron, A., Naider, F. (1993) Critical Reviews in Biochemistry and Molecular Biology 28(1):31-81; Scheibel, R., Buchner, J. (1998) Biochemical Pharmacology 56(6):675-682; Ellis, R.J., Hartl, F.U. (1996) FASEB Journal 10(1):20-26; Wawrzynow, A., et al. (1996) Molecular Micorbiology 21(5):895-899; Ewalt, K.L., et al. (1997) Cell 90(3):491-500).

Die bisher identifizierten Chaperone wirken auf zwei Weisen: sie binden entweder an Polypeptide und stabilisieren diese oder sie stellen eine Umgebung bereit, in der die Faltung ohne Störung stattfinden kann. Die erstere Gruppe, einschließlich z.B. DnaK, DnaJ und der Hitzeschockproteine, bindet direkt an das naszierende oder falsch gefaltete Polypeptid, häufig begleitet von ATP-Hydrolyse. Die Bindung des Chaperons verhindert, daß das Polypeptid mit anderen Polypeptiden aggregiert, und kann die Auflösung dieser Aggregate, wenn sie sich bereits gebildet haben, erzwingen. Nach der Wechselwirkung mit einem zweiten Chaperon GrpE (das das Auftreten eines ADP-ATP-Austauschs ermöglicht) wird das Polypeptid im Molten-globule-Zustand freigesetzt und kann sich falten. Wenn eine falsche Faltung auftritt, binden die Chaperone wieder an das falsch gefaltete Protein und erzwingen seine Rückkehr in einen ungefalteten Zustand. Dieser Zyklus kann wiederholt werden, bis das Protein korrekt gefaltet ist. Im Gegensatz zur ersten Chaperongruppe, die einfach an das Polypeptid bindet, bindet die zweite Gruppe (z.B. GroEL/ES) nicht nur an das Polypeptid, sondern umgibt es vollständig, so daß es vor der Umgebung geschützt ist. Der GroEL/ES-Komplex besteht aus zwei aufeinandergestapelten 14-gliedrigen Ringen mit einer hydrophoben inneren Oberfläche und einem "Deckel" aus einem 7-gliedrigen Ring. Das Polypeptid wird in einer ATP-abhängigen Reaktion in den Kanal im Zentrum dieses Komplexes gezogen, wo es sich ohne Störung durch andere Polypeptide falten kann. Falsch gefaltete Proteine werden nicht aus dem Komplex freigesetzt.

Ein wichtiger Schritt bei der Proteinfaltung ist die Bildung von Disulfidbindungen. Diese Bindungen, entweder innerhalb einer Untereinheit oder zwischen Untereinheiten von Proteinen, sind für die Proteinstabilität wichtig. Disulfidbindungen bilden sich leicht in wäßriger Lösung, und es ist schwierig, eine falsche Disulfidbrückenbildung ohne Hilfe einer reduzierenden Umgebung rückgängig zu machen. Zur Unterstützung dieses Prozesses der korrekten Disulfidbrückenbildung findet man im Cytosol der meisten Zellen thiolhaltige Moleküle, wie Glutathion oder Thioredoxin und ihre entsprechenden Oxidations-/Reduktionssysteme (Loferer, H., Hennecke, H. (1994) Trends in Biochemical Sciences 19(4):169-171).

Zu bestimmten Zeiten ist jedoch die Faltung naszierender Polypeptidkette nicht wünschenswert, bspw. wenn diese Proteine sezerniert werden sollen. Der Faltungsprozeß führt gewöhnlich dazu, daß die hydrophoben Bereiche des Proteins sich im Zentrum des Proteins, entfernt von der wäßrigen Lösung, befinden und die hydrophilen Bereiche an den äußeren Oberflächen des Proteins präsentiert werden. Diese Konformationsanordnung erzeugt zwar eine höherer Stabilität für das Protein, erschwert aber die Translokation des Proteins über Membranen, da der hydrophobe Kern der Membran an sich inkompatibel mit dem hydrophilen Äußeren des Proteins ist. So werden die von der Zelle synthetisierten Proteine, die zum Äußeren der Zelle sezerniert werden müssen (z.B. Zelloberflächenenzyme und Membranrezeptoren) oder die in die Membran selbst inseriert werden müssen (z.B. Transporterproteine und Kanalproteine), gewöhnlich vor der Faltung sezerniert oder inseriert. Die gleichen Chaperone, die die Aggregation naszierender Polypeptidketten verhindern, verhindern auch die Faltung von Polypeptiden, bis sie nicht mehr gebraucht werden. Somit können diese Proteine naszierende Polypeptidketten zu einem geeigneten Ort in der Zelle "eskortieren", wo sie entweder entfernt werden, so daß die Faltung möglich wird, oder das Protein auf ein Transportsystem übertragen, das entweder das Polypeptid sezerniert oder seine Insertion in eine Membran unterstützt.

Im Verlauf der Evolution hat sich eine spezialisierte Proteinmaschinerie gebildet, die Proteine mit spezifischen Prosequenzen (die später durch Spaltung aus dem Protein entfernt werden) erkennt, bindet, Transportiert und prozessiert. Die Maschinerie besteht aus einer Reihe von Proteinen, die man gemeinsam als sec (Typ-II-Sekretions-) System bezeichnet (eine Übersicht s. in Gilbert, M., et al. (1995) Critical Reviews in Biotechnology 15(1):13-39 und den Literaturstellen darin; Freudl, R. (1992) Journal of Biotechnology 23(3):231-240 und Literaturstellen darin; Neidhardt, F.C., et al. (1996) E. coli and Salmonella, ASM Press: Washington, D.C., S. 967-978; Binet, R., et al. (1997) Gene 192(1):7-11 und Rapoport, T.A. (1986) Critical Reviews in Biochemistry 20(1):73-137 und Literaturstellen darin). Das sec-System besteht aus Chaperonen (z.B. SecA und SecB), integralen Membranproteinen, die auch als Translokasen bezeichnet werden (z.B. SecY, SecE und SecG) und Signalpeptidasen (z.B. LepB). Das naszierende Polypeptid mit einer prosequenz, die zur Sekretion führt, wird von SecB gebunden, das es an SecA an der inneren Oberfläche der Zellmembran übergibt. SecA bindet an die Prosequenz und inseriert nach ATP-Hydrolyse in die Membran und zwingt auch einen Teil des Polypeptids durch die Membran. Der Rest des Polypeptids wird durch einen Komplex aus Translokasen, wie SecY, SecE und SecG, durch die Membran geleitet. Schließlich spaltet die Signalpeptidase die Prosequenz ab, und das Polypeptid befindet sind frei auf der extrazellulären Seite der Membran, wo es sich spontan faltet.

Auch Sec-unabhängige Sekretionsmechanismen sind bekannt. Beispielsweise umfaßt der Signalerkennungspartikel-abhängige Weg die Bindung eines Signalerkennungspartikel- (SRP-) Proteins an das naszierende Polypeptid während seiner Synthese, wodurch das Ribosom anhält. Ein Rezeptor für SRP an der inneren Oberfläche der Membran bindet dann den Ribosom-Polypeptid-SRP-Komplex. GTP-Hydrolyse liefert die Energie, die zur Übertragung des Komplexes auf den sec-Translokase-Komplex nötig ist, an dem das Polypeptid während seiner Synthese durch das Ribosom über die Membran geleitet wird. Bekanntlich existieren andere, für nur wenige Proteine spezifische Sekretionsmechanismen.

### III. Elemente und Verfahren der Erfindung

Die vorliegende Erfindung beruht zumindest teilweise auf der Entdeckung von neuen Molekülen, die hier als SES-Nukleinsäure- und Proteinmoleküle bezeichnet werden und an der Reparatur oder Rekombination von DNA in *C. glutamicum*, Transposition oder anderen Umlagerung von *C. glutamicum*-DNA, Genexpression in *C. glutamicum* (d. h. Transkriptions- oder Translationsprozessen), Proteinfaltung oder Proteinsekretion diese Mikroorganismus teilnehmen. In einer Ausführungsform nehmen die SES-Moleküle an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teil. In einer bevorzugten Ausführungsform hat die Aktivität der erfindungsgemäßen SES-Moleküle bezüglich der Reparatur oder Rekombination von DNA, Transposition von DNA, Genexpression, Proteinfaltung oder Proteinsekretion eine Auswirkung auf die Produktion einer gewünschten Feinchemikalie durch diesen Mikroorganismus. In einer besonders bevorzugten Ausführungsform ist die Aktivität der erfindungsgemäßen SES-Moleküle moduliert, so daß auch die Aktivität der *C. glutamicum-*Zellprozesse, an denen die erfindungsgemäßen SES-Proteine beteiligt sind, (z.B. Reparatur oder Rekombination von DNA, Transposition von DNA, Genexpression, Proteinfaltung oder Proteinsekretion) verändert ist, was direkt oder indirekt zu einer Modulation der Ausbeute, Produktion und/oder Effizienz der Produktion einer gewünschten Feinchemikalie durch *C. glutamicum* führt.

Der Begriff "SES-Protein" oder "SES-Polypeptid" umfaßt Proteine, die an einer Reihe von Zellprozessen beteiligt sind, die zur genetischen Stabilität, Genexpression, Proteinfaltung oder Proteinsekretion von *C. glutamicum* in Beziehung stehen. Beispielsweise kann ein SES-Protein an der DNA-Reparatur oder an Rekombinationsmechanismen bei *C. glutamicum*, Umlagerungen des genetischen Materials von *C. glutamicum* (wie den von Transposons vermittelten), der Transkription oder Translation von Genes in diesem Mikroorganismus, bei der Vermittlung der Proteinfaltung in *C. glutamicum* (wie der Aktivität von Chaperonen) oder der Sekretion von Proteinen aus *C. glutamicum*-Zellen (z.B. am sec-System) beteiligt sein. Beispiele für SES-Proteine umfassen solche, die von den in Tabelle 1 und Anhang A aufgeführten SES-Genen codiert werden. Die Ausdrücke "SES-Gen" oder "SES-Nukleinsäuresequenz" umfassen Nukleinsäuresequenzen, die ein SES-Protein codieren, das aus einem codierenden Bereich und entsprechenden untranslatierten 5'- und 3'-Sequenzbereichen besteht. Beispiele für SES-Gene sind die in Tabelle 1 aufgelisteten. Die Begriffe "Produktion" oder "Produktivität" sind im Fachgebiet bekannt und beinhalten die Konzentration des Fermentationsproduktes (bspw. der gewünschten Feinchemikalie), das innerhalb einer festgelegten Zeitspanne und eines festgelegten Fermentationsvolumens gebildet wird (bspw. kg Produkt pro Std. pro 1). Der Begriff "Effizienz der Produktion" umfaßt die Zeit, die zur Erzielung einer bestimmten Produktionsmenge nötig ist (bspw. wie lange die Zelle zur Aufrichtung einer bestimmten Ausstoßrate einer Feinchemikalie benötigt). Der Begriff "Ausbeute" oder "Produkt/Kohlenstoff-Ausbeute" ist im Fachgebiet bekannt und umfaßt die Effizienz der Umwandlung der Kohlenstoffquelle in das Produkt (d.h. die Feinchemikalie). Dies wird bspw. gewöhnlich ausgedrückt als kg Produkt pro kg Kohlenstoffquelle. Durch Vergrößern der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe "Biosynthese" oder "Biosyntheseweg" sind im Fachgebiet bekannt und umfassen die Synthese einer Verbindung, vorzugsweise einer organischen Verbindung, durch eine Zelle aus Zwischenverbindungen, bspw. in einem Mehrschritt- oder stark regulierten Prozeß. Die Begriffe "Abbau" oder "Abbauweg" sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle), bspw. in einem Mehrschritt- oder stark regulierten Prozeß. Der Begriff "Metabolismus" ist im Fachgebiet bekannt und umfaßt die Gesamtheit der biochemischen Reaktionen, die in einem Organismus stattfinden. Der Metabolismus einer bestimmten Verbindung (z.B. der Metabolismus einer Aminosäure, wie Glycin) umfaßt dann sämtliche Biosynthese-, Modifikations- und Abbauwege in der Zelle, die diese Verbindung betreffen. Der Begriff "DNA-Reparatur" ist im Fachgebiet bekannt und beinhaltet zelluläre Mechanismen, durch die Fehler in der DNA (entweder aufgrund von Schäden, wie, aber nicht beschränkt auf Ultraviolettbestrahlung, Methylasen, Low-fidelity-Replikation oder Mutagene) ausgeschnitten und korrigiert werden. Der Ausdruck "Rekombination" oder "DNA-Rekombination" ist im Fachgebiet bekannt und umfaßt zelluläre Mechanismen, durch die ausgedehnte DNA-Schäden, die beide Stränge eines DNA-Moleküls betreffen, durch homologe Rekombination mit einer anderen unbeschädigten Kopie des DNA-Moleküls innerhalb der gleichen Zelle korrigiert werden. Diese Reparaturen sind gewöhnlich low-fidelity und können zu Genumlagerungen führen. Der Begriff "Transposon" ist im Fachgebiet bekannt und umfaßt ein DNA-Element, das zufallsgemäß in das Genom eines Organismus inserieren kann und zur Disruption von Genen oder ihrer regulatorischen Bereiche oder zu Duplikationen, Inversionen, Deletionen und anderen Genumlagerungen führen kann. Der Begriff "Proteinfaltung" ist im Fachgebiet bekannt und umfaßt die Wanderung einer Polypeptidkette durch mehrere dreidimensionale Konfigurationen, bis die stabile, aktive, dreidimensionale Konfiguration erzielt wird. Die Bildung von Disulfidbindungen und die Sequestrierung hydrophober Bereich aus der umgebenden wäßrigen Lösung liefern einige der Antriebskräfte für diesen Proteinfaltungsprozeß, und die korrekte Faltung kann durch die Aktivität von Chaperonen verstärkt werden. Die Begriffe "Sekretion" oder "Proteinsekretion" sind im Fachgebiet bekannt und umfassen die Bewegung von Proteinen vom Inneren der Zelle zum Äußeren der Zelle in einem Mechanismus, bei dem ein System von Sekretionsproteinen ihren Durchtritt über die Zellmembran zum Äußeren der Zelle ermöglicht.

Die erfindungsgemäßen SES-Moleküle sind in einer anderen Ausführungsform befähigt, die Produktion eines gewünschten Moleküls, wie einer Feinchemikalie, in einem Mikroorganismus, wie *C*. *glutamicum*, zu modulieren. Es gibt eine Reihe von Mechanismen, durch die die Veränderung eines erfindungsgemäßen SES-Proteins die Ausbeute, Produktion und/oder Effizienz der Produktion einer Feinchemikalie aus einem *C*. *glutamicum*-Stamm, der dieses veränderte Protein enthält, direkt beeinflussen kann. Zum Beispiel sollte die Modulation von Proteinen, die direkt an der Transkription oder Translation beteiligt sind (z.B. Polymerasen oder Ribosomen), so daß ihre Anzahl oder Aktivität gesteigert wird, die zelluläre Transkription oder Translation (oder die Geschwindigkeiten dieser Prozesse) insgesamt steigern. Diese erhöhte zelluläre Genexpression sollte solche Proteine umfassen, die an der Feinchemikalienbiosynthese beteiligt sind, so daß eine Steigerung der Ausbeute, Produktion oder Effizienz der Produktion einer oder mehrerer gewünschten Verbindungen erfolgen kann. Modifikationen der Transkriptions-/Translations-Proteinmaschinerie von *C*. *glutamicum*, so daß die Regulation dieser Proteine verändert wird, kann auch die erhöhte Expression von Genen, die an der Produktion von Feinchemikalien beteiligt sind, ermöglichen. Die Modulation der Aktivität einer Reihe von Proteinen, die an der Peptidfaltung beteiligt sind, kann eine Erhöhung der Gesamtproduktion korrekt gefalteter Moleküle in der Zelle ermöglichen, wodurch die Möglichkeit erhöht wird, daß gewünschte Proteine (z.B. Feinchemikalienbiosynthese-Proteine) richtig funktionieren können. Ferner kann es durch Mutation von an der Sekretion aus *C*. *glutamicum* beteiligten Proteinen, so daß ihre Anzahl oder Aktivität erhöht ist, möglich sein, die Sekretion einer Feinchemikalie (z.B. eines Enzyms) aus Zellen in der Fermentationskultur zu erhöhen, aus der sie leicht gewonnen werden kann.

Die genetische Modifikation der erfindungsgemäßen SES-Moleküle kann auch zu einer indirekten Modulation der Produktion einer oder mehrerer Feinchemikalien führen. Beispielsweise kann man durch Erhöhen der Anzahl oder Aktivität eines erfindungsgemäßen DNA-Reparatur- oder -Rekombinationsproteins die Fähigkeit der Zelle, eine DNA-Schädigung zu entdecken und zu reparieren, erhöhen. Dies sollte die Fähigkeit der Zelle, ein mutiertes Gen in ihrem Genom zu halten, wirksam erhöhen und dadurch die Wahrscheinlichkeit erhöhen, daß ein gentechnologisch in *C*. *glutami*cum eingebrachtes Transgen (das z.B. ein Protein codiert, das die Biosynthese einer Feinchemikalie steigert) nicht während der Züchtung des Mikroorganismus verloren geht. Dagegen kann es durch Verringern der Anzahl oder Aktivität eines oder mehrerer DNA-Reparatur- oder -Rekombinationsproteine möglich sein, die genetische Instabilität des Organismus zu steigern. Diese Manipulationen sollten die Fähigkeit des Organismus, durch Mutagenese modifiziert zu werden, verbessern, ohne daß die eingebrachte Mutation berichtigt wird. Das gleiche gilt für Proteine, die an der Transposition oder Umlagerung genetischer Elemente in *C*. *glutamicum* beteiligt sind (z.B. Transposons). Durch Mutagenese dieser Proteine, so daß ihre Anzahl oder Aktivität entweder gesteigert oder verringert wird, ist es möglich, gleichzeitig die genetische Stabilität des Mikroorganismus zu steigern oder zu verringern. Dies hat eine bedeutende Auswirkung darauf, daß eine andere Mutation in *C*. *glutamicum* eingebracht und die eingebrachte Mutation beibehalten werden kann. Transposons bieten ebenfalls einen geeigneten Mechanismus, durch den die Mutagenese von *C*. *glutamicum* durchgeführt werden kann; die Duplikation gewünschter Gene (z.B. von Feinchemikalienbiosynthese-Genen) läßt sich leicht mittels Transposonmutagenese durchführen, wie auch die Disruption ungewünschter Gene (z.B. Gene, die am Abbau gewünschter Feinchemikalien beteiligt sind).

Durch die Modulation eines oder mehrerer Proteine (z.B. Sigma-Faktoren), die an der Regulation der Transkription oder Translation in Reaktion auf besondere Umweltbedingungen beteiligt sind, kann es möglich sein, die Zelle daran zu hindern, daß sie die Proteinsynthese unter ungünstigen Umweltbedingungen, wie man sie in einer Fermenterkultur im Großmaßstab antrifft, verlangsamt oder beendet. Dies sollte zu erhöhter Genexpression führen, was wiederum die gesteigerte Biosynthese gewünschter Feinchemikalien unter diesen Bedingungen ermöglichen kann. Viele dieser sezernierten Proteine haben Funktionen, die für die Zellebensfähigkeit wichtig sind (z.B. Zelloberflächenproteasen oder - Rezeptoren). Eine Änderung des Sekretionswegs, so daß diese Proteine leichter an ihren extrazellulären Ort transportiert werden, kann die Gesamtlebensfähigkeit der Zelle erhöhen und somit zu höheren Zahlen an *C*. *glutamicum*-Zellen führen, die Feinchemikalien während eine Züchtung im Großmaßstab produzieren können. Da ferner bestimmte bakterielle Proteinsekretionswege (z.B. das sec-System) bekanntlich auch an der Insertion von integralen Membranproteinen (z.B. Rezeptoren, Kanälen, Poren oder Transportern) in die Membran beteiligt sind, kann die Modulation der Aktivität von Proteinen, die an der Proteinsekretion aus *C*. *glutamicum* beteiligt sind, die Fähigkeit der Zelle zur Ausscheidung von Abfallprodukten oder zum Import notwendiger von Abfallprodukten oder zum Import notwendiger Metabolite beeinflussen. Ist die Aktivität dieser sekretorischen Proteine erhöht, kann ebenfalls die Fähigkeit der Zelle zur Produktion von Feinchemikalien (durch ein gesteigertes Vorliegen von Transportern/Kanälen in der Membran, die Nährstoffe importieren oder Abfallprodukte ausscheiden können) erhöht sein. Ist die Aktivität dieser sekretorischen Proteine verringert, können nicht genügend Nährstoffe zur Unterstützung der Überproduktion gewünschter Verbindungen vorhanden sein, oder Abfallprodukte können diese Biosynthese stören.

Als Ausgangspunkt zur Herstellung der erfindungsgemäßen Nukleinsäuresequenzen eignet sich das Genom eines *Corynebacterium glutamicum*-Stammes, der von der American Type Culture Collection unter der Bezeichnung ATCC 13032 erhältlich ist.

Von diesen Nukleinsäuresequenzen lassen sich durch die in Tabelle 1 bezeichneten Veränderungen die erfindungsgemäßen Nukleinsäuresequenzen mit üblichen Verfahren herstellen.

Das erfindungsgemäße SES-Protein oder ein biologisch aktiver Abschnitt oder Fragment davon kann an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teilnehmen oder eine oder mehrere der in Tabelle 1 beschriebenen Aktivitäten haben.

In den nachstehenden Unterabschnitten sind verschiedene Aspekte der Erfindung ausführlicher beschrieben:

### A. Isolierte Nukleinsäuremoleküle

Ein Aspekt der Erfindung betrifft isolierte Nukleinsäuremoleküle, wie im Anspruch 1 definiert.

Ein erfindungsgemäßes Nukleinsäuremolekül wie im Anspruch 1 definiert, kann mittels molekularbiologischer Standard-Techniken und der hier bereitgestellten Sequenzinformation hergestellt werden. Bspw. kann eine *C*. *glutamicum*-SES-cDNA aus einer *C*. *glutamicum*-Bank isoliert werden, indem eine vollständige Sequenz aus Anhang A oder ein Abschnitt davon als Hybridisierungssonde und Standard-Hybridisierungstechniken (wie bspw. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies läßt sich ein Nukleinsäuremolekül, umfassend eine vollständige Sequenz aus Anhang A oder einen Abschnitt davon, durch Polymerasekettenreaktion isolieren, wobei Oligonukleotidprimer, die auf der Basis dieser Sequenz erstellt wurden, verwendet werden (z.B. kann ein Nukleinsäuremolekül, umfassend eine vollständige Sequenz aus Anhang A oder einen Abschnitt davon, durch Polymerasekettenreaktion isoliert werden, indem Oligonukleotidprimer verwendet werden, die auf der Basis dieser gleichen Sequenz aus Anhang A erstellt worden sind). Bspw. läßt sich mRNA aus normalen Endothelzellen isolieren (bspw. durch das Guanidiniumthiocyanat-Extraktionsverfahren von Chirgwin et al. (1979) Biochemistry 18:5294-5299), und die cDNA kann mittels reverser Transkriptase (bspw. Moloney-MLV-Reverse-Transkriptase, erhältlich bei Gibco/BRL, Bethesda, MD, oder AMV-Reverse-Transkriptase, erhältlich von Seikagaku America, Inc., St. Petersburg, FL) hergestellt werden. Synthetische Oligonukleotidprimer für die Amplifizierung via Polymerasekettenreaktion lassen sich auf der Basis einer der in Anhang A gezeigten Nukleotidsequenzen erstellen. Eine erfindungsgemäße Nukleinsäure kann mittels cDNA oder alternativ genomischer DNA als Matrize und geeigneten Oligonukleotidprimern gemäß PCR-Standard-Amplifikationstechniken amplifiziert werden. Die so amplifizierte Nukleinsäure kann in einen geeigneten Vektor kloniert werden und durch DNA-Sequenzanalyse charakterisiert werden. Oligonukleotide, die einer SES-Nukleotidsequenz entsprechen, können ferner durch Standard-Syntheseverfahren, bspw. mit einem automatischen DNA-Synthesegerät, hergestellt werden.

Bei einer Ausführungsform codiert das erfindungsgemäße Nukleinsäuremolekül ein Protein.

Abschnitte von Proteinen, die von den beschriebenen SES-Nukleinsäuremolekülen codiert werden, sind vorzugsweise biologisch aktive Abschnitte von einem der SES-Proteine. Der Begriff "biologisch aktiver Abschnitt eines SES-Proteins", wie er hier verwendet wird, soll einen Abschnitt, bspw. eine Domäne/ein Motiv eines SES-Proteins, umfassen, die/das an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teilnimmt oder eine der in Tabelle 1 dargestellten Aktivitäten hat. Zur Bestimmung, ob ein SES-Protein oder ein biologisch aktiver Abschnitt davon an der Reparatur oder Rekombination von DNA, Transposition von genetischem Material, Genexpression (d. h. Transkriptions- oder Translationsprozessen), Proteinfaltung oder Proteinsekretion in *Corynebacterium glutamicum* teilnehmen kann, kann ein Test der enzymatischen Aktivität durchgeführt werden. Diese Testverfahren, wie eingehend in Beispiel 8 des Beispielteils beschrieben, sind dem Fachmann geläufig.

Zusätzlich zu natürlich vorkommenden Varianten der SES-Sequenz, die in der Population existieren können, ist der Fachmann sich ebenfalls bewußt darüber, daß Änderungen durch Mutation in eine Nukleotidsequenz von Anhang A eingebracht werden können, was zur Änderung der Aminosäuresequenz des codierten SES-Proteins führt, ohne daß die Funktionsfähigkeit des SES-Proteins beeinträchtigt wird. Bspw. lassen sich Nukleotidsusbtitutionen, die an "nicht-essentiellen" Aminosäureresten zu Aminosäuresubstitutionen führen, in einer Sequenz von Anhang A herstellen. Ein "nichtessentieller" Aminosäurerest ist ein Rest, der sich in der Wildtypsequenz von einem der SES-Proteine (Anhang B) verändern läßt, ohne daß die Aktivität des SES-Proteins verändert wird, wohingegen ein "essentieller" Aminosäurerest für die SES-Proteinaktivität erforderlich ist. Andere Aminosäurereste jedoch (bspw. nicht-konservierte oder lediglich semikonservierte Aminosäurereste in der Domäne mit SES-Aktivität) können für die Aktivität nicht essentiell sein und lassen sich somit wahrscheinlich verändern, ohne daß die SES-Aktivität verändert wird.

Ein isoliertes Nukleinsäuremolekül, das ein SES-Protein codiert, das zu einer Proteinsequenz aus Anhang B homolog ist, kann durch Einbringen von einer oder mehreren Nukleotidsubstitutionen, - additionen oder -deletionen in eine Nukleotidsequenz aus Anhang A erzeugt werden, so daß eine oder mehrere Aminosäuresubstitutionen, -additionen oder -deletionen in das codierte Protein eingebracht werden. Die Mutationen können in eine der Sequenzen aus Anhang A durch Standard-Techniken, wie stellengerichtete Mutagenese und PCR-vermittelte Mutagenese, eingebracht werden. Vorzugsweise werden konservative Aminosäuresubstitutionen an einem oder mehreren der vorhergesagten nicht-essentiellen Aminosäurereste eingeführt. Bei einer "konservativen Aminosäuresubstitution" wird der Aminosäurerest durch einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), nicht-polaren Seitenketten, (bspw. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einem SES-Protein wird somit vorzugsweise durch einen anderen Aminosäurerest der gleichen Seitenkettenfamilie ausgetauscht. In einer weiteren Ausführungsform können die Mutationen alternativ zufallsgemäß über die gesamte oder einen Teil der SES-codierenden Sequenz eingebracht werden, bspw. durch Sättigungsmutagenese, und die resultierenden Mutanten können auf eine hier beschriebene SES-Aktivität untersucht werden, um Mutanten zu identifizieren, die eine SES-Aktivität beibehalten. Nach der Mutagenese von einer der Sequenzen aus Anhang A kann das codierte Protein rekombinant exprimiert werden, und die Aktivität des Proteins kann bspw. mit den hier beschriebenen Tests (siehe Beispiel 8 des Beispielteils) bestimmt werden.

### B. Rekombinante Expressionsvektoren und Wirtszellen

Ein weiterer Aspekt der Erfindung betrifft Vektoren, vorzugsweise Expressionsvektoren, die eine Nukleinsäure wie in Anspruch 1 definiert enthalten. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzliche DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (bspw. Bakterienvektoren mit bakteriellem Replikationsursprung und episomale Säugetiervektoren). Andere Vektoren (z.B. nicht-episomale Säugetiervektoren) werden in das Genom einer Wirtszelle beim Einbringen in die Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben die Expressionsvektoren, die bei DNA-Rekombinationstechniken verwendet werden können, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch andere Expressionsvektorformen, wie virale Vektoren (bspw. replikationsdefiziente Retroviren, Adenoviren und adenoverwandte Viren), die ähnliche Punktionen ausüben, umfassen.

Die erfindungsgemäßen rekombinanten Expressionsvektoren umfassen eine erfindungsgemäße Nukleinsäure in einer Form, die sich zur Expression der Nukleinsäure in einer Wirtszelle eignet, d.h. daß die rekombinanten Expressionsvektoren eine oder mehrere regulatorische Sequenzen, ausgewählt auf der Basis der zur Expression zu verwendenden Wirtszellen, umfassen, die mit der zu exprimierenden Nukleinsäuresequenz funktionsfähig verbunden sind. In einem rekombinanten Expressionsvektor bedeutet "funktionsfähig verbunden", daß die Nukleotidsequenz von Interesse derart an die regulatorische(n) Sequenz(en) gebunden ist, daß die Expression der Nukleotidsequenz möglich ist (bspw. in einem in-vitro-Transkriptions-/Translationssystem oder in einer Wirtszelle, wenn der Vektor in die Wirtszelle eingebracht ist). Der Begriff "regulatorische Sequenz" soll Promotoren, Enhancer und andere Expressionskontrollelemente (bspw. Polyadenylierungssignale) umfassen. Diese regulatorischen Sequenzen sind bspw beschrieben in Goeddel: Gene Expression Technology: Methods in Enzymology 185, Academic Press, San Diego, CA (1990). Regulatorische Sequenzen umfassen solche, die die konstitutive Expression einer Nukleotidsequenz in vielen Wirtszelltypen steuern, und solche, die die Expression der Nukleotidsequenz nur in bestimmten Wirtszellen steuern. Der Fachmann ist sich dessen bewußt, daß die Gestaltung eines Expressionsvektors von Faktoren abhängen kann, wie der Wahl der zu transformierenden Wirtszelle, dem gewünschten Ausmaß der Proteinexpression usw. Die erfindungsgemäßen Expressionsvektoren können in die Wirtszellen eingebracht werden, so daß dadurch Proteine oder Peptide, einschließlich der Fusionsproteine oder -peptide, die von den Nukleinsäuren, wie hier beschrieben, codiert werden, hergestellt werden (bspw. SES-Proteine, mutierte Formen von SES-Proteinen, Fusionsproteine, usw.).

Die erfindungsgemäßen rekombinanten Expressionsvektoren können zur Expression von SES-Proteinen in prokaryotischen oder eukaryotischen Zellen ausgestaltet sein. Bspw. können SES-Gene in bakteriellen Zellen, wie *C*. *glutamicum*, Insektenzellen (mit Baculovirus-Expressionsvektoren), Hefe- und anderen Pilzzellen (siehe Romanos, M.A. et al. (1992) "Foreign gene expression in yeast: a review", Yeast 8: 423-488; van den Hondel, C.A.M.J.J. et al. (1991) "Heterologous gene expression in filamentous fungi" in: More Gene Manipulations in Fungi, J.W. Bennet & L.L. Lasure, Hrsg., S. 396-428: Academic Press: San Diego; und van den Hondel, C.A.M.J.J. & Punt, P.J. (1991) "Gene transfer systems and vector development for filamentous fungi. in: Applied Molecular Genetics of Fungi, Peberdy, J.F. et al., Hrsg, S. 1-28, Cambridge University Press: Cambridge), Algenzellen und Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.: 583-586) oder Säugetierzellen exprimiert werden. Geeignete Wirtszellen werden weiter erörtert in Goeddel, Gene Expression Technology: Methods in Enzymology 185, Academic Press, San Diego, CA (1990). Der rekombinante Expressionsvektor kann alternativ, bspw. mit regulatorischen Sequenzen des T7-Promotors und T7-Polymerase, in vitro transkribiert und translatiert werden.

Die Expression von Proteinen in Prokaryoten erfolgt meist mit Vektoren, die konstitutive oder induzierbare Promotoren enthalten, die die Expression von Fusions- oder Nicht-Fusionsproteinen steuern. Fusionsvektoren steuern eine Reihe von Aminosäuren zu einem darin codierten Protein, gewöhnlich am Aminoterminus des rekombinanten Proteins, bei. Diese Fusionsvektoren haben gewöhnlich drei Aufgaben: 1) die Verstärkung der Expression von rekombinantem Protein; 2) die Erhöhung der Löslichkeit des rekombinanten Proteins; und 3) die Unterstützung der Reinigung des rekombinanten Proteins durch Wirkung als Ligand bei der Affinitätsreinigung. Bei Fusions-Expressionsvektoren wird oft eine proteolytische Spaltstelle an der Verbindungsstelle der Fusionseinheit und des rekombinanten Proteins eingebracht, so daß die Trennung des rekombinanten Proteins von der Fusionseinheit nach der Reinigung des Fusionsproteins möglich ist. Diese Enzyme und ihre entsprechenden Erkennungssequenzen umfassen Faktor Xa, Thrombin und Enterokinase.

Übliche Fusionsexpressionsvektoren umfassen pGEX (Pharmacia Biotech Inc; Smith, D.B. und Johnson, K.S. (1988) Gene 67:31-40), pMAL (New England Biolabs, Beverly, MA) und pRIT 5 (Pharmacia, Piscataway, NJ), bei denen Glutathion-S-Transferase (GST), Maltose E-bindendes Protein bzw. Protein A an das rekombinante Zielprotein fusioniert wird. Bei einer Ausführungsform ist die codierende Sequenz des SES-Proteins in einen pGEX-Expressionsvektor kloniert, so daß ein Vektor erzeugt wird, der ein Fusionsprotein codiert, umfassend vom N-Terminus zum C-Terminus: GST - Thrombin-Spaltstelle - X-Protein. Das Fusionsprotein kann durch Affinitätschromatographie mittels Glutathion-Agarose-Harz gereinigt werden. Das rekombinante SES-Protein, das nicht mit GST fusioniert ist, kann durch Spaltung des Fusionsproteins mit Thrombin gewonnen werden.

Beispiele geeigneter induzierbarer Nicht-Fusions-*E.-coli-*Expressionsvektoren umfassen pTrc (Amann et al., (1988) Gene 69:301-315), und pET 11d (Studier et al. Gene Expression Technology: Methods in Enzymology 185, Academic Press, San Diego, Kalifornien (1990) 60-89). Die Zielgenexpression aus dem pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL 21 (DE3) oder HMS174 (DE3) von einem residenten λ-Prophagen geliefert, der ein T7 gn1-Gen unter der Transkriptiönskontrolle des lacUV 5-Promotors birgt.

Eine Strategie zur Maximierung der Expression des rekombinanten Proteins ist die Expression des Proteins in einem Wirtsbakterium, dessen Fähigkeit zur proteolytischen Spaltung des rekombinanten Proteins gestört ist (Gottesman, S. Gene Expression Technology: Methods in Enzymology 185, Academic Press, San Diego, Kalifornien (1990) 119-128). Eine weitere Strategie ist die Veränderung der Nukleinsäuresequenz der in einen Expressionsvektor zu inserierenden Nukleinsäure, so daß die einzelnen Codons für jede Aminosäure diejenigen sind, die vorzugsweise in einem zur Expression ausgewählten Bakterium, wie *C*. *glutamicum,* verwendet werden (Wada et al. (1992) Nucleic Acids Res. 20:2111-2118). Diese Veränderung der erfindungsgemäßen Nukleinsäuresequenzen kann durch Standard-DNA-Synthesetechniken erfolgen.

Bei einer weiteren Ausführungsform ist der SES-Protein-Expressionsvektor ein Hefe-Expressionsvektor. Beispiele für Vektoren zur Expression in der Hefe *S*. *cerevisiae* umfassen pYepSec1 (Baldari et al., (1987) Embo J. 6:229-234), pMFa (Kurjan und Herskowitz (1982) Cell 30:933-943), pJRY88 (Schultz et al. (1987) Gene 54:1113-123) sowie pYES2 (Invitrogen Corporation, San Diego, CA). Vektoren und Verfahren zur Konstruktion von Vektoren, die sich zur Verwendung in anderen Pilzen, wie filamentösen Pilzen, eignen, umfassen diejenigen, die eingehend beschrieben sind in: van den Hondel, C.A.M.J.J. & Punt, P.J. (1991) "Gene transfer systems and vector development for filamentous fungi, in: Applied Molecular Genetics of Fungi, J.F. Peberdy et al., Hrsg., S. 1-28, Cambridge University Press: Cambridge.

Alternativ können die erfindungsgemäßen SES-Proteine in Insektenzellen unter Verwendung von Baculovirus-Expressionsvektoren exprimiert werden. Baculovirus-Vektoren, die zur Expression von Proteinen in gezüchteten Insektenzellen (bspw. Sf9-Zellen) verfügbar sind, umfassen die pAc-Reihe (Smith et al., (1983) Mol. Cell Biol.. 3:2156-2165) und die pVL-Reihe (Lucklow und Summers (1989) Virology 170:31-39).

In einer weiteren Ausführungsform können die erfindungsgemäßen SES-Proteine in Zellen einzelliger Pflanzen (wie Algen) oder in Pflanzenzellen höherer Pflanzen (bspw. Spermatophyten, wie Feldfrüchte) exprimiert werden. Beispiele für Pflanzen-Expressionsvektoren umfassen solche, die eingehend beschrieben sind in: Becker, D., Kemper, E., Schell, J. und Masterson, R. (1992) "New plant binary vectors with selectable markers located proximal to the left border", Plant Mol. Biol. 20:1195-1197; und Bevan, M.W: (1984) "Binary Agrobacterium vectors for plant transformation", Nucl. Acids Res. 12:8711-8721.

In einer weiteren Ausführungsform wird eine erfindungsgemäße Nukleinsäure in Säugetierzellen mit einem Säugetier-Expressionsvektor exprimiert. Beispiele für Säugetier-Expressionsvektoren umfassen pCDM8 (Seed, B. (1987) Nature 329:840) und pMT2PC (Kaufman et al. (1987) EMBO J. 6:187-195). Bei der Verwendung in Säugetierzellen werden die Kontrollfunktionen des Expressionsvektors oft von viralen regulatorischen Elementen bereitgestellt. Gemeinhin verwendete Promotoren stammen bspw. aus Polyoma, Adenovirus 2, Cytomegalievirus und Simian Virus 40. Weitere geeignete Expressionssysteme für prokaryotische und eukaryotische Zellen siehe in Kapitel 16 und 17 von Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989.

Bei einer weiteren Ausführungsform kann der rekombinante Säugetier-Expressionsvektor die Expression der Nukleinsäure vorzugsweise in einem bestimmten Zelltyp bewirken (bspw. werden gewebespezifische regulatorische Elemente zur Expression der Nukleinsäure verwendet). Gewebespezifische regulatorische Elemente sind im Fachgebiet bekannt. Nicht-einschränkende Beispiele für geeignete gewebespezifische Promotoren umfassen den Albuminpromotor (leberspezifisch; Pinkert et al. (1987) Genes Dev. 1:268-277), lymphoid-spezifische Promotoren (Calame und Eaton (1988) Adv. Immunol. 43:235-275), insbesondere Promotoren von T-Zellrezeptoren (Winoto und Baltimore (1989) EMBO J. 8:729-733) und Immunglobulinen (Banerji et al. (1983) Cell 33:729-740; Queen und Baltimore (1983) Cell 33:741-748), neuronenspezifische Promotoren (bspw. der Neurofilament-Promotor; Byrne und Ruddle (1989) PNAS 86:5473-5477), pankreasspezifische Promotoren (Edlund et al., (1985) Science 230:912-916) und milchdrüsenspezifische Promotoren (bspw. Milchserum-Promotor; US-Patent Nr. 4 873 316 und europäische Patentanmeldungsveröffentlichung Nr. 264 166). Entwicklungsregulierte Promotoren sind ebenfalls umfaßt, bspw. die Maus-hox-Promotoren (Kessel und Gruss (1990) Science 249:374-379) und der α-Fetoprotein-Promotor (Campes und Tilghman (1989) Genes Dev. 3:537-546).

Die Erfindung stellt zudem einen rekombinanten Expressionsvektor bereit, umfassend ein erfindungsgemäßes DNA Molekül, das in Antisense-Richtung in den Expressionsvektor kloniert ist. D.h. daß das DNA-Molekül derart mit einer regulatorischen Sequenz funktionsfähig verbunden ist, daß die Expression (durch Transkription des DNA-Moleküls) eines RNA-Moleküls, das zur SES-mRNA antisense ist, möglich wird. Es können regulatorische Sequenzen ausgewählt werden, die funktionsfähig an eine in Antisense-Richtung klonierte Nukleinsäure gebunden sind und die kontinuierliche Expression des Antisense-RNA-Moleküls in einer Vielzahl von Zelltypen steuern, bspw. können virale Promotoren und/oder Enhancer oder regulatorische Sequenzen ausgewählt werden, die die konstitutive, gewebespezifische oder zelltypspezifische Expression von Antisense-RNA steuern. Der Antisense-Expressionsvektor kann in Form eines rekombinanten Plasmids, Phagemids oder attenuierten Virus vorliegen, in dem Antisense-Nukleinsäuren unter der Kontrolle eines hochwirksamen regulatorischen Bereichs produziert werden, dessen Aktivität durch den Zelltyp bestimmt wird, in den der Vektor eingebracht wird. Für eine Diskussion der Regulation der Genexpression mittels Antisense-Genen siehe Weintraub, H. et al., Antisense-RNA as a molecular tool for genetic analysis, Reviews - Trends in Genetics, Bd. 1(1) (1986).

Ein weiterer Aspekt der Erfindung betrifft Wirtszellen, in die ein erfindungsgemäßer rekombinanter Expressionsvektor eingebracht worden ist. Die Begriffe "Wirtszelle" und "rekombinante Wirtszelle" werden hier untereinander austauschbar verwendet. Es ist selbstverständlich, daß diese Begriffe nicht nur eine bestimmte Zielzelle, sondern auch die Nachkommen oder potentiellen Nachkommen dieser Zelle betreffen. Da in aufeinanderfolgenden Generationen aufgrund von Mutation oder Umwelteinflüssen bestimmte Modifikationen auftreten können, sind diese Nachkommen nicht unbedingt mit der Parentalzelle identisch, sind jedoch im Umfang des Begriffs, wie er hier verwendet wird, noch umfaßt.

Eine Wirtszelle kann eine prokaryotische oder eukaryotische Zelle sein. Bspw. kann ein SES-Protein in Bakterienzellen, wie *C*. *glutamicum*, Insektenzellen, Hefe- oder Säugetierzellen (wie O-varzellen des chinesischen Hamsters (CHO) oder COS-Zellen) exprimiert werden. Andere geeignete Wirtszellen sind dem Flachmann geläufig. Mikroorganismen, die mit *Corynebacterium glutamicum* verwandt sind, sind als Wirtszellen für die erfindungsgemäßen Nukleinsäure- und Proteinmoleküle geeignet.

Durch herkömmliche Transformations- oder Transfektionsverfahren läßt sich Vektor-DNA in prokaryotische oder eukaryotische Zellen einbringen. Die Begriffe "Transformation" und "Transfektion", "Konjugation" und "Transduktion", wie sie hier verwendet werden, sollen eine Vielzahl von im Stand der Technik bekannten Verfahren zum Einbringen fremder Nukleinsäure (bspw. DNA) in eine Wirtszelle umfassen, einschließlich Calciumphosphat- oder Calciumchlorid-Copräzipitation, DEAE-Dextran-vermittelter Transfektion, Lipofektion, natürlicher Kompetenz, chemisch vermittelter Übertragung oder Elektroporation. Geeignete Verfahren zur Transformation oder Transfektion von Wirtszellen lassen sich nachlesen in Sambrook et al. (Molecular Cloning: A Laboratory Manual. 2. Aufl., Cold Spring Harbor Laboratory, Cold Spring Harbor Laboratory Press, Cold Spring Harbor, NY, 1989) und anderen Labor-Handbüchern.

Es ist bekannt, daß für die stabile Transfektion von Säugetierzellen je nach dem verwendeten Expressionsvektor und der verwendeten Transfektionstechnik nur ein kleiner Teil der Zellen die fremde DNA in ihr Genom integrieren kann. Zur Identifizierung und Selektion dieser Integranten wird gewöhnlich ein Gen, das einen selektierbaren Marker (z.B. Resistenz gegen Antibiotika) codiert, zusammen mit dem Gen von Interesse in die Wirtszellen eingebracht. Bevorzugte selektierbare Marker umfassen solche, die die Resistenz gegen Medikamente, wie G418, Hygromycin und Methotrexat, verleihen. Eine Nukleinsäure, die einen selektierbaren Marker codiert, kann in eine Wirtszelle auf dem gleichen Vektor eingebracht werden, wie derjenige, der ein SES-Protein codiert, oder kann auf einem gesonderten Vektor eingebracht werden. Zellen, die mit der eingebrachten Nukleinsäure stabil transfiziert worden sind, können bspw. durch Medikamentenselektion identifiziert werden (z.B. überleben Zellen, die den selektierbaren Marker integriert haben, wohingegen die anderen Zellen sterben).

Zur Erzeugung eines homolog rekombinierten Mikroorganismus wird ein Vektor hergestellt, der zumindest einen Abschnitt eines SES-Gens enthält, in den eine Deletion, Addition oder Substitution eingebracht worden ist, um das SES-Gen zu verändern, bspw. funktionell zu disrumpieren. Dieses SES-Gen ist vorzugsweise ein *Corynebacterium glutamicum*-SES-Gen, jedoch kann ein Homologon von einem verwandten Bakterium oder sogar aus einer Säugetier-, Hefe- oder Insektenquelle verwendet werden. Bei einer bevorzugten Ausführungsform ist der Vektor derart ausgestaltet, daß das endogene SES-Gen bei homologer Rekombination funktionell disrumpiert ist (d.h. nicht länger ein funktionelles Protein codiert; auch als "Knockout"-Vektor bezeichnet). Der Vektor kann alternativ derart ausgestaltet sein, daß das endogene SES-Gen bei homologer Rekombination mutiert oder anderweitig verändert ist, jedoch noch das funktionelle Protein codiert (z.B. kann der stromaufwärts gelegene regulatorische Bereich derart verändert sein, daß dadurch die Expression des endogenen SES-Proteins verändert wird.). Der veränderte Abschnitt des SES-Gens ist im homologen Rekombinationsvektor an seinem 5'- und 3'-Ende von zusätzlicher Nukleinsäure des SES-Gens flankiert, die eine homologe Rekombination zwischen dem exogenen SES-Gen, das von dem Vektor getragen wird, und einem endogenen SES-Gen in einem Mikroorganismus ermöglicht. Die zusätzliche flankierende SES-Nukleinsäure ist für eine erfolgreiche homologe Rekombination mit dem endogenen Gen hinreichend lang. Gewöhnlich enthält der Vektor mehrere Kilobasen flankierende DNA (sowohl am 5'- als auch am 3'-Ende) (siehe z.B. Thomas, K.R. und Capecchi, M.R. (1987) Cell 51:503 für eine Beschreibung von homologen Rekombinationsvektoren). Der Vektor wird in einen Mikroorganismus (z.B. durch Elektroporation) eingebracht, und Zellen, in denen das eingebrachte SES-Gen mit dem endogenen SES-Gen homolog rekombiniert ist, werden unter Verwendung im Fachgebiet bekannter Verfahren selektiert.

Bei einer anderen Ausführungsform können rekombinante Mikroorganismen produziert werden, die ausgewählte Systeme enthalten, die eine regulierte Expression des eingebrachten Gens ermöglichen. Der Einschluß eines SES-Gens in einen Vektor, wodurch es unter die Kontrolle des Lac-Operons gebracht wird, ermöglicht z.B. die Expression des SES-Gens nur in Gegenwart von IPTG. Diese regulatorischen Systeme sind im Fachgebiet bekannt.

Eine erfindungsgemäße Wirtszelle, wie eine prokaryotische oder eukaryotische Wirtszelle in Kultur, kann zur Produktion (d.h. Expression) eines SES-Proteins verwendet werden. Die Erfindung stellt zudem Verfahren zur Produktion von SES-Proteinen unter Verwendung der erfindungsgemäßen Wirtszellen bereit. Bei einer Ausführungsform umfaßt das Verfahren die Anzucht der erfindungsgemäßen Wirtszelle (in die ein rekombinanter Expressionsvektor, der ein SES-Protein codiert, eingebracht worden ist, oder in deren Genom ein Gen eingebracht worden ist, das ein Wildtyp- oder verändertes SES-Protein codiert) in einem geeigneten Medium, bis das SES-Protein produziert worden ist. Das Verfahren umfaßt in einer weiteren Ausführungsform das Isolieren der SES-Proteine aus dem Medium oder der Wirtszelle.

### C. Erfindungsgemäße Verwendungen und Verfahren

Die hier beschriebenen Nukleinsäuremoleküle, Proteine, Proteinhomologa, Fusionsproteine, Primer, Vektoren und Wirtszellen können in einem oder mehreren nachstehenden Verfahren verwendet werden: Identifikation von *C*. *glutamicum* und verwandten Organismen, Kartierung von Genomen von Organismen, die mit *C. glutamicum* verwandt sind, Identifikation und Lokalisation von *C. glutamicum*-Sequenzen von Interesse, Evolutionsstudien, Bestimmung von SES-Proteinbereichen, die für die Funktion notwendig sind, Modulation der Aktivität eines SES-Proteins; Modulation des Stoffwechsels einer oder mehrerer Zellmembrankomponenten; Modulation des Transmembrantransports einer oder mehrerer Verbindungen und Modulation der zellulären Produktion einer gewünschten Verbindung, wie einer Feinchemikalie. Die erfindungsgemäßen SES-Nukleinsäuremoleküle haben eine Vielzahl von Verwendungen. Sie können zunächst zur Identifikation eines Organismus als *Corynebacterium glutamicum* oder naher Verwandter davon verwendet werden. Sie können zudem zur Identifikation des Vorliegens von *C. glutamicum* oder eines Verwandten davon in einer Mischpopulation von Mikroorganismen verwendet werden. Die Erfindung stellt die Nukleinsäuresequenzen einer Reihe von *C. glutamicum*-Genen bereit. Durch Sondieren der extrahierten genomischen DNA einer Kultur einer einheitlichen oder gemischten Population von Mikroorganismen unter stringenten Bedingungen mit einer Sonde, die einen Bereich eines *C. glutamicum*-Gens überspannt, das für diesen Organismus einzigartig ist, kann man bestimmen, ob dieser Organismus zugegen ist. *Corynebacterium glutamicum* selbst ist zwar nicht pathogen, jedoch ist es mit pathogenen Arten, wie *Corynebacterium diptheriae*, verwandt. Der Nachweis eines solchen Organismus ist von signifikanter klinischer Bedeutung.

Die erfindungsgemäßen Nukleinsäure- und Proteinmoleküle können ferner als marker für bestimmte Bereiche des Genoms dienen. Dies eignet sich nicht nur zum Kartieren des Genoms, sondern auch für funktionelle Studien von *C. glutainicum*-Proteinen. Zur Identifikation des Genombereichs, an den ein bestimmtes *C. glutamicum-*DNA-bindendes Protein bindet, kann das *C. glutamicum*-Genom bspw. gespalten und die Fragmente mit dem DNA-bindenden Protein inkubiert werden. Diejenigen, die das Protein binden, können zusätzlich mit den erfindungsgemäßen Nukleinsäuremolekülen, vorzugsweise mit leicht nachweisbaren Markierungen, sondiert werden; die Bindung eines solchen Nukleinsäuremoleküls an das Genomfragment ermöglicht die Lokalisation des Fragmentes auf der genomischen Karte von *C. glutamicum*, und wenn dies mehrmals mit unterschiedlichen Enzymen durchgeführt wird, erleichtert es eine rasche Bestimmung der Nukleinsäuresequenz, an die das Protein bindet. Die erfindungsgemäßen Nukleinsäuremoleküle können zudem hinreichend homolog zu den Sequenzen verwandter Arten sein, so daß diese Nukleinsäuremoleküle als Marker zur Konstruktion einer genomischen Karte in verwandten Bakterien (z.B. *Brevibacterium lactofermentum*) dienen können.

Die erfindungsgemäßen SES-Nukleinsäuremoleküle eignen sich ebenfalls für Evolutions- und Proteinstruktur-Untersuchungen. Die Stoffwechsel- und Transportprozesse, an denen die erfindungsgemäßen Moleküle beteiligt sind, werden von einer Vielzahl von prokaryotischen und eukaryotischen Zellen ausgenutzt; durch Vergleich der Sequenzen der erfindungsgemäßen Nukleinsäuremoleküle mit solchen, die ähnliche Enzyme aus anderen Organismen codieren, kann der Evolutions-Verwandschaftsgrad der Organismen bestimmt werden. Entsprechend ermöglicht ein solcher Vergleich die Bestimmung, welche Sequenzbereiche konserviert sind und welche nicht, was bei der Bestimmung solcher Bereiche des Proteins hilfreich sein kann, die für die Enzymfunktion essentiell sind. Dieser Typ der Bestimmung ist für Proteintechnologie-Untersuchungen wertvoll und kann einen Hinweis darauf geben, wieviel Mutagenese das Protein tolerieren kann ohne die Funktion zu verlieren.

Die Manipulation der erfindungsgemäßen SES-Nukleinsäuremoleküle kann die Produktion von SES-Proteinen mit funktionellen Unterschieden zu den Wildtyp-SES-Proteinen bewirken. Diese Proteine können hinsichtlich ihrer Effizienz oder Aktivität verbessert werden, können in größerer Anzahl als gewöhnlich in der Zelle zugegen sein oder können hinsichtlich ihrer Effizienz oder Aktivität geschwächt sein.

Diese Modulation der Aktivität von Proteinen, die an der DNA-Reparatur, Rekombination oder Transposition in C. *glutamicum* beteiligt sind, sollte die genetische Stabilität der Zelle beeinflussen. Beispielsweise kann man durch Verringern der Anzahl oder Aktivität von Proteinen, die an DNA-Reparaturmechanismen beteiligt sind, die Fähigkeit der Zelle, genetische Fehler zu korrigieren, verringern, was das einfachere Einbringen gewünschter Mutationen in das Genom (wie solchen, die an der Feinchemikalienproduktion beteiligte Proteine codieren) ermöglichen sollte. Die Steigerung der Aktivität oder Anzahl von Transposons sollte ebenso zu einer erhöhten Mutationsrate im Genom führen und kann die leichte Verdopplung gewünschter Gene (bspw. solcher, die an der Feinchemikalienproduktion beteiligte Proteine codieren) oder Disruption unerwünschter Gene (z.B. solcher, die Feinchemikalien-Abbauproteine codieren) möglich machen. Dagegen kann es durch Verringern der Anzahl oder Aktivität von Transposons oder Erhöhen der Anzahl oder Aktivität von DNA-Reparaturproteinen möglich sind, die genetische Stabilität von *C. glutamicum* zu erhöhen, was wiederum zur besseren Aufrechterhaltung eingebrachter Mutationen in diesen Mikroorganismen durch mehrere Generationen in Kultur führen sollte. Idealerweise wird während der Mutagenese und Stammkonstruktion die Aktivität eines oder mehrerer DNA-Reparatursysteme verringert und die Aktivität eines oder mehrerer Transposons erhöht, aber wenn die gewünschte Mutation in dem Stamm erzielt worden ist, tritt das Gegenteil auf. Diese Manipulation ist möglich, indem ein oder mehrere DNA-Reparaturgene oder Transposons unter die Kontrolle eines induzierbaren Repressors gestellt wird/werden.

Die Modulation von an der Transkription und Translation in *C*. *glutamicum* beteiligten Proteinen kann direkte und indirekte Wirkungen auf die Produktion einer Feinchemikalien von diesen Mikroorganismen haben. Beispielsweise ist es durch Manipulation eines Proteins, das direkt ein Gen translatiert (z.B. einer Polymerase) oder direkt die Transkription reguliert (eines Repressor- oder Aktivatorproteins) möglich, die Expression des Zielgens direkt zu beeinflussen. Bei Genen, die ein Protein codieren, das an der Biosynthese oder am Abbau einer Feinchemikalie beteiligt ist sollte dieser Typ der genetischen Manipulation eine direkte Wirkung auf die Produktion dieser Feinchemikalie haben. Die Mutagenese eines Repressorproteins, so daß es sein Zielgen nicht länger reprimieren kann, oder die Mutagenese eines Aktivatorproteins, so daß seine Aktivität optimiert wird, sollte zu einer erhöhten Transkription des Zielgens führen. Ist das Zielgen z.B. ein Feinchemikalienbiosynthesegen, kann eine erhöhte Produktion dieser Chemikalie aufgrund der insgesamt größeren Anzahl an für vorliegenden Transkripten dieses Gens resultieren, was ebenfalls zu einer vergrößerten Anzahl des Proteins führen sollte. Die Erhöhung der Anzahl oder Aktivität eines Repressorproteins für eine Zielsequenz oder die Verringerung der Anzahl oder Aktivität eines Aktivatorproteins für eine Zielsequenz sollte, wenn diese Sequenz bspw. ein Feinchemikalien-Abbauprotein ist, zu einer ähnlichen Steigerung der Produktion der Feinchemikalie führen.

Auch indirekte Wirkungen auf die Feinchemikalienproduktion können aus der Manipulation von Proteinen, die an der Transkription und Translation beteiligt sind, hervorgehen. Durch die Modulation der Aktivität oder Anzahl von Transkriptionsfaktoren (z.B. der Sigma-Faktoren) oder von Translationsrepressoren/-aktivatoren, die die Transkription in *C. glutamicum* in Reaktion auf Umwelt- oder Stoffwechselfaktoren global regulieren, sollte es möglich sein, die zelluläre Transkription von der Umwelt- oder Stoffwechselregulation abzukoppeln. Dies könnte wiederum eine kontinuierliche Transkription unter Bedingungen ermöglichen, die gewöhnlich die Genexpression verlangsamen oder beenden würden, wie ungünstigen Bedingungen (z.B. hohe Temperatur, niedriger Sauerstoffgehalt, hoher Spiegel an Abfallprodukten), die in einer Fermenterkultur im Großmaßstab vorliegen. Durch Erhöhen der Rate der Expression des Gens (z.B. Feinchemikalienbiosynthesegens) in diesen Situationen kann auch, zumindest aufgrund der vergleichsweise größeren Anzahl der Feinchemikalienbiosynthese-proteine in der Zelle, die Gesamtrate der Feinproduktproduktion erhöht werden. Prinzipien und Beispiele für die Modifikation der Transkriptions- und Translationsregulation sind beschrieben in z.B. Lewin, B. (1990) Genes IV, Teil 3: "Controlling procaryotic genes by transcription", Oxford Univ. Press: Oxford, S. 213-301.

Die Modulation der Aktivität oder Anzahl von Proteinen, die an der Polypeptidfaltung beteiligt sind (z.B. Chaperone) kann insgesamt die Erhöhung der Produktion korrekt gefalteter Moleküle in der Zelle ermöglichten. Dies hat zwei Auswirkungen: zunächst insgesamt eine Erhöhung der Anzahl an Proteinen in der Zelle aufgrund der Tatsache, daß weniger Proteine falsch gefaltet und abgebaut werden und, zweitens, eine Erhöhung der Anzahl eines gegebenen Proteins, das korrekt gefaltet und somit aktiv ist (s. z.B. Thomas, J.G., Baneyx, F. (1997) Protein Expression and Purification 11(3):289-296; Luo, Z.H., und Hua, Z.C. (1998) Biochemistry and Molecular Biology International 46(3):471-477; Dale, G.E., et al. (1994) Protein Engineering 7(7):925-931; Amrein, K.E. et al. (1995) Proc. Natl. Acad. Sci. U.S.A. 92(4):1048-1052 und Caspers, P., et al. (1994) Cell. Mol. Biol. 40(5):635-644). Solche Mutationen führen zwar zu einer Erhöhung der Anzahl aktiver Proteine jeder Art, aber wenn sie mit zusätzlichen Mutationen, die die Aktivität oder Anzahl von z.B. einem Feinchemikalienbiosyntheseprotein erhöhen, gekoppelt werden, kann eine additive Wirkung auf die Menge an korrekt gefaltetem aktivem gewünschtem Protein erhalten werden.

Die Manipulation von Proteinen, die an der Sekretion von Polypeptiden aus *C. glutamicum* beteiligt sind, so daß ihre Aktivität oder Anzahl verbessert ist, kann die Sekretion einer proteinartigen Feinchemikalie (z.B. eines Enzyms) aus diesem Mikroorganismus direkt verbessern. Es ist erheblich leichter, Feinchemikalien zu ernten und zu reinigen, wenn sie in das Medium einer Kultur im Großmaßstab sezerniert werden als wenn sie in der Zelle zurückgehalten werden, so daß die Ausbeute und Produktion einer Feinchemikalie durch diese Veränderung des Sekretionssystems erhöht werden sollte. Die genetische Manipulationen dieser Sekretionsproteine kann auch zu direkten Verbesserungen der Produktion einer oder mehrerer Feinchemikalien führen. Erstens kann die gesteigerte oder verringerte Aktivität eines oder mehrerer *C. glutamicum*-Sekretionssysteme (wie sie durch Mutagenese eines oder mehrerer SES-Proteine, die an diesen Wegen beteiligt sind, erzielt wird) zu insgesamt erhöhten oder verringerten Sekretionsraten aus der Zelle führen. Viele dieser sezernierten Proteine haben Funktionen, die für die Zellebensfähigkeit wichtig sind (z.B. Zelloberflächenproteasen oder -Rezeptoren). Eine Änderung des Sekretionswegs, so daß diese Proteine leichter an ihren extrazellulären Ort transportiert werden, kann die Gesamtlebensfähigkeit der Zelle erhöhen und somit zu höheren Zahlen an *C. glutamicum*-Zellen führen, die Feinchemikalien während eine Züchtung im Großmaßstab produzieren können. Zweitens spielen bestimmte bakterielle Sekretionssysteme (z.B. das sec-System) bekanntlich auch eine signifikante Rolle bei dem Prozeß, durch den integrale Membranproteinen (z.B. Kanäle, Poren oder Transporter) in die Zellmembran inserieren. Wird die Aktivität eines oder mehrerer Sekretionswegproteine gesteigert, kann die Fähigkeit der Zelle, Feinchemikalien zu produzieren, aufgrund des Vorliegens erhöhten intrazellulärer Nährstoffmengen oder verringerter Mengen an intrazellulären Abfallstoffen ebenfalls erhöht werden. Ist die Aktivität eines oder mehrerer dieser Sekretionswegproteine verringert, können nicht genügend Nährstoffe zur Unterstützung der Überproduktion gewünschter Verbindungen vorhanden sein, oder Abfallprodukte können diese Biosynthese stören.

Diese vorstehend genannten Mutagenesestrategien für SES-Proteine, die erhöhte Ausbeuten einer Feinchemikalie aus *C. glutamicum* bewirken sollen, sollen nicht einschränken sein; Variationen dieser Mutagenesestrategien sind dem Fachmann leicht ersichtlich. Unter Verwendung dieser Strategien und einschließlich der hier offenbarten Mechanismen können die erfindungsgemäßen Nukleinsäure- und Proteinmoleküle verwendet werden, um *C. glutamicum-* oder verwandte Bakterienstämme, die mutierte SES-Nukleinsäure- und Proteinmoleküle exprimieren, zu erzeugen, so daß die Ausbeute, Produktion und/oder Effizienz der Produktion einer gewünschten Verbindung verbessert wird. Die gewünschte Verbindung kann jedes von *C. glueamicum* hergestellte Produkt sein, einschließlich der Endprodukte von Biosynthesewegen und Zwischenprodukte natürlich vorkommender Stoffwechselwege sowie Moleküle, die im Metabolismus von *C. glutamicum* nicht natürlich vorkommen, die jedoch von einem erfindungsgemäßen *C. glutamicum-*Stamm produziert werden.

Diese Erfindung wird durch die nachstehenden Beispiele weiter veranschaulicht, die nicht als einschränkend aufgefaßt werden sollen. Die Inhalte sämtlicher, in dieser Patentanmeldung zitierter Literaturstellen, Patentanmeldungen, Patente und veröffentlichter Patentanmeldungen sind hiermit durch Bezugnahme aufgenommen.

### Beispiele

### Beispiel 1: Präparation der gesamten genomischen DNA aus Corynebacterium glutamicum ATCC13032

Eine Kultur von *Corynebacterium glutamicum* (ATCC 13032) wurde über Nacht bei 30°C unter starkem Schütteln in BHI-Medium (Difco) gezüchtet. Die Zellen wurden durch Zentrifugation geerntet, der Überstand wurde verworfen, und die Zellen wurden in 5ml Puffer 1 (5% des Ursprungsvolumens der Kultur - sämtliche angegebenen Volumina sind für 100 ml Kulturvolumen berechnet) resuspendiert. Zusammensetzung von Puffer I: 140,34 g/l Saccharose, 2,46 g/l MgSO₄ · 7 H₂O, 10 ml/l KH₂PO₄-Lösung (100g/l, mit KOH auf pH-Wert 6,7 eingestellt), 50 ml/l M12-Konzentrat (10 g/l (NH₄)₂SO₄, 1 g/l NaCl, 2 g/l MgSO₄ · 7 H₂O, 0,2 g/l CaCl₂, 0,5 g/l HefeExtrakt (Difco), 10 ml/l Spurenelemente-Mischung (200 mg/l FeSO₄ · H₂O, 10 mg/l ZnSO₄ · 7 H₂O, 3 mg/l MnCl₂ · 4 H₂O, 30 mg/l H₃BO₃, 20 mg/l CoCl₂ · 6 H₂O, 1 mg/l NiCl₂ · 6 H₂O, 3 mg/l Na₂MoO₄ · 2 H₂O, 500 mg/l Komplexbildner (EDTA oder Citronensäure), 100 ml/l Vitamingemisch (0,2 ml/l Biotin, 0,2 mg/l Folsäure, 20 mg/l p-Aminobenzoesäure, 20 mg/l Riboflavin, 40 mg/l Ca-Panthothenat, 140 mg/l Nikotinsäure, 40 mg/l Pyridoxolhydrochlorid, 200 mg/l Myo-Inositol). Lysozym wurde in einer Endkonzentration von 2,5 mg/ml zur Suspension gegeben. Nach etwa 4 Std. Inkubation bei 37°C wurde die Zellwand abgebaut, und die erhaltenen Protoplasten wurden durch Zentrifugation geerntet. Das Pellet wurde einmal mit 5 ml Puffer I und einmal mit 5 ml TE-Puffer (10 mM Tris-HCl, 1 mM EDTA, pH-Wert 8) gewaschen. Das Pellet wurde in 4 ml TE-Puffer resuspendiert, und 0,5 ml SDS-Lösung (10%) und 0,5 ml NaCl-LÖsung (5 M) wurden zugegeben. Nach Zugabe von Proteinase K in einer Endkonzentration von 200 ∝g/ml wurde die Suspension etwa 18 Std. bei 37°C inkubiert. Die DNA wurde durch Extraktion mit Phenol, Phenol-Chloroform-Isoamylalkohol und Chloroform-Isoamylalkohol mittels Standard-Verfahren gereinigt. Dann wurde die DNA durch Zugabe von 1/50 Volumen 3 M Natriumacetat und 2 Volumina Ethanol, anschließender Inkubation für 30 min bei -20°C und 30 min Zentrifugation bei 12000 U/min in einer Hochgeschwindigkeitszentrifuge mit einem SS34-Rotor (Sorvall) gefällt. Die DNA wurde in 1 ml TE-Puffer gelöst, der 20 ∝g/ml RNase A enthielt, und für mindestens 3 Std. bei 4°C gegen 1000 ml TE-Puffer dialysiert. Während dieser Zeit wurde der Puffer 3mal ausgetauscht. Zu Aliquots von 0,4 ml der dialysierten DNA-Lösung wurden 0,4 ml 2 M LiCl und 0,8 ml Ethanol zugegeben. Nach 30 min Inkubation bei -20°C wurde die DNA durch Zentrifugation gesammelt (13000 U/min, Biofuge Fresco, Heraeus, Hanau, Deutschland). Das DNA-Pellet wurde in TE-Puffer gelöst. Durch dieses Verfahren hergestellte DNA konnte für alle Zwecke verwendet werden, einschließlich Southern-Blotting oder zur Konstruktion genomischer Banken.

### Beispiel 2: Konstruktion genomischer Corynebacterium glutamicum (ATCC13032)-Banken in Escherichia coli

Ausgehend von DNA, die wie in Beispiel 1 beschrieben hergestellt wurde, wurden gemäß bekannter und gut eingeführter Verfahren (siehe bspw. Sambrook, J. et al. (1989) "Molecular Cloning: A Laboratory Manual". Cold Spring Harbor Laboratory Press oder Ausubel, F.M. et al. (1994) "Current Protocols in Molecular Biology", John Wiley & Sons) Cosmid- und Plasmid-Banken hergestellt.

Es ließ sich jedes Plasmid oder Cosmid einsetzen. Besondere Verwendung fanden die Plasmide pBR322 (Sutcliffe, J.G. (1979) Proc. Natl Acad. Sci. USA, 75:3737-3741); pACYC177 (Change & Cohen (1978) J. Bacteriol. 134:1141-1156); Plasmide der pBS-Reihe (pBSSK+, pBSSK- und andere; Stratagene, LaJolla, USA) oder Cosmide, wie SuperCos1 (Stratagene, LaJolla, USA) oder Lorist6 (Gibson, T.J. Rosenthal, A., und Waterson, R.H. (1987) Gene 53: 283-286.

### Beispiel 3: DNA-Sequenzierung und Computer-Funktionsanalyse

Genomische Banken, wie in Beispiel 2 beschrieben, wurden zur DNA-Sequenzierung gemäß Standard-Verfahren, insbesondere dem Kettenabbruchverfahren mit ABI377-Sequenziermaschinen (s. z.B. Fleischman, R.D. et al. (1995) "Whole-genome Random Sequencing and Assembly of Haemophilus Influenzae Rd., Science 269:496-512) verwendet. Die Sequenzierprimer mit den folgenden Nukleotidsequenzen wurden verwendet: 5'-GGAAACAGTATGACCATG-3' oder 5'-GTAAAACGACGGCCAGT-3'.

### Beispiel 4: In-vivo-Mutagenese

In vivo-Mutagenese von *Corynebacterium glutamicum* kann durchgeführt werden, indem eine Plasmid- (oder andere Vektor-) DNA durch *E.coli* oder andere Mikroorganismen (z.B. *Bacillus* spp. oder Hefen, wie *Saccharomyces cerevisiae*) geschleust wird, die die Integrität ihrer genetischen Information nicht aufrechterhalten können. Übliche Mutatorstämme weisen Mutationen in den Genen für das DNA-Reparatursystem auf (z.B., mutHLS, mutD, mutT, usw., zum Vergleich siehe Rupp, W.D. (1996) DNA repair mechanisms, in: Escherichia coli and Salmonella, S. 2277-2294, ASM: Washington). Diese Stämme sind dem Fachmann bekannt. Die Verwendung dieser Stämme ist bspw. in Greener, A. und Callahan, M. (1994) Strategies 7:32-34 veranschaulicht.

### Beispiel 5: DNA-Transfer zwischen Escherichia coli und Corynebacterium glutamicum

Mehrere *Corynebacterium-* und *Brevibacterium*-Arten enthalten endogene Plasmide (wie bspw. pHM1519 oder pBL1) die autonom replizieren (für einen Überblick siehe bspw. Martin, J.F. et al. (1987) Biotechnology 5:137-146). Shuttle-Vektoren für *Escherichia coli* und *Corynebacterium glutamicum* lassen sich leicht mittels Standard-Vektoren für *E. coli* konstruieren (Sambrook, J. et al., (1989), "Molecular Cloning: A Laboratory Manual", Cold Spring Harbor Laboratory Press oder Ausubel, F.M. et al. (1994) "Current Protocols in Molecular Biology", John Wiley & Sons), denen ein Replikationsursprung für und ein geeigneter Marker aus *Corynebacterium glutamicum* beigegeben wird. Solche Replikationsursprünge werden vorzugsweise von endogenen Plasmiden entnommen, die aus *Corynebacterium-* und Brevibactertium-Arten isoliert worden sind. Besondere Verwendung als Transformationsmarker für diese Arten sind Gene für Kanamycin-Resistenz (wie solche, die vom Tn5- oder Tn-903-Transposon stammen) oder für Chloramphenicol (Winnacker, E.L. (1987) "From Genes to Clones - Introduction to Gene Technology, VCH, Weinheim). Es gibt zahlreiche Beispiele in der Literatur für die Herstellung einer großen Vielzahl von Shuttle-Vektoren, die in *E. coli* und *C. glutamicum* replizieren und für verschiedene Zwecke verwendet werden können, einschließlich Gen-Überexpression (siehe bspw. Yoshihama, M. et al. (1985) J. Bacteriol. 162:591-597, Martin, J.F. et al., (1987) Biotechnology, 5:137-146 und Eikmanns, B.J. et al. (1992) Gene 102:93-98).

Mittels Standard-Verfahren ist es möglich, ein Gen von Interesse in einen der vorstehend beschriebenen Shuttle-Vektoren zu klonieren und solche Hybrid-Vektoren in *Corynebacterium glutamicum-*Stämme einzubringen. Die Transformation von *C. glutamicum* läßt sich durch Protoplastentransformation (Kastsumata, R. et al., (1984) J. Bacteriol. 159:306-311), Elektroporation (Liebl, E. et al., (1989) FEMS Microbiol. Letters, 53:399-303) und in Fällen, bei denen spezielle Vektoren verwendet werden, auch durch Konjugation erzielen (wie z.B. beschrieben in Schäfer, A., et (1990) J. Bacteriol. 172:1663-1666). Es ist ebenfalls möglich, die Shuttle-Vektoren für *C. glutamicum* auf *E. coli* zu übertragen, indem Plasmid-DNA aus *C. glutamicum* (mittels im Fachgebiet bekannter Standard-Verfahren) präpariert und in *E. coli* transformiert wird. Dieser Transformationsschritt kann mit Standard-Verfahren erfolgen, jedoch wird vorteilhafterweise ein Mcrdefizienter *E. coli*-Stamm verwendet, wie NM522 (Gough & Murray (1983) J. Mol. Biol. 166:1-19).

### Beispiel 6: Bestimmung der Expression des mutanten Proteins

Die Beobachtungen der Aktivität eines mutierten Proteins in einer transformierten Wirtszelle beruhen auf der Tatsache, daß das mutante Protein auf ähnliche Weise und in ähnlicher Menge exprimiert wird wie das Wildtyp-Protein. Ein geeignetes Verfahren zur Bestimmung der Transkriptionsmenge des mutanten Gens (ein Anzeichen für die mRNA-Menge, die für die Translation des Genprodukts verfügbar ist) ist die Durchführung eines Northern-Blots (s. bspw. Ausubel et al., (1988) Current Protocols in Molecular Biology, Wiley: New York), wobei ein Primer, der so ausgestaltet ist, daß er an das Gen von Interesse bindet, mit einer nachweisbaren (gewöhnlich radioaktiven oder chemilumineszierenden) Markierung versehen wird, so daß - wenn die Gesamt-RNA einer Kultur des Organismus extrahiert, auf einem Gel aufgetrennt, auf eine stabile Matrix übertragen und mit dieser Sonde inkubiert wird - die Bindung und die Quantität der Bindung der Sonde das Vorliegen und auch die Menge der mRNA für dieses Gen anzeigt. Diese Information ist ein Hinweis auf das Ausmaß der Transkription des mutanten Gens. Gesamt-Zell-RNA läßt sich durch verschiedene Verfahren aus *Corynebacterium glutamicum* isolieren, die im Fachgebiet bekannt sind, wie in Bormann, E.R. et al., (1992) Mol. Microbiol. 6:317-326 beschrieben.

Zur Bestimmung des Vorliegens oder der relativen Menge an Protein, das von dieser mRNA translatiert wird, können Standard-Techniken, wie Western-Blot, eingesetzt werden (s. bspw. Ausubel et al. (1988) "Current Protocols in Molecular Biology", Wiley, New York). Bei diesem Verfahren werden Gesamt-Zellproteine extrahiert, durch Gelelektrophorese aufgetrennt, auf eine Matrix, wie Nitrocellulose, übertragen und mit einer Sonde, wie einem Antikörper, die an das gewünschte Protein spezifisch bindet, inkubiert, Diese Sonde ist gewöhnlich mit einer chemilumineszierenden oder colorimetrischen Markierung versehen, die sich leicht nachweisen läßt. Das vorliegen und die beobachtete Menge an Markierung zeigt das Vorliegen und die Menge des gesuchten Mutantenproteins in der Zelle an.

### Beispiel 7: Wachstum von genetisch verändertem Corynebacterium glutamicum - Medien und Anzuchtbedingungen

Genetisch veränderte *Corynebakterien* werden in synthetischen oder natürlichen Wachstumsmedien gezüchtet. Eine Anzahl unterschiedlicher Wachstumsmedien für Corynebakterian sind bekannt und leicht erhältlich (Lieb et al. (1989) Appl. Microbiol. Biotechnol. 32:205-210; von der Osten et al. (1998) Biotechnology Letters 11:11-16; Patent DE 4 120 867; Liebl (1992) "The Genus Corynebacterium", in: The Procaryotes, Bd. II, Balows, A., et al., Hrsg. Springer-Verlag). Diese Medien bestehen aus einer oder mehreren Kohlenstoffquellen, Stickstoffquellen, anorganisehen Salzen, Vitaminen und Spurenelementen. Bevorzugte Kohlenstoffquellen sind Zucker, wie Mono-, Di- oder Polysaccharide. Sehr gute Kohlenstoffquellen sind bspw. Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Alkohole und organische Säuren, wie Methanol, Ethanol, Essigsäure oder Milchsäure. Stickstoffquellen sind gewöhnlich organische oder anorganische Stickstoffverbindungen oder Materialien, die diese Verbindungen enthalten. Beispielhafte Stickstoffquellen umfassen. Ammoniak-Gas oder Ammoniumsalze, wie NH₄Cl oder (NH₄)₂SO₄, NH₄OH, Nitrate, Harnstoff, Aminosäuren oder komplexe Stickstoffquellen, wie Maisquellwasser, Sojamehl, Sojaprotein, Hefeextrakt, Fleischextrakt und andere.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen. Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure. Die Medien enthalten üblicherweise auch andere Wachstumsfaktoren, wie Vitamine oder Wachstumsförderer, zu denen bspw. Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) oder anderen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Anzuchtbedingungen werden für jedes Experiment gesondert definiert. Die Temperatur sollte zwischen 15°C und 45°C liegen und kann während des Experimentes konstant gehalten oder verändert werden. Der pH-Wert des Mediums sollte im Bereich von 5 bis 8,5, vorzugsweise um 7,0 liegen, und kann durch Zugabe von Puffern zu den Medien aufrechterhalten werden. Ein beispielhafter Puffer für diesen Zweck ist ein Kaliumphosphatpuffer. Synthetische Puffer, wie MOPS, HEPES; ACES usw., können alternativ oder gleichzeitig verwendet werden. Der Anzucht-pH-Wert läßt sich während der Anzucht auch durch Zugabe von NaOH oder NH₄OH konstant halten. Werden komplexe Medienkomponenten, wie HefeExtrakt, verwendet, sinkt der Bedarf an zusätzlichen Puffern, da viele komplexe Verbindungen eine hohe Pufferkapazität aufweisen. Beim Einsatz eines Fermenters für die Anzucht von Mikroorganismen kann der pH-Wert auch mit gasförmigem Ammoniak reguliert werden.

Die Inkubationsdauer liegt gewöhnlich in einem Bereich von mehreren Stunden bis zu mehreren Tagen. Diese Zeit wird so ausgewählt, daß sich die maximale Menge Produkt in der Brühe ansammelt. Die offenbarten Wachstumsexperimente können in einer Vielzahl von Behältern, wie Mikrotiterplatten, Glasröhrchen, Glaskolben oder Glas- oder Metallfermentern unterschiedlicher Größen durchgeführt werden. Zum Screening einer großen Anzahl von Klonen sollten die Mikroorganismen in Mikrotiterplatten, Glasröhrchen oder Schüttelkolben, entweder mit oder ohne Schikanen, gezüchtet werden. Vorzugsweise werden 100-ml-Schüttelkolben verwendet, die mit 10% (bezogen auf das Volumen) des erforderlichen Wachstumsmediums gefüllt sind. Die Kolben sollten auf einem Kreiselschüttler (Amplitude 25 mm) mit einer Geschwindigkeit im Bereich von 100-300 U/min geschüttelt werden. Verdampfungsverluste können durch Aufrechterhalten einer feuchten Atmosphäre verringert werden; alternativ sollte für die Verdampfungsverluste eine mathematische Korrektur durchgeführt werden.

Werden genetisch modifizierte Klone untersucht, sollte auch ein unmodifizierter Kontrollklon oder ein Kontrollklon getestet werden, der das Basisplasmid ohne Insertion enthält. Das Medium wird auf eine OD₆₀₀ von 0,5 - 1,5 angeimpft, wobei Zellen verwendet werden, die auf Agarplatten, wie CM-Platten (10 g/l Glucose, 2,5 g/l NaCl, 2 g/l Harnstoff, 10 g/l Polypepton, 5 g/l Hefeextrakt, 5 g/l Fleischextrakt, 22 g/l Agar pH-Wert 6,8 mit 2 M NaOH), die bei 30°C inkubiert worden sind, gezüchtet wurden. Das Animpfen der Medien erfolgt entweder durch Einbringen einer Kochsalzlösung von *C. glutamicum*-Zellen von CM-Platten oder durch Zugabe einer flüssigen Vorkultur dieses Bakteriums.

### Beispiel 8: In-vitro-Analyse der Funktion mutanter Proteine

Die Bestimmung der Aktivitäten und kinetischen Parameter von Enzymen ist im Fachgebiet gut bekannt. Experimente zur Bestimmung der Aktivität eines bestimmten veränderten Enzyms müssen an die spezifische Aktivität des Wildtypenzyms angepaßt werden, was innerhalb der Fähigkeiten des Fachmann liegt. Überblicke über Enzyme im allgemeinen sowie spezifische Einzelheiten, die die Struktur, Kinetiken, Prinzipien, Verfahren, Anwendungen und Beispiele zur Bestimmung vieler Enzymaktivitäten betreffen, können bspw. in den nachstehenden Literaturstellen gefunden werden: Dixon, M., und Webb, E.C: (1979) Enzymes, Longmans, London; Fersht (1985) Enzyme Structure and Mechanism, Freeman, New York; Walsh (1979) Enzymatic Reaction Mechanisms. Freeman, San Francisco; Price, N.C., Stevens, L. (1982) Fundamentals of Enzymology. Oxford Univ. Press: Oxford; Boyer, P.D: Hrsg. (1983) The Enzymes, 3. Aufl., Academic Press, New York; Bisswanger, H. (1994) Enzymkinetik, 2. Aufl. VCH, Weinheim (ISBN 3527300325); Bergmeyer, H.U., Bergmeyer, J., Graßl, M. Hrsg. (1983-1986) Methods of Enzymatic Analysis, 3. Aufl. Bd. I-XII, Verlag Chemie: Weinheim; und Ullmann's Encyclopedia of Industrial Chemistry (1987) Bd. A9, "Enzymes", VCH, Weinheim, S. 352-363.

Die Aktivität von Proteinen, die an DNA binden, kann durch viele gut eingeführte Verfahren gemessen werden, wie DNA-Banden-Shift-Assays (die auch als Gelretardations-Assays bezeichnet werden). Die Wirkung dieser Proteine auf die Expression anderer Moleküle kann mit Reportergen-Assays (wie in Kolmar, H. et al., (1995) EMBO J. 14:3895-3904 und den darin zitierten Literaturstellen beschrieben) gemessen werden. Reportergen-Testsysteme sind wohlbekannt und für Anwendungen in pro- und eukaryotischen Zellen etabliert, wobei Enzyme, wie beta-Galactosidase, Grün-Fluoreszenz-Protein und mehrere andere verwendet werden.

Die Bestimmung der Aktivität von Membran-Transportproteinen kann gemäß Techniken, wie sie in Gennis, R.B. (1989) "Pores, Channels and Transporters", in Biomembranes, Molecular Structure and Function, Springer: Heidelberg, S. 85-137; 199-234; und 270-322 beschrieben sind, erfolgen.

### Beispiel 9: Analyse des Einflusses von mutiertem Protein auf die Produktion des gewünschten Produktes

Die Wirkung der genetischen Modifikation in *C. glutamicum* auf die Produktion einer gewünschten Verbindung (wie einer Aminosäure) kann bestimmt werden, indem die modifizierten Mikroorganismen unter geeigneten Bedingungen (wie den vorstehend beschriebenen) gezüchtet werden und das Medium und/oder die zellulären Komponenten bezüglich der erhöhten Produktion des gewünschten Produktes (d.h. einer Aminosäure) untersucht wird/werden. Solche Analysetechniken sind dem Fachmann wohlbekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (s. bspw. Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A. et al. (1988) Bioseparations: downstream processing for Biotechnology, John Wiley and Sons; Kennedy, J.F. und Cabral, J.M.S. (1992) Recovery processes for biological Materials, John Wiley and Sons; Shaeiwitz, J.A. und Henry, J.D. (1988) Biochemical Separations, in Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Zusätzlich zur Messung des Fermentationsendproduktes ist es ebenfalls möglich, andere Komponenten der Stoffwechselwege zu analysieren, die zur Produktion der gewünschten Verbindung verwendet werden, wie Zwischen- und Nebenprodukte, um die Gesamt-Effizienz der Produktion der Verbindung zu bestimmen. Die Analyseverfahren umfassen Messungen der Nährstoffmengen im Medium (bspw. Zucker, Kohlenwasserstoffe, Stickstoffquellen, Phosphat und andere Ionen), Messungen der Biomassezusammensetzung und des Wachstums, Analyse der Produktion gemeinsamer Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsg. IRL Press, S. 103-129; 131-163 und 165-192 (ISBN: 0199635773) und den darin angegebenen Literaturstellen beschrieben.

### Beispiel 10: Reinigung des gewünschten Produktes aus C. glutamicum-Kultur

Die Gewinnung des gewünschten Produktes aus *C. glutamicum*-Zellen oder aus dem Überstand der vorstehend beschriebenen Kultur kann durch verschiedene, im Fachgebiet bekannte Verfahren erfolgen. Wird das gewünschte Produkt von den Zellen nicht sezerniert, können die Zellen aus der Kultur durch langsame Zentrifugation geerntet werden, die Zellen können durch Standard-Techniken, wie mechanische Kraft oder Ultraschallbehandlung, lysiert werden. Die Zelltrümmer werden durch Zentrifugation entfernt, und die Überstandsfraktion, die die löslichen Proteine enthält, wird zur weiteren Reinigung der gewünschten Verbindung erhalten. Wird das Produkt von den *C. glutamicum*-Zellen sezerniert, werden die Zellen durch langsame Zentrifugation aus der Kultur entfernt, und die Überstandsfraktion wird zur weiteren Reinigung behalten.

Die Überstandsfraktion aus beiden Reinigungsverfahren wird einer Chromatographie mit einem geeigneten Harz unterworfen, wobei das gewünschte Molekül entweder auf dem Chromatographieharz zurückgehalten wird, viele Verunreinigungen in der Probe jedoch nicht, oder die Verunreinigungen auf dem Harz zurückbleiben, die Probe hingegen nicht. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung für ein bestimmtes zu reinigendes Molekül bewandert. Das gereinigte Produkt kann durch Filtration oder Ultrafiltration konzentriert und bei einer Temperatur aufbewahrt werden, bei der die Stabilität des Produktes maximal ist.

Im Fachgebiet sind viele Reinigungsverfahren bekannt, und das vorhergehende Reinigungsverfahren soll nicht einschränkend sein. Diese Reinigungstechniken sind bspw. beschrieben in Bailey, J.E. & Ollis, D.F. Biochemical Engineering Fundamentals, McGraw-Hill: New York (1986).

Die Identität und Reinheit der isolierten Verbindungen kann durch Techniken des Standes der Technik bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

### Äquivalente

Der Fachmann erkennt oder kann - indem er lediglich Routineverfahren verwendet - viele Äquivalente der erfindungsgemäßen spezifischen Ausführungsformen feststellen. Diese Äquivalente sollen von den nachstehenden Patentansprüchen umfaßt sein.

Die Angaben in Tabelle 1 sind folgendermassen zu verstehen:

In Spalte 1"DNA-ID" bezieht sich die jeweilige Zahl auf die SEQ ID NO des anhängenden Sequenzprotokolls. Eine "5" in der Spalte "DNA-ID" bedeutet demzufolge ein Verweis auf SEQ ID NO:5.

In Spalte 2"AS-ID" bezieht sich die jeweilige Zahl auf die SEQ ID NO des anhängenden Sequenzprotokolls. Eine "6" in der Spalte "AS-ID" bedeutet demzufolge ein Verweis auf SEQ ID NO:6.

In Spalte 3"Identifikation" wird eine eindeutige interne Bezeichnung für jede Sequenz aufgeführt.

In Spalte 4 "AS-POS" bezieht sich die jeweilige Zahl auf die Aminosäureposition der Polypeptidsequenz "AS-ID" in der gleichen Zeile. Eine "26" in der Spalte "AS-POS" bedeutet demzufolge die Aminosäureposition 26 der entsprechend angegebenen Polypeptidsequenz. Die Zählung der Position beginnt N-Terminal bei +1.

In Spalte 5 "AS-Wildtyp" bezeichnet der jeweilige Buchstabe die Aminosäure - dargestellt im Ein-Buchstaben-Code- an der in Spalte 4 angegebenen Position beim entsprechenden Wildtyp-Stamm.

In Spalte 6 "AS-Mutante" bezeichnet der jeweilige Buchstabe die Aminosäure - dargestellt im Ein-Buchstaben-Code- an der in Spalte 4 angegebenen Position beim entsprechenden Mutanten-Stamm.

In Spalte 7"Funktion" wird die physiologische Funktion der entsprechenden Polypeptidsequenz aufgeführt.

Ein-Buchstaben-Code der proteinogenen Aminosäuren:
- A: Alanin
- C: cystein
- D: Aspartat
- E: Glutamat
- F: Phenylalanin
- G: Glycin
- H: His
- I: Isoleucin
- K: Lysin
- L: Leucin
- M: Methionin
- N: Asparagin
- P: Prolin
- Q: Glutamin
- R: Arginin
- S: Serin
- T: Threonin
- V: Valin
- W: Tryptophan
- Y: Tyrosin

**Tabelle 1**

| Gene die für genetische Stabilitäts-, Genexpressions- und Faltungsproteine codieren | | | | | | |
|---|---|---|---|---|---|---|
| **DNA ID:** | **AS ID:** | **Identifikation:** | **AS Pos:** | **AS Wildtyp** | **AS Mutante** | **Funktion:** |
| 1 | 2 | RXA00019 | 337 | P | S | SINGLE-STRANDED-DNA-SPECIFIC EX- |
| ONUCLEASE RECJ (EC 3.1.-.-) | | | | | | |
| | | | 405 | T | I | SINGLE-STRANDED-DNA-SPECIFIC EX- |
| ONUCLEASE RECJ (EC 3.1.-.-) | | | | | | |
| | | | 504 | P | S | SINGLE-STRANDED-DNA-SPECIFIC EX- |
| ONUCLEASE RECJ (EC 3.1.-.-) | | | | | | |
| 3 | 4 | RXA00061 | 754 | S | N | DNA POLYMERASE I (EC 2.7.7.7) |
| 5 | 6 | RXA00209 | 414 | V | A | GLUTAMYL-TRNA(GLN) AMIDOTRANSFE- |
| RASE SUBUNIT A (EC 6.3.5.-) | | | | | | |
| | | | 454 | L | F | GLUTAMYL-TRNA(GLN) AMIDOTRANSFE- |
| RASE SUBUNIT A (EC 6.3.5.-) | | | | | | |
| 7 | 8 | RXA00211 | 44 | V | I | GLUTAMYL-TRNA(GLN) AMIDOTRANSFE- |
| RASE SUBUNIT B (EC 6.3.5.-) | | | | | | |
| 9 | 10 | RXA00314 | 319 | E | K | CYSTEINYL-TRNA SYNTHETASE (EC |
| 6.1.1.16) | | | | | | |
| 11 | 12 | RXA00458 | 170 | L | F | GLUTAMYL-TRNA SYNTHETASE (EC |
| 6.1.1.17) | | | | | | |
| 13 | 14 | RXA00493 | 363 | A | T | 60 KD CHAPERONIN GROEL |
| 15 | 16 | RXA00588 | 23 | A | V | TRANSCRIPTION ELONGATION FACTOR |
| GREA | | | | | | |
| 17 | 18 | RXA00669 | 68 | A | T | TRNA PSEUDOURIDINE SYNTHASE A (EC |
| 4.2.1.70) | | | | | | |
| 19 | 20 | RXA01061 | 686 | P | S | LEUCYL-TRNA SYNTHETASE (EC 6.1.1.4) |
| 21 | 22 | RXA01277 | 704 | G | S | PROLYL ENDOPEPTIDASE (EC 3.4.21.26) |
| 23 | 24 | RXA01278 | 543 | T | I | Protein Translation Elongation Factor G (EF-G) |
| 25 | 26 | RXA01284 | 164 | D | N | Bacterial Protein Translation Elongation Factor |
| Tu (EF-TU) | | | | | | |
| | | | 362 | S | F | Bacterial Protein Translation Elongation Factor |
| Tu (EF-TU) | | | | | | |
| 27 | 28 | RXA01344 | 5 | P | L | DNA-DIRECTED RNA POLYMERASE BETA |
| CHAIN (EC 2.7.7.6) | | | | | | |
| | | | 429 | D | V | DNA-DIRECTED RNA POLYMERASE BETA |
| CHAIN (EC 2.7.7.6) | | | | | | |
| 29 | 30 | RXA01345 | 308 | A | V | DNAK PROTEIN |
| 31 | 32 | RXA01404 | 108 | T | I | TRANSCRIPTIONAL REPRESSOR |
| 33 | 34 | RXA01431 | 46 | A | T | THIOREDOXIN |
| 35 | 36 | RXA01438 | 182 | A | T | FERREDOXIN-NADP REDUCTASE (EC |
| 1.18.1.2) | | | | | | |
| 37 | 38 | RXA01490 | 277 | A | V | TRNA PSEUDOURIDINE SYNTHASE B (EC |
| 4.2.1.70) | | | | | | |
| 39 | 40 | RXA01493 | 32 | A | V | Na+ DRIVEN MULTIDRUG EFFLUX PUMP |
| 41 | 42 | RXA01559 | 400 | T | A | PROTEIN TRANSLOCASE SUBUNIT SECD |
| 43 | 44 | RXA01596 | 334 | R | C | DNA REPAIR PROTEIN RECN |
| | | | 493 | G | D | DNA REPAIR PROTEIN RECN |
| 45 | 46 | RXA01651 | 7 | S | F | TRANSPOSASE |
| | | | 33 | L | F | TRANSPOSASE |
| 47 | 48 | RXA01710 | 69 | P | S | TRANSCRIPTIONAL REGULATOR |
| 49 | 50 | RXA01852 | 120 | P | S | HISTIDYL-TRNA SYNTHETASE (EC 6.1.1.21) |
| 51 | 52 | RXA01913 | 61 | L | F | Protein Translation Elongation Factor Ts (EF. |
| Ts) | | | | | | |
| 53 | 54 | RXA02145 | 321 | P | L | MENAQUINOL-CYTOCHROME C REDUCTA- |
| SE CYTOCHROME B SUBUNIT | | | | | | |
| 55 | 56 | RXA02236 | 87 | L | F | integration host factor |
| 57 | 58 | RXA02267 | 65 | A | T | DNA (CYTOSINE-5)-METHYLTRANSFERASE |
| (EC 2.1.1.37) | | | | | | |
| 59 | 60 | RXA02280 | 502 | A | V | HEAT SHOCK PROTEIN HTPG |
| 61 | 62 | RXA02388 | 401 | E | K | COME OPERON PROTEIN 3 |
| | | | 451 | V | M | COME OPERON PROTEIN 3 |
| 63 | 64 | RXA02416 | 484 | G | D | EXCINUCLEASE ABC SUBUNIT A |
| 65 | 66 | RXA02418 | 45 | V | I | Bacterial Protein Translation Initiation Faktor 3 ( |
| IF-3) | | | | | | |
| 67 | 68 | RXA02429 | 670 | M | I | PROTEIN TRANSLOCASE SUBUNIT SECA |
| 69 | 70 | RXA02431 | 73 | A | V | DNA POLYMERASE IV |
| 71 | 72 | RXA02445 | 17 | G | E | ATP-DEPENDENT DNA HELICASE RECG |
| 73 | 74 | RXA02476 | 167 | S | F | A/G-SPECIFIC ADENINE GLYCOSYLASE (EC |
| 3.2.2.-) | | | | | | |
| 75 | 76 | RXA02726 | 286 | A | V | ISOLEUCYL-TRNA SYNTHETASE (EC |
| 6.1.1.5) | | | | | | |
| 77 | 78 | RXA02731 | 374 | E | K | EXCINUCLEASE ABC SUBUNIT B |
| | | | 398 | M | L | EXCINUCLEASE ABC SUBUNIT B |
| | | | 410 | R | L | EXCINUCLEASE ABC SUBUNIT B |
| 79 | 80 | RXA02736 | 312 | S | F | PUTATIVE OXPPCYCLE PROTEIN OPCA |
| 81 | 82 | RXA02742 | 179 | G | S | DNA/RNA HELICASE (DEADIDEAH BOX |
| FAMILY) | | | | | | |
| 83 | 84 | RXA02748 | 100 | P | S | SIGNAL RECOGNITION PARTICLE, SUBUNIT |
| FFH/SRP54 | | | | | | |
| | | | 164 | G | D | SIGNAL RECOGNITION PARTICLE, SUBUNIT |
| FFH/SRP54 | | | | | | |
| 85 | 86 | RXA03070 | 249 | A | V | TRANSPOSASE |
| 87 | 88 | RXA03098 | 164 | S | N | DNA alkylation repair enzyme |
| 89 | 90 | RXA03206 | 98 | G | D | D-Tyr-tRNATyr deacylase |
| 91 | 92 | RXA03260 | 56 | S | F | TRANSPOSASE |
| 93 | 94 | RXA03394 | 11 | S | F | METHIONYL-TRNA SYNTHETASE (EC |
| 6.1.1.10) | | | | | | |
| 95 | 96 | RXA03674 | 342 | V | I | ATP-DEPENDENT HELICASE HEPA |
| 97 | 98 | RXA03793 | 414 | A | V | RNA POLYMERASE SIGMA FACTOR RPOD |
| 99 | 100 | RXA06048 | 3 | L | F | PS1 PROTEIN PRECURSOR |
| | | | 4 | L | P | PS1 PROTEIN PRECURSOR |
| | | | 5 | T | S | PS1 PROTEIN PRECURSOR |
| | | | 9 | A | T | PS1 PROTEIN PRECURSOR |
| | | | 26 | I | V | PS1 PROTEIN PRECURSOR |
| | | | 31 | S | T | PS1 PROTEIN PRECURSOR |
| | | | 66 | S | N | PS1 PROTEIN PRECURSOR |
| | | | 158 | N | D | PS1 PROTEIN PRECURSOR |
| 101 | 102 | RXA07005 | 339 | P | S | PROBABLE ATP-DEPENDENT HELICASE |
| LHR (EC 3.6.1.-) | | | | | | |
| 103 | 104 | RXA07006 | 239 | P | L | EXODEOXYRIBONUCLEASE VII LARGE |
| SUBUNIT (EC 3.1.11.6) | | | | | | |

## Patentansprüche

1. Isoliertes Nukleinsäuremolekül codierend für ein OpcA Polypeptid mit der in SEQ ID NO: 80 dargestellten Aminosäuresequenz, wobei das Nukleinsäuremolekül in der Aminosäureposition 312 anstelle Serin für die Aminosäure Phenylalanin codiert.

2. Ein Vektor, der wenigstens eine Nukleinsäuresequenz nach Anspruch 1 enthält.

3. Eine isolierte Wirtszelle, die mit wenigstens einem Vektor nach Anspruch 2 transfiziert ist.

4. Eine isolierte Wirtszelle nach Anspruch 3, wobei die Expression des besagten Nukleinsäuremoleküls zur Modulation der Produktion einer Feinchemikalie aus besagter Zelle führt.

5. Verfahren zur Herstellung einer Feinchemikalie welches die Kultivierung einer isolierten Zelle beinhaltet, die mit wenigstens einen Vektor nach Anspruch 2 transfiziert worden ist, so dass die Feinchemikalie produziert wird.

6. Verfahren nach Anspruch 5, **dadurch gekennzeichnet, dass** die Feinchemikalie eine Aminosäure ist.

7. Verfahren nach Anspruch 6, **dadurch gekennzeichnet, dass** die Aminosäure Lysin ist.

## Claims

1. Isolated nucleic acid molecule coding for an OpcA polypeptide with the amino acid sequence depicted in SEQ ID NO: 80, wherein the nucleic acid molecule codes for a serine instead of the amino acid phenylalanine at amino acid position 312.

2. A vector comprising at least a nucleic acid sequence according to claim 1.

3. An isolated host cell transfected with at least one vector according to claim 2.

4. An isolated host cell according to claim 3, wherein the expression of said nucleic acid molecule leads to the modulation of the production of a fine chemical from said cell.

5. Method of producing a fine chemical comprising the cultivation of an isolated cell which has been transfected with at least one vector according to claim 2 such that the fine chemical is produced.

6. Method according to claim 5, **characterised in that** the fine chemical is an amino acid.

7. Method according to claim 6, **characterised in that** the amino acid is lysine.

## Revendications

1. Molécule d'acide nucléique isolée codant pour un polypeptide OpcA avec la séquence d'acides aminés SEQ ID N° 80, la molécule d'acide nucléique codant, au niveau de la position d'acide aminé 312, pour la sérine au lieu de l'acide aminé phénylalanine.

2. Vecteur contenant au moins une séquence d'acides nucléiques selon la revendication 1.

3. Cellule hôte isolée transfectée avec au moins un vecteur selon la revendication 2.

4. Cellule hôte isolée selon la revendication 3, l'expression de ladite molécule d'acide nucléique permettant la modulation de la production d'une substance chimique noble par ladite cellule.

5. Procédé de production d'une substance chimique noble qui comprend la culture d'une cellule isolée transfectée avec au moins un vecteur selon la revendication 2 de façon à ce que la substance chimique noble soit produite.

6. Procédé selon la revendication 5, **caractérisé en ce que** la substance chimique noble est un acide aminé.

7. Procédé selon la revendication 6, **caractérisé en ce que** l'acide aminé est la lysine.


### SEQUENCE LISTING

<110> BASF Aktiengesellschaft
<120> Gene die für genetische Stabilitäts-, Genexpressions- und Faltungsproteine codieren
<130> O.Z. 0050/52974-3
<160> 104
<210> 1
   <211> 1990
   <212> DNA
   <213> Corynebacterium glutamicum
<220>
   <221> CDS
   <222> (101)..(1960)
   <223> RXA00019
<400> 1
   accggatacc ttatgaaaca cctggtgagc ggtgtgtttc accccaacaa ccgagtaaaa 60
   tatatctagt actattttac gattgaaagt agatttttct atg acc gtt acc tca   115
                                               Met Thr Val Thr Ser
                                               1               5
   cca gca gcg ctc gca ctc agc gac atg tcc tat gtg gac atc att aag   163
   Pro Ala Ala Leu Ala Leu Ser Asp Met Ser Tyr Val Asp Ile Ile Lys
                   10                  15                  20
   aag aag cgc gga tgg aca acc gag ttt ttc cac agc acc atc aac acc   211
   Lys Lys Arg Gly Trp Thr Thr Glu Phe Phe His Ser Thr Ile Asn Thr
               25                  30                  35
   ggt gaa acc acc aca ccg cta cca gac agc gac cgt gcc aca gca cta   259
   Gly Glu Thr Thr Thr Pro Leu Pro Asp Ser Asp Arg Ala Thr Ala Leu
           40                  45                  50
   atc cat gac cac atc acc aag gct caa gag ata acc atc atc acc gac   307
   Ile His Asp His Ile Thr Lys Ala Gln Glu Ile Thr Ile Ile Thr Asp
       55                  60                  65
   ttt gat atg gac ggt att tca gcc ggt gtc att gcc tat gca ggt ctt   355
   Phe Asp Met Asp Gly Ile Ser Ala Gly Val Ile Ala Tyr Ala Gly Leu
   70                  75                  80                  85
   gcc gaa ctg ggc gca cag gtc aat atg gtg gtg ccc gac tat cgt ggc   403
   Ala Glu Leu Gly Ala Gln Val Asn Met Val Val Pro Asp Tyr Arg Gly
                   90                  95                  100
   gaa cga aat gtc aca gcc agc gat att gat cgt gcg cta gag ctc tac   451
   Glu Arg Asn Val Thr Ala Ser Asp Ile Asp Arg Ala Leu Glu Leu Tyr
               105                 110                 115
   cct gca acc tca ctc atc atc acc tgc gat gtc ggc atc ggc tcc cat   499
   Pro Ala Thr Ser Leu Ile Ile Thr Cys Asp Val Gly Ile Gly Ser His
           120                 125                 130
   gaa ggt att gcc cgt gct cac gaa cgc agt atc gcc gtc ctg gtc aca    547
   Glu Gly Ile Ala Arg Ala His Glu Arg Ser Ile Ala Val Leu Val Thr
       135                 140                 145
   gat cac cac atg gag gtc gaa ccc tgc cag gcc gat gtg gtt ctt aac    595
   Asp His His Met Glu Val Glu Pro Cys Gln Ala Asp Val Val Leu Asn
   150                 155                 160                 165
   ccc aac aga att gac tct gac tac ccc aac aaa gat att tgc ggt gcg    643
   Pro Asn Arg Ile Asp Ser Asp Tyr Pro Asn Lys Asp Ile Cys Gly Ala
                   170                 175                 180
   cag gtc att ttc gcc aca ttg agt gac tat gca cgt cgt tat cgg gcg    691
   Gln Val Ile Phe Ala Thr Leu Ser Asp Tyr Ala Arg Arg Tyr Arg Ala
               185                 190                 195
   gac aag att atc gac att aat ttg ttg gct gtt ttc tca ggc att ggt    739
   Asp Lys Ile Ile Asp Ile Asn Leu Leu Ala Val Phe Ser Gly Ile Gly
           200                 205                 210
   gca ctc gcc gat gtc atg cct ctc acc cgt gac act cga cca aca gtg    787
   Ala Leu Ala Asp Val Met Pro Leu Thr Arg Asp Thr Arg Pro Thr Val
       215                 220                 225
   aag cag gct att gcg ttg ctt cgg ctt gct atc cca caa gta agt aaa    835
   Lys Gln Ala Ile Ala Leu Leu Arg Leu Ala Ile Pro Gln Val Ser Lys
   230                 235                 240                 245
   aac cgt ttc ggc ggt tgg gat acc tat gct gca cgc tct gtt aat cct    883
   Asn Arg Phe Gly Gly Trp Asp Thr Tyr Ala Ala Arg Ser Val Asn Pro
                   250                 255                 260
   gat acg tcc aca ctc atg cat att gtc aat gcc agc cag cat gat cac    931
   Asp Thr Ser Thr Leu Met His Ile Val Asn Ala Ser Gln His Asp His
               265                 270                 275
   cgc ttc att gca gcc ttc caa ggc atc tca att ctt ctt ggt gaa ctg    979
   Arg Phe Ile Ala Ala Phe Gln Gly Ile Ser Ile Leu Leu Gly Glu Leu
           280                 285                 290
   att gcg caa aag aag cta gta aac atc gac aat att tct gag tca ttc    1027
   Ile Ala Gln Lys Lys Leu Val Asn Ile Asp Asn Ile Ser Glu Ser Phe
       295                 300                 305
   att ggc ttc act ctt ggt ccg atg ttt aac gct act cgt cgt gtt ggt    1075
   Ile Gly Phe Thr Leu Gly Pro Met Phe Asn Ala Thr Arg Arg Val Gly
   310                 315                 320                 325
   ggc gac atg cac gat tca ttt ctc gtg ttt gcg ccc cat gcc gca cta    1123
   Gly Asp Met His Asp Ser Phe Leu Val Phe Ala Pro His Ala Ala Leu
                   330                 335                 340
   gca tca cag ccg tcg atg aat cca aat cga cat gct gcg atc tct cgc    1171
   Ala Ser Gln Pro Ser Met Asn Pro Asn Arg His Ala Ala Ile Ser Arg
               345                 350                 355
   atc att gat aac aac gaa cgt cgc aaa gag ctc tcc aag tcc tct tat    1219
   Ile Ile Asp Asn Asn Glu Arg Arg Lys Glu Leu Ser Lys Ser Ser Tyr
           360                 365                 370
   gct gcc gta cac agc tca gat cag ccc tac gcg ccc ttt gtg tgg ctc    1267
   Ala Ala Val His Ser Ser Asp Gln Pro Tyr Ala Pro Phe Val Trp Leu
       375                 380                 385
   tct gag gca cca agc ggc att ctt ggt ctc att gcc tca cag ctc act     1315
   Ser Glu Ala Pro Ser Gly Ile Leu Gly Leu Ile Ala Ser Gln Leu Thr
   390                 395                 400                 405
   cgt gag tct gac gtg cct gcc att gtc att aat cca gat acc ttg tcc     1363
   Arg Glu Ser Asp Val Pro Ala Ile Val Ile Asn Pro Asp Thr Leu Ser
                   410                 415                 420
   ggt tca gct cgc tca cct gag tgg gca ccg atc atc acc caa gta aac     1411
   Gly Ser Ala Arg Ser Pro Glu Trp Ala Pro Ile Ile Thr Gln Val Asn
               425                 430                 435
   acc ctc agc gca caa ggt cac ggc ggt att cat gct gca ggc cat gag     1459
   Thr Leu Ser Ala Gln Gly His Gly Gly Ile His Ala Ala Gly His Glu
           440                 445                 450
   tac gcc tgt ggt atg cgt ttt gat aac cat gat gac att gtg acc ttt     1507
   Tyr Ala Cys Gly Met Arg Phe Asp Asn His Asp Asp Ile Val Thr Phe
       455                 460                 465
   gtt gca aca ctc gac gca ctc gat aaa aac acg cca cgg gaa gca cag     1555
   Val Ala Thr Leu Asp Ala Leu Asp Lys Asn Thr Pro Arg Glu Ala Gln
   470                 475                 480                 485
   ccg gca gat ctg cat ttg gtt gac att gac cac gcg cgt cct gtg ctt     1603
   Pro Ala Asp Leu His Leu Val Asp Ile Asp His Ala Arg Pro Val Leu
                   490                 495                 500
   gat aac ccc tca ctc acc caa gag ctc agt acg gtc gat gct gca gtg     1651
   Asp Asn Pro Ser Leu Thr Gln Glu Leu Ser Thr Val Asp Ala Ala Val
               505                 510                 515
   gat gct gca cag ttg ctt gtt ctc att gat cag ctt gat caa ctg cag     1699
   Asp Ala Ala Gln Leu Leu Val Leu Ile Asp Gln Leu Asp Gln Leu Gln
           520                 525                 530
   cca ttt gga cat ggt ttt acc tat ccg cgc atc gac gtg acg ttc agg     1747
   Pro Phe Gly His Gly Phe Thr Tyr Pro Arg Ile Asp Val Thr Phe Arg
       535                 540                 545
   ccg gca gaa aca gaa ttc aag gtt atg ggt cag cac cat caa cat ctc     1795
   Pro Ala Glu Thr Glu Phe Lys Val Met Gly Gln His His Gln His Leu
   550                 555                 560                 565
   aag gtg atc act cac tca ggg ttg acc tta ttg tgg tgg aat aag gct     1843
   Lys Val Ile Thr His Ser Gly Leu Thr Leu Leu Trp Trp Asn Lys Ala
                   570                 575                 580
   cag cag ctc gat gag atc gca cag tct gaa tta gtc acc atg tct gtg     1891
   Gln Gln Leu Asp Glu Ile Ala Gln Ser Glu Leu Val Thr Met Ser Val
               585                 590                 595
   gag ctc gat gtc aat atg ttc cgt ggg ttt att tcc ccg caa ggc att     1939
   Glu Leu Asp Val Asn Met Phe Arg Gly Phe Ile Ser Pro Gln Gly Ile
           600                 605                 610
   gtc tct gcg tgc aca gtt atc tagcttggtt gcataagcac caaaaacaac        1990
   Val Ser Ala Cys Thr Val Ile
       615                 620
<210> 2
   <211> 620
   <212> PRT
   <213> Corynebacterium glutamicum
<400> 2
   Met Thr Val Thr Ser Pro Ala Ala Leu Ala Leu Ser Asp Met Ser Tyr
   1 5 10 15
   Val Asp Ile Ile Lys Lys Lys Arg Gly Trp Thr Thr Glu Phe Phe His
   20 25 30
   Ser Thr Ile Asn Thr Gly Glu Thr Thr Thr Pro Leu Pro Asp Ser Asp
   35 40 45
   Arg Ala Thr Ala Leu Ile His Asp His Ile Thr Lys Ala Gln Glu Ile
   50 55 60
   Thr Ile Ile Thr Asp Phe Asp Met Asp Gly Ile Ser Ala Gly Val Ile
   65 70 75 80
   Ala Tyr Ala Gly Leu Ala Glu Leu Gly Ala Gln Val Asn Met Val Val
   85 90 95
   Pro Asp Tyr Arg Gly Glu Arg Asn Val Thr Ala Ser Asp Ile Asp Arg
   100 105 110
   Ala Leu Glu Leu Tyr Pro Ala Thr Ser Leu Ile Ile Thr Cys Asp Val
   115 120 125
   Gly Ile Gly Ser His Glu Gly Ile Ala Arg Ala His Glu Arg Ser Ile
   130 135 140
   Ala Val Leu Val Thr Asp His His Met Glu Val Glu Pro Cys Gln Ala
   145 150 155 160
   Asp Val Val Leu Asn Pro Asn Arg Ile Asp Ser Asp Tyr Pro Asn Lys
   165 170 175
   Asp Ile Cys Gly Ala Gln Val Ile Phe Ala Thr Leu Ser Asp Tyr Ala
   180 185 190
   Arg Arg Tyr Arg Ala Asp Lys Ile Ile Asp Ile Asn Leu Leu Ala Val
   195 200 205
   Phe Ser Gly Ile Gly Ala Leu Ala Asp Val Met Pro Leu Thr Arg Asp
   210 215 220
   Thr Arg Pro Thr Val Lys Gln Ala Ile Ala Leu Leu Arg Leu Ala Ile
   225 230 235 240
   Pro Gln Val Ser Lys Asn Arg Phe Gly Gly Trp Asp Thr Tyr Ala Ala
   245 250 255
   Arg Ser Val Asn Pro Asp Thr Ser Thr Leu Met His Ile Val Asn Ala
   260 265 270
   Ser Gln His Asp His Arg Phe Ile Ala Ala Phe Gln Gly Ile Ser Ile
   275 280 285
   Leu Leu Gly Glu Leu Ile Ala Gln Lys Lys Leu Val Asn Ile Asp Asn
   290 295 300
   Ile Ser Glu Ser Phe Ile Gly Phe Thr Leu Gly Pro Met Phe Asn Ala
   305 310 315 320
   Thr Arg Arg Val Gly Gly Asp Met His Asp Ser Phe Leu Val Phe Ala
   325 330 335
   Pro His Ala Ala Leu Ala Ser Gln Pro Ser Met Asn Pro Asn Arg His
   340 345 350
   Ala Ala Ile Ser Arg Ile Ile Asp Asn Asn Glu Arg Arg Lys Glu Leu
   355 360 365
   Ser Lys Ser Ser Tyr Ala Ala Val His Ser Ser Asp Gln Pro Tyr Ala
   370 375 380
   Pro Phe Val Trp Leu Ser Glu Ala Pro Ser Gly Ile Leu Gly Leu Ile
   385 390 395 400
   Ala Ser Gln Leu Thr Arg Glu Ser Asp Val Pro Ala Ile Val Ile Asn
   405 410 415
   Pro Asp Thr Leu Ser Gly Ser Ala Arg Ser Pro Glu Trp Ala Pro Ile
   420 425 430
   Ile Thr Gln Val Asn Thr Leu Ser Ala Gln Gly His Gly Gly Ile His
   435 440 445
   Ala Ala Gly His Glu Tyr Ala Cys Gly Met Arg Phe Asp Asn His Asp
   450 455 460
   Asp Ile Val Thr Phe Val Ala Thr Leu Asp Ala Leu Asp Lys Asn Thr
   465 470 475 480
   Pro Arg Glu Ala Gln Pro Ala Asp Leu His Leu Val Asp Ile Asp His
   485 490 495
   Ala Arg Pro Val Leu Asp Asn Pro Ser Leu Thr Gln Glu Leu Ser Thr
   500 505 510
   Val Asp Ala Ala Val Asp Ala Ala Gln Leu Leu Val Leu Ile Asp Gln
   515 520 525
   Leu Asp Gln Leu Gln Pro Phe Gly His Gly Phe Thr Tyr Pro Arg Ile
   530 535 540
   Asp Val Thr Phe Arg Pro Ala Glu Thr Glu Phe Lys Val Met Gly Gln
   545 550 555 560
   His His Gln His Leu Lys Val Ile Thr His Ser Gly Leu Thr Leu Leu
   565 570 575
   Trp Trp Asn Lys Ala Gln Gln Leu Asp Glu Ile Ala Gln Ser Glu Leu
   580 585 590
   Val Thr Met Ser Val Glu Leu Asp Val Asn Met Phe Arg Gly Phe Ile
   595 600 605
   Ser Pro Gln Gly Ile Val Ser Ala Cys Thr Val Ile
   610 615 620
<210> 3
   <211> 2845
   <212> DNA
   <213> Corynebacterium glutamicum
<220>
   <221> CDS
   <222> (101)..(2815)
   <223> RXA00061
<400> 3
   aatcaattgc agaactaacc cggttgtttc cgagccagtc tgaatgactg aaagcaatat 60
   tagaccatca atgattagga atggaaatta ggggtctggt ttg ggt gaa tgt gtc    115
                                               Leu Gly Glu Cys Val
                                                1               5
   gct aat ttt tcc act cgc cta cac tcg gga ggc gtg act gag aag act    163
   Ala Asn Phe Ser Thr Arg Leu His Ser Gly Gly Val Thr Glu Lys Thr
                   10                  15                  20
   gac cag acc tta atg ctt atc gac ggc cac tcg atg gct ttc cgc gca    211
   Asp Gln Thr Leu Met Leu Ile Asp Gly His Ser Met Ala Phe Arg Ala
               25                  30                  35
   ttc ttt gct ttg ccg gct gag aat ttc tcc acg tcg ggc ggg cag gcc    259
   Phe Phe Ala Leu Pro Ala Glu Asn Phe Ser Thr Ser Gly Gly Gln Ala
           40                  45                  50
   acc aat gct gtc tat ggc ttt ctc tcg atg ctg tcc acg ttg ttg aag    307
   Thr Asn Ala Val Tyr Gly Phe Leu Ser Met Leu Ser Thr Leu Leu Lys
       55                  60                  65
   gat gag cag cct act cat gtg gcg gtg gct ttc gat gtg ggg cgt aag    355
   Asp Glu Gln Pro Thr His Val Ala Val Ala Phe Asp Val Gly Arg Lys
   70                  75                  80                  85
   acg ttc cgt acc gat atg ttc ccg gcg tat aag gcg cag cgt gaa gca    403
   Thr Phe Arg Thr Asp Met Phe Pro Ala Tyr Lys Ala Gln Arg Glu Ala
                   90                  95                 100
   acg cca cct gag ttt aag ggc cag gtg gaa atc ctc aag gag gtg ttg    451
   Thr Pro Pro Glu Phe Lys Gly Gln Val Glu Ile Leu Lys Glu Val Leu
               105                 110                 115
   tcc act ttg gga att acg act att gag aaa atc gat ttt gag gct gat    499
   Ser Thr Leu Gly Ile Thr Thr Ile Glu Lys Ile Asp Phe Glu Ala Asp
           120                 125                 130
   gat gtg atc gcc acg ttg tct gtg gcg gcg aaa cct tta ggc ttt aag    547
   Asp Val Ile Ala Thr Leu Ser Val Ala Ala Lys Pro Leu Gly Phe Lys
       135                 140                 145
   acg ctg att gtt acg ggt gac cgt gat tcc ttc cag ttg gtc aat gac    595
   Thr Leu Ile Val Thr Gly Asp Arg Asp Ser Phe Gln Leu Val Asn Asp
   150                 155                 160                 165
   acc acc acg gtg ttg tat ccg atg aag ggc gtg tct gtg ctg cac cgt    643
   Thr Thr Thr Val Leu Tyr Pro Met Lys Gly Val Ser Val Leu His Arg
                   170                 175                 180
   ttc acg ccg gaa gca gtg gag gag aag tat gga ctg aca ccg agg cag    691
   Phe Thr Pro Glu Ala Val Glu Glu Lys Tyr Gly Leu Thr Pro Arg Gln
               185                 190                 195
   tat ccg gag ttt gca gcg ctg cgt ggt gat cct tcc gat aac ttg cct    739
   Tyr Pro Glu Phe Ala Ala Leu Arg Gly Asp Pro Ser Asp Asn Leu Pro
           200                 205                 210
   aat att cct ggc gtg ggc gag aag act gct acc aag tgg att gcc cag      787
   Asn Ile Pro Gly Val Gly Glu Lys Thr Ala Thr Lys Trp Ile Ala Gln
       215                 220                 225
   tat gaa act ttg gat aat ttg ctt gat cac gct gat gag atc aag ggc      835
   Tyr Glu Thr Leu Asp Asn Leu Leu Asp His Ala Asp Glu Ile Lys Gly
   230                 235                 240                 245
   aag gtt ggc gcc agc ctg cgt gag cgc att gag cag gtc cgg atg aac      883
   Lys Val Gly Ala Ser Leu Arg Glu Arg Ile Glu Gln Val Arg Met Asn
                   250                 255                 260
   cgc aag ctc acg gag atg gtg aag gat ctg gag ctg ccg ctt ggt ccg      931
   Arg Lys Leu Thr Glu Met Val Lys Asp Leu Glu Leu Pro Leu Gly Pro
               265                 270                 275
   gac gat ttt gag atg aag cct gtg cag gtt gcg gag gtt gcg gcg aag      979
   Asp Asp Phe Glu Met Lys Pro Val Gln Val Ala Glu Val Ala Ala Lys
           280                 285                 290
   ttt gac gat ctg gag ttt ggt acc aat ttg cgt gag cgg gtg ctg gcg      1027
   Phe Asp Asp Leu Glu Phe Gly Thr Asn Leu Arg Glu Arg Val Leu Ala
       295                 300                 305
   gtg gtg aag gcc gag ggt tcc gct gcc ccc gtg gag gaa gtg gaa gcg      1075
   Val Val Lys Ala Glu Gly Ser Ala Ala Pro Val Glu Glu Val Glu Ala
   310                 315                 320                 325
   gaa cag gtt gtc gtc gat acg caa tct ttg gcg caa tgg ctg cct gct      1123
   Glu Gln Val Val Val Asp Thr Gln Ser Leu Ala Gln Trp Leu Pro Ala
                   330                 335                 340
   agg gct ggc cag gcg ctt gct tta gcg ctg gct gga gtg gct aaa cct      1171
   Arg Ala Gly Gln Ala Leu Ala Leu Ala Leu Ala Gly Val Ala Lys Pro
               345                 350                 355
   gct gct ggc gac acg tat gcg cta gcg att gcg gat acc aag cgc cat      1219
   Ala Ala Gly Asp Thr Tyr Ala Leu Ala Ile Ala Asp Thr Lys Arg His
           360                 365                 370
   gcg gtg ttg gtt gat gtg gct gat att tca gcg gag gat gaa aag gcg      1267
   Ala Val Leu Val Asp Val Ala Asp Ile Ser Ala Glu Asp Glu Lys Ala
       375                 380                 385
   ctg gcc acg tgg ttg gcg tcg gaa gat cca aag atg ctg cac ggc gct      1315
   Leu Ala Thr Trp Leu Ala Ser Glu Asp Pro Lys Met Leu His Gly Ala
   390                 395                 400                 405
   aag gcc gcc tat cat atg ctc gct ggg cgc ggt ttt gag ctg cac ggc      1363
   Lys Ala Ala Tyr His Met Leu Ala Gly Arg Gly Phe Glu Leu His Gly
                   410                 415                 420
   gtg gtg cat gac acg gcg atc gcg gca tac ttg ctg cgt ccg ggc caa      1411
   Val Val His Asp Thr Ala Ile Ala Ala Tyr Leu Leu Arg Pro Gly Gln
               425                 430                 435
   cgc acc tat gag ctt gcc gac gtc tac cag cgg cat ctt caa cga cag      1459
   Arg Thr Tyr Glu Leu Ala Asp Val Tyr Gln Arg His Leu Gln Arg Gln
           440                 445                 450
   ttg tct aca aac gac aat ggc ggc cag ctc acg ctg ctc gac gca gct      1507
   Leu Ser Thr Asn Asp Asn Gly Gly Gln Leu Thr Leu Leu Asp Ala Ala
       455                 460                 465
   gat gac caa tcg ctt gtt gat gat gtc att gca atc ctt gag ctg tct    1555
   Asp Asp Gln Ser Leu Val Asp Asp Val Ile Ala Ile Leu Glu Leu Ser
   470                 475                 480                 485
   gaa gaa ttg acc aaa cag ctt cag gag att caa gct ttt gag ctt tac    1603
   Glu Glu Leu Thr Lys Gln Leu Gln Glu Ile Gln Ala Phe Glu Leu Tyr
                   490                 495                 500
   cat gac ctg gaa att ccg ctg tcg gga att ctg gcg cgc atg gag gcc    1651
   His Asp Leu Glu Ile Pro Leu Ser Gly Ile Leu Ala Arg Met Glu Ala
               505                 510                 515
   atc ggt atc gct gtt gat gtt gcc act ttg gaa gag cag ttg aag act    1699
   Ile Gly Ile Ala Val Asp Val Ala Thr Leu Glu Glu Gln Leu Lys Thr
           520                 525                 530
   ttc att ggt cag gtt gct cag gaa gag gaa gca gct cgc gag ctc gct    1747
   Phe Ile Gly Gln Val Ala Gln Glu Glu Glu Ala Ala Arg Glu Leu Ala
       535                 540                 545
   gag gat cca acc ctg aat ctc tcg agc ccg aag cag ctg caa gtg gtg    1795
   Glu Asp Pro Thr Leu Asn Leu Ser Ser Pro Lys Gln Leu Gln Val Val
   550                 555                 560                 565
   ctt ttt gag acg ttc gga atg ccg aaa acc aag aaa acc aag acc ggc    1843
   Leu Phe Glu Thr Phe Gly Met Pro Lys Thr Lys Lys Thr Lys Thr Gly
                   570                 575                 580
   tac tct acg gct gcc gcg gaa att gaa gcc cta gcg atc aag aat ccg    1891
   Tyr Ser Thr Ala Ala Ala Glu Ile Glu Ala Leu Ala Ile Lys Asn Pro
               585                 590                 595
   cac cca ttc cta gat cac ctg ttg gca cac cgt cag tac caa aag atg    1939
   His Pro Phe Leu Asp His Leu Leu Ala His Arg Gln Tyr Gln Lys Met
           600                 605                 610
   aag acc act ctg gaa ggt ctc atc cgt gag gtg gct cct gat ggc cgt    1987
   Lys Thr Thr Leu Glu Gly Leu Ile Arg Glu Val Ala Pro Asp Gly Arg
       615                 620                 625
   att cac acc acc ttc aac cag acg gtg gcg tct acg gga cgt ttg tca    2035
   Ile His Thr Thr Phe Asn Gln Thr Val Ala Ser Thr Gly Arg Leu Ser
   630                 635                 640                 645
   tcc act gat ccc aac ctg caa aac att cct gtg cgc act gag gct ggc    2083
   Ser Thr Asp Pro Asn Leu Gln Asn Ile Pro Val Arg Thr Glu Ala Gly
                   650                 655                 660
   cga aag att cgt tcg gga ttc gtc gta ggc gag ggg tat gaa acc ttg    2131
   Arg Lys Ile Arg Ser Gly Phe Val Val Gly Glu Gly Tyr Glu Thr Leu
               665                 670                 675
   ctg act gcc gac tat tcg cag att gaa atg cgc gtg atg gct cac ctt    2179
   Leu Thr Ala Asp Tyr Ser Gln Ile Glu Met Arg Val Met Ala His Leu
           680                 685                 690
   tcc cag gac cca ggc ttg att gag gcg tac cgc gaa ggc gaa gac ctg    2227
   Ser Gln Asp Pro Gly Leu Ile Glu Ala Tyr Arg Glu Gly Glu Asp Leu
       695                 700                 705
   cac aat tac gtg ggt tcc aag gtg ttt aat gtg ccc atc gat ggc gtg    2275
   His Asn Tyr Val Gly Ser Lys Val Phe Asn Val Pro Ile Asp Gly Val
   710                 715                 720                 725
   acc cct gag ctg cgt cgc cag gtc aag gcc atg tct tac ggt ctg gtg     2323
   Thr Pro Glu Leu Arg Arg Gln Val Lys Ala Met Ser Tyr Gly Leu Val
                   730                 735                 740
   tac ggc ttg tcc gcg ttt ggt ttg tct cag cag ctg agc att cct gct     2371
   Tyr Gly Leu Ser Ala Phe Gly Leu Ser Gln Gln Leu Ser Ile Pro Ala
               745                 750                 755
   ggc gaa gcg aag cag atc atg gag tcc tac ttc gag cgc ttc ggc gga     2419
   Gly Glu Ala Lys Gln Ile Met Glu Ser Tyr Phe Glu Arg Phe Gly Gly
           760                 765                 770
   gta cag cgc tac ctc cgg gag atc gtg gag gag gct cga aaa gct ggc     2467
   Val Gln Arg Tyr Leu Arg Glu Ile Val Glu Glu Ala Arg Lys Ala Gly
       775                 780                 785
   tac acg gaa acg ctg ttt ggg cgt cgt cgc tac ctg ccg gaa ctg acc     2515
   Tyr Thr Glu Thr Leu Phe Gly Arg Arg Arg Tyr Leu Pro Glu Leu Thr
   790                 795                 800                 805
   tcg gat aac cgt gtc gct cgt gaa aac gct gaa cgt gcc gca ctg aac     2563
   Ser Asp Asn Arg Val Ala Arg Glu Asn Ala Glu Arg Ala Ala Leu Asn
                   810                 815                 820
   gcc ccg att cag gga act gcc gca gac atc atc aag gtg gcc atg atc     2611
   Ala Pro Ile Gln Gly Thr Ala Ala Asp Ile Ile Lys Val Ala Met Ile
               825                 830                 835
   cgg gtg gac cgt tca ctc aag gaa gct gcc gtg aaa tct cgc gtg ctg     2659
   Arg Val Asp Arg Ser Leu Lys Glu Ala Ala Val Lys Ser Arg Val Leu
           840                 845                 850
   ctt cag gtg cat gat gaa ttg gtc gtg gaa gta gcg gcc ggt gag ttg     2707
   Leu Gln Val His Asp Glu Leu Val Val Glu Val Ala Ala Gly Glu Leu
       855                 860                 865
   gaa caa gtc cgt gag att ctg gaa cgc gaa atg gat aac gcc atc aag     2755
   Glu Gln Val Arg Glu Ile Leu Glu Arg Glu Met Asp Asn Ala Ile Lys
   870                 875                 880                 885
   ctg tcc gtt cct ttg gaa gtt tca gct ggt gat ggc gtt aac tgg gat     2803
   Leu Ser Val Pro Leu Glu Val Ser Ala Gly Asp Gly Val Asn Trp Asp
                   890                 895                 900
   gct gca gcg cac taagaggtaa ctgccttttc gtcgacgagc                   2845
   Ala Ala Ala His
               905
<210> 4
   <211> 905
   <212> PRT
   <213> Corynebacterium glutamicum
<400> 4
   Leu Gly Glu Cys Val Ala Asn Phe Ser Thr Arg Leu His Ser Gly Gly
   1 5 10 15
   Val Thr Glu Lys Thr Asp Gln Thr Leu Met Leu Ile Asp Gly His Ser
   20 25 30
   Met Ala Phe Arg Ala Phe Phe Ala Leu Pro Ala Glu Asn Phe Ser Thr
   35 40 45
   Ser Gly Gly Gln Ala Thr Asn Ala Val Tyr Gly Phe Leu Ser Met Leu
   50 55 60
   Ser Thr Leu Leu Lys Asp Glu Gln Pro Thr His Val Ala Val Ala Phe
   65 70 75 80
   Asp Val Gly Arg Lys Thr Phe Arg Thr Asp Met Phe Pro Ala Tyr Lys
   85 90 95
   Ala Gln Arg Glu Ala Thr Pro Pro Glu Phe Lys Gly Gln Val Glu Ile
   100 105 110
   Leu Lys Glu Val Leu Ser Thr Leu Gly Ile Thr Thr Ile Glu Lys Ile
   115 120 125
   Asp Phe Glu Ala Asp Asp Val Ile Ala Thr Leu Ser Val Ala Ala Lys
   130 135 140
   Pro Leu Gly Phe Lys Thr Leu Ile Val Thr Gly Asp Arg Asp Ser Phe
   145 150 155 160
   Gln Leu Val Asn Asp Thr Thr Thr Val Leu Tyr Pro Met Lys Gly Val
   165 170 175
   Ser Val Leu His Arg Phe Thr Pro Glu Ala Val Glu Glu Lys Tyr Gly
   180 185 190
   Leu Thr Pro Arg Gln Tyr Pro Glu Phe Ala Ala Leu Arg Gly Asp Pro
   195 200 205
   Ser Asp Asn Leu Pro Asn Ile Pro Gly Val Gly Glu Lys Thr Ala Thr
   210 215 220
   Lys Trp Ile Ala Gln Tyr Glu Thr Leu Asp Asn Leu Leu Asp His Ala
   225 230 235 240
   Asp Glu Ile Lys Gly Lys Val Gly Ala Ser Leu Arg Glu Arg Ile Glu
   245 250 255
   Gln Val Arg Met Asn Arg Lys Leu Thr Glu Met Val Lys Asp Leu Glu
   260 265 270
   Leu Pro Leu Gly Pro Asp Asp Phe Glu Met Lys Pro Val Gln Val Ala
   275 280 285
   Glu Val Ala Ala Lys Phe Asp Asp Leu Glu Phe Gly Thr Asn Leu Arg
   290 295 300
   Glu Arg Val Leu Ala Val Val Lys Ala Glu Gly Ser Ala Ala Pro Val
   305 310 315 320
   Glu Glu Val Glu Ala Glu Gln Val Val Val Asp Thr Gln Ser Leu Ala
   325 330 335
   Gln Trp Leu Pro Ala Arg Ala Gly Gln Ala Leu Ala Leu Ala Leu Ala
   340 345 350
   Gly Val Ala Lys Pro Ala Ala Gly Asp Thr Tyr Ala Leu Ala Ile Ala
   355 360 365
   Asp Thr Lys Arg His Ala Val Leu Val Asp Val Ala Asp Ile Ser Ala
   370 375 380
   Glu Asp Glu Lys Ala Leu Ala Thr Trp Leu Ala Ser Glu Asp Pro Lys
   385 390 395 400
   Met Leu His Gly Ala Lys Ala Ala Tyr His Met Leu Ala Gly Arg Gly
   405 410 415
   Phe Glu Leu His Gly Val Val His Asp Thr Ala Ile Ala Ala Tyr Leu
   420 425 430
   Leu Arg Pro Gly Gln Arg Thr Tyr Glu Leu Ala Asp Val Tyr Gln Arg
   435 440 445
   His Leu Gln Arg Gln Leu Ser Thr Asn Asp Asn Gly Gly Gln Leu Thr
   450 455 460
   Leu Leu Asp Ala Ala Asp Asp Gln Ser Leu Val Asp Asp Val Ile Ala
   465 470 475 480
   Ile Leu Glu Leu Ser Glu Glu Leu Thr Lys Gln Leu Gln Glu Ile Gln
   485 490 495
   Ala Phe Glu Leu Tyr His Asp Leu Glu Ile Pro Leu Ser Gly Ile Leu
   500 505 510
   Ala Arg Met Glu Ala Ile Gly Ile Ala Val Asp Val Ala Thr Leu Glu
   515 520 525
   Glu Gln Leu Lys Thr Phe Ile Gly Gln Val Ala Gln Glu Glu Glu Ala
   530 535 540
   Ala Arg Glu Leu Ala Glu Asp Pro Thr Leu Asn Leu Ser Ser Pro Lys
   545 550 555 560
   Gln Leu Gln Val Val Leu Phe Glu Thr Phe Gly Met Pro Lys Thr Lys
   565 570 575
   Lys Thr Lys Thr Gly Tyr Ser Thr Ala Ala Ala Glu Ile Glu Ala Leu
   580 585 590
   Ala Ile Lys Asn Pro His Pro Phe Leu Asp His Leu Leu Ala His Arg
   595 600 605
   Gln Tyr Gln Lys Met Lys Thr Thr Leu Glu Gly Leu Ile Arg Glu Val
   610 615 620
   Ala Pro Asp Gly Arg Ile His Thr Thr Phe Asn Gln Thr Val Ala Ser
   625 630 635 640
   Thr Gly Arg Leu Ser Ser Thr Asp Pro Asn Leu Gln Asn Ile Pro Val
   645 650 655
   Arg Thr Glu Ala Gly Arg Lys Ile Arg Ser Gly Phe Val Val Gly Glu
   660 665 670
   Gly Tyr Glu Thr Leu Leu Thr Ala Asp Tyr Ser Gln Ile Glu Met Arg
   675 680 685
   Val Met Ala His Leu Ser Gln Asp Pro Gly Leu Ile Glu Ala Tyr Arg
   690 695 700
   Glu Gly Glu Asp Leu His Asn Tyr Val Gly Ser Lys Val Phe Asn Val
   705 710 715 720
   Pro Ile Asp Gly Val Thr Pro Glu Leu Arg Arg Gln Val Lys Ala Met
                   725                 730                 735
   Ser Tyr Gly Leu Val Tyr Gly Leu Ser Ala Phe Gly Leu Ser Gln Gln
               740                 745                 750
   Leu Ser Ile Pro Ala Gly Glu Ala Lys Gln Ile Met Glu Ser Tyr Phe
           755                 760                 765
   Glu Arg Phe Gly Gly Val Gln Arg Tyr Leu Arg Glu Ile Val Glu Glu
       770                 775                 780
   Ala Arg Lys Ala Gly Tyr Thr Glu Thr Leu Phe Gly Arg Arg Arg Tyr
   785                 790                 795                 800
   Leu Pro Glu Leu Thr Ser Asp Asn Arg Val Ala Arg Glu Asn Ala Glu
                   805                 810                 815
   Arg Ala Ala Leu Asn Ala Pro Ile Gln Gly Thr Ala Ala Asp Ile Ile
               820                 825                 830
   Lys Val Ala Met Ile Arg Val Asp Arg Ser Leu Lys Glu Ala Ala Val
           835                 840                 845
   Lys Ser Arg Val Leu Leu Gln Val His Asp Glu Leu Val Val Glu Val
       850                 855                 860
   Ala Ala Gly Glu Leu Glu Gln Val Arg Glu Ile Leu Glu Arg Glu Met
   865                 870                 875                 880
   Asp Asn Ala Ile Lys Leu Ser Val Pro Leu Glu Val Ser Ala Gly Asp
                   885                 890                 895
   Gly Val Asn Trp Asp Ala Ala Ala His
               900                 905
<210> 5
   <211> 1621
   <212> DNA
   <213> Corynebacterium glutamicum
<220>
   <221> CDS
   <222> (101)..(1591)
   <223> RXA00209
<400> 5
   acaagaccct cgatgctgcg gctgcgttgg accaagcgcc cgctgtcgag gatggacgtt 60
   ttatggttcc gcagattctg ggtgagggcg actaataatt atg acc aac aag tac   115
                                               Met Thr Asn Lys Tyr
                                               1               5
   ctg gtt gaa ggc tct gaa aac gag ctg acc aca aag acc gca gca gag   163
   Leu Val Glu Gly Ser Glu Asn Glu Leu Thr Thr Lys Thr Ala Ala Glu
                   10                  15                  20
   ctg gca ggt ctt att cat tcc cgc gag gta act tcc cgc gag gtt act   211
   Leu Ala Gly Leu Ile His Ser Arg Glu Val Thr Ser Arg Glu Val Thr
               25                  30                  35
   caa gcg cac cta gat cgc att gct gcg gtt gac ggc gat att cat gca   259
   Gln Ala His Leu Asp Arg Ile Ala Ala Val Asp Gly Asp Ile His Ala
           40                  45                  50
   ttt ctc cac gtt ggc cag gag gag gcc ctg aac gcg gcg gat gac gtc    307
   Phe Leu His Val Gly Gln Glu Glu Ala Leu Asn Ala Ala Asp Asp Val
        55                  60                  65
   gat aag cgt cta gac gct gga gag gca cct gcc tcg gct ttg gct ggc    355
   Asp Lys Arg Leu Asp Ala Gly Glu Ala Pro Ala Ser Ala Leu Ala Gly
    70                  75                  80                  85
   gtg ccg ctt gcg ctg aag gat gtc ttt acc acc act gat gcg ccg acc    403
   Val Pro Leu Ala Leu Lys Asp Val Phe Thr Thr Thr Asp Ala Pro Thr
                    90                  95                 100
   acg gcg gca tcg aag atg ctt gag ggc tac atg agc cct tat gac gcg    451
   Thr Ala Ala Ser Lys Met Leu Glu Gly Tyr Met Ser Pro Tyr Asp Ala
               105                 110                 115
   act gtg acc cgc aag atc cgt gag gct ggc atc cca att ttg ggt aag    499
   Thr Val Thr Arg Lys Ile Arg Glu Ala Gly Ile Pro Ile Leu Gly Lys
           120                 125                 130
   acc aac atg gat gag ttt gcg atg ggt tcc tcc act gag aac tcc gca    547
   Thr Asn Met Asp Glu Phe Ala Met Gly Ser Ser Thr Glu Asn Ser Ala
       135                 140                 145
   tac ggc cca acc cac aat ccg tgg gat ctg gag cgc acc gca ggt ggt    595
   Tyr Gly Pro Thr His Asn Pro Trp Asp Leu Glu Arg Thr Ala Gly Gly
   150                 155                 160                 165
   tct ggt ggt ggc tct tca gct gct ctt gct gca ggt cag gcg cca ctt    643
   Ser Gly Gly Gly Ser Ser Ala Ala Leu Ala Ala Gly Gln Ala Pro Leu
                   170                 175                 180
   gcg att ggt act gac act ggt gga tcc atc cgt cag cca gct gcg ctg    691
   Ala Ile Gly Thr Asp Thr Gly Gly Ser Ile Arg Gln Pro Ala Ala Leu
               185                 190                 195
   acc aac act gtc ggt gtg aag cca acc tac gga acc gta tcc cgt tac    739
   Thr Asn Thr Val Gly Val Lys Pro Thr Tyr Gly Thr Val Ser Arg Tyr
           200                 205                 210
   ggt ctg att gcg tgt gcg tcc tcc ctg gat cag ggt ggc cca acc gct    787
   Gly Leu Ile Ala Cys Ala Ser Ser Leu Asp Gln Gly Gly Pro Thr Ala
       215                 220                 225
   cgt act gtt ctg gat acc gcg ctt ttg cac gag gtt atc gca ggc cac    835
   Arg Thr Val Leu Asp Thr Ala Leu Leu His Glu Val Ile Ala Gly His
   230                 235                 240                 245
   gac gct ttt gat gcg acc tcc gtg aat cgt ccg gtt gct cct gtt gtg    883
   Asp Ala Phe Asp Ala Thr Ser Val Asn Arg Pro Val Ala Pro Val Val
                   250                 255                 260
   cag gct gcc cgt gaa ggc gcg aac ggt gac ctg aaa ggc gtg aag gtc    931
   Gln Ala Ala Arg Glu Gly Ala Asn Gly Asp Leu Lys Gly Val Lys Val
               265                 270                 275
   ggt gtg gtc aag cag ttc gac cgc gac ggc tac cag cct ggc gtg ctt    979
   Gly Val Val Lys Gln Phe Asp Arg Asp Gly Tyr Gln Pro Gly Val Leu
           280                 285                 290
   gag gca ttc cac gct tct gtt gag cag atg cgc tcc cag ggt gcg gaa   1027
   Glu Ala Phe His Ala Ser Val Glu Gln Met Arg Ser Gln Gly Ala Glu
       295                 300                 305
   atc gtc gag gtt gat tgc cct cac ttt gat gac gct ctt ggc gcg tac    1075
   Ile Val Glu Val Asp Cys Pro His Phe Asp Asp Ala Leu Gly Ala Tyr
   310                 315                 320                 325
   tac ctg att ctt cct tgt gaa gtt tcc tcc aac ctc gcg cgt ttt gac    1123
   Tyr Leu Ile Leu Pro Cys Glu Val Ser Ser Asn Leu Ala Arg Phe Asp
                   330                 335                 340
   ggc atg cgt tac ggt ttg cgc gct ggt gat gac gga act cgt tcc gcc    1171
   Gly Met Arg Tyr Gly Leu Arg Ala Gly Asp Asp Gly Thr Arg Ser Ala
               345                 350                 355
   gat gag gtc atg gcg tac acc cgt gcg cag gga ttc ggc cct gag gtt    1219
   Asp Glu Val Met Ala Tyr Thr Arg Ala Gln Gly Phe Gly Pro Glu Val
           360                 365                 370
   aag cgc cgt atc atc ctc ggc act tac gcg ttg tct gtt ggt tac tac    1267
   Lys Arg Arg Ile Ile Leu Gly Thr Tyr Ala Leu Ser Val Gly Tyr Tyr
       375                 380                 385
   gac gcg tac tac ctg cag gct cag cgc gtt cgt acc ctc att gca cag    1315
   Asp Ala Tyr Tyr Leu Gln Ala Gln Arg Val Arg Thr Leu Ile Ala Gln
   390                 395                 400                 405
   gac ttc gcc aag gct tac gag cag gtc gac atc ttg gtg tcc cca acc    1363
   Asp Phe Ala Lys Ala Tyr Glu Gln Val Asp Ile Leu Val Ser Pro Thr
                   410                 415                 420
   act cca acc acc gcg ttc aag ctg ggg gag aag gtc acc gat ccg ctg    1411
   Thr Pro Thr Thr Ala Phe Lys Leu Gly Glu Lys Val Thr Asp Pro Leu
               425                 430                 435
   gag atg tac aac ttc gac ttg tgc acc ctg cca ctg aac ctg gct ggt    1459
   Glu Met Tyr Asn Phe Asp Leu Cys Thr Leu Pro Leu Asn Leu Ala Gly
           440                 445                 450
   ctc gcg ggc atg tcc ctg cct tcc ggc ttg gca tca gat act ggt ctg    1507
   Leu Ala Gly Met Ser Leu Pro Ser Gly Leu Ala Ser Asp Thr Gly Leu
       455                 460                 465
   cct gtt ggt ttg cag ctg atg gct cct gct ttc cag gac gat cgt ctc    1555
   Pro Val Gly Leu Gln Leu Met Ala Pro Ala Phe Gln Asp Asp Arg Leu
   470                 475                 480                 485
   tac cgc gtc ggc gct gct ttt gaa gct gga cgc aag taggttctaa         1601
   Tyr Arg Val Gly Ala Ala Phe Glu Ala Gly Arg Lys
                   490                 495
   acccttttta agaaattggc                                               1621
<210> 6
   <211> 497
   <212> PRT
   <213> Corynebacterium glutamicum
<400> 6
   Met Thr Asn Lys Tyr Leu Val Glu Gly Ser Glu Asn Glu Leu Thr Thr
   1               5                   10                  15
   Lys Thr Ala Ala Glu Leu Ala Gly Leu Ile His Ser Arg Glu Val Thr
               20                  25                  30
   Ser Arg Glu Val Thr Gln Ala His Leu Asp Arg Ile Ala Ala Val Asp
           35                  40                  45
   Gly Asp Ile His Ala Phe Leu His Val Gly Gln Glu Glu Ala Leu Asn
       50                  55                  60
   Ala Ala Asp Asp Val Asp Lys Arg Leu Asp Ala Gly Glu Ala Pro Ala
   65                  70                  75                  80
   Ser Ala Leu Ala Gly Val Pro Leu Ala Leu Lys Asp Val Phe Thr Thr
                   85                  90                  95
   Thr Asp Ala Pro Thr Thr Ala Ala Ser Lys Met Leu Glu Gly Tyr Met
               100                 105                 110
   Ser Pro Tyr Asp Ala Thr Val Thr Arg Lys Ile Arg Glu Ala Gly Ile
           115                 120                 125
   Pro Ile Leu Gly Lys Thr Asn Met Asp Glu Phe Ala Met Gly Ser Ser
       130                 135                 140
   Thr Glu Asn Ser Ala Tyr Gly Pro Thr His Asn Pro Trp Asp Leu Glu
   145                 150                 155                 160
   Arg Thr Ala Gly Gly Ser Gly Gly Gly Ser Ser Ala Ala Leu Ala Ala
                   165                 170                 175
   Gly Gln Ala Pro Leu Ala Ile Gly Thr Asp Thr Gly Gly Ser Ile Arg
               180                 185                 190
   Gln Pro Ala Ala Leu Thr Asn Thr Val Gly Val Lys Pro Thr Tyr Gly
           195                 200                 205
   Thr Val Ser Arg Tyr Gly Leu Ile Ala Cys Ala Ser Ser Leu Asp Gln
       210                 215                 220
   Gly Gly Pro Thr Ala Arg Thr Val Leu Asp Thr Ala Leu Leu His Glu
   225                 230                 235                 240
   Val Ile Ala Gly His Asp Ala Phe Asp Ala Thr Ser Val Asn Arg Pro
                   245                 250                 255
   Val Ala Pro Val Val Gln Ala Ala Arg Glu Gly Ala Asn Gly Asp Leu
               260                 265                 270
   Lys Gly Val Lys Val Gly Val Val Lys Gln Phe Asp Arg Asp Gly Tyr
           275                 280                 285
   Gln Pro Gly Val Leu Glu Ala Phe His Ala Ser Val Glu Gln Met Arg
       290                 295                 300
   Ser Gln Gly Ala Glu Ile Val Glu Val Asp Cys Pro His Phe Asp Asp
   305                 310                 315                 320
   Ala Leu Gly Ala Tyr Tyr Leu Ile Leu Pro Cys Glu Val Ser Ser Asn
                   325                 330                 335
   Leu Ala Arg Phe Asp Gly Met Arg Tyr Gly Leu Arg Ala Gly Asp Asp
               340                 345                 350
   Gly Thr Arg Ser Ala Asp Glu Val Met Ala Tyr Thr Arg Ala Gln Gly
           355                 360                 365
   Phe Gly Pro Glu Val Lys Arg Arg Ile Ile Leu Gly Thr Tyr Ala Leu
   370 375 380
   Ser Val Gly Tyr Tyr Asp Ala Tyr Tyr Leu Gln Ala Gln Arg Val Arg
   385 390 395 400
   Thr Leu Ile Ala Gln Asp Phe Ala Lys Ala Tyr Glu Gln Val Asp Ile
   405 410 415
   Leu Val Ser Pro Thr Thr Pro Thr Thr Ala Phe Lys Leu Gly Glu Lys
   420 425 430
   Val Thr Asp Pro Leu Glu Met Tyr Asn Phe Asp Leu Cys Thr Leu Pro
   435 440 445
   Leu Asn Leu Ala Gly Leu Ala Gly Met Ser Leu Pro Ser Gly Leu Ala
   450 455 460
   Ser Asp Thr Gly Leu Pro Val Gly Leu Gln Leu Met Ala Pro Ala Phe
   465 470 475 480
   Gln Asp Asp Arg Leu Tyr Arg Val Gly Ala Ala Phe Glu Ala Gly Arg
   485 490 495
   Lys
<210> 7
   <211> 793
   <212> DNA
   <213> Corynebacterium glutamicum
<220>
   <221> CDS
   <222> (101)..(763)
   <223> RXA00211
<400> 7
   tgagccaaaa tcaataaggt gtttttcagc ctgaggtaaa aatacggtgg tactgtcgaa 60
   accaatcatc ccctagtttt gaaaagaagg aagcgagcca atg tca ttc ctg atc   115
                                               Met Ser Phe Leu Ile
                                               1               5
   cgc gtc ctg ttg tcc gac acc cca ggc agc ctc gcg tta ctc gct gaa   163
   Arg Val Leu Leu Ser Asp Thr Pro Gly Ser Leu Ala Leu Leu Ala Glu
                   10                  15                  20
   gcc ctt ggg att gta gag gcc aat att caa tcc gtg gac gtg gtg gaa   211
   Ala Leu Gly Ile Val Glu Ala Asn Ile Gln Ser Val Asp Val Val Glu
               25                  30                  35
   cgc ttc ccc aat ggc acg gtc atg gac gat ctg gtg atc tcc atc cct   259
   Arg Phe Pro Asn Gly Thr Val Met Asp Asp Leu Val Ile Ser Ile Pro
           40                  45                  50
   cgc gat gtc atg gca gac acc atc atc acc gca gct gaa gaa gtc gac   307
   Arg Asp Val Met Ala Asp Thr Ile Ile Thr Ala Ala Glu Glu Val Asp
       55                  60                  65
   ggc gtg gag att gat tcc atc cgc cca ttc tcc ggg act gtt gac cgc   355
   Gly Val Glu Ile Asp Ser Ile Arg Pro Phe Ser Gly Thr Val Asp Arg
   70                  75                  80                  85
   cgc gga cag atc caa atg ctg gct gct gtt gct cac caa cgc cgc gat     403
   Arg Gly Gln Ile Gln Met Leu Ala Ala Val Ala His Gln Arg Arg Asp
                       90                  95                  100
   atc acc gca gcg atg gaa gaa atg gtc gat gtc atc ccc cgc acc atg     451
   Ile Thr Ala Ala Met Glu Glu Met Val Asp Val Ile Pro Arg Thr Met
                   105                 110                 115
   acc tct ggt tgg gct ttg gtc att gat cta aaa gga ccc atc act cgc     499
   Thr Ser Gly Trp Ala Leu Val Ile Asp Leu Lys Gly Pro Ile Thr Arg
           120                 125                 130
   atc gct ggt tcc cta gca gcg ccc gaa gat gac ggc acc gtt ccg gag     547
   Ile Ala Gly Ser Leu Ala Ala Pro Glu Asp Asp Gly Thr Val Pro Glu
       135                 140                 145
   aac atc gtt ctc aaa gaa gct cgc atg ctc aac ccg gaa aac gat ccg     595
   Asn Ile Val Leu Lys Glu Ala Arg Met Leu Asn Pro Glu Asn Asp Pro
   150                 155                 160                 165
   tgg att cca gag tcc tgg aca ctg ctt gat tct tcc ctt gcc atc gct     643
   Trp Ile Pro Glu Ser Trp Thr Leu Leu Asp Ser Ser Leu Ala Ile Ala
                   170                 175                 180
   ccg atc ggc aag cac ggc ctg gct ctg att atc ggt cgc cct ggt ggc     691
   Pro Ile Gly Lys His Gly Leu Ala Leu Ile Ile Gly Arg Pro Gly Gly
               185                 190                 195
   cct gat ttc ttg gcc agc gaa gtg gag cac tta ggc caa gtc ggt gac     739
   Pro Asp Phe Leu Ala Ser Glu Val Glu His Leu Gly Gln Val Gly Asp
           200                 205                 210
   att atc gga gca atg ctt caa aaa taatctgagc tgtttaaaaa atgccccaag    793
   Ile Ile Gly Ala Met Leu Gln Lys
       215                 220
<210> 8
   <211> 221
   <212> PRT
   <213> Corynebacterium glutamicum
<400> 8
   Met Ser Phe Leu Ile Arg Val Leu Leu Ser Asp Thr Pro Gly Ser Leu
   1               5                   10                  15
   Ala Leu Leu Ala Glu Ala Leu Gly Ile Val Glu Ala Asn Ile Gln Ser
               20                  25                  30
   Val Asp Val Val Glu Arg Phe Pro Asn Gly Thr Val Met Asp Asp Leu
           35                  40                  45
   Val Ile Ser Ile Pro Arg Asp Val Met Ala Asp Thr Ile Ile Thr Ala
       50                  55                  60
   Ala Glu Glu Val Asp Gly Val Glu Ile Asp Ser Ile Arg Pro Phe Ser
   65                  70                  75                  80
   Gly Thr Val Asp Arg Arg Gly Gln Ile Gln Met Leu Ala Ala Val Ala
                   85                  90                  95
   His Gln Arg Arg Asp Ile Thr Ala Ala Met Glu Glu Met Val Asp Val
               100                 105                 110
   Ile Pro Arg Thr Met Thr Ser Gly Trp Ala Leu Val Ile Asp Leu Lys
           115                 120                 125
   Gly Pro Ile Thr Arg Ile Ala Gly Ser Leu Ala Ala Pro Glu Asp Asp
       130                 135                 140
   Gly Thr Val Pro Glu Asn Ile Val Leu Lys Glu Ala Arg Met Leu Asn
   145                 150                 155                 160
   Pro Glu Asn Asp Pro Trp Ile Pro Glu Ser Trp Thr Leu Leu Asp Ser
                   165                 170                 175
   Ser Leu Ala Ile Ala Pro Ile Gly Lys His Gly Leu Ala Leu Ile Ile
               180                 185                 190
   Gly Arg Pro Gly Gly Pro Asp Phe Leu Ala Ser Glu Val Glu His Leu
           195                 200                 205
   Gly Gln Val Gly Asp Ile Ile Gly Ala Met Leu Gln Lys
       210                 215                 220
<210> 9
   <211> 1543
   <212> DNA
   <213> Corynebacterium glutamicum
<220>
   <221> CDS
   <222> (101)..(1513)
   <223> RXA00314
<400> 9
   acctgtaaac acttacggtt tgggcgaaat tgaagcggga gccaacctgc tcaacgtcgc 60
   aaagaaagaa gcggtgccag caacaccata agttgaaacc ttg agt gtt cgc aca   115
                                              Leu Ser Val Arg Thr
                                               1               5
   cag gtt aga cta ggg gac gtg act cta cgc atc ttt gac acc ggt acc   163
   Gln Val Arg Leu Gly Asp Val Thr Leu Arg Ile Phe Asp Thr Gly Thr
                        10                  15                  20
   cgt acg ctt cga gat ttt aaa cct gtt caa cca ggt cat gcc tcg gtg   211
   Arg Thr Leu Arg Asp Phe Lys Pro Val Gln Pro Gly His Ala Ser Val
                    25                  30                  35
   tac ctg tgt ggt gcc acc ccg caa tct tca ccc cac att gga cat gtt   259
   Tyr Leu Cys Gly Ala Thr Pro Gln Ser Ser Pro His Ile Gly His Val
                40                  45                  50
   cgt tca gca gta gcg ttt gat att ttg cgc cgc tgg ctc atg gct aag   307
   Arg Ser Ala Val Ala Phe Asp Ile Leu Arg Arg Trp Leu Met Ala Lys
            55                  60                  65
   gga ctt gat gtg gca ttt gtt cgc aat gtc act gat atc gat gac aag   355
   Gly Leu Asp Val Ala Phe Val Arg Asn Val Thr Asp Ile Asp Asp Lys
        70                  75                  80                  85
   att ctc acc aag gca tct gaa aat ggt cgc cct tgg tgg gaa tgg gtg   403
   Ile Leu Thr Lys Ala Ser Glu Asn Gly Arg Pro Trp Trp Glu Trp Val
                        90                  95                 100
   tcc acc tat gaa cgt gaa ttc acc tgg acg tac aac acg ttg ggt gtg    451
   Ser Thr Tyr Glu Arg Glu Phe Thr Trp Thr Tyr Asn Thr Leu Gly Val
               105                 110                 115
   ctt cct cca tca acg gag cct cgt gca aca ggc cac gtc act cag atg    499
   Leu Pro Pro Ser Thr Glu Pro Arg Ala Thr Gly His Val Thr Gln Met
           120                 125                 130
   att aag tac atg cag cgc ttg att gat aac ggc ttt gct tac gcc gtt    547
   Ile Lys Tyr Met Gln Arg Leu Ile Asp Asn Gly Phe Ala Tyr Ala Val
       135                 140                 145
   gat ggc tct gtg tac ttt gat gtc gca gcg tgg tcc aag gct gaa gga    595
   Asp Gly Ser Val Tyr Phe Asp Val Ala Ala Trp Ser Lys Ala Glu Gly
   150                 155                 160                 165
   tct gac tat ggt tct ttg tcc gga aac cgt gtt gaa gat atg gag cag    643
   Ser Asp Tyr Gly Ser Leu Ser Gly Asn Arg Val Glu Asp Met Glu Gln
                   170                 175                 180
   ggc gag ccc gat aac ttt ggt aag cgg ggg cca cag gac ttt gct ctg    691
   Gly Glu Pro Asp Asn Phe Gly Lys Arg Gly Pro Gln Asp Phe Ala Leu
               185                 190                 195
   tgg aag gct gcc aaa ccg ggt gag ccg tca tgg cca acc cct tgg gga    739
   Trp Lys Ala Ala Lys Pro Gly Glu Pro Ser Trp Pro Thr Pro Trp Gly
           200                 205                 210
   gac ggc cgg ccg ggt tgg cat ttg gaa tgc tct gcc atg gcc acc tac    787
   Asp Gly Arg Pro Gly Trp His Leu Glu Cys Ser Ala Met Ala Thr Tyr
       215                 220                 225
   tat ttg ggt gag caa ttt gat att cac tgt ggt ggt ttg gat ctg caa    835
   Tyr Leu Gly Glu Gln Phe Asp Ile His Cys Gly Gly Leu Asp Leu Gln
   230                 235                 240                 245
   ttt cca cac cat gaa aat gaa att gcc cag gca cat gcg gct ggc gat    883
   Phe Pro His His Glu Asn Glu Ile Ala Gln Ala His Ala Ala Gly Asp
                   250                 255                 260
   aaa ttt gcc aac tac tgg atg cac aat cac tgg gta aca atg gcc ggc    931
   Lys Phe Ala Asn Tyr Trp Met His Asn His Trp Val Thr Met Ala Gly
               265                 270                 275
   gag aaa atg tcc aag tct ttg ggc aat gtt ttg gct gtg ccg gaa atg    979
   Glu Lys Met Ser Lys Ser Leu Gly Asn Val Leu Ala Val Pro Glu Met
           280                 285                 290
   cta aag cag gtt cgt cct gtc gag ctt cgt tat tac ctt ggg tct gcc    1027
   Leu Lys Gln Val Arg Pro Val Glu Leu Arg Tyr Tyr Leu Gly Ser Ala
       295                 300                 305
   cat tac cgt tcc gtc ctt gag tat tcc gag agc gct ttg agt gaa gct    1075
   His Tyr Arg Ser Val Leu Glu Tyr Ser Glu Ser Ala Leu Ser Glu Ala
   310                 315                 320                 325
   gcg gtg ggt tac cgt cgc att gag tct ttc ctt gag cgt gtg ggg gat    1123
   Ala Val Gly Tyr Arg Arg Ile Glu Ser Phe Leu Glu Arg Val Gly Asp
                   330                 335                 340
   gtt gag gta ggc gag tgg acg cca ggt ttt gaa gtt gcg atg gat gag    1171
   Val Glu Val Gly Glu Trp Thr Pro Gly Phe Glu Val Ala Met Asp Glu
               345                 350                 355
   gat att gca gtt cct aag gct ttg gct gaa atc cat aac gct gtc cgc   1219
   Asp Ile Ala Val Pro Lys Ala Leu Ala Glu Ile His Asn Ala Val Arg
           360                 365                 370
   gag ggc aat gct gcc ttg gat aag ggt gat cgt gag gca gcg gag aag   1267
   Glu Gly Asn Ala Ala Leu Asp Lys Gly Asp Arg Glu Ala Ala Glu Lys
       375                 380                 385
   ctt gct tcc tcg gtt cgt gcg atg act ggc gtt ttg ggc ttc gac ccc   1315
   Leu Ala Ser Ser Val Arg Ala Met Thr Gly Val Leu Gly Phe Asp Pro
   390                 395                 400                 405
   gtt gaa tgg ggt tca gat gca ggc gct gat ggc aag gca gat aag gcg   1363
   Val Glu Trp Gly Ser Asp Ala Gly Ala Asp Gly Lys Ala Asp Lys Ala
                   410                 415                 420
   ctt gat gtg ctg att tct tcg gag ctt gag cgt cgt gca act gct cgt   1411
   Leu Asp Val Leu Ile Ser Ser Glu Leu Glu Arg Arg Ala Thr Ala Arg
               425                 430                 435
   gct gag aag aat tgg gcg gtt gct gat gag gtt cga gat cgt ctt gcc   1459
   Ala Glu Lys Asn Trp Ala Val Ala Asp Glu Val Arg Asp Arg Leu Ala
           440                 445                 450
   gat gct ggt att gag gtt gtg gat acc gca gat ggc gct aca tgg aaa   1507
   Asp Ala Gly Ile Glu Val Val Asp Thr Ala Asp Gly Ala Thr Trp Lys
       455                 460                 465
   ttg cag taattacaga cacttttaag gagataattt                          1543
   Leu Gln
   470
<210> 10
   <211> 471
   <212> PRT
   <213> Corynebacterium glutamicum
<400> 10
   Leu Ser Val Arg Thr Gln Val Arg Leu Gly Asp Val Thr Leu Arg Ile
    1               5                  10                  15
   Phe Asp Thr Gly Thr Arg Thr Leu Arg Asp Phe Lys Pro Val Gln Pro
               20                  25                  30
   Gly His Ala Ser Val Tyr Leu Cys Gly Ala Thr Pro Gln Ser Ser Pro
           35                  40                  45
   His Ile Gly His Val Arg Ser Ala Val Ala Phe Asp Ile Leu Arg Arg
       50                  55                  60
   Trp Leu Met Ala Lys Gly Leu Asp Val Ala Phe Val Arg Asn Val Thr
    65                  70                  75                  80
   Asp Ile Asp Asp Lys Ile Leu Thr Lys Ala Ser Glu Asn Gly Arg Pro
                    85                  90                  95
   Trp Trp Glu Trp Val Ser Thr Tyr Glu Arg Glu Phe Thr Trp Thr Tyr
               100                 105                 110
   Asn Thr Leu Gly Val Leu Pro Pro Ser Thr Glu Pro Arg Ala Thr Gly
           115                 120                 125
   His Val Thr Gln Met Ile Lys Tyr Met Gln Arg Leu Ile Asp Asn Gly
       130                 135                 140
   Phe Ala Tyr Ala Val Asp Gly Ser Val Tyr Phe Asp Val Ala Ala Trp
   145                 150                 155                 160
   Ser Lys Ala Glu Gly Ser Asp Tyr Gly Ser Leu Ser Gly Asn Arg Val
                   165                 170                 175
   Glu Asp Met Glu Gln Gly Glu Pro Asp Asn Phe Gly Lys Arg Gly Pro
               180                 185                 190
   Gln Asp Phe Ala Leu Trp Lys Ala Ala Lys Pro Gly Glu Pro Ser Trp
           195                 200                 205
   Pro Thr Pro Trp Gly Asp Gly Arg Pro Gly Trp His Leu Glu Cys Ser
       210                 215                 220
   Ala Met Ala Thr Tyr Tyr Leu Gly Glu Gln Phe Asp Ile His Cys Gly
   225                 230                 235                 240
   Gly Leu Asp Leu Gln Phe Pro His His Glu Asn Glu Ile Ala Gln Ala
                   245                 250                 255
   His Ala Ala Gly Asp Lys Phe Ala Asn Tyr Trp Met His Asn His Trp
               260                 265                 270
   Val Thr Met Ala Gly Glu Lys Met Ser Lys Ser Leu Gly Asn Val Leu
           275                 280                 285
   Ala Val Pro Glu Met Leu Lys Gln Val Arg Pro Val Glu Leu Arg Tyr
       290                 295                 300
   Tyr Leu Gly Ser Ala His Tyr Arg Ser Val Leu Glu Tyr Ser Glu Ser
   305                 310                 315                 320
   Ala Leu Ser Glu Ala Ala Val Gly Tyr Arg Arg Ile Glu Ser Phe Leu
                   325                 330                 335
   Glu Arg Val Gly Asp Val Glu Val Gly Glu Trp Thr Pro Gly Phe Glu
               340                 345                 350
   Val Ala Met Asp Glu Asp Ile Ala Val Pro Lys Ala Leu Ala Glu Ile
           355                 360                 365
   His Asn Ala Val Arg Glu Gly Asn Ala Ala Leu Asp Lys Gly Asp Arg
       370                 375                 380
   Glu Ala Ala Glu Lys Leu Ala Ser Ser Val Arg Ala Met Thr Gly Val
   385                 390                 395                 400
   Leu Gly Phe Asp Pro Val Glu Trp Gly Ser Asp Ala Gly Ala Asp Gly
                   405                 410                 415
   Lys Ala Asp Lys Ala Leu Asp Val Leu Ile Ser Ser Glu Leu Glu Arg
               420                 425                 430
   Arg Ala Thr Ala Arg Ala Glu Lys Asn Trp Ala Val Ala Asp Glu Val
           435                 440                 445
   Arg Asp Arg Leu Ala Asp Ala Gly Ile Glu Val Val Asp Thr Ala Asp
       450                 455                 460
   Gly Ala Thr Trp Lys Leu Gln
   465 470
<210> 11
   <211> 1009
   <212> DNA
   <213> Corynebacterium glutamicum
<220>
   <221> CDS
   <222> (101)..(979)
   <223> RXA00458
<400> 11
   cacccctgaa aacctcctca actatcccgg agtgatcatc tccaccgttc aggagaaccc 60
   atccgaaaca tggcggcaag tgaacatcta atctagaaac atg gca gga cga tac   115
                                               Met Ala Gly Arg Tyr
                                               1               5
   gca cca tca cca agc ggc gac ctt cac ttt ggc aac ctc cgc aca gca   163
   Ala Pro Ser Pro Ser Gly Asp Leu His Phe Gly Asn Leu Arg Thr Ala
                   10                  15                  20
   ctg ctg gcc tgg ctg ttc gcg cgc tcc gaa gga aaa aaa ttc ctc atg   211
   Leu Leu Ala Trp Leu Phe Ala Arg Ser Glu Gly Lys Lys Phe Leu Met
               25                  30                  35
   cgg gtc gaa gac atc gat gaa caa cgc tca tcc aag gaa tcc gcc gaa   259
   Arg Val Glu Asp Ile Asp Glu Gln Arg Ser Ser Lys Glu Ser Ala Glu
           40                  45                  50
   agc caa ctc gca gac cta tcc gcc ctg ggt ctc gat tgg gat ggc gac   307
   Ser Gln Leu Ala Asp Leu Ser Ala Leu Gly Leu Asp Trp Asp Gly Asp
       55                  60                  65
   gtc ctc tac caa tcc aca cgc tac gac gcc tac cgc gca gcc ctt gaa   355
   Val Leu Tyr Gln Ser Thr Arg Tyr Asp Ala Tyr Arg Ala Ala Leu Glu
   70                  75                  80                  85
   aaa cta gac acc tac gaa tgt tat tgc tcg cgc cgg gac atc caa gaa   403
   Lys Leu Asp Thr Tyr Glu Cys Tyr Cys Ser Arg Arg Asp Ile Gln Glu
                   90                  95                  100
   gcc tcg cgg gca ccc cat gtg gct ccg gga gtg tat ccg gga acg tgt   451
   Ala Ser Arg Ala Pro His Val Ala Pro Gly Val Tyr Pro Gly Thr Cys
               105                 110                 115
   agg gga ttg aag gag gag gaa cgc gtc gaa aag cgt gca acc ttg gct   499
   Arg Gly Leu Lys Glu Glu Glu Arg Val Glu Lys Arg Ala Thr Leu Ala
           120                 125                 130
   gcg caa aac cgg cac ccc gcc atc cgc ctg cgc gcg cag gta acc tcg   547
   Ala Gln Asn Arg His Pro Ala Ile Arg Leu Arg Ala Gln Val Thr Ser
       135                 140                 145
   ttt gat ttt cac gac cga ctt cgc ggc cca caa act ggc ccc gta gac   595
   Phe Asp Phe His Asp Arg Leu Arg Gly Pro Gln Thr Gly Pro Val Asp
   150                 155                 160                 165
   gat ttc att ctg ctc cgc ggc ggg cag gaa ccc gga tgg gca tac aac   643
   Asp Phe Ile Leu Leu Arg Gly Gly Gln Glu Pro Gly Trp Ala Tyr Asn
                   170                 175                 180
   tta gct gtc gtc gtc gac gat gcc tac caa ggc gtt gac cag gta gtc    691
   Leu Ala Val Val Val Asp Asp Ala Tyr Gln Gly Val Asp Gln Val Val
               185                 190                 195
   cgc ggc gac gac cta ctc gat tcc gcc gcg cgc caa gcc tac ctc ggc    739
   Arg Gly Asp Asp Leu Leu Asp Ser Ala Ala Arg Gln Ala Tyr Leu Gly
           200                 205                 210
   tcg ctg ctg ggc acc ccc gcg ccc gaa tac att cac gtg ccg ctc gtg    787
   Ser Leu Leu Gly Thr Pro Ala Pro Glu Tyr Ile His Val Pro Leu Val
       215                 220                 225
   ctc aac gcc cac ggc cag cgc ctc gcc aaa cgc gac ggg gca gtg acg    835
   Leu Asn Ala His Gly Gln Arg Leu Ala Lys Arg Asp Gly Ala Val Thr
   230                 235                 240                 245
   ctt aaa gaa atg ctt atc gac gcc ccc ctc cac acc att ttc tcc cgc    883
   Leu Lys Glu Met Leu Ile Asp Ala Pro Leu His Thr Ile Phe Ser Arg
                   250                 255                 260
   ctc gca tca tcg ctc ggc tac gaa ggg gta aat tcc gca ccc caa ttg    931
   Leu Ala Ser Ser Leu Gly Tyr Glu Gly Val Asn Ser Ala Pro Gln Leu
               265                 270                 275
   ttg gaa att ttc gac ccc aca acc ctc agc cgg gag ccg ttt att tac    979
   Leu Glu Ile Phe Asp Pro Thr Thr Leu Ser Arg Glu Pro Phe Ile Tyr
           280                 285                 290
   tgaggctcag agggaggggt cattccatct                                   1009
<210> 12
   <211> 293
   <212> PRT
   <213> Corynebacterium glutamicum
<400> 12
   Met Ala Gly Arg Tyr Ala Pro Ser Pro Ser Gly Asp Leu His Phe Gly
   1 5 10 15
   Asn Leu Arg Thr Ala Leu Leu Ala Trp Leu Phe Ala Arg Ser Glu Gly
   20 25 30
   Lys Lys Phe Leu Met Arg Val Glu Asp Ile Asp Glu Gln Arg Ser Ser
   35 40 45
   Lys Glu Ser Ala Glu Ser Gln Leu Ala Asp Leu Ser Ala Leu Gly Leu
   50 55 60
   Asp Trp Asp Gly Asp Val Leu Tyr Gln Ser Thr Arg Tyr Asp Ala Tyr
   65 70 75 80
   Arg Ala Ala Leu Glu Lys Leu Asp Thr Tyr Glu Cys Tyr Cys Ser Arg
   85 90 95
   Arg Asp Ile Gln Glu Ala Ser Arg Ala Pro His Val Ala Pro Gly Val
   100 105 110
   Tyr Pro Gly Thr Cys Arg Gly Leu Lys Glu Glu Glu Arg Val Glu Lys
   115 120 125
   Arg Ala Thr Leu Ala Ala Gln Asn Arg His Pro Ala Ile Arg Leu Arg
   130 135 140
   Ala Gln Val Thr Ser Phe Asp Phe His Asp Arg Leu Arg Gly Pro Gln
   145 150 155 160
   Thr Gly Pro Val Asp Asp Phe Ile Leu Leu Arg Gly Gly Gln Glu Pro
   165 170 175
   Gly Trp Ala Tyr Asn Leu Ala Val Val Val Asp Asp Ala Tyr Gln Gly
   180 185 190
   Val Asp Gln Val Val Arg Gly Asp Asp Leu Leu Asp Ser Ala Ala Arg
   195 200 205
   Gln Ala Tyr Leu Gly Ser Leu Leu Gly Thr Pro Ala Pro Glu Tyr Ile
   210 215 220
   His Val Pro Leu Val Leu Asn Ala His Gly Gln Arg Leu Ala Lys Arg
   225 230 235 240
   Asp Gly Ala Val Thr Leu Lys Glu Met Leu Ile Asp Ala Pro Leu His
   245 250 255
   Thr Ile Phe Ser Arg Leu Ala Ser Ser Leu Gly Tyr Glu Gly Val Asn
   260 265 270
   Ser Ala Pro Gln Leu Leu Glu Ile Phe Asp Pro Thr Thr Leu Ser Arg
   275 280 285
   Glu Pro Phe Ile Tyr
   290
<210> 13
   <211> 1744
   <212> DNA
   <213> Corynebacterium glutamicum
<220>
   <221> CDS
   <222> (101)..(1714)
   <223> RXA00493
<400> 13
   cccgttacgg cggcaccgag atcaagttcg gtggcgtgga gtacttgctt ctctccgctc 60
   gtgacatcct cgcaatcgtc gagaagtagg ggataagttc atg gca aag ctc att   115
                                             Met Ala Lys Leu Ile
                                             1               5
   gct ttt gac cag gac gcc cgc gaa ggc att ctc cgg ggc gtt gac gct   163
   Ala Phe Asp Gln Asp Ala Arg Glu Gly Ile Leu Arg Gly Val Asp Ala
                   10                  15                  20
   ctg gca aac gct gtc aag gta acc ctc ggc cca cgc ggc cgt aac gtg   211
   Leu Ala Asn Ala Val Lys Val Thr Leu Gly Pro Arg Gly Arg Asn Val
               25                  30                  35
   gtt ctt gat aag gca ttc ggc gga cct ctg gtc acc aac gac ggt gtc   259
   Val Leu Asp Lys Ala Phe Gly Gly Pro Leu Val Thr Asn Asp Gly Val
           40                  45                  50
   acc att gcc cgc gac atc gac ctt gag gat cct ttt gag aac ctc ggt   307
   Thr Ile Ala Arg Asp Ile Asp Leu Glu Asp Pro Phe Glu Asn Leu Gly
       55                  60                  65
   gcg cag ctg gtg aag tcc gtt gct gtt aag acc aac gac atc gct ggt    355
   Ala Gln Leu Val Lys Ser Val Ala Val Lys Thr Asn Asp Ile Ala Gly
    70                  75                  80                  85
   gac ggc acc acg act gca act ctg ctt gct cag gca ctc att gct gaa    403
   Asp Gly Thr Thr Thr Ala Thr Leu Leu Ala Gln Ala Leu Ile Ala Glu
                    90                  95                 100
   ggc ctg cgc aac gtt gct gct ggc gca aac cca atg gag ctc aac aag    451
   Gly Leu Arg Asn Val Ala Ala Gly Ala Asn Pro Met Glu Leu Asn Lys
               105                 110                 115
   ggt att tct gca gct gca gaa aag acc ttg gaa gag ttg aag gca cgc    499
   Gly Ile Ser Ala Ala Ala Glu Lys Thr Leu Glu Glu Leu Lys Ala Arg
           120                 125                 130
   gca acc gag gtg tct gac acc aag gaa atc gca aac gtc gct acc gtt    547
   Ala Thr Glu Val Ser Asp Thr Lys Glu Ile Ala Asn Val Ala Thr Val
       135                 140                 145
   tca tcc cgc gat gaa gtt gtc ggc gag atc gtt gct gca gcg atg gaa    595
   Ser Ser Arg Asp Glu Val Val Gly Glu Ile Val Ala Ala Ala Met Glu
   150                 155                 160                 165
   aag gtt ggc aag gac ggt gtc gtc acc gtt gag gag tcc cag tcc atc    643
   Lys Val Gly Lys Asp Gly Val Val Thr Val Glu Glu Ser Gln Ser Ile
                   170                 175                 180
   gag act gct ctc gag gtc acc gaa ggt att tct ttc gac aag ggc tac    691
   Glu Thr Ala Leu Glu Val Thr Glu Gly Ile Ser Phe Asp Lys Gly Tyr
               185                 190                 195
   ctt tcc cct tat ttc atc aac gac aac gac act cag cag gct gtc ctg    739
   Leu Ser Pro Tyr Phe Ile Asn Asp Asn Asp Thr Gln Gln Ala Val Leu
           200                 205                 210
   gac aac cct gca gtg ctg ctt gtt cgc aac aag att tct tcc ctc cca    787
   Asp Asn Pro Ala Val Leu Leu Val Arg Asn Lys Ile Ser Ser Leu Pro
       215                 220                 225
   gac ttc ctc cca ttg ctg gag aag gtt gtg gag tcc aac cgt cct ttg    835
   Asp Phe Leu Pro Leu Leu Glu Lys Val Val Glu Ser Asn Arg Pro Leu
   230                 235                 240                 245
   ctg atc atc gca gaa gac gtc gag ggc gag cct ttg cag acc ctg gtt    883
   Leu Ile Ile Ala Glu Asp Val Glu Gly Glu Pro Leu Gln Thr Leu Val
                   250                 255                 260
   gtg aac tcc atc cgc aag acc atc aag gtc gtt gca gtg aag tcc cct    931
   Val Asn Ser Ile Arg Lys Thr Ile Lys Val Val Ala Val Lys Ser Pro
               265                 270                 275
   tac ttc ggt gac cga cgc aag gcg ttc atg gat gac ctg gct att gtc    979
   Tyr Phe Gly Asp Arg Arg Lys Ala Phe Met Asp Asp Leu Ala Ile Val
           280                 285                 290
   acc aag gca act gtc gtg gat cca gaa gtg ggc atc aac ctc aac gaa    1027
   Thr Lys Ala Thr Val Val Asp Pro Glu Val Gly Ile Asn Leu Asn Glu
       295                 300                 305
   gct ggc gaa gaa gtt ttc ggt acc gca cgc cgc atc acc gtt tcc aag    1075
   Ala Gly Glu Glu Val Phe Gly Thr Ala Arg Arg Ile Thr Val Ser Lys
   310                 315                 320                 325
   gac gaa acc atc atc gtt gat ggt gca ggt tcc gca gaa gac gtt gaa     1123
   Asp Glu Thr Ile Ile Val Asp Gly Ala Gly Ser Ala Glu Asp Val Glu
                   330                 335                 340
   gca cgt cgc ggc cag atc cgt cgc gaa atc gcc aac acc gat tcc acc     1171
   Ala Arg Arg Gly Gln Ile Arg Arg Glu Ile Ala Asn Thr Asp Ser Thr
               345                 350                 355
   tgg gat cgc gaa aag gca gaa gag cgt ttg gct aag ctc tcc ggt ggt     1219
   Trp Asp Arg Glu Lys Ala Glu Glu Arg Leu Ala Lys Leu Ser Gly Gly
           360                 365                 370
   att gct gtc atc cgc gtt ggt gca gca act gaa acc gaa gtc aac gac     1267
   Ile Ala Val Ile Arg Val Gly Ala Ala Thr Glu Thr Glu Val Asn Asp
       375                 380                 385
   cgc aag ctg cgt gtc gaa gat gcc atc aac gct gct cgc gca gca gca     1315
   Arg Lys Leu Arg Val Glu Asp Ala Ile Asn Ala Ala Arg Ala Ala Ala
   390                 395                 400                 405
   caa gaa ggc gtt atc gct ggt ggc ggt tcc gct ttg gtt cag atc gct     1363
   Gln Glu Gly Val Ile Ala Gly Gly Gly Ser Ala Leu Val Gln Ile Ala
                   410                 415                 420
   gag act ctg aag gct tac gcc gaa gag ttc gaa ggc gac cag aag gtc     1411
   Glu Thr Leu Lys Ala Tyr Ala Glu Glu Phe Glu Gly Asp Gln Lys Val
               425                 430                 435
   ggc gtt cgc gca ctg gct act gct ttg ggc aag cca gcg tac tgg atc     1459
   Gly Val Arg Ala Leu Ala Thr Ala Leu Gly Lys Pro Ala Tyr Trp Ile
           440                 445                 450
   gcc tcc aac gca ggt ctt gac ggc tct gtt gtt gtt gca cgc act gct     1507
   Ala Ser Asn Ala Gly Leu Asp Gly Ser Val Val Val Ala Arg Thr Ala
       455                 460                 465
   gct ctg cca aac ggc gag ggc ttc aac gct gca act ttg gaa tac gga     1555
   Ala Leu Pro Asn Gly Glu Gly Phe Asn Ala Ala Thr Leu Glu Tyr Gly
   470                 475                 480                 485
   aac ctg atc aac gac ggt gtc atc gac cca gtc aag gtc acc cat tcc     1603
   Asn Leu Ile Asn Asp Gly Val Ile Asp Pro Val Lys Val Thr His Ser
                   490                 495                 500
   gca gta gtg aat gca acc tct gtt gca cgc atg gtt ctg acc act gag     1651
   Ala Val Val Asn Ala Thr Ser Val Ala Arg Met Val Leu Thr Thr Glu
               505                 510                 515
   gct tct gtt gtt gag aag cct gca gaa gaa gca gcc gat gca cat gca     1699
   Ala Ser Val Val Glu Lys Pro Ala Glu Glu Ala Ala Asp Ala His Ala
           520                 525                 530
   gga cat cat cac cac taaagttctg tgaaaaacac cgtggggcag               1744
   Gly His His His His
       535
<210> 14
   <211> 538
   <212> PRT
   <213> Corynebacterium glutamicum
<400> 14
   Met Ala Lys Leu Ile Ala Phe Asp Gln Asp Ala Arg Glu Gly Ile Leu
   1 5 10 15
   Arg Gly Val Asp Ala Leu Ala Asn Ala Val Lys Val Thr Leu Gly Pro
   20 25 30
   Arg Gly Arg Asn Val Val Leu Asp Lys Ala Phe Gly Gly Pro Leu Val
   35 40 45
   Thr Asn Asp Gly Val Thr Ile Ala Arg Asp Ile Asp Leu Glu Asp Pro
   50 55 60
   Phe Glu Asn Leu Gly Ala Gln Leu Val Lys Ser Val Ala Val Lys Thr
   65 70 75 80
   Asn Asp Ile Ala Gly Asp Gly Thr Thr Thr Ala Thr Leu Leu Ala Gln
   85 90 95
   Ala Leu Ile Ala Glu Gly Leu Arg Asn Val Ala Ala Gly Ala Asn Pro
   100 105 110
   Met Glu Leu Asn Lys Gly Ile Ser Ala Ala Ala Glu Lys Thr Leu Glu
   115 120 125
   Glu Leu Lys Ala Arg Ala Thr Glu Val Ser Asp Thr Lys Glu Ile Ala
   130 135 140
   Asn Val Ala Thr Val Ser Ser Arg Asp Glu Val Val Gly Glu Ile Val
   145 150 155 160
   Ala Ala Ala Met Glu Lys Val Gly Lys Asp Gly Val Val Thr Val Glu
   165 170 175
   Glu Ser Gln Ser Ile Glu Thr Ala Leu Glu Val Thr Glu Gly Ile Ser
   180 185 190
   Phe Asp Lys Gly Tyr Leu Ser Pro Tyr Phe Ile Asn Asp Asn Asp Thr
   195 200 205
   Gln Gln Ala Val Leu Asp Asn Pro Ala Val Leu Leu Val Arg Asn Lys
   210 215 220
   Ile Ser Ser Leu Pro Asp Phe Leu Pro Leu Leu Glu Lys Val Val Glu
   225 230 235 240
   Ser Asn Arg Pro Leu Leu Ile Ile Ala Glu Asp Val Glu Gly Glu Pro
   245 250 255
   Leu Gln Thr Leu Val Val Asn Ser Ile Arg Lys Thr Ile Lys Val Val
   260 265 270
   Ala Val Lys Ser Pro Tyr Phe Gly Asp Arg Arg Lys Ala Phe Met Asp
   275 280 285
   Asp Leu Ala Ile Val Thr Lys Ala Thr Val Val Asp Pro Glu Val Gly
   290 295 300
   Ile Asn Leu Asn Glu Ala Gly Glu Glu Val Phe Gly Thr Ala Arg Arg
   305 310 315 320
   Ile Thr Val Ser Lys Asp Glu Thr Ile Ile Val Asp Gly Ala Gly Ser
   325 330 335
   Ala Glu Asp Val Glu Ala Arg Arg Gly Gln Ile Arg Arg Glu Ile Ala
   340 345 350
   Asn Thr Asp Ser Thr Trp Asp Arg Glu Lys Ala Glu Glu Arg Leu Ala
   355 360 365
   Lys Leu Ser Gly Gly Ile Ala Val Ile Arg Val Gly Ala Ala Thr Glu
   370 375 380
   Thr Glu Val Asn Asp Arg Lys Leu Arg Val Glu Asp Ala Ile Asn Ala
   385 390 395 400
   Ala Arg Ala Ala Ala Gln Glu Gly Val Ile Ala Gly Gly Gly Ser Ala
   405 410 415
   Leu Val Gln Ile Ala Glu Thr Leu Lys Ala Tyr Ala Glu Glu Phe Glu
   420 425 430
   Gly Asp Gln Lys Val Gly Val Arg Ala Leu Ala Thr Ala Leu Gly Lys
   435 440 445
   Pro Ala Tyr Trp Ile Ala Ser Asn Ala Gly Leu Asp Gly Ser Val Val
   450 455 460
   Val Ala Arg Thr Ala Ala Leu Pro Asn Gly Glu Gly Phe Asn Ala Ala
   465 470 475 480
   Thr Leu Glu Tyr Gly Asn Leu Ile Asn Asp Gly Val Ile Asp Pro Val
   485 490 495
   Lys Val Thr His Ser Ala Val Val Asn Ala Thr Ser Val Ala Arg Met
   500 505 510
   Val Leu Thr Thr Glu Ala Ser Val Val Glu Lys Pro Ala Glu Glu Ala
   515 520 525
   Ala Asp Ala His Ala Gly His His His His
   530 535
<210> 15
   <211> 652
   <212> DNA
   <213> Corynebacterium glutamicum
<220>
   <221> CDS
   <222> (101)..(622)
   <223> RXA00588
<400> 15
   tcatacatct tggccccgga aaaccggggc caatcttatg gctcaagtcg ctagttagcc 60
   gatgatccac ctctactgtt ccccaggagg gtaagtaatt atg gca agt gta gat   115
                                               Met Ala Ser Val Asp
                                                1               5
   aag caa tac atc acc cca gaa acc aag gcc aag ctg gag gaa gag ctc   163
   Lys Gln Tyr Ile Thr Pro Glu Thr Lys Ala Lys Leu Glu Glu Glu Leu
                   10                  15                  20
   aac gcc ctc atc gca cac cgc cct gca gtt gct gcg gaa atc aat gag   211
   Asn Ala Leu Ile Ala His Arg Pro Ala Val Ala Ala Glu Ile Asn Glu
               25                  30                  35
   cgc cgt gaa gaa ggc gac ctc aag gaa aac gct ggc tat gac gcc gct   259
   Arg Arg Glu Glu Gly Asp Leu Lys Glu Asn Ala Gly Tyr Asp Ala Ala
           40                  45                  50
   cgt gaa atg cag gac cag gaa gag gcc cgc atc aag cag atc tct gag   307
   Arg Glu Met Gln Asp Gln Glu Glu Ala Arg Ile Lys Gln Ile Ser Glu
       55                  60                  65
   ctg ctg gcc aac tcc acc act gag cgc gaa ggc atc atc gaa ggt gtc   355
   Leu Leu Ala Asn Ser Thr Thr Glu Arg Glu Gly Ile Ile Glu Gly Val
    70                  75                  80                  85
   gca aac gtt ggc tcc gtt gtt cac gtc tac tac gac ggc gac gag aac   403
   Ala Asn Val Gly Ser Val Val His Val Tyr Tyr Asp Gly Asp Glu Asn
                   90                  95                  100
   gac aag gaa acc ttc ctc atc ggt acc cgt gct ggc gct tcc gag aac   451
   Asp Lys Glu Thr Phe Leu Ile Gly Thr Arg Ala Gly Ala Ser Glu Asn
               105                 110                 115
   cca gat ctt gag acc tac tct gag cag tcc cca ctc ggc gct gca att   499
   Pro Asp Leu Glu Thr Tyr Ser Glu Gln Ser Pro Leu Gly Ala Ala Ile
           120                 125                 130
   ctc gga gct cag gaa ggc gac acc cgt cag tac acc gct cca aat ggt   547
   Leu Gly Ala Gln Glu Gly Asp Thr Arg Gln Tyr Thr Ala Pro Asn Gly
       135                 140                 145
   tcc gtt atc tcc gta act gtt gtt tct gca gaa cca tac aac tca gca   595
   Ser Val Ile Ser Val Thr Val Val Ser Ala Glu Pro Tyr Asn Ser Ala
   150                 155                 160                 165
   aaa gcc gcg aca ctc cgc ggc aaa aac taaccaagga tttaaaagtc         642
   Lys Ala Ala Thr Leu Arg Gly Lys Asn
                   170
   ttcaaaatga                                                        652
<210> 16
   <211> 174
   <212> PRT
   <213> Corynebacterium glutamicum
<400> 16
   Met Ala Ser Val Asp Lys Gln Tyr Ile Thr Pro Glu Thr Lys Ala Lys
   1               5                   10                  15
   Leu Glu Glu Glu Leu Asn Ala Leu Ile Ala His Arg Pro Ala Val Ala
               20                  25                  30
   Ala Glu Ile Asn Glu Arg Arg Glu Glu Gly Asp Leu Lys Glu Asn Ala
           35                  40                  45
   Gly Tyr Asp Ala Ala Arg Glu Met Gln Asp Gln Glu Glu Ala Arg Ile
       50                  55                  60
   Lys Gln Ile Ser Glu Leu Leu Ala Asn Ser Thr Thr Glu Arg Glu Gly
   65                  70                  75                  80
   Ile Ile Glu Gly Val Ala Asn Val Gly Ser Val Val His Val Tyr Tyr
                   85                  90                  95
   Asp Gly Asp Glu Asn Asp Lys Glu Thr Phe Leu Ile Gly Thr Arg Ala
               100                 105                 110
   Gly Ala Ser Glu Asn Pro Asp Leu Glu Thr Tyr Ser Glu Gln Ser Pro
           115                 120                 125
   Leu Gly Ala Ala Ile Leu Gly Ala Gln Glu Gly Asp Thr Arg Gln Tyr
       130                 135                 140
   Thr Ala Pro Asn Gly Ser Val Ile Ser Val Thr Val Val Ser Ala Glu
   145                 150                 155                 160
   Pro Tyr Asn Ser Ala Lys Ala Ala Thr Leu Arg Gly Lys Asn
                   165                 170
<210> 17
   <211> 1012
   <212> DNA
   <213> Corynebacterium glutamicum
<220>
   <221> CDS
   <222> (101)..(982)
   <223> RXA00669
<400> 17
   tttactgcgg gcattttacg tatctgcacc ccgcccggct gcgctgagca gccgtaaagc 60
   gtggggcgtg acgtcgaaaa gcaaaaaatg aaaggcagac atg gac aat tca acg   115
                                               Met Asp Asn Ser Thr
                                                1               5
   gtg cga atc cgg ctg gat cta gcg tat gac ggc acg gat ttt cat ggc   163
   Val Arg Ile Arg Leu Asp Leu Ala Tyr Asp Gly Thr Asp Phe His Gly
                   10                  15                  20
   tgg gcg aag cag ggg acc agc gat cta cgc acc gtg caa aaa gtg ttg   211
   Trp Ala Lys Gln Gly Thr Ser Asp Leu Arg Thr Val Gln Lys Val Leu
               25                  30                  35
   gaa gac aat ttg agc atg gtg ctg cgt gag act gtt gaa ttg act gtg   259
   Glu Asp Asn Leu Ser Met Val Leu Arg Glu Thr Val Glu Leu Thr Val
           40                  45                  50
   gcc ggg cga acc gat gcg ggg gtg cat gcg gcg ggc cag gtg gcg cac   307
   Ala Gly Arg Thr Asp Ala Gly Val His Ala Ala Gly Gln Val Ala His
       55                  60                  65
   ttt gat att ccg gca cac gct tta gag cag cgc agt att gat ggc gat   355
   Phe Asp Ile Pro Ala His Ala Leu Glu Gln Arg Ser Ile Asp Gly Asp
   70                  75                  80                  85
   cca agc aag ttg gtt cgg cgc ttg ggt cgg ttg ctg ccc gat gat att   403
   Pro Ser Lys Leu Val Arg Arg Leu Gly Arg Leu Leu Pro Asp Asp Ile
                   90                  95                  100
   cgg gtg cat ggc gta cgt ttt gcc gag ccc ggg ttt gat gcg cga ttt   451
   Arg Val His Gly Val Arg Phe Ala Glu Pro Gly Phe Asp Ala Arg Phe
               105                 110                 115
   tcc gcg atg cgc agg cac tac gtt tat cgc att acg acg cat ccc gcc   499
   Ser Ala Met Arg Arg His Tyr Val Tyr Arg Ile Thr Thr His Pro Ala
           120                 125                 130
   ggc gcg ctg cct acg cgc cgc cac gac acg gcg cag tgg cca aaa cct    547
   Gly Ala Leu Pro Thr Arg Arg His Asp Thr Ala Gln Trp Pro Lys Pro
       135                 140                 145
   gtc gaa cta gag cgg atg caa tta gcc gcc gat gca ctg ctg ggg ctg    595
   Val Glu Leu Glu Arg Met Gln Leu Ala Ala Asp Ala Leu Leu Gly Leu
   150                 155                 160                 165
   cat gat ttt gtg gcg ttt tgc aaa gct aag cca cat gcg acg acg gtg    643
   His Asp Phe Val Ala Phe Cys Lys Ala Lys Pro His Ala Thr Thr Val
                   170                 175                 180
   cgt gaa cta caa aaa ttt gcg tgg aaa gac gtc tcc act gac atc gaa    691
   Arg Glu Leu Gln Lys Phe Ala Trp Lys Asp Val Ser Thr Asp Ile Glu
               185                 190                 195
   ccg cag gtg tat gaa gca cac gtg gtg gcc gat gct ttt tgc tgg tcg    739
   Pro Gln Val Tyr Glu Ala His Val Val Ala Asp Ala Phe Cys Trp Ser
           200                 205                 210
   atg gtg cgc tcg ctg gtc ggc tcc tgc atg gcc gtg ggc gaa gga cgc    787
   Met Val Arg Ser Leu Val Gly Ser Cys Met Ala Val Gly Glu Gly Arg
       215                 220                 225
   cgc gga tca ggg ttt act gca gaa ttg ctt gat gca agc gaa cgc agc    835
   Arg Gly Ser Gly Phe Thr Ala Glu Leu Leu Asp Ala Ser Glu Arg Ser
   230                 235                 240                 245
   ccc atg gtt cca gta gca cct gcg aaa ggt ttg agc ttg gtt ggc gtg    883
   Pro Met Val Pro Val Ala Pro Ala Lys Gly Leu Ser Leu Val Gly Val
                   250                 255                 260
   gat tat cct tcc gct gat aag tta cag gaa aga gcg ctg gaa acc cga    931
   Asp Tyr Pro Ser Ala Asp Lys Leu Gln Glu Arg Ala Leu Glu Thr Arg
               265                 270                 275
   gct gtt cgc gag ttt ccg gac gcg tcc gcg agc cta aaa cta gat gat    979
   Ala Val Arg Glu Phe Pro Asp Ala Ser Ala Ser Leu Lys Leu Asp Asp
           280                 285                 290
   gag taaaagggac taaactcgtc tctcgtatct                              1012
   Glu
<210> 18
   <211> 294
   <212> PRT
   <213> Corynebacterium glutamicum
<400> 18
   Met Asp Asn Ser Thr Val Arg Ile Arg Leu Asp Leu Ala Tyr Asp Gly
   1               5                   10                  15
   Thr Asp Phe His Gly Trp Ala Lys Gln Gly Thr Ser Asp Leu Arg Thr
               20                  25                  30
   Val Gln Lys Val Leu Glu Asp Asn Leu Ser Met Val Leu Arg Glu Thr
           35                  40                  45
   Val Glu Leu Thr Val Ala Gly Arg Thr Asp Ala Gly Val His Ala Ala
       50                  55                  60
   Gly Gln Val Ala His Phe Asp Ile Pro Ala His Ala Leu Glu Gln Arg
   65                  70                  75                  80
   Ser Ile Asp Gly Asp Pro Ser Lys Leu Val Arg Arg Leu Gly Arg Leu
                   85                  90                  95
   Leu Pro Asp Asp Ile Arg Val His Gly Val Arg Phe Ala Glu Pro Gly
               100                 105                 110
   Phe Asp Ala Arg Phe Ser Ala Met Arg Arg His Tyr Val Tyr Arg Ile
           115                 120                 125
   Thr Thr His Pro Ala Gly Ala Leu Pro Thr Arg Arg His Asp Thr Ala
       130                 135                 140
   Gln Trp Pro Lys Pro Val Glu Leu Glu Arg Met Gln Leu Ala Ala Asp
   145                 150                 155                 160
   Ala Leu Leu Gly Leu His Asp Phe Val Ala Phe Cys Lys Ala Lys Pro
                   165                 170                 175
   His Ala Thr Thr Val Arg Glu Leu Gln Lys Phe Ala Trp Lys Asp Val
               180                 185                 190
   Ser Thr Asp Ile Glu Pro Gln Val Tyr Glu Ala His Val Val Ala Asp
           195                 200                 205
   Ala Phe Cys Trp Ser Met Val Arg Ser Leu Val Gly Ser Cys Met Ala
       210                 215                 220
   Val Gly Glu Gly Arg Arg Gly Ser Gly Phe Thr Ala Glu Leu Leu Asp
   225                 230                 235                 240
   Ala Ser Glu Arg Ser Pro Met Val Pro Val Ala Pro Ala Lys Gly Leu
                   245                 250                 255
   Ser Leu Val Gly Val Asp Tyr Pro Ser Ala Asp Lys Leu Gln Glu Arg
               260                 265                 270
   Ala Leu Glu Thr Arg Ala Val Arg Glu Phe Pro Asp Ala Ser Ala Ser
           275                 280                 285
   Leu Lys Leu Asp Asp Glu
       290
<210> 19
   <211> 3022
   <212> DNA
   <213> Corynebacterium glutamicum
<220>
   <221> CDS
   <222> (101)..(2992)
   <223> RXA01061
<400> 19
   gcagacaaga ctgagcagtc cgacggcgat aagcagtggt ttccacataa ttcttcaagt 60
   ctatctactt attgagggga ggaagaattg ccctccacac atg aga tgt ccc gtg   115
                                             Met Arg Cys Pro Val
                                                 1               5
   tac cta cta cac tgt tta acc atg act aac ccg agc gaa ggc acc act   163
   Tyr Leu Leu His Cys Leu Thr Met Thr Asn Pro Ser Glu Gly Thr Thr
                   10                  15                  20
   ccc ctg gcg ttc cgt tat acc ccg gaa ctc gcc aac aag atc gaa ggt   211
   Pro Leu Ala Phe Arg Tyr Thr Pro Glu Leu Ala Asn Lys Ile Glu Gly
               25                  30                  35
   gag tgg cag aat tac tgg act gac aac ggc aca ttc aac gca ccc aac   259
   Glu Trp Gln Asn Tyr Trp Thr Asp Asn Gly Thr Phe Asn Ala Pro Asn
           40                  45                  50
   cca gtg ggt gat tta gcg cct gcg gac ggt aaa gca ctt cct gag gac   307
   Pro Val Gly Asp Leu Ala Pro Ala Asp Gly Lys Ala Leu Pro Glu Asp
       55                  60                  65
   aag ctc ttt gtc cag gat atg ttc ccg tac cca tcc gga gct ggc ctg   355
   Lys Leu Phe Val Gln Asp Met Phe Pro Tyr Pro Ser Gly Ala Gly Leu
   70                  75                  80                  85
   cac gta ggc cac cca ctc ggt tac atc gca acg gat gtt ttc gcc cgc   403
   His Val Gly His Pro Leu Gly Tyr Ile Ala Thr Asp Val Phe Ala Arg
                   90                  95                  100
   tac aac cgc atg ctg ggc aag aac gtt ctg cac acc ttg ggc tat gac   451
   Tyr Asn Arg Met Leu Gly Lys Asn Val Leu His Thr Leu Gly Tyr Asp
               105                 110                 115
   gcc ttc gga ctg cca gca gag cag tac gcg atc caa acc ggt aca cac   499
   Ala Phe Gly Leu Pro Ala Glu Gln Tyr Ala Ile Gln Thr Gly Thr His
           120                 125                 130
   cca cgc acc acc acc atg gcc aac att gag aac atg aag cgc cag ctc   547
   Pro Arg Thr Thr Thr Met Ala Asn Ile Glu Asn Met Lys Arg Gln Leu
       135                 140                 145
   ggt gcg ctg ggt ctt ggc cat gat tcc cgt cgt gcg gtg gcc acc acg   595
   Gly Ala Leu Gly Leu Gly His Asp Ser Arg Arg Ala Val Ala Thr Thr
   150                 155                 160                 165
   gat cct gag ttc tac aag tgg act cag tgg atc ttc ctg cag att ttc   643
   Asp Pro Glu Phe Tyr Lys Trp Thr Gln Trp Ile Phe Leu Gln Ile Phe
                   170                 175                 180
   aat tcg tgg ttc gat gca gag cag cag aag gca cgt ccc atc agt gag   691
   Asn Ser Trp Phe Asp Ala Glu Gln Gln Lys Ala Arg Pro Ile Ser Glu
               185                 190                 195
   ctg att ccg ttg ctg gag tcc ggc gag ctg aag act aag gac ggg gcg   739
   Leu Ile Pro Leu Leu Glu Ser Gly Glu Leu Lys Thr Lys Asp Gly Ala
           200                 205                 210
   gat tac aac gcg ctg gga gac gtc gaa aag caa aaa gcg gtg gat gac   787
   Asp Tyr Asn Ala Leu Gly Asp Val Glu Lys Gln Lys Ala Val Asp Asp
       215                 220                 225
   tac cgc ctt gtt tat cgc tcg aac tcc acc gtg aac tgg tgc cca ggc   835
   Tyr Arg Leu Val Tyr Arg Ser Asn Ser Thr Val Asn Trp Cys Pro Gly
   230                 235                 240                 245
   ttg ggc acc gtg ttg gca aac gag gaa gtg acc gcg gac ggc cgt tcc   883
   Leu Gly Thr Val Leu Ala Asn Glu Glu Val Thr Ala Asp Gly Arg Ser
                   250                 255                 260
   gag cgt ggc aat ttc cct gtt ttc cgt aag aat ttg tcc cag tgg atg     931
   Glu Arg Gly Asn Phe Pro Val Phe Arg Lys Asn Leu Ser Gln Trp Met
               265                 270                 275
   atg cgc att acc gcg tac tcg gat cgt ctg atc gat gat ctg gag ctg     979
   Met Arg Ile Thr Ala Tyr Ser Asp Arg Leu Ile Asp Asp Leu Glu Leu
           280                 285                 290
   ctc gat tgg act gag aag gtc aag tcc atg cag cgt aac tgg att ggc    1027
   Leu Asp Trp Thr Glu Lys Val Lys Ser Met Gln Arg Asn Trp Ile Gly
       295                 300                 305
   cgt tcc cgc ggc gct gaa gtt gat ttc agt gca gag ggc gaa acc gtc    1075
   Arg Ser Arg Gly Ala Glu Val Asp Phe Ser Ala Glu Gly Glu Thr Val
   310                 315                 320                 325
   acc gtg ttt acc acc cgc cca gat act ctg ttc ggc gcg acc tac atg    1123
   Thr Val Phe Thr Thr Arg Pro Asp Thr Leu Phe Gly Ala Thr Tyr Met
                   330                 335                 340
   gtt ctt gca cct gag cat gag ctg gtc gac gtg ctg ctg gag aag gct    1171
   Val Leu Ala Pro Glu His Glu Leu Val Asp Val Leu Leu Glu Lys Ala
               345                 350                 355
   ggt tcc tac gag ggc gtt gat gcc cgt tgg acc aat ggc cag gcg agc    1219
   Gly Ser Tyr Glu Gly Val Asp Ala Arg Trp Thr Asn Gly Gln Ala Ser
           360                 365                 370
   cct gcg gaa gct gtc gct gca tac cgc gcc tcc atc gcc gcg aag tcc    1267
   Pro Ala Glu Ala Val Ala Ala Tyr Arg Ala Ser Ile Ala Ala Lys Ser
       375                 380                 385
   gac ctg gag cgt cag gaa aac aag gaa aag acc ggc gtc ttc ctg ggc    1315
   Asp Leu Glu Arg Gln Glu Asn Lys Glu Lys Thr Gly Val Phe Leu Gly
   390                 395                 400                 405
   gtt tac gcg acc aac cca gtc aac ggc gat cag atc aca gtg ttc atc    1363
   Val Tyr Ala Thr Asn Pro Val Asn Gly Asp Gln Ile Thr Val Phe Ile
                   410                 415                 420
   gct gac tac gtt ctg acc ggc tac ggc acc ggc gcc atc atg gcg gtt    1411
   Ala Asp Tyr Val Leu Thr Gly Tyr Gly Thr Gly Ala Ile Met Ala Val
               425                 430                 435
   cct gct cac gac gag cgc gac tac gaa ttc gcc acc gtt ttg ggt ctg    1459
   Pro Ala His Asp Glu Arg Asp Tyr Glu Phe Ala Thr Val Leu Gly Leu
           440                 445                 450
   cct atc aag gaa gtt gtc gca ggt ggc aac atc gaa gag gct gct ttc    1507
   Pro Ile Lys Glu Val Val Ala Gly Gly Asn Ile Glu Glu Ala Ala Phe
       455                 460                 465
   acc gaa tct ggc gaa gca gtc aac tct gcg aac gac aac ggc ctg gat    1555
   Thr Glu Ser Gly Glu Ala Val Asn Ser Ala Asn Asp Asn Gly Leu Asp
   470                 475                 480                 485
   atc aac ggc ctt gcc aag gat gag gct att gcc aag acc atc gaa tgg    1603
   Ile Asn Gly Leu Ala Lys Asp Glu Ala Ile Ala Lys Thr Ile Glu Trp
                   490                 495                 500
   ttg gaa gaa aag gaa ctt ggc cgc ggc acc atc cag tac aag ctg cgc      1651
   Leu Glu Glu Lys Glu Leu Gly Arg Gly Thr Ile Gln Tyr Lys Leu Arg
               505                 510                 515
   gac tgg ctg ttc gct cgc cag cgt tac tgg ggc gag cct ttc cca atc      1699
   Asp Trp Leu Phe Ala Arg Gln Arg Tyr Trp Gly Glu Pro Phe Pro Ile
           520                 525                 530
   gtc tac gac gaa aac ggc caa gca cat gct ctg cca gac tcc atg ctt      1747
   Val Tyr Asp Glu Asn Gly Gln Ala His Ala Leu Pro Asp Ser Met Leu
       535                 540                 545
   cca gtc gag ctg cca gag gta gag gac tac aag cct gtc tcc ttc gac      1795
   Pro Val Glu Leu Pro Glu Val Glu Asp Tyr Lys Pro Val Ser Phe Asp
   550                 555                 560                 565
   cct gaa gac gca gac tcc gag cct tcc cca cca ctg gct aag gcc cgc      1843
   Pro Glu Asp Ala Asp Ser Glu Pro Ser Pro Pro Leu Ala Lys Ala Arg
                   570                 575                 580
   gaa tgg gtt gag gtg gaa ctc gat ctc ggc gat ggc aag aag aag tac      1891
   Glu Trp Val Glu Val Glu Leu Asp Leu Gly Asp Gly Lys Lys Lys Tyr
               585                 590                 595
   acc cgc gac acc aac gtc atg cca cag tgg gca ggt tcc tcc tgg tac      1939
   Thr Arg Asp Thr Asn Val Met Pro Gln Trp Ala Gly Ser Ser Trp Tyr
           600                 605                 610
   cag ctg cgc tac gtc gat cca agc aac gat gag cag ttc tgc aac atc      1987
   Gln Leu Arg Tyr Val Asp Pro Ser Asn Asp Glu Gln Phe Cys Asn Ile
       615                 620                 625
   gaa aat gaa cgc tac tgg acc ggc cca cgc cca gaa acc cac gga cca      2035
   Glu Asn Glu Arg Tyr Trp Thr Gly Pro Arg Pro Glu Thr His Gly Pro
   630                 635                 640                 645
   aac gat cca ggc ggc gta gac ctc tac gtc ggt ggc gtc gag cac gca      2083
   Asn Asp Pro Gly Gly Val Asp Leu Tyr Val Gly Gly Val Glu His Ala
                   650                 655                 660
   gtt ctc cac ctg ctc tac gca cgt ttc tgg cac aag gtc ctc ttc gac      2131
   Val Leu His Leu Leu Tyr Ala Arg Phe Trp His Lys Val Leu Phe Asp
               665                 670                 675
   ctg ggc cac gtc tcc tcc aag gag cca tac cgt cgc ctg tac aac cag      2179
   Leu Gly His Val Ser Ser Lys Glu Pro Tyr Arg Arg Leu Tyr Asn Gln
           680                 685                 690
   ggc tac atc cag gcc ttc gcc tac acc gat tcc cgt ggc gtc tac gtg      2227
   Gly Tyr Ile Gln Ala Phe Ala Tyr Thr Asp Ser Arg Gly Val Tyr Val
       695                 700                 705
   cct gcc gat gat gtc gaa gag aag gac gga aag ttc ttc tac cag ggc      2275
   Pro Ala Asp Asp Val Glu Glu Lys Asp Gly Lys Phe Phe Tyr Gln Gly
   710                 715                 720                 725
   gaa gaa gtc aac cag gaa tac gga aag atg ggc aag tcc ctg aag aac      2323
   Glu Glu Val Asn Gln Glu Tyr Gly Lys Met Gly Lys Ser Leu Lys Asn
                   730                 735                 740
   gcc gtt gcc cca gac gat atc tgc aac aac ttc ggt gct gac acc ctg      2371
   Ala Val Ala Pro Asp Asp Ile Cys Asn Asn Phe Gly Ala Asp Thr Leu
               745                 750                 755
   cgc gtt tac gag atg gcc atg gga cct ttg gac acc tcc cgt cca tgg     2419
   Arg Val Tyr Glu Met Ala Met Gly Pro Leu Asp Thr Ser Arg Pro Trp
           760                 765                 770
   gca acc aag gac gtc gtc ggt gcg cag cgc ttc ctc cag cgt ctg tgg     2467
   Ala Thr Lys Asp Val Val Gly Ala Gln Arg Phe Leu Gln Arg Leu Trp
       775                 780                 785
   cgt ctc gtc gtc gat gaa aac acc ggc gaa gtg ctc act cgc gat gaa     2515
   Arg Leu Val Val Asp Glu Asn Thr Gly Glu Val Leu Thr Arg Asp Glu
   790                 795                 800                 805
   gtc ctc acc gac gat gac aac aag caa ctg cac cgc acc atc gca ggc     2563
   Val Leu Thr Asp Asp Asp Asn Lys Gln Leu His Arg Thr Ile Ala Gly
                   810                 815                 820
   gtc cgc gac gac tac acc aac ttg cgc gtt aac acc gtg gtt gcc aag     2611
   Val Arg Asp Asp Tyr Thr Asn Leu Arg Val Asn Thr Val Val Ala Lys
               825                 830                 835
   ctc atc gaa tac gtc aac tac ctg acc aaa aca tac cca gac acc atc     2659
   Leu Ile Glu Tyr Val Asn Tyr Leu Thr Lys Thr Tyr Pro Asp Thr Ile
           840                 845                 850
   cca gct ggc gca gtc ctg cca ctg atc gtc atg gtc tcc cct atc gca     2707
   Pro Ala Gly Ala Val Leu Pro Leu Ile Val Met Val Ser Pro Ile Ala
       855                 860                 865
   cca cac atc gcg gag gaa ctc tgg aag aag ctc ggc cac gac gac acc     2755
   Pro His Ile Ala Glu Glu Leu Trp Lys Lys Leu Gly His Asp Asp Thr
   870                 875                 880                 885
   gtc acc tac gaa cca ttc ccc acc ttt gag gaa aaa tgg ctc acc gac     2803
   Val Thr Tyr Glu Pro Phe Pro Thr Phe Glu Glu Lys Trp Leu Thr Asp
                   890                 895                 900
   gat gaa atc gaa ctg cca gtc cag gtc aac ggc aag gtc cgc ggt cgc     2851
   Asp Glu Ile Glu Leu Pro Val Gln Val Asn Gly Lys Val Arg Gly Arg
               905                 910                 915
   atc acc gtt gca gcc gac gcc agc cag gag cag gtc atc gag gca gcg     2899
   Ile Thr Val Ala Ala Asp Ala Ser Gln Glu Gln Val Ile Glu Ala Ala
           920                 925                 930
   ctt gcc gac gag aag gtg cag gag caa atc tcc ggc aag aac ctg atc     2947
   Leu Ala Asp Glu Lys Val Gln Glu Gln Ile Ser Gly Lys Asn Leu Ile
       935                 940                 945
   aag cag atc gtt gtt cca gga cgc atg gtt aac ctt gtg gtg aag         2992
   Lys Gln Ile Val Val Pro Gly Arg Met Val Asn Leu Val Val Lys
   950                 955                 960
   taatccccct cggtttagat tcccctagaa                                    3022
<210> 20
   <211> 964
   <212> PRT
   <213> Corynebacterium glutamicum
<400> 20
   Met Arg Cys Pro Val Tyr Leu Leu His Cys Leu Thr Met Thr Asn Pro
   1               5                   10                  15
   Ser Glu Gly Thr Thr Pro Leu Ala Phe Arg Tyr Thr Pro Glu Leu Ala
               20                  25                  30
   Asn Lys Ile Glu Gly Glu Trp Gln Asn Tyr Trp Thr Asp Asn Gly Thr
           35                  40                  45
   Phe Asn Ala Pro Asn Pro Val Gly Asp Leu Ala Pro Ala Asp Gly Lys
       50                  55                  60
   Ala Leu Pro Glu Asp Lys Leu Phe Val Gln Asp Met Phe Pro Tyr Pro
   65                  70                  75                  80
   Ser Gly Ala Gly Leu His Val Gly His Pro Leu Gly Tyr Ile Ala Thr
                   85                  90                  95
   Asp Val Phe Ala Arg Tyr Asn Arg Met Leu Gly Lys Asn Val Leu His
               100                 105                 110
   Thr Leu Gly Tyr Asp Ala Phe Gly Leu Pro Ala Glu Gln Tyr Ala Ile
           115                 120                 125
   Gln Thr Gly Thr His Pro Arg Thr Thr Thr Met Ala Asn Ile Glu Asn
       130                 135                 140
   Met Lys Arg Gln Leu Gly Ala Leu Gly Leu Gly His Asp Ser Arg Arg
   145                 150                 155                 160
   Ala Val Ala Thr Thr Asp Pro Glu Phe Tyr Lys Trp Thr Gln Trp Ile
                   165                 170                 175
   Phe Leu Gln Ile Phe Asn Ser Trp Phe Asp Ala Glu Gln Gln Lys Ala
               180                 185                 190
   Arg Pro Ile Ser Glu Leu Ile Pro Leu Leu Glu Ser Gly Glu Leu Lys
           195                 200                 205
   Thr Lys Asp Gly Ala Asp Tyr Asn Ala Leu Gly Asp Val Glu Lys Gln
       210                 215                 220
   Lys Ala Val Asp Asp Tyr Arg Leu Val Tyr Arg Ser Asn Ser Thr Val
   225                 230                 235                 240
   Asn Trp Cys Pro Gly Leu Gly Thr Val Leu Ala Asn Glu Glu Val Thr
                   245                 250                 255
   Ala Asp Gly Arg Ser Glu Arg Gly Asn Phe Pro Val Phe Arg Lys Asn
               260                 265                 270
   Leu Ser Gln Trp Met Met Arg Ile Thr Ala Tyr Ser Asp Arg Leu Ile
           275                 280                 285
   Asp Asp Leu Glu Leu Leu Asp Trp Thr Glu Lys Val Lys Ser Met Gln
       290                 295                 300
   Arg Asn Trp Ile Gly Arg Ser Arg Gly Ala Glu Val Asp Phe Ser Ala
   305                 310                 315                 320
   Glu Gly Glu Thr Val Thr Val Phe Thr Thr Arg Pro Asp Thr Leu Phe
                   325                 330                 335
   Gly Ala Thr Tyr Met Val Leu Ala Pro Glu His Glu Leu Val Asp Val
               340                 345                 350
   Leu Leu Glu Lys Ala Gly Ser Tyr Glu Gly Val Asp Ala Arg Trp Thr
   355 360 365
   Asn Gly Gln Ala Ser Pro Ala Glu Ala Val Ala Ala Tyr Arg Ala Ser
   370 375 380
   Ile Ala Ala Lys Ser Asp Leu Glu Arg Gln Glu Asn Lys Glu Lys Thr
   385 390 395 400
   Gly Val Phe Leu Gly Val Tyr Ala Thr Asn Pro Val Asn Gly Asp Gln
   405 410 415
   Ile Thr Val Phe Ile Ala Asp Tyr Val Leu Thr Gly Tyr Gly Thr Gly
   420 425 430
   Ala Ile Met Ala Val Pro Ala His Asp Glu Arg Asp Tyr Glu Phe Ala
   435 440 445
   Thr Val Leu Gly Leu Pro Ile Lys Glu Val Val Ala Gly Gly Asn Ile
   450 455 460
   Glu Glu Ala Ala Phe Thr Glu Ser Gly Glu Ala Val Asn Ser Ala Asn
   465 470 475 480
   Asp Asn Gly Leu Asp Ile Asn Gly Leu Ala Lys Asp Glu Ala Ile Ala
   485 490 495
   Lys Thr Ile Glu Trp Leu Glu Glu Lys Glu Leu Gly Arg Gly Thr Ile
   500 505 510
   Gln Tyr Lys Leu Arg Asp Trp Leu Phe Ala Arg Gln Arg Tyr Trp Gly
   515 520 525
   Glu Pro Phe Pro Ile Val Tyr Asp Glu Asn Gly Gln Ala His Ala Leu
   530 535 540
   Pro Asp Ser Met Leu Pro Val Glu Leu Pro Glu Val Glu Asp Tyr Lys
   545 550 555 560
   Pro Val Ser Phe Asp Pro Glu Asp Ala Asp Ser Glu Pro Ser Pro Pro
   565 570 575
   Leu Ala Lys Ala Arg Glu Trp Val Glu Val Glu Leu Asp Leu Gly Asp
   580 585 590
   Gly Lys Lys Lys Tyr Thr Arg Asp Thr Asn Val Met Pro Gln Trp Ala
   595 600 605
   Gly Ser Ser Trp Tyr Gln Leu Arg Tyr Val Asp Pro Ser Asn Asp Glu
   610 615 620
   Gln Phe Cys Asn Ile Glu Asn Glu Arg Tyr Trp Thr Gly Pro Arg Pro
   625 630 635 640
   Glu Thr His Gly Pro Asn Asp Pro Gly Gly Val Asp Leu Tyr Val Gly
   645 650 655
   Gly Val Glu His Ala Val Leu His Leu Leu Tyr Ala Arg Phe Trp His
   660 665 670
   Lys Val Leu Phe Asp Leu Gly His Val Ser Ser Lys Glu Pro Tyr Arg
   675 680 685
   Arg Leu Tyr Asn Gln Gly Tyr Ile Gln Ala Phe Ala Tyr Thr Asp Ser
           690                 695                 700
   Arg Gly Val Tyr Val Pro Ala Asp Asp Val Glu Glu Lys Asp Gly Lys
   705                 710                 715                 720
   Phe Phe Tyr Gln Gly Glu Glu Val Asn Gln Glu Tyr Gly Lys Met Gly
                   725                 730                 735
   Lys Ser Leu Lys Asn Ala Val Ala Pro Asp Asp Ile Cys Asn Asn Phe
               740                 745                 750
   Gly Ala Asp Thr Leu Arg Val Tyr Glu Met Ala Met Gly Pro Leu Asp
           755                 760                 765
   Thr Ser Arg Pro Trp Ala Thr Lys Asp Val Val Gly Ala Gln Arg Phe
       770                 775                 780
   Leu Gln Arg Leu Trp Arg Leu Val Val Asp Glu Asn Thr Gly Glu Val
   785                 790                 795                 800
   Leu Thr Arg Asp Glu Val Leu Thr Asp Asp Asp Asn Lys Gln Leu His
                   805                 810                 815
   Arg Thr Ile Ala Gly Val Arg Asp Asp Tyr Thr Asn Leu Arg Val Asn
               820                 825                 830
   Thr Val Val Ala Lys Leu Ile Glu Tyr Val Asn Tyr Leu Thr Lys Thr
           835                 840                 845
   Tyr Pro Asp Thr Ile Pro Ala Gly Ala Val Leu Pro Leu Ile Val Met
       850                 855                 860
   Val Ser Pro Ile Ala Pro His Ile Ala Glu Glu Leu Trp Lys Lys Leu
   865                 870                 875                 880
   Gly His Asp Asp Thr Val Thr Tyr Glu Pro Phe Pro Thr Phe Glu Glu
                   885                 890                 895
   Lys Trp Leu Thr Asp Asp Glu Ile Glu Leu Pro Val Gln Val Asn Gly
               900                 905                 910
   Lys Val Arg Gly Arg Ile Thr Val Ala Ala Asp Ala Ser Gln Glu Gln
           915                 920                 925
   Val Ile Glu Ala Ala Leu Ala Asp Glu Lys Val Gln Glu Gln Ile Ser
       930                 935                 940
   Gly Lys Asn Leu Ile Lys Gln Ile Val Val Pro Gly Arg Met Val Asn
   945                 950                 955                 960
   Leu Val Val Lys
<210> 21
   <211> 2248
   <212> DNA
   <213> Corynebacterium glutamicum
<220>
   <221> CDS
   <222> (101)..(2218)
   <223> RXA01277
<400> 21
   gaccagccga atctacattc cttattctgc tggcgttaca attcagggcc aaacccgtat 60
   gatgaaaaag acaccgggga aatcggagtg cgcgtagatt ttg aaa acg gcc ggt   115
                                               Leu Lys Thr Ala Gly
                                                1               5
   act act cgg ttc acg ttt acg tcg gct gat cca att gga ggc gcc ctc   163
   Thr Thr Arg Phe Thr Phe Thr Ser Ala Asp Pro Ile Gly Gly Ala Leu
                   10                  15                  20
   gga agc cgc ctt aaa aaa cct gcc ggt caa aag atc act aac ctg aac   211
   Gly Ser Arg Leu Lys Lys Pro Ala Gly Gln Lys Ile Thr Asn Leu Asn
               25                  30                  35
   ttc atg act gat tac acg ttc ctc gaa gac att gac acc ccg gaa gcg   259
   Phe Met Thr Asp Tyr Thr Phe Leu Glu Asp Ile Asp Thr Pro Glu Ala
           40                  45                  50
   ctc gcg tgg gcg gaa aaa tgg tcg ggg gaa agc gtc gaa aag cta aaa   307
   Leu Ala Trp Ala Glu Lys Trp Ser Gly Glu Ser Val Glu Lys Leu Lys
       55                  60                  65
   agc cca gcc aag gac gcc ctg gaa gcc agg ctg ctg gct gcg ttg gac   355
   Ser Pro Ala Lys Asp Ala Leu Glu Ala Arg Leu Leu Ala Ala Leu Asp
   70                  75                  80                  85
   acc gat gat cgc att gcc tac gtg agc cgg cgc ggt gag aag ctg tac   403
   Thr Asp Asp Arg Ile Ala Tyr Val Ser Arg Arg Gly Glu Lys Leu Tyr
                   90                  95                  100
   aac ttt tgg cgg gac gcg cag cat ccg cgt gga gtg tgg cgc acg acc   451
   Asn Phe Trp Arg Asp Ala Gln His Pro Arg Gly Val Trp Arg Thr Thr
               105                 110                 115
   acg ttg gag tcg tat gaa agt gac cag ccg gag tgg gac gtg ctc att   499
   Thr Leu Glu Ser Tyr Glu Ser Asp Gln Pro Glu Trp Asp Val Leu Ile
           120                 125                 130
   gat gtg gat gcg ttg gcg gag gat gag ggc gaa aac tgg gta tgg aag   547
   Asp Val Asp Ala Leu Ala Glu Asp Glu Gly Glu Asn Trp Val Trp Lys
       135                 140                 145
   ggc gcg gtt gtg cgc tcg ccg gag ttt gat cgg gcg ttg gtg aag ttc   595
   Gly Ala Val Val Arg Ser Pro Glu Phe Asp Arg Ala Leu Val Lys Phe
   150                 155                 160                 165
   tcg cgg ggc ggg gct gat gcg acg gtg att agg gag ttt gat ctg gcc   643
   Ser Arg Gly Gly Ala Asp Ala Thr Val Ile Arg Glu Phe Asp Leu Ala
                   170                 175                 180
   acg gct gct ttc gtg gat gat tcg ccg ttt gaa ttg gag gag gcg aag   691
   Thr Ala Ala Phe Val Asp Asp Ser Pro Phe Glu Leu Glu Glu Ala Lys
               185                 190                 195
   tcc gat gtc acg tgg gtt gat ctg gat acg ttg ctg gtg ggc acg gat   739
   Ser Asp Val Thr Trp Val Asp Leu Asp Thr Leu Leu Val Gly Thr Asp
           200                 205                 210
   acc ggc gag ggg tca ctg acg gat tct ggg tac ccg gcg cgg gtg ctc   787
   Thr Gly Glu Gly Ser Leu Thr Asp Ser Gly Tyr Pro Ala Arg Val Leu
       215                 220                 225
   acg tgg aag cgt ggg act ccg ctt gag cag gcg gag ttg ttc ttt gag     835
   Thr Trp Lys Arg Gly Thr Pro Leu Glu Gln Ala Glu Leu Phe Phe Glu
   230                 235                 240                 245
   ggg tcg cgt cag gat gtg gcg act cat gcg tgg cgg gat tca aca cct     883
   Gly Ser Arg Gln Asp Val Ala Thr His Ala Trp Arg Asp Ser Thr Pro
                   250                 255                 260
   ggt ttt gag cgg acg ttt gtg tca agg tcg ttg gat ttc tat aat tcg     931
   Gly Phe Glu Arg Thr Phe Val Ser Arg Ser Leu Asp Phe Tyr Asn Ser
               265                 270                 275
   gag acg tcg ctg gaa acc gag ggt ggc ctg gtc aag ctt gat gtg ccg     979
   Glu Thr Ser Leu Glu Thr Glu Gly Gly Leu Val Lys Leu Asp Val Pro
           280                 285                 290
   acc gat tgc gat gtc att gtg aag aag cag tgg att ttt gtg agt cct    1027
   Thr Asp Cys Asp Val Ile Val Lys Lys Gln Trp Ile Phe Val Ser Pro
       295                 300                 305
   cgg acg gat ttc gct ggg att cca gca ggt ggc ttg gga gtg ctg ctg    1075
   Arg Thr Asp Phe Ala Gly Ile Pro Ala Gly Gly Leu Gly Val Leu Leu
   310                 315                 320                 325
   tta aag gag ttc ctt gag ggc ggg cgc gat ttt cag cct gtg ttt acg    1123
   Leu Lys Glu Phe Leu Glu Gly Gly Arg Asp Phe Gln Pro Val Phe Thr
                   330                 335                 340
   cct act gag tcg acg tcg ctg cag gga ttg gcc acg aca aag aat ttc    1171
   Pro Thr Glu Ser Thr Ser Leu Gln Gly Leu Ala Thr Thr Lys Asn Phe
               345                 350                 355
   ctg gtt tta acg ctc ctt aat aat gtc tcc aca gaa atc gtc aca gtg    1219
   Leu Val Leu Thr Leu Leu Asn Asn Val Ser Thr Glu Ile Val Thr Val
           360                 365                 370
   ccg ctc aat gat ccg aca acg gag cat gaa cac att gac ctc cca gag    1267
   Pro Leu Asn Asp Pro Thr Thr Glu His Glu His Ile Asp Leu Pro Glu
       375                 380                 385
   cat gtc acc gcg cat gtg gtt gct acc tcc ccg ttg gat ggc gat gaa    1315
   His Val Thr Ala His Val Val Ala Thr Ser Pro Leu Asp Gly Asp Glu
   390                 395                 400                 405
   att tgg gtg cag gca gcg agt ttc acc gaa gcg cca acg ttg ctg cgt    1363
   Ile Trp Val Gln Ala Ala Ser Phe Thr Glu Ala Pro Thr Leu Leu Arg
                   410                 415                 420
   gcg gag ctg cct ggt gcg ctt gag gct gtg aag aag gcg ccg ttg cag    1411
   Ala Glu Leu Pro Gly Ala Leu Glu Ala Val Lys Lys Ala Pro Leu Gln
               425                 430                 435
   ttt gaa aat gct ggt cag gag act cgt cag cat tgg gca acc tcg gcg    1459
   Phe Glu Asn Ala Gly Gln Glu Thr Arg Gln His Trp Ala Thr Ser Ala
           440                 445                 450
   gat gga acg aag att ccg tac ttt att aca gga gcc ttc gag gag gaa    1507
   Asp Gly Thr Lys Ile Pro Tyr Phe Ile Thr Gly Ala Phe Glu Glu Glu
       455                 460                 465
   cca caa aac acc ctg gtc cac gcc tac ggc ggc ttc gag gtt tcc ctt    1555
   Pro Gln Asn Thr Leu Val His Ala Tyr Gly Gly Phe Glu Val Ser Leu
   470                 475                 480                 485
   acc cca agc cac tcc ccg acc cgc ggc atc gca tgg ttg gaa aag ggc    1603
   Thr Pro Ser His Ser Pro Thr Arg Gly Ile Ala Trp Leu Glu Lys Gly
                   490                 495                 500
   tac tac ttt gtg gaa gcc aac ctg cgt ggt ggc ggt gaa ttc ggt ccg    1651
   Tyr Tyr Phe Val Glu Ala Asn Leu Arg Gly Gly Gly Glu Phe Gly Pro
               505                 510                 515
   gaa tgg cat tcg cag gca acc aag ctg aac cgc atg aag gtg tgg gag    1699
   Glu Trp His Ser Gln Ala Thr Lys Leu Asn Arg Met Lys Val Trp Glu
           520                 525                 530
   gat cac cgc gcg gtg ctc gcc gac ctt gtg gag cgc ggc tac gca acg    1747
   Asp His Arg Ala Val Leu Ala Asp Leu Val Glu Arg Gly Tyr Ala Thr
       535                 540                 545
   ccg gag cag att gcg att cgt ggc gga tcc aac ggt ggt ttg ctg aca    1795
   Pro Glu Gln Ile Ala Ile Arg Gly Gly Ser Asn Gly Gly Leu Leu Thr
   550                 555                 560                 565
   agt ggc gcg tta act cag tac cca gaa gca ttc ggt gcg gca gtt gtg    1843
   Ser Gly Ala Leu Thr Gln Tyr Pro Glu Ala Phe Gly Ala Ala Val Val
                   570                 575                 580
   cag gtg ccg ttg gct gat atg ttg cgc tat cac acc tgg tca gcg ggt    1891
   Gln Val Pro Leu Ala Asp Met Leu Arg Tyr His Thr Trp Ser Ala Gly
               585                 590                 595
   gct tcg tgg atg gcg gag tac ggc aac cct gac gat ccg gag gaa cgg    1939
   Ala Ser Trp Met Ala Glu Tyr Gly Asn Pro Asp Asp Pro Glu Glu Arg
           600                 605                 610
   gcg gtg att gag cag tac tcg ccg gtg cag gcg gtg gtg ggc gtc gag    1987
   Ala Val Ile Glu Gln Tyr Ser Pro Val Gln Ala Val Val Gly Val Glu
       615                 620                 625
   aag cga att tat cca ccc gca ttg gtg acg acc tca acc cgg gac gac    2035
   Lys Arg Ile Tyr Pro Pro Ala Leu Val Thr Thr Ser Thr Arg Asp Asp
   630                 635                 640                 645
   cgc gtc cac ccc gcg cac gcg cgc ctt ttt gct caa gct ttg ctt gat    2083
   Arg Val His Pro Ala His Ala Arg Leu Phe Ala Gln Ala Leu Leu Asp
                   650                 655                 660
   gcg ggc cag gcc gtg gat tac tac gaa aac acc gag ggc ggc cat gcc    2131
   Ala Gly Gln Ala Val Asp Tyr Tyr Glu Asn Thr Glu Gly Gly His Ala
               665                 670                 675
   ggc gcg gcg gat aac aag cag acc gcg ttt gtg gaa tcg ctg atc tac    2179
   Gly Ala Ala Asp Asn Lys Gln Thr Ala Phe Val Glu Ser Leu Ile Tyr
           680                 685                 690
   acc tgg atc gag aag act ttg gat cag cag ggt agc att taatacctat     2228
   Thr Trp Ile Glu Lys Thr Leu Asp Gln Gln Gly Ser Ile
       695                 700                 705
   gattatgcga aggctgcgct                                              2248
<210> 22
   <211> 706
   <212> PRT
   <213> Corynebacterium glutamicum
<400> 22
   Leu Lys Thr Ala Gly Thr Thr Arg Phe Thr Phe Thr Ser Ala Asp Pro
    1               5                  10                  15
   Ile Gly Gly Ala Leu Gly Ser Arg Leu Lys Lys Pro Ala Gly Gln Lys
               20                  25                  30
   Ile Thr Asn Leu Asn Phe Met Thr Asp Tyr Thr Phe Leu Glu Asp Ile
           35                  40                  45
   Asp Thr Pro Glu Ala Leu Ala Trp Ala Glu Lys Trp Ser Gly Glu Ser
       50                  55                  60
   Val Glu Lys Leu Lys Ser Pro Ala Lys Asp Ala Leu Glu Ala Arg Leu
   65                  70                  75                  80
   Leu Ala Ala Leu Asp Thr Asp Asp Arg Ile Ala Tyr Val Ser Arg Arg
                   85                  90                  95
   Gly Glu Lys Leu Tyr Asn Phe Trp Arg Asp Ala Gln His Pro Arg Gly
               100                 105                 110
   Val Trp Arg Thr Thr Thr Leu Glu Ser Tyr Glu Ser Asp Gln Pro Glu
           115                 120                 125
   Trp Asp Val Leu Ile Asp Val Asp Ala Leu Ala Glu Asp Glu Gly Glu
       130                 135                 140
   Asn Trp Val Trp Lys Gly Ala Val Val Arg Ser Pro Glu Phe Asp Arg
   145                 150                 155                 160
   Ala Leu Val Lys Phe Ser Arg Gly Gly Ala Asp Ala Thr Val Ile Arg
                   165                 170                 175
   Glu Phe Asp Leu Ala Thr Ala Ala Phe Val Asp Asp Ser Pro Phe Glu
               180                 185                 190
   Leu Glu Glu Ala Lys Ser Asp Val Thr Trp Val Asp Leu Asp Thr Leu
           195                 200                 205
   Leu Val Gly Thr Asp Thr Gly Glu Gly Ser Leu Thr Asp Ser Gly Tyr
       210                 215                 220
   Pro Ala Arg Val Leu Thr Trp Lys Arg Gly Thr Pro Leu Glu Gln Ala
   225                 230                 235                 240
   Glu Leu Phe Phe Glu Gly Ser Arg Gln Asp Val Ala Thr His Ala Trp
                   245                 250                 255
   Arg Asp Ser Thr Pro Gly Phe Glu Arg Thr Phe Val Ser Arg Ser Leu
               260                 265                 270
   Asp Phe Tyr Asn Ser Glu Thr Ser Leu Glu Thr Glu Gly Gly Leu Val
           275                 280                 285
   Lys Leu Asp Val Pro Thr Asp Cys Asp Val Ile Val Lys Lys Gln Trp
       290                 295                 300
   Ile Phe Val Ser Pro Arg Thr Asp Phe Ala Gly Ile Pro Ala Gly Gly
   305                 310                 315                 320
   Leu Gly Val Leu Leu Leu Lys Glu Phe Leu Glu Gly Gly Arg Asp Phe
                   325                 330                 335
   Gln Pro Val Phe Thr Pro Thr Glu Ser Thr Ser Leu Gln Gly Leu Ala
               340                 345                 350
   Thr Thr Lys Asn Phe Leu Val Leu Thr Leu Leu Asn Asn Val Ser Thr
           355                 360                 365
   Glu Ile Val Thr Val Pro Leu Asn Asp Pro Thr Thr Glu His Glu His
       370                 375                 380
   Ile Asp Leu Pro Glu His Val Thr Ala His Val Val Ala Thr Ser Pro
   385                 390                 395                 400
   Leu Asp Gly Asp Glu Ile Trp Val Gln Ala Ala Ser Phe Thr Glu Ala
                   405                 410                 415
   Pro Thr Leu Leu Arg Ala Glu Leu Pro Gly Ala Leu Glu Ala Val Lys
               420                 425                 430
   Lys Ala Pro Leu Gln Phe Glu Asn Ala Gly Gln Glu Thr Arg Gln His
           435                 440                 445
   Trp Ala Thr Ser Ala Asp Gly Thr Lys Ile Pro Tyr Phe Ile Thr Gly
       450                 455                 460
   Ala Phe Glu Glu Glu Pro Gln Asn Thr Leu Val His Ala Tyr Gly Gly
   465                 470                 475                 480
   Phe Glu Val Ser Leu Thr Pro Ser His Ser Pro Thr Arg Gly Ile Ala
                   485                 490                 495
   Trp Leu Glu Lys Gly Tyr Tyr Phe Val Glu Ala Asn Leu Arg Gly Gly
               500                 505                 510
   Gly Glu Phe Gly Pro Glu Trp His Ser Gln Ala Thr Lys Leu Asn Arg
           515                 520                 525
   Met Lys Val Trp Glu Asp His Arg Ala Val Leu Ala Asp Leu Val Glu
       530                 535                 540
   Arg Gly Tyr Ala Thr Pro Glu Gln Ile Ala Ile Arg Gly Gly Ser Asn
   545                 550                 555                 560
   Gly Gly Leu Leu Thr Ser Gly Ala Leu Thr Gln Tyr Pro Glu Ala Phe
                   565                 570                 575
   Gly Ala Ala Val Val Gln Val Pro Leu Ala Asp Met Leu Arg Tyr His
               580                 585                 590
   Thr Trp Ser Ala Gly Ala Ser Trp Met Ala Glu Tyr Gly Asn Pro Asp
           595                 600                 605
   Asp Pro Glu Glu Arg Ala Val Ile Glu Gln Tyr Ser Pro Val Gln Ala
       610                 615                 620
   Val Val Gly Val Glu Lys Arg Ile Tyr Pro Pro Ala Leu Val Thr Thr
   625                 630                 635                 640
   Ser Thr Arg Asp Asp Arg Val His Pro Ala His Ala Arg Leu Phe Ala
                   645                 650                 655
   Gln Ala Leu Leu Asp Ala Gly Gln Ala Val Asp Tyr Tyr Glu Asn Thr
               660                 665                 670
   Glu Gly Gly His Ala Gly Ala Ala Asp Asn Lys Gln Thr Ala Phe Val
   675 680 685
   Glu Ser Leu Ile Tyr Thr Trp Ile Glu Lys Thr Leu Asp Gln Gln Gly
   690 695 700
   Ser Ile
   705
<210> 23
   <211> 2257
   <212> DNA
   <213> Corynebacterium glutamicum
<220>
   <221> CDS
   <222> (101)..(2227)
   <223> RXA01278
<400> 23
   ttatccgtag gtgacaaact ttttaatact tgggtatctg tcatggatac cccggtaata 60
   aataagtgaa ttaccgtaac caacaagttg gggtaccact gtg gca caa gaa gtg   115
                                               Val Ala Gln Glu Val
                                                1               5
   ctt aag gat cta aac aag gtc cgc aac atc ggc atc atg gcg cac atc   163
   Leu Lys Asp Leu Asn Lys Val Arg Asn Ile Gly Ile Met Ala His Ile
                    10                  15                  20
   gat gct ggt aag acc acg acc acc gaa cgc atc ctc ttc tac acc ggc   211
   Asp Ala Gly Lys Thr Thr Thr Thr Glu Arg Ile Leu Phe Tyr Thr Gly
                25                  30                  35
   atc aac cgt aag gtc ggt gag acc cac gac ggt ggc gca acc acc gac   259
   Ile Asn Arg Lys Val Gly Glu Thr His Asp Gly Gly Ala Thr Thr Asp
            40                  45                  50
   tgg atg gag cag gag aag gaa cgc ggc atc acc att acc tcc gcc gcg   307
   Trp Met Glu Gln Glu Lys Glu Arg Gly Ile Thr Ile Thr Ser Ala Ala
        55                  60                  65
   gtt acc tgt ttc tgg gat aac aac cag gtc aac atc att gac acc cct   355
   Val Thr Cys Phe Trp Asp Asn Asn Gln Val Asn Ile Ile Asp Thr Pro
    70                  75                  80                  85
   ggc cac gtt gac ttc acc gtt gag gtt gag cgt tcc ctc cgc gtg ctt   403
   Gly His Val Asp Phe Thr Val Glu Val Glu Arg Ser Leu Arg Val Leu
                    90                  95                  100
   gac ggc gca gtt gct gtg ttc gac ggc aag gaa ggc gtt gag cca cag   451
   Asp Gly Ala Val Ala Val Phe Asp Gly Lys Glu Gly Val Glu Pro Gln
                105                 110                 115
   tct gag cag gtt tgg cgt cag gct acc aag tac gac gtt cca cgt atc   499
   Ser Glu Gln Val Trp Arg Gln Ala Thr Lys Tyr Asp Val Pro Arg Ile
            120                 125                 130
   tgc ttc gtg aac aag atg gac aag ctc ggt gct gac ttc tac ttc acc   547
   Cys Phe Val Asn Lys Met Asp Lys Leu Gly Ala Asp Phe Tyr Phe Thr
        135                 140                 145
   gtt ggc acc atc gag gac cgc ctg ggt gca aag cca ttg gtt atg cag   595
   Val Gly Thr Ile Glu Asp Arg Leu Gly Ala Lys Pro Leu Val Met Gln
   150                 155                 160                 165
   ctc cca atc ggt gct gag gac aac ttc gac ggc gtc atc gac ctt ctt      643
   Leu Pro Ile Gly Ala Glu Asp Asn Phe Asp Gly Val Ile Asp Leu Leu
                   170                 175                 180
   gaa atg aag gca ctg acc tgg cgt gga gtt acc cca att ggt acc gaa      691
   Glu Met Lys Ala Leu Thr Trp Arg Gly Val Thr Pro Ile Gly Thr Glu
               185                 190                 195
   gct acc gtt gag gag atc cca gca gag ctc gca gac cgc gca gct gag      739
   Ala Thr Val Glu Glu Ile Pro Ala Glu Leu Ala Asp Arg Ala Ala Glu
           200                 205                 210
   tac cgt gag aag ctt ctc gag acc gtt gca gag tcc gac gaa gag ctc      787
   Tyr Arg Glu Lys Leu Leu Glu Thr Val Ala Glu Ser Asp Glu Glu Leu
       215                 220                 225
   atg gag aag tac ttc ggt ggc gaa gag ctc agc atc gct gag atc aag      835
   Met Glu Lys Tyr Phe Gly Gly Glu Glu Leu Ser Ile Ala Glu Ile Lys
   230                 235                 240                 245
   gca gct atc cgt aag atg gtt gtt aac tct gag atc tac cct gtt tac      883
   Ala Ala Ile Arg Lys Met Val Val Asn Ser Glu Ile Tyr Pro Val Tyr
                   250                 255                 260
   tgt ggc acc gcc tac aag aac aag ggc atc cag cca ctg ctc gac gca      931
   Cys Gly Thr Ala Tyr Lys Asn Lys Gly Ile Gln Pro Leu Leu Asp Ala
               265                 270                 275
   gtc gtt gac ttc ctg cct tcc cca ctg gat ctc ggc gag acc aag ggc      979
   Val Val Asp Phe Leu Pro Ser Pro Leu Asp Leu Gly Glu Thr Lys Gly
           280                 285                 290
   act gac gtt aag gat cct gag aag gtt ctg acc cgt aag cct tcc gac     1027
   Thr Asp Val Lys Asp Pro Glu Lys Val Leu Thr Arg Lys Pro Ser Asp
       295                 300                 305
   gaa gag cca ctg tct gca ctt gca ttc aag att gca gct cac cca ttc     1075
   Glu Glu Pro Leu Ser Ala Leu Ala Phe Lys Ile Ala Ala His Pro Phe
   310                 315                 320                 325
   ttc ggt aag ctg acc ttc gtt cgt ctg tac tcc ggc aag gtt gag cca     1123
   Phe Gly Lys Leu Thr Phe Val Arg Leu Tyr Ser Gly Lys Val Glu Pro
                   330                 335                 340
   ggc gag cag gtt ctt aac tcc acc aag aac aag aag gaa cgc att ggt     1171
   Gly Glu Gln Val Leu Asn Ser Thr Lys Asn Lys Lys Glu Arg Ile Gly
               345                 350                 355
   aag ctg ttc cag atg cac gcc aac aag gaa aac cct gtt gag gtt gca     1219
   Lys Leu Phe Gln Met His Ala Asn Lys Glu Asn Pro Val Glu Val Ala
           360                 365                 370
   cac gct ggt aac atc tac gcg ttc atc ggc ctg aag gac acc acc acc     1267
   His Ala Gly Asn Ile Tyr Ala Phe Ile Gly Leu Lys Asp Thr Thr Thr
       375                 380                 385
   ggt gac acc ctc tgt gac gca aac gct cca atc att ctt gag tcc atg     1315
   Gly Asp Thr Leu Cys Asp Ala Asn Ala Pro Ile Ile Leu Glu Ser Met
   390                 395                 400                 405
   gac ttc ccg gat cca gtt atc cag gtt gct att gag cct aag acc aag    1363
   Asp Phe Pro Asp Pro Val Ile Gln Val Ala Ile Glu Pro Lys Thr Lys
                   410                 415                 420
   tct gac cag gag aag ctc ggc gta gct atc cag aag ctt gct gaa gaa    1411
   Ser Asp Gln Glu Lys Leu Gly Val Ala Ile Gln Lys Leu Ala Glu Glu
               425                 430                 435
   gac cca acc ttc acc gtt cac ttg gac gat gag tcc ggc cag acc gtc    1459
   Asp Pro Thr Phe Thr Val His Leu Asp Asp Glu Ser Gly Gln Thr Val
           440                 445                 450
   att ggc ggc atg ggc gag ctg cac ctc gat gtt ctt gtt gac cgc atg    1507
   Ile Gly Gly Met Gly Glu Leu His Leu Asp Val Leu Val Asp Arg Met
       455                 460                 465
   aag cgc gag ttc aag gtt gag gca aac atc ggt gac cca cag gtt gct    1555
   Lys Arg Glu Phe Lys Val Glu Ala Asn Ile Gly Asp Pro Gln Val Ala
   470                 475                 480                 485
   tac cgt gag acc atc cgt aag cct gtt gag tcc ctc agc tac acc cac    1603
   Tyr Arg Glu Thr Ile Arg Lys Pro Val Glu Ser Leu Ser Tyr Thr His
                   490                 495                 500
   aag aag cag act ggt ggt tcc ggt cag ttc gct aag gtc atc atc acc    1651
   Lys Lys Gln Thr Gly Gly Ser Gly Gln Phe Ala Lys Val Ile Ile Thr
               505                 510                 515
   att gag cct tac gca cct gag gca gac gag ctt gaa gag ggc gag tcc    1699
   Ile Glu Pro Tyr Ala Pro Glu Ala Asp Glu Leu Glu Glu Gly Glu Ser
           520                 525                 530
   gca atc tac aag ttc gag aac gct gtc acc ggt ggt cgt gtt cca cgt    1747
   Ala Ile Tyr Lys Phe Glu Asn Ala Val Thr Gly Gly Arg Val Pro Arg
       535                 540                 545
   gaa tac atc cca tcc gtt gac gct ggt atc cag gac gca atg cag tac    1795
   Glu Tyr Ile Pro Ser Val Asp Ala Gly Ile Gln Asp Ala Met Gln Tyr
   550                 555                 560                 565
   ggc ttc ctg gct ggc tac cca ctg gtt aac gtc aag gca acc ctt gaa    1843
   Gly Phe Leu Ala Gly Tyr Pro Leu Val Asn Val Lys Ala Thr Leu Glu
                   570                 575                 580
   gat ggc gct tac cac gac gtt gac tcc tct gaa atg gcc ttc aag ctc    1891
   Asp Gly Ala Tyr His Asp Val Asp Ser Ser Glu Met Ala Phe Lys Leu
               585                 590                 595
   gcc ggt tcc cag gcg ttc aag gaa gct gtt gca aag gca aag cca gtc    1939
   Ala Gly Ser Gln Ala Phe Lys Glu Ala Val Ala Lys Ala Lys Pro Val
           600                 605                 610
   ctc ctc gag cca atc atg tcc gtt gaa atc acc act cct gag gag tac    1987
   Leu Leu Glu Pro Ile Met Ser Val Glu Ile Thr Thr Pro Glu Glu Tyr
       615                 620                 625
   atg ggt gaa gtc atc ggt gac gtg aac tcc cgc cgt ggc cag atc gct    2035
   Met Gly Glu Val Ile Gly Asp Val Asn Ser Arg Arg Gly Gln Ile Ala
   630                 635                 640                 645
   tcc atg gat gac cgt gca ggc gcc aag ctg gtt aag gct aag gtt cca    2083
   Ser Met Asp Asp Arg Ala Gly Ala Lys Leu Val Lys Ala Lys Val Pro
                   650                 655                 660
   ctg tct cag atg ttc ggt tac gtc ggt gac ctt cgc tct aag acc cag    2131
   Leu Ser Gln Met Phe Gly Tyr Val Gly Asp Leu Arg Ser Lys Thr Gln
               665                 670                 675
   ggt cgt gca aac tac tcc atg gtc ttc gat tcc tac gct gag gtc cca    2179
   Gly Arg Ala Asn Tyr Ser Met Val Phe Asp Ser Tyr Ala Glu Val Pro
           680                 685                 690
   gcc aac gtt gcc gca gat gtt att gct gag cgc aac ggc acc gct tcc    2227
   Ala Asn Val Ala Ala Asp Val Ile Ala Glu Arg Asn Gly Thr Ala Ser
       695                 700                 705
   taaagatcgt ttagatccga aggaaaacgt                                   2257
<210> 24
   <211> 709
   <212> PRT
   <213> Corynebacterium glutamicum
<400> 24
   Val Ala Gln Glu Val Leu Lys Asp Leu Asn Lys Val Arg Asn Ile Gly
   1 5 10 15
   Ile Met Ala His Ile Asp Ala Gly Lys Thr Thr Thr Thr Glu Arg Ile
   20 25 30
   Leu Phe Tyr Thr Gly Ile Asn Arg Lys Val Gly Glu Thr His Asp Gly
   35 40 45
   Gly Ala Thr Thr Asp Trp Met Glu Gln Glu Lys Glu Arg Gly Ile Thr
   50 55 60
   Ile Thr Ser Ala Ala Val Thr Cys Phe Trp Asp Asn Asn Gln Val Asn
   65 70 75 80
   Ile Ile Asp Thr Pro Gly His Val Asp Phe Thr Val Glu Val Glu Arg
   85 90 95
   Ser Leu Arg Val Leu Asp Gly Ala Val Ala Val Phe Asp Gly Lys Glu
   100 105 110
   Gly Val Glu Pro Gln Ser Glu Gln Val Trp Arg Gln Ala Thr Lys Tyr
   115 120 125
   Asp Val Pro Arg Ile Cys Phe Val Asn Lys Met Asp Lys Leu Gly Ala
   130 135 140
   Asp Phe Tyr Phe Thr Val Gly Thr Ile Glu Asp Arg Leu Gly Ala Lys
   145 150 155 160
   Pro Leu Val Met Gln Leu Pro Ile Gly Ala Glu Asp Asn Phe Asp Gly
   165 170 175
   Val Ile Asp Leu Leu Glu Met Lys Ala Leu Thr Trp Arg Gly Val Thr
   180 185 190
   Pro Ile Gly Thr Glu Ala Thr Val Glu Glu Ile Pro Ala Glu Leu Ala
   195 200 205
   Asp Arg Ala Ala Glu Tyr Arg Glu Lys Leu Leu Glu Thr Val Ala Glu
   210 215 220
   Ser Asp Glu Glu Leu Met Glu Lys Tyr Phe Gly Gly Glu Glu Leu Ser
   225 230 235 240
   Ile Ala Glu Ile Lys Ala Ala Ile Arg Lys Met Val Val Asn Ser Glu
   245 250 255
   Ile Tyr Pro Val Tyr Cys Gly Thr Ala Tyr Lys Asn Lys Gly Ile Gln
   260 265 270
   Pro Leu Leu Asp Ala Val Val Asp Phe Leu Pro Ser Pro Leu Asp Leu
   275 280 285
   Gly Glu Thr Lys Gly Thr Asp Val Lys Asp Pro Glu Lys Val Leu Thr
   290 295 300
   Arg Lys Pro Ser Asp Glu Glu Pro Leu Ser Ala Leu Ala Phe Lys Ile
   305 310 315 320
   Ala Ala His Pro Phe Phe Gly Lys Leu Thr Phe Val Arg Leu Tyr Ser
   325 330 335
   Gly Lys Val Glu Pro Gly Glu Gln Val Leu Asn Ser Thr Lys Asn Lys
   340 345 350
   Lys Glu Arg Ile Gly Lys Leu Phe Gln Met His Ala Asn Lys Glu Asn
   355 360 365
   Pro Val Glu Val Ala His Ala Gly Asn Ile Tyr Ala Phe Ile Gly Leu
   370 375 380
   Lys Asp Thr Thr Thr Gly Asp Thr Leu Cys Asp Ala Asn Ala Pro Ile
   385 390 395 400
   Ile Leu Glu Ser Met Asp Phe Pro Asp Pro Val Ile Gln Val Ala Ile
   405 410 415
   Glu Pro Lys Thr Lys Ser Asp Gln Glu Lys Leu Gly Val Ala Ile Gln
   420 425 430
   Lys Leu Ala Glu Glu Asp Pro Thr Phe Thr Val His Leu Asp Asp Glu
   435 440 445
   Ser Gly Gln Thr Val Ile Gly Gly Met Gly Glu Leu His Leu Asp Val
   450 455 460
   Leu Val Asp Arg Met Lys Arg Glu Phe Lys Val Glu Ala Asn Ile Gly
   465 470 475 480
   Asp Pro Gln Val Ala Tyr Arg Glu Thr Ile Arg Lys Pro Val Glu Ser
   485 490 495
   Leu Ser Tyr Thr His Lys Lys Gln Thr Gly Gly Ser Gly Gln Phe Ala
   500 505 510
   Lys Val Ile Ile Thr Ile Glu Pro Tyr Ala Pro Glu Ala Asp Glu Leu
   515 520 525
   Glu Glu Gly Glu Ser Ala Ile Tyr Lys Phe Glu Asn Ala Val Thr Gly
   530 535 540
   Gly Arg Val Pro Arg Glu Tyr Ile Pro Ser Val Asp Ala Gly Ile Gln
   545 550 555 560
   Asp Ala Met Gln Tyr Gly Phe Leu Ala Gly Tyr Pro Leu Val Asn Val
                       565                     570                     575
   Lys Ala Thr Leu Glu Asp Gly Ala Tyr His Asp Val Asp Ser Ser Glu
                   580                     585                     590
   Met Ala Phe Lys Leu Ala Gly Ser Gln Ala Phe Lys Glu Ala Val Ala
               595                     600                     605
   Lys Ala Lys Pro Val Leu Leu Glu Pro Ile Met Ser Val Glu Ile Thr
       610                     615                     620
   Thr Pro Glu Glu Tyr Met Gly Glu Val Ile Gly Asp Val Asn Ser Arg
   625                     630                     635                     640
   Arg Gly Gln Ile Ala Ser Met Asp Asp Arg Ala Gly Ala Lys Leu Val
                       645                     650                     655
   Lys Ala Lys Val Pro Leu Ser Gln Met Phe Gly Tyr Val Gly Asp Leu
                   660                     665                     670
   Arg Ser Lys Thr Gln Gly Arg Ala Asn Tyr Ser Met Val Phe Asp Ser
               675                     680                     685
   Tyr Ala Glu Val Pro Ala Asn Val Ala Ala Asp Val Ile Ala Glu Arg
       690                     695                     700
   Asn Gly Thr Ala Ser
   705
<210> 25
   <211> 1318
   <212> DNA
   <213> Corynebacterium glutamicum
<220>
   <221> CDS
   <222> (101)..(1288)
   <223> RXA01284
<400> 25
   atctgtgtgc tcagtcttcc aggctgctta tcacagtgaa agcaaaacca attcgtggct 60
   gcgaaagtcg tagccaccac gaagtccagg aggacataca gtg gca aag gcg aag   115
                                               Val Ala Lys Ala Lys
                                               1               5
   ttc gag cgt acc aag ccc cac gta aac atc ggc acc atc ggt cac gtt   163
   Phe Glu Arg Thr Lys Pro His Val Asn Ile Gly Thr Ile Gly His Val
                   10                  15                  20
   gac cac ggt aag acc acc acc acc gcg gct atc acc aag gtt ctg gct   211
   Asp His Gly Lys Thr Thr Thr Thr Ala Ala Ile Thr Lys Val Leu Ala
               25                  30                  35
   gac act tac cct gag ctc aac gag gct ttc gcc ttc gac tcc atc gat   259
   Asp Thr Tyr Pro Glu Leu Asn Glu Ala Phe Ala Phe Asp Ser Ile Asp
           40                  45                  50
   aag gct cct gag gag aag gag cgt ggc atc acg atc aac atc tcc cac   307
   Lys Ala Pro Glu Glu Lys Glu Arg Gly Ile Thr Ile Asn Ile Ser His
       55                  60                  65
   gtt gag tac cag act gaa aag cgc cac tac gca cac gtt gac gct cca   355
   Val Glu Tyr Gln Thr Glu Lys Arg His Tyr Ala His Val Asp Ala Pro
    70                 75                  80                  85
   ggc cac gcc gac tac atc aag aac atg att acc ggc gct gct cag atg    403
   Gly His Ala Asp Tyr Ile Lys Asn Met Ile Thr Gly Ala Ala Gln Met
                    90                  95                 100
   gac ggc gca atc ctc gtt gtt gct gct acc gac ggc cca atg cct cag    451
   Asp Gly Ala Ile Leu Val Val Ala Ala Thr Asp Gly Pro Met Pro Gln
               105                 110                 115
   acc cgt gag cac gtt ctt ctt gct cgc cag gtt ggc gtt cct tac atc    499
   Thr Arg Glu His Val Leu Leu Ala Arg Gln Val Gly Val Pro Tyr Ile
           120                 125                 130
   ctc gtt gct ctt aac aag tgc gac atg gtt gag gat gag gaa atc atc    547
   Leu Val Ala Leu Asn Lys Cys Asp Met Val Glu Asp Glu Glu Ile Ile
       135                 140                 145
   gag ctc gtc gag atg gaa gtt cgt gaa ctt ctt gct gag cag gac tac    595
   Glu Leu Val Glu Met Glu Val Arg Glu Leu Leu Ala Glu Gln Asp Tyr
   150                 155                 160                 165
   gac gaa gag gct cca att gtt cac atc tcc gct ctg aag gct ctt gag    643
   Asp Glu Glu Ala Pro Ile Val His Ile Ser Ala Leu Lys Ala Leu Glu
                   170                 175                 180
   ggc gac gag aag tgg ggc aag cag atc ctt gag ctc atg cag gct tgc    691
   Gly Asp Glu Lys Trp Gly Lys Gln Ile Leu Glu Leu Met Gln Ala Cys
               185                 190                 195
   gat gac aac atc cct gac cca gtt cgt gag acc gac aag cca ttc ctc    739
   Asp Asp Asn Ile Pro Asp Pro Val Arg Glu Thr Asp Lys Pro Phe Leu
           200                 205                 210
   atg cct atc gag gac atc ttc acc atc acc ggt cgt ggc acc gtt gtt    787
   Met Pro Ile Glu Asp Ile Phe Thr Ile Thr Gly Arg Gly Thr Val Val
       215                 220                 225
   acc ggt cgt gtt gag cgc ggt acc ctg aac gtg aac gat gat gtt gac    835
   Thr Gly Arg Val Glu Arg Gly Thr Leu Asn Val Asn Asp Asp Val Asp
   230                 235                 240                 245
   atc atc ggc atc aag gag aag tcc acc tcc acc acc gtt acc ggt atc    883
   Ile Ile Gly Ile Lys Glu Lys Ser Thr Ser Thr Thr Val Thr Gly Ile
                   250                 255                 260
   gag atg ttc cgt aag ctt ctt gac tcc gct gag gct ggc gac aac tgt    931
   Glu Met Phe Arg Lys Leu Leu Asp Ser Ala Glu Ala Gly Asp Asn Cys
               265                 270                 275
   ggt ctg ctt ctc cgt ggt atc aag cgc gaa gat gtt gag cgt ggc cag    979
   Gly Leu Leu Leu Arg Gly Ile Lys Arg Glu Asp Val Glu Arg Gly Gln
           280                 285                 290
   gtt atc gtt aag cca ggc gct tac acc cct cac acc gag ttc gag ggc   1027
   Val Ile Val Lys Pro Gly Ala Tyr Thr Pro His Thr Glu Phe Glu Gly
       295                 300                 305
   tct gtc tac gtt ctg tcc aag gat gaa ggt ggc cgc cac acc cca ttc   1075
   Ser Val Tyr Val Leu Ser Lys Asp Glu Gly Gly Arg His Thr Pro Phe
   310                 315                 320                 325
   ttc gac aac tac cgt cct cag ttc tac ttc cgc acc acc gac gtt acc    1123
   Phe Asp Asn Tyr Arg Pro Gln Phe Tyr Phe Arg Thr Thr Asp Val Thr
                   330                 335                 340
   ggt gtt gtg aag ctt cca gag ggc acc gag atg gtc atg cct ggc gac    1171
   Gly Val Val Lys Leu Pro Glu Gly Thr Glu Met Val Met Pro Gly Asp
               345                 350                 355
   aac gtc gac atg tcc gtc acc ctg atc cag cct gtc gct atg gac gag    1219
   Asn Val Asp Met Ser Val Thr Leu Ile Gln Pro Val Ala Met Asp Glu
           360                 365                 370
   ggc ctg cgt ttc gct atc cgc gaa ggc tcc cgc acc gtt ggc gct ggt    1267
   Gly Leu Arg Phe Ala Ile Arg Glu Gly Ser Arg Thr Val Gly Ala Gly
       375                 380                 385
   cgt gtc acc aag atc atc aag taatttgatg ctctaactgt tgaggtcttt       1318
   Arg Val Thr Lys Ile Ile Lys
   390                 395
<210> 26
   <211> 396
   <212> PRT
   <213> Corynebacterium glutamicum
<400> 26
   Val Ala Lys Ala Lys Phe Glu Arg Thr Lys Pro His Val Asn Ile Gly
    1               5                  10                  15
   Thr Ile Gly His Val Asp His Gly Lys Thr Thr Thr Thr Ala Ala Ile
               20                  25                  30
   Thr Lys Val Leu Ala Asp Thr Tyr Pro Glu Leu Asn Glu Ala Phe Ala
           35                  40                  45
   Phe Asp Ser Ile Asp Lys Ala Pro Glu Glu Lys Glu Arg Gly Ile Thr
       50                  55                  60
   Ile Asn Ile Ser His Val Glu Tyr Gln Thr Glu Lys Arg His Tyr Ala
   65                  70                  75                  80
   His Val Asp Ala Pro Gly His Ala Asp Tyr Ile Lys Asn Met Ile Thr
                   85                  90                  95
   Gly Ala Ala Gln Met Asp Gly Ala Ile Leu Val Val Ala Ala Thr Asp
               100                 105                 110
   Gly Pro Met Pro Gln Thr Arg Glu His Val Leu Leu Ala Arg Gln Val
           115                 120                 125
   Gly Val Pro Tyr Ile Leu Val Ala Leu Asn Lys Cys Asp Met Val Glu
       130                 135                 140
   Asp Glu Glu Ile Ile Glu Leu Val Glu Met Glu Val Arg Glu Leu Leu
   145                 150                 155                 160
   Ala Glu Gln Asp Tyr Asp Glu Glu Ala Pro Ile Val His Ile Ser Ala
                   165                 170                 175
   Leu Lys Ala Leu Glu Gly Asp Glu Lys Trp Gly Lys Gln Ile Leu Glu
               180                 185                 190
   Leu Met Gln Ala Cys Asp Asp Asn Ile Pro Asp Pro Val Arg Glu Thr
   195 200 205
   Asp Lys Pro Phe Leu Met Pro Ile Glu Asp Ile Phe Thr Ile Thr Gly
   210 215 220
   Arg Gly Thr Val Val Thr Gly Arg Val Glu Arg Gly Thr Leu Asn Val
   225 230 235 240
   Asn Asp Asp Val Asp Ile Ile Gly Ile Lys Glu Lys Ser Thr Ser Thr
   245 250 255
   Thr Val Thr Gly Ile Glu Met Phe Arg Lys Leu Leu Asp Ser Ala Glu
   260 265 270
   Ala Gly Asp Asn Cys Gly Leu Leu Leu Arg Gly Ile Lys Arg Glu Asp
   275 280 285
   Val Glu Arg Gly Gln Val Ile Val Lys Pro Gly Ala Tyr Thr Pro His
   290 295 300
   Thr Glu Phe Glu Gly Ser Val Tyr Val Leu Ser Lys Asp Glu Gly Gly
   305 310 315 320
   Arg His Thr Pro Phe Phe Asp Asn Tyr Arg Pro Gln Phe Tyr Phe Arg
   325 330 335
   Thr Thr Asp Val Thr Gly Val Val Lys Leu Pro Glu Gly Thr Glu Met
   340 345 350
   Val Met Pro Gly Asp Asn Val Asp Met Ser Val Thr Leu Ile Gln Pro
   355 360 365
   Val Ala Met Asp Glu Gly Leu Arg Phe Ala Ile Arg Glu Gly Ser Arg
   370 375 380
   Thr Val Gly Ala Gly Arg Val Thr Lys Ile Ile Lys
   385 390 395
<210> 27
   <211> 3625
   <212> DNA
   <213> Corynebacterium glutamicum
<220>
   <221> CDS
   <222> (101)..(3595)
   <223> RXA01344
<400> 27
   gggggatcgg gttcctcagc agaccaattg ctcaaaaata ccagcggtgt tgatctgcac 60
   ttaatggcct tgaccagcca ggtgcaatta cccgcgtgag gtg ctg gaa gga ccc   115
                                              Val Leu Glu Gly Pro
                                              1               5
   atc ttg gca gtc tcc cgc cag acc aag tca gtc gtc gat att ccc ggt   163
   Ile Leu Ala Val Ser Arg Gln Thr Lys Ser Val Val Asp Ile Pro Gly
                   10                  15                  20
   gca ccg cag cgt tat tct ttc gcg aag gtg tcc gca ccc att gag gtg   211
   Ala Pro Gln Arg Tyr Ser Phe Ala Lys Val Ser Ala Pro Ile Glu Val
               25                  30                  35
   ccc ggg cta cta gat ctt caa ctg gat tct tac tcc tgg ctg att ggt      259
   Pro Gly Leu Leu Asp Leu Gln Leu Asp Ser Tyr Ser Trp Leu Ile Gly
           40                  45                  50
   acg cct gag tgg cgt gct cgt cag aag gaa gaa ttc ggc gag gga gcc      307
   Thr Pro Glu Trp Arg Ala Arg Gln Lys Glu Glu Phe Gly Glu Gly Ala
       55                  60                  65
   cgc gta acc agc ggc ctt gag aac att ctc gag gag ctc tcc cca atc      355
   Arg Val Thr Ser Gly Leu Glu Asn Ile Leu Glu Glu Leu Ser Pro Ile
   70                  75                  80                  85
   cag gat tac tct gga aac atg tcc ctg agc ctt tcg gag cca cgc ttc      403
   Gln Asp Tyr Ser Gly Asn Met Ser Leu Ser Leu Ser Glu Pro Arg Phe
                   90                  95                  100
   gaa gac gtc aag aac acc att gac gag gcg aaa gaa aag gac atc aac      451
   Glu Asp Val Lys Asn Thr Ile Asp Glu Ala Lys Glu Lys Asp Ile Asn
               105                 110                 115
   tac gcg gcg cca ctg tat gtg acc gcg gag ttc gtc aac aac acc acc      499
   Tyr Ala Ala Pro Leu Tyr Val Thr Ala Glu Phe Val Asn Asn Thr Thr
           120                 125                 130
   ggt gaa atc aag tct cag act gtc ttc atc ggc gat ttc cca atg atg      547
   Gly Glu Ile Lys Ser Gln Thr Val Phe Ile Gly Asp Phe Pro Met Met
       135                 140                 145
   acg gac aag gga acg ttc atc atc aac gga acc gaa cgc gtt gtg gtc      595
   Thr Asp Lys Gly Thr Phe Ile Ile Asn Gly Thr Glu Arg Val Val Val
   150                 155                 160                 165
   agc cag ctc gtc cgc tcc ccg ggc gtg tac ttt gac cag acc atc gat      643
   Ser Gln Leu Val Arg Ser Pro Gly Val Tyr Phe Asp Gln Thr Ile Asp
                   170                 175                 180
   aag tca act gag cgt cca ctg cac gcc gtg aag gtt att cct tcc cgt      691
   Lys Ser Thr Glu Arg Pro Leu His Ala Val Lys Val Ile Pro Ser Arg
               185                 190                 195
   ggt gct tgg ctt gag ttt gac gtc gat aag cgc gat tcg gtt ggt gtt      739
   Gly Ala Trp Leu Glu Phe Asp Val Asp Lys Arg Asp Ser Val Gly Val
           200                 205                 210
   cgt att gac cgc aag cgt cgc cag cca gtc acc gta ctg ctg aag gct      787
   Arg Ile Asp Arg Lys Arg Arg Gln Pro Val Thr Val Leu Leu Lys Ala
       215                 220                 225
   ctt ggc tgg acc act gag cag atc acc gag cgt ttc ggt ttc tct gaa      835
   Leu Gly Trp Thr Thr Glu Gln Ile Thr Glu Arg Phe Gly Phe Ser Glu
   230                 235                 240                 245
   atc atg atg tcc acc ctc gag tcc gat ggt gta gca aac acc gat gag      883
   Ile Met Met Ser Thr Leu Glu Ser Asp Gly Val Ala Asn Thr Asp Glu
                   250                 255                 260
   gca ttg ctg gag atc tac cgc aag cag cgt cca ggc gag cag cct acc      931
   Ala Leu Leu Glu Ile Tyr Arg Lys Gln Arg Pro Gly Glu Gln Pro Thr
               265                 270                 275
   cgc gac ctt gcg cag tcc ctc ctg gac aac agc ttc ttc cgt gca aag      979
   Arg Asp Leu Ala Gln Ser Leu Leu Asp Asn Ser Phe Phe Arg Ala Lys
           280                 285                 290
   cgc tac gac ctg gct cgc gtt ggt cgt tac aag atc aac cgc aag ctc    1027
   Arg Tyr Asp Leu Ala Arg Val Gly Arg Tyr Lys Ile Asn Arg Lys Leu
       295                 300                 305
   ggc ctt ggt ggc gac cac gat ggt ttg atg act ctt act gaa gag gac    1075
   Gly Leu Gly Gly Asp His Asp Gly Leu Met Thr Leu Thr Glu Glu Asp
   310                 315                 320                 325
   atc gca acc acc atc gag tac ctg gtg cgt ctg cac gca ggt gag cgc    1123
   Ile Ala Thr Thr Ile Glu Tyr Leu Val Arg Leu His Ala Gly Glu Arg
                   330                 335                 340
   gtc atg act tct cca aat ggt gaa gag atc cca gtc gag acc gat gac    1171
   Val Met Thr Ser Pro Asn Gly Glu Glu Ile Pro Val Glu Thr Asp Asp
               345                 350                 355
   atc gac cac ttt ggt aac cgt cgt ctg cgt acc gtt ggc gaa ctg atc    1219
   Ile Asp His Phe Gly Asn Arg Arg Leu Arg Thr Val Gly Glu Leu Ile
           360                 365                 370
   cag aac cag gtc cgt gtc ggc ctg tcc cgc atg gag cgc gtt gtt cgt    1267
   Gln Asn Gln Val Arg Val Gly Leu Ser Arg Met Glu Arg Val Val Arg
       375                 380                 385
   gag cgt atg acc acc cag gat gcg gag tcc att act cct act tcc ttg    1315
   Glu Arg Met Thr Thr Gln Asp Ala Glu Ser Ile Thr Pro Thr Ser Leu
   390                 395                 400                 405
   atc aac gtt cgt cct gtc tct gca gct atc cgt gag ttc ttc gga act    1363
   Ile Asn Val Arg Pro Val Ser Ala Ala Ile Arg Glu Phe Phe Gly Thr
                   410                 415                 420
   tcc cag ctg tct cag ttc atg gac cag aac aac tcc ctg tct ggt ttg    1411
   Ser Gln Leu Ser Gln Phe Met Asp Gln Asn Asn Ser Leu Ser Gly Leu
               425                 430                 435
   act cac aag cgt cgt ctg tcg gct ctg ggc ccg ggt ggt ctg tcc cgt    1459
   Thr His Lys Arg Arg Leu Ser Ala Leu Gly Pro Gly Gly Leu Ser Arg
           440                 445                 450
   gag cgc gcc ggc atc gag gtt cga gac gtt cac cca tct cac tac ggc    1507
   Glu Arg Ala Gly Ile Glu Val Arg Asp Val His Pro Ser His Tyr Gly
       455                 460                 465
   cgt atg tgc cca att gag act ccg gaa ggt cca aac att ggc ctg atc    1555
   Arg Met Cys Pro Ile Glu Thr Pro Glu Gly Pro Asn Ile Gly Leu Ile
   470                 475                 480                 485
   ggt tcc ttg gct tcc tat gct cga gtg aac cca ttc ggt ttc att gag    1603
   Gly Ser Leu Ala Ser Tyr Ala Arg Val Asn Pro Phe Gly Phe Ile Glu
                   490                 495                 500
   acc cca tac cgt cgc atc atc gac ggc aag ctg acc gac cag att gac    1651
   Thr Pro Tyr Arg Arg Ile Ile Asp Gly Lys Leu Thr Asp Gln Ile Asp
               505                 510                 515
   tac ctt acc gct gat gag gaa gac cgc ttc gtt gtt gcg cag gca aac    1699
   Tyr Leu Thr Ala Asp Glu Glu Asp Arg Phe Val Val Ala Gln Ala Asn
           520                 525                 530
   acg cac tac gac gaa gag ggc aac atc acc gat gag acc gtc act gtt    1747
   Thr His Tyr Asp Glu Glu Gly Asn Ile Thr Asp Glu Thr Val Thr Val
       535                 540                 545
   cgt ctg aag gac ggc gac atc gcc atg gtt ggc cgc aac gcg gtt gat   1795
   Arg Leu Lys Asp Gly Asp Ile Ala Met Val Gly Arg Asn Ala Val Asp
   550                 555                 560                 565
   tac atg gac gtt tcc cct cgt cag atg gtt tct gtt ggt acc gcg atg   1843
   Tyr Met Asp Val Ser Pro Arg Gln Met Val Ser Val Gly Thr Ala Met
                   570                 575                 580
   att cca ttc ctg gag cac gac gat gct aac cgt gca ctg atg ggc gcg   1891
   Ile Pro Phe Leu Glu His Asp Asp Ala Asn Arg Ala Leu Met Gly Ala
               585                 590                 595
   aac atg cag aag cag gct gtg cca ctg att cgt gcc gag gct cct ttc   1939
   Asn Met Gln Lys Gln Ala Val Pro Leu Ile Arg Ala Glu Ala Pro Phe
           600                 605                 610
   gtg ggc acc ggt atg gag cag cgc gca gca tac gac gcc ggc gac ctg   1987
   Val Gly Thr Gly Met Glu Gln Arg Ala Ala Tyr Asp Ala Gly Asp Leu
       615                 620                 625
   gtt att acc cca gtc gca ggt gtg gtg gaa aac gtt tca gct gac ttc   2035
   Val Ile Thr Pro Val Ala Gly Val Val Glu Asn Val Ser Ala Asp Phe
   630                 635                 640                 645
   atc acc atc atg gct gat gac ggc aag cgc gaa acc tac ctg ctg cgt   2083
   Ile Thr Ile Met Ala Asp Asp Gly Lys Arg Glu Thr Tyr Leu Leu Arg
                   650                 655                 660
   aag ttc cag cgc acc aac cag ggc acc agc tac aac cag aag cct ttg   2131
   Lys Phe Gln Arg Thr Asn Gln Gly Thr Ser Tyr Asn Gln Lys Pro Leu
               665                 670                 675
   gtt aac ttg ggc gag cgc gtt gaa gct ggc cag gtt att gct gat ggt   2179
   Val Asn Leu Gly Glu Arg Val Glu Ala Gly Gln Val Ile Ala Asp Gly
           680                 685                 690
   cca ggt acc ttc aat ggt gaa atg tcc ctt ggc cgt aac ctt ctg gtt   2227
   Pro Gly Thr Phe Asn Gly Glu Met Ser Leu Gly Arg Asn Leu Leu Val
       695                 700                 705
   gcg ttc atg cct tgg gaa ggc cac aac tac gag gat gcg atc atc ctc   2275
   Ala Phe Met Pro Trp Glu Gly His Asn Tyr Glu Asp Ala Ile Ile Leu
   710                 715                 720                 725
   aac cag aac atc gtt gag cag gac atc ttg acc tcg atc cac atc gag   2323
   Asn Gln Asn Ile Val Glu Gln Asp Ile Leu Thr Ser Ile His Ile Glu
                   730                 735                 740
   gag cac gag atc gat gcc cgc gac act aag ctt ggc gcc gaa gaa atc   2371
   Glu His Glu Ile Asp Ala Arg Asp Thr Lys Leu Gly Ala Glu Glu Ile
               745                 750                 755
   acc cgc gac atc cct aat gtg tct gaa gaa gtc ctc aag gac ctc gac   2419
   Thr Arg Asp Ile Pro Asn Val Ser Glu Glu Val Leu Lys Asp Leu Asp
           760                 765                 770
   gac cgc ggt att gtc cgc atc ggt gct gat gtt cgt gac ggc gac atc   2467
   Asp Arg Gly Ile Val Arg Ile Gly Ala Asp Val Arg Asp Gly Asp Ile
       775                 780                 785
   ctg gtc ggt aag gtc acc cct aag ggc gag acc gag ctc acc ccg gaa   2515
   Leu Val Gly Lys Val Thr Pro Lys Gly Glu Thr Glu Leu Thr Pro Glu
   790                 795                 800                 805
   gag cgc ttg ctg cgc gca atc ttc ggt gag aag gcc cgc gaa gtt cgc    2563
   Glu Arg Leu Leu Arg Ala Ile Phe Gly Glu Lys Ala Arg Glu Val Arg
                   810                 815                 820
   gat acc tcc atg aag gtg cct cac ggt gag acc ggc aag gtc atc ggc    2611
   Asp Thr Ser Met Lys Val Pro His Gly Glu Thr Gly Lys Val Ile Gly
               825                 830                 835
   gtg cgt cac ttc tcc cgc gag gac gac gac gat ctg gct cct ggc gtc    2659
   Val Arg His Phe Ser Arg Glu Asp Asp Asp Asp Leu Ala Pro Gly Val
           840                 845                 850
   aac gag atg atc cgt atc tac gtt gct cag aag cgt aag atc cag gac    2707
   Asn Glu Met Ile Arg Ile Tyr Val Ala Gln Lys Arg Lys Ile Gln Asp
       855                 860                 865
   ggc gat aag ctc gct ggc cgc cac ggt aac aag ggt gtt gtc ggt aaa    2755
   Gly Asp Lys Leu Ala Gly Arg His Gly Asn Lys Gly Val Val Gly Lys
   870                 875                 880                 885
   att ttg cct cag gaa gat atg cca ttc ctt cca gac ggc act cct gtt    2803
   Ile Leu Pro Gln Glu Asp Met Pro Phe Leu Pro Asp Gly Thr Pro Val
                   890                 895                 900
   gac atc atc ttg aac acc cac ggt gtt cca cgt cgt atg aac att ggt    2851
   Asp Ile Ile Leu Asn Thr His Gly Val Pro Arg Arg Met Asn Ile Gly
               905                 910                 915
   cag gtt ctt gag acc cac ctt ggc tgg ctg gca tct gct ggt tgg tcc    2899
   Gln Val Leu Glu Thr His Leu Gly Trp Leu Ala Ser Ala Gly Trp Ser
           920                 925                 930
   gtg gat cct gaa gat cct gag aac gct gag ctc gtc aag act ctg cct    2947
   Val Asp Pro Glu Asp Pro Glu Asn Ala Glu Leu Val Lys Thr Leu Pro
       935                 940                 945
   gca gac ctc ctc gag gtt cct gct ggt tcc ttg act gca act cct gtg    2995
   Ala Asp Leu Leu Glu Val Pro Ala Gly Ser Leu Thr Ala Thr Pro Val
   950                 955                 960                 965
   ttc gac ggt gcg tca aac gaa gag ctc gca ggc ctg ctc gct aat tca    3043
   Phe Asp Gly Ala Ser Asn Glu Glu Leu Ala Gly Leu Leu Ala Asn Ser
                   970                 975                 980
   cgt cca aac cgc gac ggc gac gtc atg gtt aac gcg gat ggt aaa gca    3091
   Arg Pro Asn Arg Asp Gly Asp Val Met Val Asn Ala Asp Gly Lys Ala
               985                 990                 995
   acg ctt atc gac ggt cgc tcc ggt gag cct tac ccg tac ccg gtt tcc    3139
   Thr Leu Ile Asp Gly Arg Ser Gly Glu Pro Tyr Pro Tyr Pro Val Ser
           1000                1005                1010
   atc ggc tac atg tac atg ctg aag ctg cac cac ctc gtt gac gag aag    3187
   Ile Gly Tyr Met Tyr Met Leu Lys Leu His His Leu Val Asp Glu Lys
       1015                1020                1025
   atc cac gca cgt tcc act ggt cct tac tcc atg att acc cag cag cca    3235
   Ile His Ala Arg Ser Thr Gly Pro Tyr Ser Met Ile Thr Gln Gln Pro
   1030                1035                1040                1045
   ctg ggt ggt aaa gca cag ttc ggt gga cag cgt ttc ggc gaa atg gag    3283
   Leu Gly Gly Lys Ala Gln Phe Gly Gly Gln Arg Phe Gly Glu Met Glu
                   1050                1055                1060
   gtg tgg gca atg cag gca tac ggc gct gcc tac aca ctt cag gag ctg   3331
   Val Trp Ala Met Gln Ala Tyr Gly Ala Ala Tyr Thr Leu Gln Glu Leu
               1065                1070                1075
   ctg acc atc aag tct gat gac gtg gtt ggc cgt gtc aag gtc tac gaa   3379
   Leu Thr Ile Lys Ser Asp Asp Val Val Gly Arg Val Lys Val Tyr Glu
           1080                1085                1090
   gca att gtg aag ggc gag aac atc ccg gat cca ggt att cct gag tcc   3427
   Ala Ile Val Lys Gly Glu Asn Ile Pro Asp Pro Gly Ile Pro Glu Ser
       1095                1100                1105
   ttc aag gtt ctc ctc aag gag ctc cag tcc ttg tgc ctg aac gtg gag   3475
   Phe Lys Val Leu Leu Lys Glu Leu Gln Ser Leu Cys Leu Asn Val Glu
   1110                1115                1120                1125
   gtt ctc tcc gca gac ggc act cca atg gag ctc gcg ggt gac gac gac   3523
   Val Leu Ser Ala Asp Gly Thr Pro Met Glu Leu Ala Gly Asp Asp Asp
                   1130                1135                1140
   gac ttc gat cag gca ggc gcc tca ctt ggc atc aac ctg tcc cgt gac   3571
   Asp Phe Asp Gln Ala Gly Ala Ser Leu Gly Ile Asn Leu Ser Arg Asp
               1145                1150                1155
   gag cgt tcc gac gcc gac acc gca tagcagatca gaaaacaacc gctagaaatc  3625
   Glu Arg Ser Asp Ala Asp Thr Ala
           1160                1165
<210> 28
   <211> 1165
   <212> PRT
   <213> Corynebacterium glutamicum
<400> 28
   Val Leu Glu Gly Pro Ile Leu Ala Val Ser Arg Gln Thr Lys Ser Val
   1               5                   10                  15
   Val Asp Ile Pro Gly Ala Pro Gln Arg Tyr Ser Phe Ala Lys Val Ser
               20                  25                  30
   Ala Pro Ile Glu Val Pro Gly Leu Leu Asp Leu Gln Leu Asp Ser Tyr
           35                  40                  45
   Ser Trp Leu Ile Gly Thr Pro Glu Trp Arg Ala Arg Gln Lys Glu Glu
       50                  55                  60
   Phe Gly Glu Gly Ala Arg Val Thr Ser Gly Leu Glu Asn Ile Leu Glu
   65                  70                  75                  80
   Glu Leu Ser Pro Ile Gln Asp Tyr Ser Gly Asn Met Ser Leu Ser Leu
                   85                  90                  95
   Ser Glu Pro Arg Phe Glu Asp Val Lys Asn Thr Ile Asp Glu Ala Lys
               100                 105                 110
   Glu Lys Asp Ile Asn Tyr Ala Ala Pro Leu Tyr Val Thr Ala Glu Phe
           115                 120                 125
   Val Asn Asn Thr Thr Gly Glu Ile Lys Ser Gln Thr Val Phe Ile Gly
       130                 135                 140
   Asp Phe Pro Met Met Thr Asp Lys Gly Thr Phe Ile Ile Asn Gly Thr
   145 150 155 160
   Glu Arg Val Val Val Ser Gln Leu Val Arg Ser Pro Gly Val Tyr Phe
   165 170 175
   Asp Gln Thr Ile Asp Lys Ser Thr Glu Arg Pro Leu His Ala Val Lys
   180 185 190
   Val Ile Pro Ser Arg Gly Ala Trp Leu Glu Phe Asp Val Asp Lys Arg
   195 200 205
   Asp Ser Val Gly Val Arg Ile Asp Arg Lys Arg Arg Gln Pro Val Thr
   210 215 220
   Val Leu Leu Lys Ala Leu Gly Trp Thr Thr Glu Gln Ile Thr Glu Arg
   225 230 235 240
   Phe Gly Phe Ser Glu Ile Met Met Ser Thr Leu Glu Ser Asp Gly Val
   245 250 255
   Ala Asn Thr Asp Glu Ala Leu Leu Glu Ile Tyr Arg Lys Gln Arg Pro
   260 265 270
   Gly Glu Gln Pro Thr Arg Asp Leu Ala Gln Ser Leu Leu Asp Asn Ser
   275 280 285
   Phe Phe Arg Ala Lys Arg Tyr Asp Leu Ala Arg Val Gly Arg Tyr Lys
   290 295 300
   Ile Asn Arg Lys Leu Gly Leu Gly Gly Asp His Asp Gly Leu Met Thr
   305 310 315 320
   Leu Thr Glu Glu Asp Ile Ala Thr Thr Ile Glu Tyr Leu Val Arg Leu
   325 330 335
   His Ala Gly Glu Arg Val Met Thr Ser Pro Asn Gly Glu Glu Ile Pro
   340 345 350
   Val Glu Thr Asp Asp Ile Asp His Phe Gly Asn Arg Arg Leu Arg Thr
   355 360 365
   Val Gly Glu Leu Ile Gln Asn Gln Val Arg Val Gly Leu Ser Arg Met
   370 375 380
   Glu Arg Val Val Arg Glu Arg Met Thr Thr Gln Asp Ala Glu Ser Ile
   385 390 395 400
   Thr Pro Thr Ser Leu Ile Asn Val Arg Pro Val Ser Ala Ala Ile Arg
   405 410 415
   Glu Phe Phe Gly Thr Ser Gln Leu Ser Gln Phe Met Asp Gln Asn Asn
   420 425 430
   Ser Leu Ser Gly Leu Thr His Lys Arg Arg Leu Ser Ala Leu Gly Pro
   435 440 445
   Gly Gly Leu Ser Arg Glu Arg Ala Gly Ile Glu Val Arg Asp Val His
   450 455 460
   Pro Ser His Tyr Gly Arg Met Cys Pro Ile Glu Thr Pro Glu Gly Pro
   465 470 475 480
   Asn Ile Gly Leu Ile Gly Ser Leu Ala Ser Tyr Ala Arg Val Asn Pro
   485 490 495
   Phe Gly Phe Ile Glu Thr Pro Tyr Arg Arg Ile Ile Asp Gly Lys Leu
   500 505 510
   Thr Asp Gln Ile Asp Tyr Leu Thr Ala Asp Glu Glu Asp Arg Phe Val
   515 520 525
   Val Ala Gln Ala Asn Thr His Tyr Asp Glu Glu Gly Asn Ile Thr Asp
   530 535 540
   Glu Thr Val Thr Val Arg Leu Lys Asp Gly Asp Ile Ala Met Val Gly
   545 550 555 560
   Arg Asn Ala Val Asp Tyr Met Asp Val Ser Pro Arg Gln Met Val Ser
   565 570 575
   Val Gly Thr Ala Met Ile Pro Phe Leu Glu His Asp Asp Ala Asn Arg
   580 585 590
   Ala Leu Met Gly Ala Asn Met Gln Lys Gln Ala Val Pro Leu Ile Arg
   595 600 605
   Ala Glu Ala Pro Phe Val Gly Thr Gly Met Glu Gln Arg Ala Ala Tyr
   610 615 620
   Asp Ala Gly Asp Leu Val Ile Thr Pro Val Ala Gly Val Val Glu Asn
   625 630 635 640
   Val Ser Ala Asp Phe Ile Thr Ile Met Ala Asp Asp Gly Lys Arg Glu
   645 650 655
   Thr Tyr Leu Leu Arg Lys Phe Gln Arg Thr Asn Gln Gly Thr Ser Tyr
   660 665 670
   Asn Gln Lys Pro Leu Val Asn Leu Gly Glu Arg Val Glu Ala Gly Gln
   675 680 685
   Val Ile Ala Asp Gly Pro Gly Thr Phe Asn Gly Glu Met Ser Leu Gly
   690 695 700
   Arg Asn Leu Leu Val Ala Phe Met Pro Trp Glu Gly His Asn Tyr Glu
   705 710 715 720
   Asp Ala Ile Ile Leu Asn Gln Asn Ile Val Glu Gln Asp Ile Leu Thr
   725 730 735
   Ser Ile His Ile Glu Glu His Glu Ile Asp Ala Arg Asp Thr Lys Leu
   740 745 750
   Gly Ala Glu Glu Ile Thr Arg Asp Ile Pro Asn Val Ser Glu Glu Val
   755 760 765
   Leu Lys Asp Leu Asp Asp Arg Gly Ile Val Arg Ile Gly Ala Asp Val
   770 775 780
   Arg Asp Gly Asp Ile Leu Val Gly Lys Val Thr Pro Lys Gly Glu Thr
   785 790 795 800
   Glu Leu Thr Pro Glu Glu Arg Leu Leu Arg Ala Ile Phe Gly Glu Lys
   805 810 815
   Ala Arg Glu Val Arg Asp Thr Ser Met Lys Val Pro His Gly Glu Thr
   820 825 830
   Gly Lys Val Ile Gly Val Arg His Phe Ser Arg Glu Asp Asp Asp Asp
           835                 840                 845
   Leu Ala Pro Gly Val Asn Glu Met Ile Arg Ile Tyr Val Ala Gln Lys
       850                 855                 860
   Arg Lys Ile Gln Asp Gly Asp Lys Leu Ala Gly Arg His Gly Asn Lys
   865                 870                 875                 880
   Gly Val Val Gly Lys Ile Leu Pro Gln Glu Asp Met Pro Phe Leu Pro
                   885                 890                 895
   Asp Gly Thr Pro Val Asp Ile Ile Leu Asn Thr His Gly Val Pro Arg
               900                 905                 910
   Arg Met Asn Ile Gly Gln Val Leu Glu Thr His Leu Gly Trp Leu Ala
           915                 920                 925
   Ser Ala Gly Trp Ser Val Asp Pro Glu Asp Pro Glu Asn Ala Glu Leu
       930                 935                 940
   Val Lys Thr Leu Pro Ala Asp Leu Leu Glu Val Pro Ala Gly Ser Leu
   945                 950                 955                 960
   Thr Ala Thr Pro Val Phe Asp Gly Ala Ser Asn Glu Glu Leu Ala Gly
                   965                 970                 975
   Leu Leu Ala Asn Ser Arg Pro Asn Arg Asp Gly Asp Val Met Val Asn
               980                 985                 990
   Ala Asp Gly Lys Ala Thr Leu Ile Asp Gly Arg Ser Gly Glu Pro Tyr
           995                 1000                1005
   Pro Tyr Pro Val Ser Ile Gly Tyr Met Tyr Met Leu Lys Leu His His
      1010                 1015                1020
   Leu Val Asp Glu Lys Ile His Ala Arg Ser Thr Gly Pro Tyr Ser Met
   1025                1030                1035                1040
   Ile Thr Gln Gln Pro Leu Gly Gly Lys Ala Gln Phe Gly Gly Gln Arg
                  1045                1050                1055
   Phe Gly Glu Met Glu Val Trp Ala Met Gln Ala Tyr Gly Ala Ala Tyr
              1060                1065                1070
   Thr Leu Gln Glu Leu Leu Thr Ile Lys Ser Asp Asp Val Val Gly Arg
          1075                1080                1085
   Val Lys Val Tyr Glu Ala Ile Val Lys Gly Glu Asn Ile Pro Asp Pro
      1090                1095                1100
   Gly Ile Pro Glu Ser Phe Lys Val Leu Leu Lys Glu Leu Gln Ser Leu
   1105                1110                1115                1120
   Cys Leu Asn Val Glu Val Leu Ser Ala Asp Gly Thr Pro Met Glu Leu
                  1125                1130                1135
   Ala Gly Asp Asp Asp Asp Phe Asp Gln Ala Gly Ala Ser Leu Gly Ile
              1140                1145                1150
   Asn Leu Ser Arg Asp Glu Arg Ser Asp Ala Asp Thr Ala
          1155                1160                1165
<210> 29
   <211> 1582
   <212> DNA
   <213> Corynebacterium glutamicum
<220>
   <221> CDS
   <222> (101)..(1552)
   <223> RXA01345
<400> 29
   cataacctca ttgaacatgc aaaactaatg cttttggggg gtatgcataa attcgtttcg 60
   ttccactgca cagcccgaaa atgctgctag ggtcaagttc atg cgt ttt gga ctt   115
                                               Met Arg Phe Gly Leu
                                                1               5
   gac ttg gga act acc cgc aca atc gcg gcc gcc gtg gac cgc gga aac   163
   Asp Leu Gly Thr Thr Arg Thr Ile Ala Ala Ala Val Asp Arg Gly Asn
                   10                  15                  20
   tat ccc atc gtc act gtg gaa gat tct tta ggc gac acc cac gat ttc   211
   Tyr Pro Ile Val Thr Val Glu Asp Ser Leu Gly Asp Thr His Asp Phe
               25                  30                  35
   att cca tct gtg gtg gcc ctc aag gca gat agg att gtc gcg ggt tgg   259
   Ile Pro Ser Val Val Ala Leu Lys Ala Asp Arg Ile Val Ala Gly Trp
           40                  45                  50
   gat gct att gag gtt ggg cag gac cac cct tcc ttc gta cgt tct ttc   307
   Asp Ala Ile Glu Val Gly Gln Asp His Pro Ser Phe Val Arg Ser Phe
       55                  60                  65
   aaa cgc cta ctc tct gaa ccc aat gtc acg gaa gcc acc ccg gtc tac   355
   Lys Arg Leu Leu Ser Glu Pro Asn Val Thr Glu Ala Thr Pro Val Tyr
   70                  75                  80                  85
   ttg ggc gat cat gta cac cct ttg ggc gcc gtc ctg gag gct ttt gcg   403
   Leu Gly Asp His Val His Pro Leu Gly Ala Val Leu Glu Ala Phe Ala
                   90                  95                  100
   gaa aac gtg gtc act gcg ctg cgt gca ttt cag acg caa ttg gga gat   451
   Glu Asn Val Val Thr Ala Leu Arg Ala Phe Gln Thr Gln Leu Gly Asp
               105                 110                 115
   acc tcc ccg atc gaa gta gtc att ggt gtg ccc gcc aac tcc cac agc   499
   Thr Ser Pro Ile Glu Val Val Ile Gly Val Pro Ala Asn Ser His Ser
           120                 125                 130
   gcc cag cga ctg ctc acc atg tcc gcc ttc agc gcc aca ggc atc acc   547
   Ala Gln Arg Leu Leu Thr Met Ser Ala Phe Ser Ala Thr Gly Ile Thr
       135                 140                 145
   gtt gtc ggt ttg gtc aat gag ccc agc gcc gca gct ttc gag tac acc   595
   Val Val Gly Leu Val Asn Glu Pro Ser Ala Ala Ala Phe Glu Tyr Thr
   150                 155                 160                 165
   cac cgc cac gcc cgc acc tta aac tcc aag cgc caa gcc atc gtg gtt   643
   His Arg His Ala Arg Thr Leu Asn Ser Lys Arg Gln Ala Ile Val Val
                   170                 175                 180
   tat gat ttg gga ggc gga aca ttc gac tcc tcg ctc atc cgc atc gac   691
   Tyr Asp Leu Gly Gly Gly Thr Phe Asp Ser Ser Leu Ile Arg Ile Asp
               185                 190                 195
   ggc acc cac cac gag gtt gtg tcc tcc att ggc att tca cgc ctt ggt     739
   Gly Thr His His Glu Val Val Ser Ser Ile Gly Ile Ser Arg Leu Gly
               200                 205                 210
   ggc gat gat ttc gat gaa atc ctc ctc caa tgc gcg ctc aag gcc gca     787
   Gly Asp Asp Phe Asp Glu Ile Leu Leu Gln Cys Ala Leu Lys Ala Ala
           215                 220                 225
   ggc aga cag cac gat gcg ttt ggc aag cgt gct aaa aac acg ctt ctc     835
   Gly Arg Gln His Asp Ala Phe Gly Lys Arg Ala Lys Asn Thr Leu Leu
   230                 235                 240                 245
   gac gaa tcc cgc aac gcg aag gaa gct ctt gtt ccg caa tcc cgt cgc     883
   Asp Glu Ser Arg Asn Ala Lys Glu Ala Leu Val Pro Gln Ser Arg Arg
                   250                 255                 260
   ttg gtt cta gaa att ggc gac gac gac atc acc gtt cca gtg aac aag     931
   Leu Val Leu Glu Ile Gly Asp Asp Asp Ile Thr Val Pro Val Asn Lys
                   265                 270                 275
   ttc tac gag gct gcc act ccc ctg gtg gaa aaa tcc ttg tcc atc atg     979
   Phe Tyr Glu Ala Ala Thr Pro Leu Val Glu Lys Ser Leu Ser Ile Met
               280                 285                 290
   gaa ccc ctc atc ggc gtc gat gat ctt aaa gat tcc gac atc gca ggc    1027
   Glu Pro Leu Ile Gly Val Asp Asp Leu Lys Asp Ser Asp Ile Ala Gly
           295                 300                 305
   atc tac ctt gtt ggt gga gga tcc tcg ctc cca ctc gtt tcc agg ttg    1075
   Ile Tyr Leu Val Gly Gly Gly Ser Ser Leu Pro Leu Val Ser Arg Leu
   310                 315                 320                 325
   ctc cgc gag cgt ttc ggc cgc cgt gtc cac cgc tcc cca ttc ccc tca    1123
   Leu Arg Glu Arg Phe Gly Arg Arg Val His Arg Ser Pro Phe Pro Ser
                   330                 335                 340
   ggt tcc act gcg gtg ggt ctg gcc atc gcg gct gac cct tcc tct ggt    1171
   Gly Ser Thr Ala Val Gly Leu Ala Ile Ala Ala Asp Pro Ser Ser Gly
                   345                 350                 355
   ttc cac cta agg gac cgc gtt gcg cga ggc atc ggt gtg ttc cgt gag    1219
   Phe His Leu Arg Asp Arg Val Ala Arg Gly Ile Gly Val Phe Arg Glu
               360                 365                 370
   cac gat tct ggt cgt gcc gtg agc ttt gac ccg ctg atc gcc ccg gac    1267
   His Asp Ser Gly Arg Ala Val Ser Phe Asp Pro Leu Ile Ala Pro Asp
           375                 380                 385
   acc gat tct gcg acc gtg gcg aaa cga tgc tac aag gcg gtg cac aac    1315
   Thr Asp Ser Ala Thr Val Ala Lys Arg Cys Tyr Lys Ala Val His Asn
   390                 395                 400                 405
   att ggt tgg ttc agg ttc gtg gaa tac tcc acc gtg tcc gag gat ggc    1363
   Ile Gly Trp Phe Arg Phe Val Glu Tyr Ser Thr Val Ser Glu Asp Gly
                   410                 415                 420
   agc ccc gga gat att tcc ctg ctc agt gaa atc aag att cct ttt gat    1411
   Ser Pro Gly Asp Ile Ser Leu Leu Ser Glu Ile Lys Ile Pro Phe Asp
               425                 430                 435
   agc tcc atc acc gat gtg gat gct acc gag att tca cgt ttc gat ggc    1459
   Ser Ser Ile Thr Asp Val Asp Ala Thr Glu Ile Ser Arg Phe Asp Gly
               440                 445                 450
   cca gaa gta gaa gaa acc atc aca gtc aat gac aac ggc gtg gct tcc    1507
   Pro Glu Val Glu Glu Thr Ile Thr Val Asn Asp Asn Gly Val Ala Ser
       455                 460                 465
   att tcc atc aag ata ctc ggc ggc gtt acc gtc gag cac aca att        1552
   Ile Ser Ile Lys Ile Leu Gly Gly Val Thr Val Glu His Thr Ile
   470                 475                 480
   tagttaccat tttggtgctg gtggagtcca                                   1582
<210> 30
   <211> 484
   <212> PRT
   <213> Corynebacterium glutamicum
<400> 30
   Met Arg Phe Gly Leu Asp Leu Gly Thr Thr Arg Thr Ile Ala Ala Ala
   1 5 10 15
   Val Asp Arg Gly Asn Tyr Pro Ile Val Thr Val Glu Asp Ser Leu Gly
   20 25 30
   Asp Thr His Asp Phe Ile Pro Ser Val Val Ala Leu Lys Ala Asp Arg
   35 40 45
   Ile Val Ala Gly Trp Asp Ala Ile Glu Val Gly Gln Asp His Pro Ser
   50 55 60
   Phe Val Arg Ser Phe Lys Arg Leu Leu Ser Glu Pro Asn Val Thr Glu
   65 70 75 80
   Ala Thr Pro Val Tyr Leu Gly Asp His Val His Pro Leu Gly Ala Val
   85 90 95
   Leu Glu Ala Phe Ala Glu Asn Val Val Thr Ala Leu Arg Ala Phe Gln
   100 105 110
   Thr Gln Leu Gly Asp Thr Ser Pro Ile Glu Val Val Ile Gly Val Pro
   115 120 125
   Ala Asn Ser His Ser Ala Gln Arg Leu Leu Thr Met Ser Ala Phe Ser
   130 135 140
   Ala Thr Gly Ile Thr Val Val Gly Leu Val Asn Glu Pro Ser Ala Ala
   145 150 155 160
   Ala Phe Glu Tyr Thr His Arg His Ala Arg Thr Leu Asn Ser Lys Arg
   165 170 175
   Gln Ala Ile Val Val Tyr Asp Leu Gly Gly Gly Thr Phe Asp Ser Ser
   180 185 190
   Leu Ile Arg Ile Asp Gly Thr His His Glu Val Val Ser Ser Ile Gly
   195 200 205
   Ile Ser Arg Leu Gly Gly Asp Asp Phe Asp Glu Ile Leu Leu Gln Cys
   210 215 220
   Ala Leu Lys Ala Ala Gly Arg Gln His Asp Ala Phe Gly Lys Arg Ala
   225 230 235 240
   Lys Asn Thr Leu Leu Asp Glu Ser Arg Asn Ala Lys Glu Ala Leu Val
   245 250 255
   Pro Gln Ser Arg Arg Leu Val Leu Glu Ile Gly Asp Asp Asp Ile Thr
   260 265 270
   Val Pro Val Asn Lys Phe Tyr Glu Ala Ala Thr Pro Leu Val Glu Lys
   275 280 285
   Ser Leu Ser Ile Met Glu Pro Leu Ile Gly Val Asp Asp Leu Lys Asp
   290 295 300
   Ser Asp Ile Ala Gly Ile Tyr Leu Val Gly Gly Gly Ser Ser Leu Pro
   305 310 315 320
   Leu Val Ser Arg Leu Leu Arg Glu Arg Phe Gly Arg Arg Val His Arg
   325 330 335
   Ser Pro Phe Pro Ser Gly Ser Thr Ala Val Gly Leu Ala Ile Ala Ala
   340 345 350
   Asp Pro Ser Ser Gly Phe His Leu Arg Asp Arg Val Ala Arg Gly Ile
   355 360 365
   Gly Val Phe Arg Glu His Asp Ser Gly Arg Ala Val Ser Phe Asp Pro
   370 375 380
   Leu Ile Ala Pro Asp Thr Asp Ser Ala Thr Val Ala Lys Arg Cys Tyr
   385 390 395 400
   Lys Ala Val His Asn Ile Gly Trp Phe Arg Phe Val Glu Tyr Ser Thr
   405 410 415
   Val Ser Glu Asp Gly Ser Pro Gly Asp Ile Ser Leu Leu Ser Glu Ile
   420 425 430
   Lys Ile Pro Phe Asp Ser Ser Ile Thr Asp Val Asp Ala Thr Glu Ile
   435 440 445
   Ser Arg Phe Asp Gly Pro Glu Val Glu Glu Thr Ile Thr Val Asn Asp
   450 455 460
   Asn Gly Val Ala Ser Ile Ser Ile Lys Ile Leu Gly Gly Val Thr Val
   465 470 475 480
   Glu His Thr Ile
<210> 31
   <211> 1123
   <212> DNA
   <213> Corynebacterium glutamicum
<220>
   <221> CDS
   <222> (101)..(1093)
   <223> RXA01404
<220>
   <221> unsure
   <222> 12 .. 12
   <223> All occurrences of n indicate any nucleotide
<400> 31
   gtggatccgg tnttgtgatc cactacgcaa ttggagcgct ccaacacaag ctatatttgt 60
   ttaaatgtcc tgtcaatagt tcaagagaaa atcacagaag atg agc aca tcc cgc   115
                                               Met Ser Thr Ser Arg
                                                1               5
   ccc aca att tat gac gtc gcc aaa gcc gca ggc gtc tcc aaa tcc ttg   163
   Pro Thr Ile Tyr Asp Val Ala Lys Ala Ala Gly Val Ser Lys Ser Leu
                   10                  15                  20
   gtt tct ctc gtg ctt cgc ggc tcc ccc aac gtg agc aaa gaa tcc gaa   211
   Val Ser Leu Val Leu Arg Gly Ser Pro Asn Val Ser Lys Glu Ser Glu
               25                  30                  35
   gcc gcg gtc aag acc gcg ata aaa aag ctc aac tac cag cca aat cgc   259
   Ala Ala Val Lys Thr Ala Ile Lys Lys Leu Asn Tyr Gln Pro Asn Arg
           40                  45                  50
   gcc gca tca gac ctt gcg gcc aag cgc acg cag ctc att gca gtg ctt   307
   Ala Ala Ser Asp Leu Ala Ala Lys Arg Thr Gln Leu Ile Ala Val Leu
       55                  60                  65
   atc gac gac tac tcc aac ccg tgg ttc atc gac ctg att caa agc ctc   355
   Ile Asp Asp Tyr Ser Asn Pro Trp Phe Ile Asp Leu Ile Gln Ser Leu
   70                  75                  80                  85
   agc gat gtg ctc acc ccc aag ggg tac cga ctg tcc gtc att gac tca   403
   Ser Asp Val Leu Thr Pro Lys Gly Tyr Arg Leu Ser Val Ile Asp Ser
                   90                  95                  100
   tta acc tct caa gcc ggc acc gat ccc att acc agt gca cta tca atg   451
   Leu Thr Ser Gln Ala Gly Thr Asp Pro Ile Thr Ser Ala Leu Ser Met
               105                 110                 115
   cgc ccc gat gga atc atc atc gcc caa gac atc ccc gat ttc act gtc   499
   Arg Pro Asp Gly Ile Ile Ile Ala Gln Asp Ile Pro Asp Phe Thr Val
           120                 125                 130
   ccc gat tcc cta ccc cca ttt gtc atc gca ggc acc aga atc acc caa   547
   Pro Asp Ser Leu Pro Pro Phe Val Ile Ala Gly Thr Arg Ile Thr Gln
       135                 140                 145
   gcc agc acc cat gat tca gtg gcc aac gat gac ttc cgg ggc gca gaa   595
   Ala Ser Thr His Asp Ser Val Ala Asn Asp Asp Phe Arg Gly Ala Glu
   150                 155                 160                 165
   ata gcc aca aaa cac ctc atc gat ctt gga cac acc cac atc gcc cac   643
   Ile Ala Thr Lys His Leu Ile Asp Leu Gly His Thr His Ile Ala His
                   170                 175                 180
   cta cgc gtg gga agc ggc gct ggc tta cga cgc ttc gaa agc ttt gag   691
   Leu Arg Val Gly Ser Gly Ala Gly Leu Arg Arg Phe Glu Ser Phe Glu
               185                 190                 195
   gca acc atg cgt gca cat ggc ctg gag ccg ctt tcc aac gat tac ctc   739
   Ala Thr Met Arg Ala His Gly Leu Glu Pro Leu Ser Asn Asp Tyr Leu
           200                 205                 210
   ggc ccc gcc gtt gag cac gcc ggg tac acc gaa acc ctc gca cta ctc   787
   Gly Pro Ala Val Glu His Ala Gly Tyr Thr Glu Thr Leu Ala Leu Leu
       215                 220                 225
   aaa gag cac ccg gag gtc acc gcc att ttc tcc tca aac gac atc acc   835
   Lys Glu His Pro Glu Val Thr Ala Ile Phe Ser Ser Asn Asp Ile Thr
   230                 235                 240                 245
   gcc atc gga gca ctc ggt gcc gcc cgt gaa cta ggt tta cgc gta cct     883
   Ala Ile Gly Ala Leu Gly Ala Ala Arg Glu Leu Gly Leu Arg Val Pro
                   250                 255                 260
   gaa gat cta tca ata atc gga tat gac aac act ccc ctc gcc caa acc     931
   Glu Asp Leu Ser Ile Ile Gly Tyr Asp Asn Thr Pro Leu Ala Gln Thr
               265                 270                 275
   cga ctg atc aac ctc acc acc atc gac gac aac agc atc ggc gtc ggc     979
   Arg Leu Ile Asn Leu Thr Thr Ile Asp Asp Asn Ser Ile Gly Val Gly
           280                 285                 290
   tac aac gcc gct ctc ttg ttg ctg agc atg ctt gat ccc gag gca ccc     1027
   Tyr Asn Ala Ala Leu Leu Leu Leu Ser Met Leu Asp Pro Glu Ala Pro
       295                 300                 305
   cac ccg gag atc atg cat acg ttg cag ccc tcg ctg att gaa cgg ggc     1075
   His Pro Glu Ile Met His Thr Leu Gln Pro Ser Leu Ile Glu Arg Gly
   310                 315                 320                 325
   acg tgc gcg cca cgt gga tagctacccc aaatacttgg acttcctaat            1123
   Thr Cys Ala Pro Arg Gly
                   330
<210> 32
   <211> 331
   <212> PRT
   <213> Corynebacterium glutamicum
<400> 32
   Met Ser Thr Ser Arg Pro Thr Ile Tyr Asp Val Ala Lys Ala Ala Gly
   1               5                   10                  15
   Val Ser Lys Ser Leu Val Ser Leu Val Leu Arg Gly Ser Pro Asn Val
               20                  25                  30
   Ser Lys Glu Ser Glu Ala Ala Val Lys Thr Ala Ile Lys Lys Leu Asn
           35                  40                  45
   Tyr Gln Pro Asn Arg Ala Ala Ser Asp Leu Ala Ala Lys Arg Thr Gln
       50                  55                  60
   Leu Ile Ala Val Leu Ile Asp Asp Tyr Ser Asn Pro Trp Phe Ile Asp
   65                  70                  75                  80
   Leu Ile Gln Ser Leu Ser Asp Val Leu Thr Pro Lys Gly Tyr Arg Leu
                   85                  90                  95
   Ser Val Ile Asp Ser Leu Thr Ser Gln Ala Gly Thr Asp Pro Ile Thr
               100                 105                 110
   Ser Ala Leu Ser Met Arg Pro Asp Gly Ile Ile Ile Ala Gln Asp Ile
           115                 120                 125
   Pro Asp Phe Thr Val Pro Asp Ser Leu Pro Pro Phe Val Ile Ala Gly
       130                 135                 140
   Thr Arg Ile Thr Gln Ala Ser Thr His Asp Ser Val Ala Asn Asp Asp
   145                 150                 155                 160
   Phe Arg Gly Ala Glu Ile Ala Thr Lys His Leu Ile Asp Leu Gly His
   165 170 175
   Thr His Ile Ala His Leu Arg Val Gly Ser Gly Ala Gly Leu Arg Arg
   180 185 190
   Phe Glu Ser Phe Glu Ala Thr Met Arg Ala His Gly Leu Glu Pro Leu
   195 200 205
   Ser Asn Asp Tyr Leu Gly Pro Ala Val Glu His Ala Gly Tyr Thr Glu
   210 215 220
   Thr Leu Ala Leu Leu Lys Glu His Pro Glu Val Thr Ala Ile Phe Ser
   225 230 235 240
   Ser Asn Asp Ile Thr Ala Ile Gly Ala Leu Gly Ala Ala Arg Glu Leu
   245 250 255
   Gly Leu Arg Val Pro Glu Asp Leu Ser Ile Ile Gly Tyr Asp Asn Thr
   260 265 270
   Pro Leu Ala Gln Thr Arg Leu Ile Asn Leu Thr Thr Ile Asp Asp Asn
   275 280 285
   Ser Ile Gly Val Gly Tyr Asn Ala Ala Leu Leu Leu Leu Ser Met Leu
   290 295 300
   Asp Pro Glu Ala Pro His Pro Glu Ile Met His Thr Leu Gln Pro Ser
   305 310 315 320
   Leu Ile Glu Arg Gly Thr Cys Ala Pro Arg Gly
   325 330
<210> 33
   <211> 502
   <212> DNA
   <213> Corynebacterium glutamicum
<220>
   <221> CDS
   <222> (101)..(472)
   <223> RXA01431
<400> 33
   caccgcagct ggttccggtt gccgcgcagc gatcgatgca gagcattacc tagcttctct 60
   ggcctaattc acagttagcc ttaaaccaaa ccatgtacca atg aat gtc gga ttc   115
                                               Met Asn Val Gly Phe
                                               1               5
   ccc agg agt ccc gtc att gtt aat tta gga gaa acc atg agc aat gtt   163
   Pro Arg Ser Pro Val Ile Val Asn Leu Gly Glu Thr Met Ser Asn Val
                   10                  15                  20
   gtt gca gta acc gag cag acc ttc aag tcc acc gtc atc gat tcc gac   211
   Val Ala Val Thr Glu Gln Thr Phe Lys Ser Thr Val Ile Asp Ser Asp
               25                  30                  35
   aag cca gtc atc gtt gac ttc tgg gca gaa tgg tgt ggc ccc tgc aag   259
   Lys Pro Val Ile Val Asp Phe Trp Ala Glu Trp Cys Gly Pro Cys Lys
           40                  45                  50
   aag ctc agc ccc atc att gag gaa atc gca ggc gag tac ggc gac aag   307
   Lys Leu Ser Pro Ile Ile Glu Glu Ile Ala Gly Glu Tyr Gly Asp Lys
       55                  60                  65
   gca gtc gtt gcc agc gtc gac gtc gat gca gag cgt acc ttg ggt gcc   355
   Ala Val Val Ala Ser Val Asp Val Asp Ala Glu Arg Thr Leu Gly Ala
   70                  75                  80                  85
   atg ttc cag att atg tcg att cct tct gtt ctc att ttc aaa aat ggt   403
   Met Phe Gln Ile Met Ser Ile Pro Ser Val Leu Ile Phe Lys Asn Gly
                   90                  95                  100
   gca aaa gtc gag gaa ttt gtc ggt ctg cgc ccc aag aac gaa att gtg   451
   Ala Lys Val Glu Glu Phe Val Gly Leu Arg Pro Lys Asn Glu Ile Val
               105                 110                 115
   gaa aaa cta gag aag cac ctc tagctggtat tcttactgca gtcacgtgga      502
   Glu Lys Leu Glu Lys His Leu
           120
<210> 34
   <211> 124
   <212> PRT
   <213> Corynebacterium glutamicum
<400> 34
   Met Asn Val Gly Phe Pro Arg Ser Pro Val Ile Val Asn Leu Gly Glu
   1               5                   10                  15
   Thr Met Ser Asn Val Val Ala Val Thr Glu Gln Thr Phe Lys Ser Thr
               20                  25                  30
   Val Ile Asp Ser Asp Lys Pro Val Ile Val Asp Phe Trp Ala Glu Trp
           35                  40                  45
   Cys Gly Pro Cys Lys Lys Leu Ser Pro Ile Ile Glu Glu Ile Ala Gly
       50                  55                  60
   Glu Tyr Gly Asp Lys Ala Val Val Ala Ser Val Asp Val Asp Ala Glu
   65                  70                  75                  80
   Arg Thr Leu Gly Ala Met Phe Gln Ile Met Ser Ile Pro Ser Val Leu
                   85                  90                  95
   Ile Phe Lys Asn Gly Ala Lys Val Glu Glu Phe Val Gly Leu Arg Pro
               100                 105                 110
   Lys Asn Glu Ile Val Glu Lys Leu Glu Lys His Leu
           115                 120
<210> 35
   <211> 1495
   <212> DNA
   <213> Corynebacterium glutamicum
<220>
   <221> CDS
   <222> (101)..(1465)
   <223> RXA01438
<400> 35
   ccattagcag tcgcaccccg ataggagtcg aatctacaag tggaaccccc gctcacatac 60
   tccacatttt ttagaacccc tttaaggaat cgaactttat atg tct cgc cct ttg   115
                                               Met Ser Arg Pro Leu
                                                1               5
   cgt gtt gcc gtt gtc ggt gca ggt cca gca gga atc tac gcg tct gat   163
   Arg Val Ala Val Val Gly Ala Gly Pro Ala Gly Ile Tyr Ala Ser Asp
                   10                  15                  20
   ttg ttg atg aaa tcc gac acg gac gtg cag att gat ctt ttt gaa cgt   211
   Leu Leu Met Lys Ser Asp Thr Asp Val Gln Ile Asp Leu Phe Glu Arg
               25                  30                  35
   atg cca gcg cct ttc ggt ttg atc cgt tat ggt gtt gcg cct gat cac   259
   Met Pro Ala Pro Phe Gly Leu Ile Arg Tyr Gly Val Ala Pro Asp His
           40                  45                  50
   cct cgc atc aag ggc atc gtg aag tcc ctg cac aat gtg atg gac aag   307
   Pro Arg Ile Lys Gly Ile Val Lys Ser Leu His Asn Val Met Asp Lys
       55                  60                  65
   gag cag ctg cgt ttc ttg ggc aac att gag gtc ggc aag gac atc act   355
   Glu Gln Leu Arg Phe Leu Gly Asn Ile Glu Val Gly Lys Asp Ile Thr
   70                  75                  80                  85
   gtt gag gag ttg cgt gag ttt tat gac gcg atc gtg ttc tcc act ggc   403
   Val Glu Glu Leu Arg Glu Phe Tyr Asp Ala Ile Val Phe Ser Thr Gly
                   90                  95                  100
   gct act ggc gac cag gat ctt cgg gtt cca ggt tct gat ctg gaa ggt   451
   Ala Thr Gly Asp Gln Asp Leu Arg Val Pro Gly Ser Asp Leu Glu Gly
               105                 110                 115
   tcg tgg ggc gct ggc gag ttc gtt ggt ttc tat gat ggc aac ccg aac   499
   Ser Trp Gly Ala Gly Glu Phe Val Gly Phe Tyr Asp Gly Asn Pro Asn
           120                 125                 130
   ttt gaa cgc aac tgg gat ctt tct gct gag aag gta gcg gtt gtt ggt   547
   Phe Glu Arg Asn Trp Asp Leu Ser Ala Glu Lys Val Ala Val Val Gly
       135                 140                 145
   gtc ggt aac gtg gcg ttg gac gtt gct cgt att ttg gcg aag act ggc   595
   Val Gly Asn Val Ala Leu Asp Val Ala Arg Ile Leu Ala Lys Thr Gly
   150                 155                 160                 165
   gat gag ctg cta gtt act gaa atc cct gac aat gtc tat gag agc ttg   643
   Asp Glu Leu Leu Val Thr Glu Ile Pro Asp Asn Val Tyr Glu Ser Leu
                   170                 175                 180
   gct aag aat cag gct aag gaa gtg cac gtt ttt ggt cgt cgt gga cct   691
   Ala Lys Asn Gln Ala Lys Glu Val His Val Phe Gly Arg Arg Gly Pro
               185                 190                 195
   gct cag gcg aag ttc act ccg ttg gag ctg aag gaa ctt gac cat tcc   739
   Ala Gln Ala Lys Phe Thr Pro Leu Glu Leu Lys Glu Leu Asp His Ser
           200                 205                 210
   gac acc atc gag gtg atc gtg aac cct gag gac att gat tac gat gca   787
   Asp Thr Ile Glu Val Ile Val Asn Pro Glu Asp Ile Asp Tyr Asp Ala
       215                 220                 225
   gct tcg gag cag gct cgt cgt gat tcc aag tct cag gac ctc gtg tgc   835
   Ala Ser Glu Gln Ala Arg Arg Asp Ser Lys Ser Gln Asp Leu Val Cys
   230                 235                 240                 245
   cag act ttg gaa agc tac gcg atg cgc gat cct aag ggc gct cct cac    883
   Gln Thr Leu Glu Ser Tyr Ala Met Arg Asp Pro Lys Gly Ala Pro His
                   250                 255                 260
   aag ctg ttc att cac ttc ttt gag tcc cca gtg gag atc ctc ggt gag    931
   Lys Leu Phe Ile His Phe Phe Glu Ser Pro Val Glu Ile Leu Gly Glu
               265                 270                 275
   gac ggc aag gtt gtt ggc ctc aag act gag cgt act cag ctg gac ggc    979
   Asp Gly Lys Val Val Gly Leu Lys Thr Glu Arg Thr Gln Leu Asp Gly
           280                 285                 290
   aac ggt ggc gtg act ggc acc ggc gag ttc aag acc tgg gat atg cag    1027
   Asn Gly Gly Val Thr Gly Thr Gly Glu Phe Lys Thr Trp Asp Met Gln
       295                 300                 305
   tca gtt tac cgc gcg gta ggt tac cgt tct gat gcg atc gag ggt gtt    1075
   Ser Val Tyr Arg Ala Val Gly Tyr Arg Ser Asp Ala Ile Glu Gly Val
   310                 315                 320                 325
   cct ttt gac gat gag cgc gcg gtt gtc ccc aac gac ggc ggc cac atc    1123
   Pro Phe Asp Asp Glu Arg Ala Val Val Pro Asn Asp Gly Gly His Ile
                   330                 335                 340
   atc gat cct gag gtc ggc tcc ccc atc act ggc ctg tac gcc act ggc    1171
   Ile Asp Pro Glu Val Gly Ser Pro Ile Thr Gly Leu Tyr Ala Thr Gly
               345                 350                 355
   tgg atc aag cgt ggc cca att gga ctg atc ggc aac acc aag tcc gac    1219
   Trp Ile Lys Arg Gly Pro Ile Gly Leu Ile Gly Asn Thr Lys Ser Asp
           360                 365                 370
   gcc aag gaa acc act gag atg ctg ctt gct gat cac gct gct ggt tct    1267
   Ala Lys Glu Thr Thr Glu Met Leu Leu Ala Asp His Ala Ala Gly Ser
       375                 380                 385
   ttg cct gcg cct gca aag cct gag ttg gag tcc atc att gag ttc ctc    1315
   Leu Pro Ala Pro Ala Lys Pro Glu Leu Glu Ser Ile Ile Glu Phe Leu
   390                 395                 400                 405
   gat gag cgc aag gtt gcg ttc acc aca tgg gat ggc tgg cac ctg ctg    1363
   Asp Glu Arg Lys Val Ala Phe Thr Thr Trp Asp Gly Trp His Leu Leu
                   410                 415                 420
   gat gct gcg gag cgc gcg ctg ggt gag cct gag ggc cgc gag cgc aag    1411
   Asp Ala Ala Glu Arg Ala Leu Gly Glu Pro Glu Gly Arg Glu Arg Lys
               425                 430                 435
   aag atc gtt gag tgg aat gac atg gtg cgc cat gct cgt cca gaa tac    1459
   Lys Ile Val Glu Trp Asn Asp Met Val Arg His Ala Arg Pro Glu Tyr
           440                 445                 450
   gac atc taaagtcgct taaagcctca aaaaagggcg                          1495
   Asp Ile
       455
<210> 36
   <211> 455
   <212> PRT
   <213> Corynebacterium glutamicum
<400> 36
   Met Ser Arg Pro Leu Arg Val Ala Val Val Gly Ala Gly Pro Ala Gly
   1 5 10 15
   Ile Tyr Ala Ser Asp Leu Leu Met Lys Ser Asp Thr Asp Val Gln Ile
   20 25 30
   Asp Leu Phe Glu Arg Met Pro Ala Pro Phe Gly Leu Ile Arg Tyr Gly
   35 40 45
   Val Ala Pro Asp His Pro Arg Ile Lys Gly Ile Val Lys Ser Leu His
   50 55 60
   Asn Val Met Asp Lys Glu Gln Leu Arg Phe Leu Gly Asn Ile Glu Val
   65 70 75 80
   Gly Lys Asp Ile Thr Val Glu Glu Leu Arg Glu Phe Tyr Asp Ala Ile
   85 90 95
   Val Phe Ser Thr Gly Ala Thr Gly Asp Gln Asp Leu Arg Val Pro Gly
   100 105 110
   Ser Asp Leu Glu Gly Ser Trp Gly Ala Gly Glu Phe Val Gly Phe Tyr
   115 120 125
   Asp Gly Asn Pro Asn Phe Glu Arg Asn Trp Asp Leu Ser Ala Glu Lys
   130 135 140
   Val Ala Val Val Gly Val Gly Asn Val Ala Leu Asp Val Ala Arg Ile
   145 150 155 160
   Leu Ala Lys Thr Gly Asp Glu Leu Leu Val Thr Glu Ile Pro Asp Asn
   165 170 175
   Val Tyr Glu Ser Leu Ala Lys Asn Gln Ala Lys Glu Val His Val Phe
   180 185 190
   Gly Arg Arg Gly Pro Ala Gln Ala Lys Phe Thr Pro Leu Glu Leu Lys
   195 200 205
   Glu Leu Asp His Ser Asp Thr Ile Glu Val Ile Val Asn Pro Glu Asp
   210 215 220
   Ile Asp Tyr Asp Ala Ala Ser Glu Gln Ala Arg Arg Asp Ser Lys Ser
   225 230 235 240
   Gln Asp Leu Val Cys Gln Thr Leu Glu Ser Tyr Ala Met Arg Asp Pro
   245 250 255
   Lys Gly Ala Pro His Lys Leu Phe Ile His Phe Phe Glu Ser Pro Val
   260 265 270
   Glu Ile Leu Gly Glu Asp Gly Lys Val Val Gly Leu Lys Thr Glu Arg
   275 280 285
   Thr Gln Leu Asp Gly Asn Gly Gly Val Thr Gly Thr Gly Glu Phe Lys
   290 295 300
   Thr Trp Asp Met Gln Ser Val Tyr Arg Ala Val Gly Tyr Arg Ser Asp
   305 310 315 320
   Ala Ile Glu Gly Val Pro Phe Asp Asp Glu Arg Ala Val Val Pro Asn
   325 330 335
   Asp Gly Gly His Ile Ile Asp Pro Glu Val Gly Ser Pro Ile Thr Gly
   340 345 350
   Leu Tyr Ala Thr Gly Trp Ile Lys Arg Gly Pro Ile Gly Leu Ile Gly
   355 360 365
   Asn Thr Lys Ser Asp Ala Lys Glu Thr Thr Glu Met Leu Leu Ala Asp
   370 375 380
   His Ala Ala Gly Ser Leu Pro Ala Pro Ala Lys Pro Glu Leu Glu Ser
   385 390 395 400
   Ile Ile Glu Phe Leu Asp Glu Arg Lys Val Ala Phe Thr Thr Trp Asp
   405 410 415
   Gly Trp His Leu Leu Asp Ala Ala Glu Arg Ala Leu Gly Glu Pro Glu
   420 425 430
   Gly Arg Glu Arg Lys Lys Ile Val Glu Trp Asn Asp Met Val Arg His
   435 440 445
   Ala Arg Pro Glu Tyr Asp Ile
   450 455
<210> 37
   <211> 1021
   <212> DNA
   <213> Corynebacterium glutamicum
<220>
   <221> CDS
   <222> (101)..(991)
   <223> RXA01490
<400> 37
   cacaccaatg gtgactactg atccttgaag atcagccgga acgctgtcta gtccactcca 60
   aatatccact gttttagact acggcataga ctcaacagac atg aat gct cct gcc   115
                                               Met Asn Ala Pro Ala
                                                1               5
   cct aaa cct gga ctc gtg atc gtc gac aag ccc gcc gga atg aca tcc   163
   Pro Lys Pro Gly Leu Val Ile Val Asp Lys Pro Ala Gly Met Thr Ser
                       10                  15                  20
   cat gac gtg gtg tcc aaa ttg cgc cgc gca ttt tcc acc cgc aaa gta   211
   His Asp Val Val Ser Lys Leu Arg Arg Ala Phe Ser Thr Arg Lys Val
                   25                  30                  35
   ggc cac gca ggc acc ctc gac ccc atg gca acc ggc gtg tta gtc gtc   259
   Gly His Ala Gly Thr Leu Asp Pro Met Ala Thr Gly Val Leu Val Val
               40                  45                  50
   gga att gag cgc gga acc cgc ttc ctg gca cac atg gtg gcc tcc acc   307
   Gly Ile Glu Arg Gly Thr Arg Phe Leu Ala His Met Val Ala Ser Thr
       55                  60                  65
   aaa gcc tac gac gcc acc att cga ctc ggc gcc gcc acc agc acc gat   355
   Lys Ala Tyr Asp Ala Thr Ile Arg Leu Gly Ala Ala Thr Ser Thr Asp
   70                  75                  80                  85
   gat gca gaa ggc gag gtt atc tcc aca aca gac gca tcc ggc ctc gac   403
   Asp Ala Glu Gly Glu Val Ile Ser Thr Thr Asp Ala Ser Gly Leu Asp
                   90                  95                  100
   cac agc acc atc ctt gct gaa atc gtc aac ctc acc ggc gac atc atg      451
   His Ser Thr Ile Leu Ala Glu Ile Val Asn Leu Thr Gly Asp Ile Met
               105                 110                 115
   caa aaa ccc acc aaa gtc tcc gcc atc aaa atc gac ggc aaa cgc gcc      499
   Gln Lys Pro Thr Lys Val Ser Ala Ile Lys Ile Asp Gly Lys Arg Ala
           120                 125                 130
   cac gaa cgc gtc cgc gac ggc gaa gaa gta gac att ccc gca cgt ccc      547
   His Glu Arg Val Arg Asp Gly Glu Glu Val Asp Ile Pro Ala Arg Pro
       135                 140                 145
   gtc acc gtc agc gtc ttt gac gtg ctc gac tac cac gtc gac ggt gaa      595
   Val Thr Val Ser Val Phe Asp Val Leu Asp Tyr His Val Asp Gly Glu
   150                 155                 160                 165
   ttt tat gac tta gat gtg cgc gtc cac tgc tcc tcc ggc acc tac atc      643
   Phe Tyr Asp Leu Asp Val Arg Val His Cys Ser Ser Gly Thr Tyr Ile
                   170                 175                 180
   cgc gcg ctc gcc cgc gac ctc ggc aac gct ttg cag gtc ggc ggc cac      691
   Arg Ala Leu Ala Arg Asp Leu Gly Asn Ala Leu Gln Val Gly Gly His
               185                 190                 195
   ctg acc gcg ctt agg cgc aca gag gtc ggc cct ttt acg ctt aac gac      739
   Leu Thr Ala Leu Arg Arg Thr Glu Val Gly Pro Phe Thr Leu Asn Asp
           200                 205                 210
   gcg acc ccc ctc tcc aaa ctc caa gag aat cca gaa ctc tcc ctc aac      787
   Ala Thr Pro Leu Ser Lys Leu Gln Glu Asn Pro Glu Leu Ser Leu Asn
       215                 220                 225
   ctc gac cag gca ctc acc cgc agt tac cca gtc ctt gac atc acc gaa      835
   Leu Asp Gln Ala Leu Thr Arg Ser Tyr Pro Val Leu Asp Ile Thr Glu
   230                 235                 240                 245
   gac gaa ggc gtt gac ctg tcc atg ggc aaa tgg ttg gaa cct cgc gga      883
   Asp Glu Gly Val Asp Leu Ser Met Gly Lys Trp Leu Glu Pro Arg Gly
                   250                 255                 260
   ctg aaa ggc gtc cac gct gca gta aca cca tca gga aaa gcc gtg gcg      931
   Leu Lys Gly Val His Ala Ala Val Thr Pro Ser Gly Lys Ala Val Ala
               265                 270                 275
   ctc atc gaa gaa aag ggc aaa cgc ctg gcc acc gtg ttt gtt gct cac      979
   Leu Ile Glu Glu Lys Gly Lys Arg Leu Ala Thr Val Phe Val Ala His
           280                 285                 290
   ccc aac act ctt tagttggtct gccagaagcc gatttaagag                     1021
   Pro Asn Thr Leu
       295
<210> 38
   <211> 297
   <212> PRT
   <213> Corynebacterium glutamicum
<400> 38
   Met Asn Ala Pro Ala Pro Lys Pro Gly Leu Val Ile Val Asp Lys Pro
   1 5 10 15
   Ala Gly Met Thr Ser His Asp Val Val Ser Lys Leu Arg Arg Ala Phe
   20 25 30
   Ser Thr Arg Lys Val Gly His Ala Gly Thr Leu Asp Pro Met Ala Thr
   35 40 45
   Gly Val Leu Val Val Gly Ile Glu Arg Gly Thr Arg Phe Leu Ala His
   50 55 60
   Met Val Ala Ser Thr Lys Ala Tyr Asp Ala Thr Ile Arg Leu Gly Ala
   65 70 75 80
   Ala Thr Ser Thr Asp Asp Ala Glu Gly Glu Val Ile Ser Thr Thr Asp
   85 90 95
   Ala Ser Gly Leu Asp His Ser Thr Ile Leu Ala Glu Ile Val Asn Leu
   100 105 110
   Thr Gly Asp Ile Met Gln Lys Pro Thr Lys Val Ser Ala Ile Lys Ile
   115 120 125
   Asp Gly Lys Arg Ala His Glu Arg Val Arg Asp Gly Glu Glu Val Asp
   130 135 140
   Ile Pro Ala Arg Pro Val Thr Val Ser Val Phe Asp Val Leu Asp Tyr
   145 150 155 160
   His Val Asp Gly Glu Phe Tyr Asp Leu Asp Val Arg Val His Cys Ser
   165 170 175
   Ser Gly Thr Tyr Ile Arg Ala Leu Ala Arg Asp Leu Gly Asn Ala Leu
   180 185 190
   Gln Val Gly Gly His Leu Thr Ala Leu Arg Arg Thr Glu Val Gly Pro
   195 200 205
   Phe Thr Leu Asn Asp Ala Thr Pro Leu Ser Lys Leu Gln Glu Asn Pro
   210 215 220
   Glu Leu Ser Leu Asn Leu Asp Gln Ala Leu Thr Arg Ser Tyr Pro Val
   225 230 235 240
   Leu Asp Ile Thr Glu Asp Glu Gly Val Asp Leu Ser Met Gly Lys Trp
   245 250 255
   Leu Glu Pro Arg Gly Leu Lys Gly Val His Ala Ala Val Thr Pro Ser
   260 265 270
   Gly Lys Ala Val Ala Leu Ile Glu Glu Lys Gly Lys Arg Leu Ala Thr
   275 280 285
   Val Phe Val Ala His Pro Asn Thr Leu
   290 295
<210> 39
   <211> 1441
   <212> DNA
   <213> Corynebacterium glutamicum
<220>
   <221> CDS
   <222> (101)..(1411)
   <223> RXA01493
<400> 39
   cctgctgcag gctataccgc tcgtggtacg gaaatcgaag ccctcgatac gttgattgaa 60
   gcaaccgtta ccttggggga gtctttgcga agctcggcgc atg tcg atg tct aac   115
                                               Met Ser Met Ser Asn
                                                1               5
   aac gac ttt gag cat gag tcc cat gat gtt tct gca aag cag atc ttc   163
   Asn Asp Phe Glu His Glu Ser His Asp Val Ser Ala Lys Gln Ile Phe
                   10                  15                  20
   ggg ctc gcg ttc ccc gca ctg ggt gtt cta gct gcg atg ccg ctg tat   211
   Gly Leu Ala Phe Pro Ala Leu Gly Val Leu Ala Ala Met Pro Leu Tyr
               25                  30                  35
   ctc ttg ttg gat aca gcg gtt gtt ggc act ttg ggt ggc ttc gaa ttg   259
   Leu Leu Leu Asp Thr Ala Val Val Gly Thr Leu Gly Gly Phe Glu Leu
           40                  45                  50
   gct gcg ttg ggc gca gca aca aca att caa gct caa gtg aca aca cag   307
   Ala Ala Leu Gly Ala Ala Thr Thr Ile Gln Ala Gln Val Thr Thr Gln
       55                  60                  65
   ctg aca ttc ttg tcc tat gga act acc gcg aga tca tcg aga att ttc   355
   Leu Thr Phe Leu Ser Tyr Gly Thr Thr Ala Arg Ser Ser Arg Ile Phe
    70                  75                  80                  85
   gga atg ggt gat cgc cgg gga gca att gcc gaa ggt gtg caa gca acc   403
   Gly Met Gly Asp Arg Arg Gly Ala Ile Ala Glu Gly Val Gln Ala Thr
                   90                  95                  100
   tgg gtg gca ctc ttt gta ggc ttg ggc atc tta acg ctg atg ctc att   451
   Trp Val Ala Leu Phe Val Gly Leu Gly Ile Leu Thr Leu Met Leu Ile
               105                 110                 115
   gga gcc ccg act ttc gcg ttg tgg ctc agt ggt gat gaa gct cta gcc   499
   Gly Ala Pro Thr Phe Ala Leu Trp Leu Ser Gly Asp Glu Ala Leu Ala
           120                 125                 130
   caa gaa gca ggg cat tgg ctc cgg gtc gct gct ttt gcg gtg cca cta   547
   Gln Glu Ala Gly His Trp Leu Arg Val Ala Ala Phe Ala Val Pro Leu
       135                 140                 145
   att ctc atg atc atg gct ggc aac ggt tgg tta aga ggt att caa aac   595
   Ile Leu Met Ile Met Ala Gly Asn Gly Trp Leu Arg Gly Ile Gln Asn
   150                 155                 160                 165
   acc aag ctg cca ctc tat ttc acc ttg gcg gga gtc atc ccc ggc gcg   643
   Thr Lys Leu Pro Leu Tyr Phe Thr Leu Ala Gly Val Ile Pro Gly Ala
                   170                 175                 180
   atc ttg att ccg ata ttc gtg gct aag ttt gga ctt gtg ggc tct gcc   691
   Ile Leu Ile Pro Ile Phe Val Ala Lys Phe Gly Leu Val Gly Ser Ala
               185                 190                 195
   tgg gca aac ctc att gca gaa gca att act gct tcg ctg ttt ttg ggt   739
   Trp Ala Asn Leu Ile Ala Glu Ala Ile Thr Ala Ser Leu Phe Leu Gly
           200                 205                 210
   gca ttg atc aag cac cac gaa ggt tcg tgg aag ccg agc tgg acg gtg   787
   Ala Leu Ile Lys His His Glu Gly Ser Trp Lys Pro Ser Trp Thr Val
       215                 220                 225
   atg aaa aat cag ttg gtt ctt gga cgt gat ttg atc atg cgg tca atg     835
   Met Lys Asn Gln Leu Val Leu Gly Arg Asp Leu Ile Met Arg Ser Met
   230                 235                 240                 245
   tcg ttc cag gtt gct ttt ctt tcc gcg gcc gct gtg gct gca cga ttt     883
   Ser Phe Gln Val Ala Phe Leu Ser Ala Ala Ala Val Ala Ala Arg Phe
                   250                 255                 260
   ggc acg gca tcc ttg gcg gcc cac cag gtg ttg ctt cag ctg tgg aat     931
   Gly Thr Ala Ser Leu Ala Ala His Gln Val Leu Leu Gln Leu Trp Asn
               265                 270                 275
   ttc atc aca ttg gtg ctg gat tct cta gct atc gcg gcg cag acc tta     979
   Phe Ile Thr Leu Val Leu Asp Ser Leu Ala Ile Ala Ala Gln Thr Leu
           280                 285                 290
   act ggt gca gcc ctg ggc gct gga act gcg aag gtc gcc cgc agg gtg     1027
   Thr Gly Ala Ala Leu Gly Ala Gly Thr Ala Lys Val Ala Arg Arg Val
       295                 300                 305
   ggt aat cag gtg att aag tac tct ctg att ttc gct ggt ggc tta ggt     1075
   Gly Asn Gln Val Ile Lys Tyr Ser Leu Ile Phe Ala Gly Gly Leu Gly
   310                 315                 320                 325
   ttg gtg ttc gtg gtc tta cac tcg tgg att ccg cgt att ttc act cag     1123
   Leu Val Phe Val Val Leu His Ser Trp Ile Pro Arg Ile Phe Thr Gln
                   330                 335                 340
   gac gcc gac gtt tta gat gcg att gct tcc ccg tgg tgg atc atg gtc     1171
   Asp Ala Asp Val Leu Asp Ala Ile Ala Ser Pro Trp Trp Ile Met Val
               345                 350                 355
   gcg atg atc att ttg ggt ggc att gtc ttt gct att gat ggt gtg ctg     1219
   Ala Met Ile Ile Leu Gly Gly Ile Val Phe Ala Ile Asp Gly Val Leu
           360                 365                 370
   ttg ggt gct gct gac gcg gtg ttc ctc cga aat gcc tct atc ttg gcg     1267
   Leu Gly Ala Ala Asp Ala Val Phe Leu Arg Asn Ala Ser Ile Leu Ala
       375                 380                 385
   gtt gtg gtc gga ttc tta cca ggc gtc tgg att tcc tat gca tta gat     1315
   Val Val Val Gly Phe Leu Pro Gly Val Trp Ile Ser Tyr Ala Leu Asp
   390                 395                 400                 405
   gca ggg ctg aca ggc gtg tgg tgt ggt ttg ctg gcg ttt att ctg atc     1363
   Ala Gly Leu Thr Gly Val Trp Cys Gly Leu Leu Ala Phe Ile Leu Ile
                   410                 415                 420
   cga cta ttt gcg gtg att tgg cgg ttt aag tct atg aag tgg gcg cgt     1411
   Arg Leu Phe Ala Val Ile Trp Arg Phe Lys Ser Met Lys Trp Ala Arg
               425                 430                 435
   tagcttcggc gcgtggcaaa ccacatttgc                                    1441
<210> 40
   <211> 437
   <212> PRT
   <213> Corynebacterium glutamicum
<400> 40
   Met Ser Met Ser Asn Asn Asp Phe Glu His Glu Ser His Asp Val Ser
   1 5 10 15
   Ala Lys Gln Ile Phe Gly Leu Ala Phe Pro Ala Leu Gly Val Leu Ala
   20 25 30
   Ala Met Pro Leu Tyr Leu Leu Leu Asp Thr Ala Val Val Gly Thr Leu
   35 40 45
   Gly Gly Phe Glu Leu Ala Ala Leu Gly Ala Ala Thr Thr Ile Gln Ala
   50 55 60
   Gln Val Thr Thr Gln Leu Thr Phe Leu Ser Tyr Gly Thr Thr Ala Arg
   65 70 75 80
   Ser Ser Arg Ile Phe Gly Met Gly Asp Arg Arg Gly Ala Ile Ala Glu
   85 90 95
   Gly Val Gln Ala Thr Trp Val Ala Leu Phe Val Gly Leu Gly Ile Leu
   100 105 110
   Thr Leu Met Leu Ile Gly Ala Pro Thr Phe Ala Leu Trp Leu Ser Gly
   115 120 125
   Asp Glu Ala Leu Ala Gln Glu Ala Gly His Trp Leu Arg Val Ala Ala
   130 135 140
   Phe Ala Val Pro Leu Ile Leu Met Ile Met Ala Gly Asn Gly Trp Leu
   145 150 155 160
   Arg Gly Ile Gln Asn Thr Lys Leu Pro Leu Tyr Phe Thr Leu Ala Gly
   165 170 175
   Val Ile Pro Gly Ala Ile Leu Ile Pro Ile Phe Val Ala Lys Phe Gly
   180 185 190
   Leu Val Gly Ser Ala Trp Ala Asn Leu Ile Ala Glu Ala Ile Thr Ala
   195 200 205
   Ser Leu Phe Leu Gly Ala Leu Ile Lys His His Glu Gly Ser Trp Lys
   210 215 220
   Pro Ser Trp Thr Val Met Lys Asn Gln Leu Val Leu Gly Arg Asp Leu
   225 230 235 240
   Ile Met Arg Ser Met Ser Phe Gln Val Ala Phe Leu Ser Ala Ala Ala
   245 250 255
   Val Ala Ala Arg Phe Gly Thr Ala Ser Leu Ala Ala His Gln Val Leu
   260 265 270
   Leu Gln Leu Trp Asn Phe Ile Thr Leu Val Leu Asp Ser Leu Ala Ile
   275 280 285
   Ala Ala Gln Thr Leu Thr Gly Ala Ala Leu Gly Ala Gly Thr Ala Lys
   290 295 300
   Val Ala Arg Arg Val Gly Asn Gln Val Ile Lys Tyr Ser Leu Ile Phe
   305 310 315 320
   Ala Gly Gly Leu Gly Leu Val Phe Val Val Leu His Ser Trp Ile Pro
   325 330 335
   Arg Ile Phe Thr Gln Asp Ala Asp Val Leu Asp Ala Ile Ala Ser Pro
               340                 345                 350
   Trp Trp Ile Met Val Ala Met Ile Ile Leu Gly Gly Ile Val Phe Ala
           355                 360                 365
   Ile Asp Gly Val Leu Leu Gly Ala Ala Asp Ala Val Phe Leu Arg Asn
       370                 375                 380
   Ala Ser Ile Leu Ala Val Val Val Gly Phe Leu Pro Gly Val Trp Ile
   385                 390                 395                 400
   Ser Tyr Ala Leu Asp Ala Gly Leu Thr Gly Val Trp Cys Gly Leu Leu
                   405                 410                 415
   Ala Phe Ile Leu Ile Arg Leu Phe Ala Val Ile Trp Arg Phe Lys Ser
               420                 425                 430
   Met Lys Trp Ala Arg
           435
<210> 41
   <211> 2056
   <212> DNA
   <213> Corynebacterium glutamicum
<220>
   <221> CDS
   <222> (101)..(2026)
   <223> RXA01559
<400> 41
   ctttctcgct cgtgtcgcac tcacccacgc cacctggcgt gggtgagtgg cgcatggagt 60
   gggtgggcgt cgacaagcgt ggttgtctgg ttgattggaa ttg aag gag act ttc   115
                                             Leu Lys Glu Thr Phe
                                              1               5
   ttg gct cgg caa aaa aag agt gcc gct agc gcc tgg gaa cga tgg cca   163
   Leu Ala Arg Gln Lys Lys Ser Ala Ala Ser Ala Trp Glu Arg Trp Pro
                   10                  15                  20
   aaa cgc gca ata gcg ttg ttt gtg ctc atc gtc gtt ggt gtt tat gcg   211
   Lys Arg Ala Ile Ala Leu Phe Val Leu Ile Val Val Gly Val Tyr Ala
               25                  30                  35
   ttg gtg ctg ttg aca ggc gat cgt tct gcc aca cca aaa ttg ggt att   259
   Leu Val Leu Leu Thr Gly Asp Arg Ser Ala Thr Pro Lys Leu Gly Ile
           40                  45                  50
   gat ctg caa ggc gga acc cga gtg acc ctc gtg ccg cag ggg cag gat   307
   Asp Leu Gln Gly Gly Thr Arg Val Thr Leu Val Pro Gln Gly Gln Asp
       55                  60                  65
   cca act cag gac cag ctg aat cag gca cgc acc att ctg gaa aac cgt   355
   Pro Thr Gln Asp Gln Leu Asn Gln Ala Arg Thr Ile Leu Glu Asn Arg
   70                  75                  80                  85
   gtg aac ggc atg ggc gtt tca ggt gca agc gtg gtc gct gac ggt aac   403
   Val Asn Gly Met Gly Val Ser Gly Ala Ser Val Val Ala Asp Gly Asn
                   90                  95                  100
   acg ctg gtg atc act gtt ccc ggg gaa aat acc gca cag gcg caa tcc   451
   Thr Leu Val Ile Thr Val Pro Gly Glu Asn Thr Ala Gln Ala Gln Ser
   105 110 115
   cta gga cag acc tcc cag ctg ctg ttc cgt ccc gtt ggt cag gca gga 499
   Leu Gly Gln Thr Ser Gln Leu Leu Phe Arg Pro Val Gly Gln Ala Gly
   120 125 130
   atg ccc gat atg acc acg ttg atg cca gag ctg gaa gag atg gcc aac 547
   Met Pro Asp Met Thr Thr Leu Met Pro Glu Leu Glu Glu Met Ala Asn
   135 140 145
   agg tgg gtt gaa tac ggc gtc atc acc gaa gag cag gca aat gcc tcc 595
   Arg Trp Val Glu Tyr Gly Val Ile Thr Glu Glu Gln Ala Asn Ala Ser
   150 155 160 165
   ttg gag gaa atg aac acc gct gtt gca tcg acc act gcg gtg gaa ggc 643
   Leu Glu Glu Met Asn Thr Ala Val Ala Ser Thr Thr Ala Val Glu Gly
   170 175 180
   gaa gaa gca act gag cca gaa ccc gtc acc gtg tcg gcg acc cct atg 691
   Glu Glu Ala Thr Glu Pro Glu Pro Val Thr Val Ser Ala Thr Pro Met
   185 190 195
   gat gag cca gcc aac tcc att gag gca aca cag cga cgc cag gaa atc 739
   Asp Glu Pro Ala Asn Ser Ile Glu Ala Thr Gln Arg Arg Gln Glu Ile
   200 205 210
   acg gac atg ctg cgc acc gac cgc cag tcc acc gat ccc act gtc cag 787
   Thr Asp Met Leu Arg Thr Asp Arg Gln Ser Thr Asp Pro Thr Val Gln
   215 220 225
   atc gct gca agt tct ttg atg cag tgc acc act gat gag atg gat cct 835
   Ile Ala Ala Ser Ser Leu Met Gln Cys Thr Thr Asp Glu Met Asp Pro
   230 235 240 245
   ttg gcc ggc acc gat gat cca cgc ctg cca ttg gtg gca tgt gat cca 883
   Leu Ala Gly Thr Asp Asp Pro Arg Leu Pro Leu Val Ala Cys Asp Pro
   250 255 260
   gct gta ggt ggc gtg tat gta ctt gat cct gca cct ttg ctc aac ggc 931
   Ala Val Gly Gly Val Tyr Val Leu Asp Pro Ala Pro Leu Leu Asn Gly
   265 270 275
   gaa acc gat gag gaa aat ggt gcg cgc cta acc ggt aat gag atc gat 979
   Glu Thr Asp Glu Glu Asn Gly Ala Arg Leu Thr Gly Asn Glu Ile Asp
   280 285 290
   acc aac cgt ccc atc acc ggt gga ttc aac gcc cag tcc ggc cag atg 1027
   Thr Asn Arg Pro Ile Thr Gly Gly Phe Asn Ala Gln Ser Gly Gln Met
   295 300 305
   gaa atc agc ttt gcc ttc aaa tcc ggc gat ggg gaa gaa ggc tct gca 1075
   Glu Ile Ser Phe Ala Phe Lys Ser Gly Asp Gly Glu Glu Gly Ser Ala
   310 315 320 325
   act tgg tcc tct ctg acc agc cag tac ctg cag cag cag atc gcc atc 1123
   Thr Trp Ser Ser Leu Thr Ser Gln Tyr Leu Gln Gln Gln Ile Ala Ile
   330 335 340
   acc ctg gac tct cag gtg att tct gca ccc gtg att cag tca gca acc 1171
   Thr Leu Asp Ser Gln Val Ile Ser Ala Pro Val Ile Gln Ser Ala Thr
   345 350 355
   cct gtg ggt tct gca aca tcc atc acc ggt gac ttc act caa act gaa    1219
   Pro Val Gly Ser Ala Thr Ser Ile Thr Gly Asp Phe Thr Gln Thr Glu
           360                 365                 370
   gcc caa gat ctg gcg aac aac ctg cgc tac ggt gca ttg ccc ctg agc    1267
   Ala Gln Asp Leu Ala Asn Asn Leu Arg Tyr Gly Ala Leu Pro Leu Ser
       375                 380                 385
   ttc gca ggt gaa aac ggc gag cgc ggc gga act acc acc acc gtt ccg    1315
   Phe Ala Gly Glu Asn Gly Glu Arg Gly Gly Thr Thr Thr Thr Val Pro
   390                 395                 400                 405
   cca tca cta ggc gca gca tcc ttg aag gcc gga ctg atc gca ggc atc    1363
   Pro Ser Leu Gly Ala Ala Ser Leu Lys Ala Gly Leu Ile Ala Gly Ile
                   410                 415                 420
   gtc ggc atc gcg ctg gtc gcc atc ttc gtg ttc gcc tac tac cgc gtc    1411
   Val Gly Ile Ala Leu Val Ala Ile Phe Val Phe Ala Tyr Tyr Arg Val
               425                 430                 435
   ttc gga ttc gtt tcc ctg ttc acc ctg ttt gcc gca ggc gtg ttg gtc    1459
   Phe Gly Phe Val Ser Leu Phe Thr Leu Phe Ala Ala Gly Val Leu Val
           440                 445                 450
   tac ggc ctt ctg gta ctg ctg gga cgc tgg atc gga tat tcc cta gac    1507
   Tyr Gly Leu Leu Val Leu Leu Gly Arg Trp Ile Gly Tyr Ser Leu Asp
       455                 460                 465
   ctt gct ggt atc gcc ggt ttg atc atc ggt atc ggt acc acc gcc gac    1555
   Leu Ala Gly Ile Ala Gly Leu Ile Ile Gly Ile Gly Thr Thr Ala Asp
   470                 475                 480                 485
   tcc ttc gtg gtg ttc tat gag cgc atc aag gat gag atc cgt gaa gga    1603
   Ser Phe Val Val Phe Tyr Glu Arg Ile Lys Asp Glu Ile Arg Glu Gly
                   490                 495                 500
   aga tcc ttt aga tct gca gta cct cgt gca tgg gaa agc gcc aag cgc    1651
   Arg Ser Phe Arg Ser Ala Val Pro Arg Ala Trp Glu Ser Ala Lys Arg
               505                 510                 515
   acc atc gtc aca ggc aac atg gtc act ttg ctc ggc gct atc gtg att    1699
   Thr Ile Val Thr Gly Asn Met Val Thr Leu Leu Gly Ala Ile Val Ile
           520                 525                 530
   tac ttg ctc gcg gtc ggc gaa gtc aag ggc ttt gcc ttc acc ctg ggt    1747
   Tyr Leu Leu Ala Val Gly Glu Val Lys Gly Phe Ala Phe Thr Leu Gly
       535                 540                 545
   ctg acc acc gta ttc gat ctc gtt gtc acc ttc ctg atc acg gca cca    1795
   Leu Thr Thr Val Phe Asp Leu Val Val Thr Phe Leu Ile Thr Ala Pro
   550                 555                 560                 565
   ctg gtt atc ctg gca tca cgc aac cca ttc ttt gcc aag tca tcg gtc    1843
   Leu Val Ile Leu Ala Ser Arg Asn Pro Phe Phe Ala Lys Ser Ser Val
                   570                 575                 580
   aac ggc atg gga cga gtg atg aag ctc gtt gaa gaa cgc cgc gcc aac    1891
   Asn Gly Met Gly Arg Val Met Lys Leu Val Glu Glu Arg Arg Ala Asn
               585                 590                 595
   ggt gaa ttg gat gag cct gag tac ctg aaa aag atc cat gcc aag aat    1939
   Gly Glu Leu Asp Glu Pro Glu Tyr Leu Lys Lys Ile His Ala Lys Asn
           600                 605                 610
   gcg gca gct gat aag gct tcc act gac aat tct tcc act gac aat tct   1987
   Ala Ala Ala Asp Lys Ala Ser Thr Asp Asn Ser Ser Thr Asp Asn Ser
       615                 620                 625
   gaa gca cct ggc acc gat acg aac caa gag gag gag aag tagccatgac    2036
   Glu Ala Pro Gly Thr Asp Thr Asn Gln Glu Glu Glu Lys
   630                 635                 640
   tgattcccag actgaatcac                                             2056
<210> 42
   <211> 642
   <212> PRT
   <213> Corynebacterium glutamicum
<400> 42
   Leu Lys Glu Thr Phe Leu Ala Arg Gln Lys Lys Ser Ala Ala Ser Ala
   1               5                   10                  15
   Trp Glu Arg Trp Pro Lys Arg Ala Ile Ala Leu Phe Val Leu Ile Val
               20                  25                  30
   Val Gly Val Tyr Ala Leu Val Leu Leu Thr Gly Asp Arg Ser Ala Thr
           35                  40                  45
   Pro Lys Leu Gly Ile Asp Leu Gln Gly Gly Thr Arg Val Thr Leu Val
       50                  55                  60
   Pro Gln Gly Gln Asp Pro Thr Gln Asp Gln Leu Asn Gln Ala Arg Thr
   65                  70                  75                  80
   Ile Leu Glu Asn Arg Val Asn Gly Met Gly Val Ser Gly Ala Ser Val
                   85                  90                  95
   Val Ala Asp Gly Asn Thr Leu Val Ile Thr Val Pro Gly Glu Asn Thr
               100                 105                 110
   Ala Gln Ala Gln Ser Leu Gly Gln Thr Ser Gln Leu Leu Phe Arg Pro
           115                 120                 125
   Val Gly Gln Ala Gly Met Pro Asp Met Thr Thr Leu Met Pro Glu Leu
       130                 135                 140
   Glu Glu Met Ala Asn Arg Trp Val Glu Tyr Gly Val Ile Thr Glu Glu
   145                 150                 155                 160
   Gln Ala Asn Ala Ser Leu Glu Glu Met Asn Thr Ala Val Ala Ser Thr
                   165                 170                 175
   Thr Ala Val Glu Gly Glu Glu Ala Thr Glu Pro Glu Pro Val Thr Val
               180                 185                 190
   Ser Ala Thr Pro Met Asp Glu Pro Ala Asn Ser Ile Glu Ala Thr Gln
           195                 200                 205
   Arg Arg Gln Glu Ile Thr Asp Met Leu Arg Thr Asp Arg Gln Ser Thr
       210                 215                 220
   Asp Pro Thr Val Gln Ile Ala Ala Ser Ser Leu Met Gln Cys Thr Thr
   225                 230                 235                 240
   Asp Glu Met Asp Pro Leu Ala Gly Thr Asp Asp Pro Arg Leu Pro Leu
   245 250 255
   Val Ala Cys Asp Pro Ala Val Gly Gly Val Tyr Val Leu Asp Pro Ala
   260 265 270
   Pro Leu Leu Asn Gly Glu Thr Asp Glu Glu Asn Gly Ala Arg Leu Thr
   275 280 285
   Gly Asn Glu Ile Asp Thr Asn Arg Pro Ile Thr Gly Gly Phe Asn Ala
   290 295 300
   Gln Ser Gly Gln Met Glu Ile Ser Phe Ala Phe Lys Ser Gly Asp Gly
   305 310 315 320
   Glu Glu Gly Ser Ala Thr Trp Ser Ser Leu Thr Ser Gln Tyr Leu Gln
   325 330 335
   Gln Gln Ile Ala Ile Thr Leu Asp Ser Gln Val Ile Ser Ala Pro Val
   340 345 350
   Ile Gln Ser Ala Thr Pro Val Gly Ser Ala Thr Ser Ile Thr Gly Asp
   355 360 365
   Phe Thr Gln Thr Glu Ala Gln Asp Leu Ala Asn Asn Leu Arg Tyr Gly
   370 375 380
   Ala Leu Pro Leu Ser Phe Ala Gly Glu Asn Gly Glu Arg Gly Gly Thr
   385 390 395 400
   Thr Thr Thr Val Pro Pro Ser Leu Gly Ala Ala Ser Leu Lys Ala Gly
   405 410 415
   Leu Ile Ala Gly Ile Val Gly Ile Ala Leu Val Ala Ile Phe Val Phe
   420 425 430
   Ala Tyr Tyr Arg Val Phe Gly Phe Val Ser Leu Phe Thr Leu Phe Ala
   435 440 445
   Ala Gly Val Leu Val Tyr Gly Leu Leu Val Leu Leu Gly Arg Trp Ile
   450 455 460
   Gly Tyr Ser Leu Asp Leu Ala Gly Ile Ala Gly Leu Ile Ile Gly Ile
   465 470 475 480
   Gly Thr Thr Ala Asp Ser Phe Val Val Phe Tyr Glu Arg Ile Lys Asp
   485 490 495
   Glu Ile Arg Glu Gly Arg Ser Phe Arg Ser Ala Val Pro Arg Ala Trp
   500 505 510
   Glu Ser Ala Lys Arg Thr Ile Val Thr Gly Asn Met Val Thr Leu Leu
   515 520 525
   Gly Ala Ile Val Ile Tyr Leu Leu Ala Val Gly Glu Val Lys Gly Phe
   530 535 540
   Ala Phe Thr Leu Gly Leu Thr Thr Val Phe Asp Leu Val Val Thr Phe
   545 550 555 560
   Leu Ile Thr Ala Pro Leu Val Ile Leu Ala Ser Arg Asn Pro Phe Phe
   565 570 575
   Ala Lys Ser Ser Val Asn Gly Met Gly Arg Val Met Lys Leu Val Glu
   580 585 590
   Glu Arg Arg Ala Asn Gly Glu Leu Asp Glu Pro Glu Tyr Leu Lys Lys
           595                 600                 605
   Ile His Ala Lys Asn Ala Ala Ala Asp Lys Ala Ser Thr Asp Asn Ser
       610                 615                 620
   Ser Thr Asp Asn Ser Glu Ala Pro Gly Thr Asp Thr Asn Gln Glu Glu
   625                 630                 635                 640
   Glu Lys
<210> 43
   <211> 1909
   <212> DNA
   <213> Corynebacterium glutamicum
<220>
   <221> CDS
   <222> (101)..(1879)
   <223> RXA01596
<400> 43
   tcccaggtca gcggggtaat tcgaaaacca ttcgaacaat tttcgaggat ttagaaaaaa 60
   cgttcgcata aattgttaga actgatgtac actttgaggc atg ctc gta gac att   115
                                               Met Leu Val Asp Ile
                                               1               5
   gct att gag aac ctc gga gtt att cca gcg gcc tca gct gag ttc agc   163
   Ala Ile Glu Asn Leu Gly Val Ile Pro Ala Ala Ser Ala Glu Phe Ser
                   10                  15                  20
   tca ggt tta aca gtg ctc acc ggt gag acc ggc gcc gga aag acc atg   211
   Ser Gly Leu Thr Val Leu Thr Gly Glu Thr Gly Ala Gly Lys Thr Met
               25                  30                  35
   gta gtg aca ggt tta cgc ctg tta tcc ggc ggt cgc gcc gac gct tca   259
   Val Val Thr Gly Leu Arg Leu Leu Ser Gly Gly Arg Ala Asp Ala Ser
           40                  45                  50
   cgc gtg cgc aca gga tcc cct caa gct gtt gtg gag ggg cgc ttt gtt   307
   Arg Val Arg Thr Gly Ser Pro Gln Ala Val Val Glu Gly Arg Phe Val
       55                  60                  65
   acg caa ggc gtg ccc tgc gac att gtc gaa cgt gca acc gga atc gtt   355
   Thr Gln Gly Val Pro Cys Asp Ile Val Glu Arg Ala Thr Gly Ile Val
   70                  75                  80                  85
   tcg aac gcc gga ggt gcc gca gat gaa aat gga gag ttt tta gct gtc   403
   Ser Asn Ala Gly Gly Ala Ala Asp Glu Asn Gly Glu Phe Leu Ala Val
                   90                  95                  100
   cgt tcc gtc ggc gcc aac ggc cgt tca aaa gct cat ctc ggt ggt cgc   451
   Arg Ser Val Gly Ala Asn Gly Arg Ser Lys Ala His Leu Gly Gly Arg
               105                 110                 115
   tcc gta cct gcg gca acg ctg tcc gag ttc tct gat gag ctg ttg acc   499
   Ser Val Pro Ala Ala Thr Leu Ser Glu Phe Ser Asp Glu Leu Leu Thr
           120                 125                 130
   atc cac ggt caa aat gac caa ctc cgg ttg ctc tcc cca gaa cgc caa   547
   Ile His Gly Gln Asn Asp Gln Leu Arg Leu Leu Ser Pro Glu Arg Gln
       135                 140                 145
   cta gag gcg ctt gat cgt ttt gat cca gag ctg gcc caa ctg cgc aaa    595
   Leu Glu Ala Leu Asp Arg Phe Asp Pro Glu Leu Ala Gln Leu Arg Lys
   150                 155                 160                 165
   aac tac aac gcc aag tac ctc act tgg aag tcc ttg gat aaa gat ctg    643
   Asn Tyr Asn Ala Lys Tyr Leu Thr Trp Lys Ser Leu Asp Lys Asp Leu
                   170                 175                 180
   cag aag cgc ctg agt agt agg cga gag ctg gct caa gaa gtc gat cgc    691
   Gln Lys Arg Leu Ser Ser Arg Arg Glu Leu Ala Gln Glu Val Asp Arg
               185                 190                 195
   ctg caa ttc gcg att aat gag atc gag gaa gtc tcg cca cag cca ggc    739
   Leu Gln Phe Ala Ile Asn Glu Ile Glu Glu Val Ser Pro Gln Pro Gly
           200                 205                 210
   gaa gac gcc gaa ctg gtt gag cag atc cgc agg ctc cag gac gtg gac    787
   Glu Asp Ala Glu Leu Val Glu Gln Ile Arg Arg Leu Gln Asp Val Asp
       215                 220                 225
   acc ctg cgg gag caa gct gca acc gca ttg gct gcg att gat ggt gcc    835
   Thr Leu Arg Glu Gln Ala Ala Thr Ala Leu Ala Ala Ile Asp Gly Ala
   230                 235                 240                 245
   ggc tct ctc agc gac gcc atg ggt ggt tcc ggc ggc ttt gat gaa tcc    883
   Gly Ser Leu Ser Asp Ala Met Gly Gly Ser Gly Gly Phe Asp Glu Ser
                   250                 255                 260
   cag gag tca gcc tct gac cag ctc ggc cag gcg gag tcc gcg ctg gca    931
   Gln Glu Ser Ala Ser Asp Gln Leu Gly Gln Ala Glu Ser Ala Leu Ala
               265                 270                 275
   ggc agt gat gac tca aag ctg aaa gat att gcc gtt cag ctt gcg gaa    979
   Gly Ser Asp Asp Ser Lys Leu Lys Asp Ile Ala Val Gln Leu Ala Glu
           280                 285                 290
   atc acc agc cag ctc agc caa gtg tcc atg gaa ttg ggc ggg ttc ctc    1027
   Ile Thr Ser Gln Leu Ser Gln Val Ser Met Glu Leu Gly Gly Phe Leu
       295                 300                 305
   tct gat ctc ccc gca gac ccc caa gca ctc gat gac atg ctc acc cgc    1075
   Ser Asp Leu Pro Ala Asp Pro Gln Ala Leu Asp Asp Met Leu Thr Arg
   310                 315                 320                 325
   caa cag caa ttg aaa ctg ctc acg cgt aaa tac gct gca gat att gac    1123
   Gln Gln Gln Leu Lys Leu Leu Thr Arg Lys Tyr Ala Ala Asp Ile Asp
                   330                 335                 340
   ggc gtg att gag tgg cag cgg aaa gcc caa atc cgc cta gac agc att    1171
   Gly Val Ile Glu Trp Gln Arg Lys Ala Gln Ile Arg Leu Asp Ser Ile
               345                 350                 355
   gac att tcc tcc gaa gcg ctt gac aag ctg aaa gaa gac gcg aaa aag    1219
   Asp Ile Ser Ser Glu Ala Leu Asp Lys Leu Lys Glu Asp Ala Lys Lys
           360                 365                 370
   gcg cag gcc tcc atg atg cgt gcc gct aag aag ctt tca gct gtc cgt    1267
   Ala Gln Ala Ser Met Met Arg Ala Ala Lys Lys Leu Ser Ala Val Arg
       375                 380                 385
   gca aag gca gca acc aag ttg ggg aca act gtc acc gag gag ctt cag     1315
   Ala Lys Ala Ala Thr Lys Leu Gly Thr Thr Val Thr Glu Glu Leu Gln
   390                 395                 400                 405
   ggc ctg gcc atg caa aaa gcc cgc ttt gag gtt gct ttg acc tcc att     1363
   Gly Leu Ala Met Gln Lys Ala Arg Phe Glu Val Ala Leu Thr Ser Ile
                   410                 415                 420
   gag gcg tgc gcc agc ggt atc gac cag gtg gaa ttc cag ctc gca gca     1411
   Glu Ala Cys Ala Ser Gly Ile Asp Gln Val Glu Phe Gln Leu Ala Ala
               425                 430                 435
   aat gcc ttt gca cag cct cgt cca ctt gca tcc tct gcg tct ggt ggt     1459
   Asn Ala Phe Ala Gln Pro Arg Pro Leu Ala Ser Ser Ala Ser Gly Gly
           440                 445                 450
   gaa ctt tcc cgc gtt atg ttg gcg ctc gag gtg atc ttg gct gct gga     1507
   Glu Leu Ser Arg Val Met Leu Ala Leu Glu Val Ile Leu Ala Ala Gly
       455                 460                 465
   acc acg ggc acc acc ttg gtg ttc gac gag gtt gat gca ggt gtg ggc     1555
   Thr Thr Gly Thr Thr Leu Val Phe Asp Glu Val Asp Ala Gly Val Gly
   470                 475                 480                 485
   gga cgc gca gcg gtg gaa atc ggt cgc cgc ctg gcc cgc ctt gcc acc     1603
   Gly Arg Ala Ala Val Glu Ile Gly Arg Arg Leu Ala Arg Leu Ala Thr
                   490                 495                 500
   aaa aac caa gtc atc gtg gtc acc cat ctc cca cag gtc gct gct tac     1651
   Lys Asn Gln Val Ile Val Val Thr His Leu Pro Gln Val Ala Ala Tyr
               505                 510                 515
   gcc gac acg cac ctg cac gtt gcc aag aat gta gga gaa gcc tcc gtg     1699
   Ala Asp Thr His Leu His Val Ala Lys Asn Val Gly Glu Ala Ser Val
           520                 525                 530
   acc tca gga gtg gag tca ctg acc ttc gac cga cgc gtg gaa gag ctc     1747
   Thr Ser Gly Val Glu Ser Leu Thr Phe Asp Arg Arg Val Glu Glu Leu
       535                 540                 545
   tcc cgc atg ctc gct ggc ctc gac gac acc gcc acc ggc cga gcc cac     1795
   Ser Arg Met Leu Ala Gly Leu Asp Asp Thr Ala Thr Gly Arg Ala His
   550                 555                 560                 565
   gca acg gag ctg ctc gag cgt gca cag cgt gaa aag gaa gat att aac     1843
   Ala Thr Glu Leu Leu Glu Arg Ala Gln Arg Glu Lys Glu Asp Ile Asn
                   570                 575                 580
   gag gag cga gta gaa cca ctt ctc gcc gcc agt gca taagagtttt          1889
   Glu Glu Arg Val Glu Pro Leu Leu Ala Ala Ser Ala
               585                 590
   cttggaattt tttaggcgcg                                               1909
<210> 44
   <211> 593
   <212> PRT
   <213> Corynebacterium glutamicum
<400> 44
   Met Leu Val Asp Ile Ala Ile Glu Asn Leu Gly Val Ile Pro Ala Ala
   1               5                   10                  15
   Ser Ala Glu Phe Ser Ser Gly Leu Thr Val Leu Thr Gly Glu Thr Gly
               20                  25                  30
   Ala Gly Lys Thr Met Val Val Thr Gly Leu Arg Leu Leu Ser Gly Gly
           35                  40                  45
   Arg Ala Asp Ala Ser Arg Val Arg Thr Gly Ser Pro Gln Ala Val Val
       50                  55                  60
   Glu Gly Arg Phe Val Thr Gln Gly Val Pro Cys Asp Ile Val Glu Arg
   65                  70                  75                  80
   Ala Thr Gly Ile Val Ser Asn Ala Gly Gly Ala Ala Asp Glu Asn Gly
                   85                  90                  95
   Glu Phe Leu Ala Val Arg Ser Val Gly Ala Asn Gly Arg Ser Lys Ala
               100                 105                 110
   His Leu Gly Gly Arg Ser Val Pro Ala Ala Thr Leu Ser Glu Phe Ser
           115                 120                 125
   Asp Glu Leu Leu Thr Ile His Gly Gln Asn Asp Gln Leu Arg Leu Leu
       130                 135                 140
   Ser Pro Glu Arg Gln Leu Glu Ala Leu Asp Arg Phe Asp Pro Glu Leu
   145                 150                 155                 160
   Ala Gln Leu Arg Lys Asn Tyr Asn Ala Lys Tyr Leu Thr Trp Lys Ser
                   165                 170                 175
   Leu Asp Lys Asp Leu Gln Lys Arg Leu Ser Ser Arg Arg Glu Leu Ala
               180                 185                 190
   Gln Glu Val Asp Arg Leu Gln Phe Ala Ile Asn Glu Ile Glu Glu Val
           195                 200                 205
   Ser Pro Gln Pro Gly Glu Asp Ala Glu Leu Val Glu Gln Ile Arg Arg
       210                 215                 220
   Leu Gln Asp Val Asp Thr Leu Arg Glu Gln Ala Ala Thr Ala Leu Ala
   225                 230                 235                 240
   Ala Ile Asp Gly Ala Gly Ser Leu Ser Asp Ala Met Gly Gly Ser Gly
                   245                 250                 255
   Gly Phe Asp Glu Ser Gln Glu Ser Ala Ser Asp Gln Leu Gly Gln Ala
               260                 265                 270
   Glu Ser Ala Leu Ala Gly Ser Asp Asp Ser Lys Leu Lys Asp Ile Ala
           275                 280                 285
   Val Gln Leu Ala Glu Ile Thr Ser Gln Leu Ser Gln Val Ser Met Glu
       290                 295                 300
   Leu Gly Gly Phe Leu Ser Asp Leu Pro Ala Asp Pro Gln Ala Leu Asp
   305                 310                 315                 320
   Asp Met Leu Thr Arg Gln Gln Gln Leu Lys Leu Leu Thr Arg Lys Tyr
                   325                 330                 335
   Ala Ala Asp Ile Asp Gly Val Ile Glu Trp Gln Arg Lys Ala Gln Ile
               340                 345                 350
   Arg Leu Asp Ser Ile Asp Ile Ser Ser Glu Ala Leu Asp Lys Leu Lys
           355                 360                 365
   Glu Asp Ala Lys Lys Ala Gln Ala Ser Met Met Arg Ala Ala Lys Lys
       370                 375                 380
   Leu Ser Ala Val Arg Ala Lys Ala Ala Thr Lys Leu Gly Thr Thr Val
   385                 390                 395                 400
   Thr Glu Glu Leu Gln Gly Leu Ala Met Gln Lys Ala Arg Phe Glu Val
                   405                 410                 415
   Ala Leu Thr Ser Ile Glu Ala Cys Ala Ser Gly Ile Asp Gln Val Glu
               420                 425                 430
   Phe Gln Leu Ala Ala Asn Ala Phe Ala Gln Pro Arg Pro Leu Ala Ser
           435                 440                 445
   Ser Ala Ser Gly Gly Glu Leu Ser Arg Val Met Leu Ala Leu Glu Val
       450                 455                 460
   Ile Leu Ala Ala Gly Thr Thr Gly Thr Thr Leu Val Phe Asp Glu Val
   465                 470                 475                 480
   Asp Ala Gly Val Gly Gly Arg Ala Ala Val Glu Ile Gly Arg Arg Leu
                   485                 490                 495
   Ala Arg Leu Ala Thr Lys Asn Gln Val Ile Val Val Thr His Leu Pro
               500                 505                 510
   Gln Val Ala Ala Tyr Ala Asp Thr His Leu His Val Ala Lys Asn Val
           515                 520                 525
   Gly Glu Ala Ser Val Thr Ser Gly Val Glu Ser Leu Thr Phe Asp Arg
       530                 535                 540
   Arg Val Glu Glu Leu Ser Arg Met Leu Ala Gly Leu Asp Asp Thr Ala
   545                 550                 555                 560
   Thr Gly Arg Ala His Ala Thr Glu Leu Leu Glu Arg Ala Gln Arg Glu
                   565                 570                 575
   Lys Glu Asp Ile Asn Glu Glu Arg Val Glu Pro Leu Leu Ala Ala Ser
               580                 585                 590
   Ala
<210> 45
   <211> 265
   <212> DNA
   <213> Corynebacterium glutamicum
<220>
   <221> CDS
   <222> (101)..(235)
   <223> RXA01651
<400> 45
   caatctctaa ggagaaagtt tatgacaaat aggacctgac ccctgtttgg tagacaccta 60
   acatcccaac attctgggac agaaaggtaa cctacctatc atg cca acc aag acc   115
                                               Met Pro Thr Lys Thr
                                               1               5
   tac tcc gag gag ttc aaa cgc gac gcc gtt gct ttg tac gag aac tcc   163
   Tyr Ser Glu Glu Phe Lys Arg Asp Ala Val Ala Leu Tyr Glu Asn Ser
                   10                  15                  20
   gat ggg gcc tca ctc caa cag atc gcc aac gat ctc ggc atc aac cga   211
   Asp Gly Ala Ser Leu Gln Gln Ile Ala Asn Asp Leu Gly Ile Asn Arg
               25                  30                  35
   gta acc ctg aaa aac ttc gat caa taaatacggt gcgcatgcct caaccaacac  265
   Val Thr Leu Lys Asn Phe Asp Gln
           40                  45
<210> 46
   <211> 45
   <212> PRT
   <213> Corynebacterium glutamicum
<400> 46
   Met Pro Thr Lys Thr Tyr Ser Glu Glu Phe Lys Arg Asp Ala Val Ala
   1               5                   10                  15
   Leu Tyr Glu Asn Ser Asp Gly Ala Ser Leu Gln Gln Ile Ala Asn Asp
               20                  25                  30
   Leu Gly Ile Asn Arg Val Thr Leu Lys Asn Phe Asp Gln
           35                  40                  45
<210> 47
   <211> 538
   <212> DNA
   <213> Corynebacterium glutamicum
<220>
   <221> CDS
   <222> (101)..(508)
   <223> RXA01710
<400> 47
   tctcggcgct aatctggttt attggtgata tccgagccaa gggaactccg agctcaccca 60
   ttaccactga tccaacgcac gaccatcttg agaggacagc atg aca gac ttc aaa   115
                                               Met Thr Asp Phe Lys
                                               1               5
   ctc atc agc gat acc gag tgg cgc gaa cgc ctc acc ccg cag gaa ttc   163
   Leu Ile Ser Asp Thr Glu Trp Arg Glu Arg Leu Thr Pro Gln Glu Phe
                   10                  15                  20
   cat gtc ctc cgc gaa gcc ggc acc gaa cca cct cac gtc ggt gaa tac   211
   His Val Leu Arg Glu Ala Gly Thr Glu Pro Pro His Val Gly Glu Tyr
               25                  30                  35
   acc aac acc acc acc gaa ggt gtg tac tcc tgt cgc gcc tgt ggt gaa   259
   Thr Asn Thr Thr Thr Glu Gly Val Tyr Ser Cys Arg Ala Cys Gly Glu
           40                  45                  50
   gag tta ttc cgc tcc acc gag aag ttt gaa tcc cac tgc ggt tgg cct   307
   Glu Leu Phe Arg Ser Thr Glu Lys Phe Glu Ser His Cys Gly Trp Pro
       55                  60                  65
   tcc ttc ttc tcc cca ctt gct ggc gac aaa atc att gag aag gaa gat   355
   Ser Phe Phe Ser Pro Leu Ala Gly Asp Lys Ile Ile Glu Lys Glu Asp
   70                  75                  80                  85
   ctt tcc ctc ggt atg cgt cgc gtt gag att ctg tgc gct aac tgc ggc   403
   Leu Ser Leu Gly Met Arg Arg Val Glu Ile Leu Cys Ala Asn Cys Gly
                   90                  95                  100
   tct cac atg ggt cac gtc ttc gaa ggc gaa ggc tac gac acc ccc acc   451
   Ser His Met Gly His Val Phe Glu Gly Glu Gly Tyr Asp Thr Pro Thr
               105                 110                 115
   gat ctt cgt tac tgc att aac tcc atc agc ttg aag ctg gaa gaa aag   499
   Asp Leu Arg Tyr Cys Ile Asn Ser Ile Ser Leu Lys Leu Glu Glu Lys
           120                 125                 130
   cca gtt tcc taagcttccg agcacgaaac gagccttggc                      538
   Pro Val Ser
       135
<210> 48
   <211> 136
   <212> PRT
   <213> Corynebacterium glutamicum
<400> 48
   Met Thr Asp Phe Lys Leu Ile Ser Asp Thr Glu Trp Arg Glu Arg Leu
   1               5                   10                  15
   Thr Pro Gln Glu Phe His Val Leu Arg Glu Ala Gly Thr Glu Pro Pro
               20                  25                  30
   His Val Gly Glu Tyr Thr Asn Thr Thr Thr Glu Gly Val Tyr Ser Cys
           35                  40                  45
   Arg Ala Cys Gly Glu Glu Leu Phe Arg Ser Thr Glu Lys Phe Glu Ser
       50                  55                  60
   His Cys Gly Trp Pro Ser Phe Phe Ser Pro Leu Ala Gly Asp Lys Ile
   65                  70                  75                  80
   Ile Glu Lys Glu Asp Leu Ser Leu Gly Met Arg Arg Val Glu Ile Leu
                   85                  90                  95
   Cys Ala Asn Cys Gly Ser His Met Gly His Val Phe Glu Gly Glu Gly
               100                 105                 110
   Tyr Asp Thr Pro Thr Asp Leu Arg Tyr Cys Ile Asn Ser Ile Ser Leu
           115                 120                 125
   Lys Leu Glu Glu Lys Pro Val Ser
       130                 135
<210> 49
   <211> 1417
   <212> DNA
   <213> Corynebacterium glutamicum
<220>
   <221> CDS
   <222> (101)..(1387)
   <223> RXA01852
<400> 49
   aaccaccacc atgcgtgcag aacgcactgg taaccctttc ttgctggcac tgtagggcta 60
   agttccgtac tacttcttcg aataggtatc gttaataatc gtg agt caa aac aag   115
                                               Val Ser Gln Asn Lys
                                                1               5
   tcc aag tct gaa aag ctt cag tca ttt gct gca ccc aag ggt gtt cct   163
   Ser Lys Ser Glu Lys Leu Gln Ser Phe Ala Ala Pro Lys Gly Val Pro
                   10                  15                  20
   gat tac gcc cca cca aaa tct gca gcg ttt tta gca gtc cgt gat gcc   211
   Asp Tyr Ala Pro Pro Lys Ser Ala Ala Phe Leu Ala Val Arg Asp Ala
               25                  30                  35
   ttt gtt aat caa gca cat aag gcc ggg ttt gag cat att gag ctg ccg   259
   Phe Val Asn Gln Ala His Lys Ala Gly Phe Glu His Ile Glu Leu Pro
           40                  45                  50
   atc ttt gaa gac acc ggc ttg ttt gcg cgt ggt gtt ggt gag tcc act   307
   Ile Phe Glu Asp Thr Gly Leu Phe Ala Arg Gly Val Gly Glu Ser Thr
       55                  60                  65
   gac gta gtg agc aag gaa atg tac acc ttc gct gat cgt ggc gag cgc   355
   Asp Val Val Ser Lys Glu Met Tyr Thr Phe Ala Asp Arg Gly Glu Arg
   70                  75                  80                  85
   tct gtc acg ctg cgc cca gaa ggc act gca ggc gtg atg cgt gca gtt   403
   Ser Val Thr Leu Arg Pro Glu Gly Thr Ala Gly Val Met Arg Ala Val
                   90                  95                  100
   att gaa cac agc ctg gac cgt gga cag ctt ccc gta aag ctg aac tac   451
   Ile Glu His Ser Leu Asp Arg Gly Gln Leu Pro Val Lys Leu Asn Tyr
               105                 110                 115
   gcc gga cca ttc ttc cgt tat gag cgt cct cag gca ggg cgt tac cgt   499
   Ala Gly Pro Phe Phe Arg Tyr Glu Arg Pro Gln Ala Gly Arg Tyr Arg
           120                 125                 130
   cag ctt cag caa gta ggc gta gag gca att ggt gtg gat gat cca gcg   547
   Gln Leu Gln Gln Val Gly Val Glu Ala Ile Gly Val Asp Asp Pro Ala
       135                 140                 145
   ctt gat gcg gag atc att gcg ctt gct gat cgt tct tac cgc agc ttg   595
   Leu Asp Ala Glu Ile Ile Ala Leu Ala Asp Arg Ser Tyr Arg Ser Leu
   150                 155                 160                 165
   ggg ctg cag gat ttc cgt ctg gag ctc acc agc ttg ggt gat cgt cac   643
   Gly Leu Gln Asp Phe Arg Leu Glu Leu Thr Ser Leu Gly Asp Arg His
                   170                 175                 180
   tgc cgt ccc gag tat cgt cag aag ctg cag gat ttc ttg ttt gca ctt   691
   Cys Arg Pro Glu Tyr Arg Gln Lys Leu Gln Asp Phe Leu Phe Ala Leu
               185                 190                 195
   cct ttg gat gag gaa acc cgc aag cgc gca gag atc aac cca ctt cgg   739
   Pro Leu Asp Glu Glu Thr Arg Lys Arg Ala Glu Ile Asn Pro Leu Arg
           200                 205                 210
   gtg ttg gat gat aag cgt cct gaa gtc caa gag atg act gcg gat gca     787
   Val Leu Asp Asp Lys Arg Pro Glu Val Gln Glu Met Thr Ala Asp Ala
       215                 220                 225
   cca ttg atg ctg gat cac ctt gat gca gag tgc cgt gag cac ttt gaa     835
   Pro Leu Met Leu Asp His Leu Asp Ala Glu Cys Arg Glu His Phe Glu
   230                 235                 240                 245
   aca gtg act ggt ttg ctc gat gac atg ggt gtt cca tat gtg att aac     883
   Thr Val Thr Gly Leu Leu Asp Asp Met Gly Val Pro Tyr Val Ile Asn
                   250                 255                 260
   cca cgc atg gtt cgt ggt ttg gat tac tac acc aag act tgt ttt gag     931
   Pro Arg Met Val Arg Gly Leu Asp Tyr Tyr Thr Lys Thr Cys Phe Glu
               265                 270                 275
   ttc gtt cac gat ggc ctg ggc gca cag tct ggc att ggt ggc ggc gga     979
   Phe Val His Asp Gly Leu Gly Ala Gln Ser Gly Ile Gly Gly Gly Gly
           280                 285                 290
   cgc tac gac ggt ctg atg gca cag ctt ggc gga cag gat ctg tct ggc     1027
   Arg Tyr Asp Gly Leu Met Ala Gln Leu Gly Gly Gln Asp Leu Ser Gly
       295                 300                 305
   atc ggc tat ggc ctg ggt gtg gat cgc acc atg ttg gct ctg gaa gct     1075
   Ile Gly Tyr Gly Leu Gly Val Asp Arg Thr Met Leu Ala Leu Glu Ala
   310                 315                 320                 325
   gaa ggt gtg act gtt ggt gct gag cgt cgc gtt gat gtg tac ggc gtt     1123
   Glu Gly Val Thr Val Gly Ala Glu Arg Arg Val Asp Val Tyr Gly Val
                   330                 335                 340
   cca ctg ggc aag gat gct aag aag gct ctt gct gga atc gtg aac acg     1171
   Pro Leu Gly Lys Asp Ala Lys Lys Ala Leu Ala Gly Ile Val Asn Thr
               345                 350                 355
   ctg cgc gct gcg ggt att tcc acc gat atg tct tac ggc gac cgt ggc     1219
   Leu Arg Ala Ala Gly Ile Ser Thr Asp Met Ser Tyr Gly Asp Arg Gly
           360                 365                 370
   ctg aag ggt gcc atg aag ggc gct gac cgc tcc aac gcg ttg tac acc     1267
   Leu Lys Gly Ala Met Lys Gly Ala Asp Arg Ser Asn Ala Leu Tyr Thr
       375                 380                 385
   ttg gtg ctg ggc gag cag gag ctg gag aac aac acc atc gcg gtg aag     1315
   Leu Val Leu Gly Glu Gln Glu Leu Glu Asn Asn Thr Ile Ala Val Lys
   390                 395                 400                 405
   gat atg cgt gcg cat gag cag cac gat gtc gca ttg gac gag gtt gtg     1363
   Asp Met Arg Ala His Glu Gln His Asp Val Ala Leu Asp Glu Val Val
                   410                 415                 420
   gcc ttt ttg cag ggg aaa ctt att taaataattc ataagtaaaa aaccgtcaat    1417
   Ala Phe Leu Gln Gly Lys Leu Ile
               425
<210> 50
   <211> 429
   <212> PRT
   <213> Corynebacterium glutamicum
<400> 50
   Val Ser Gln Asn Lys Ser Lys Ser Glu Lys Leu Gln Ser Phe Ala Ala
   1               5                   10                  15
   Pro Lys Gly Val Pro Asp Tyr Ala Pro Pro Lys Ser Ala Ala Phe Leu
               20                  25                  30
   Ala Val Arg Asp Ala Phe Val Asn Gln Ala His Lys Ala Gly Phe Glu
           35                  40                  45
   His Ile Glu Leu Pro Ile Phe Glu Asp Thr Gly Leu Phe Ala Arg Gly
       50                  55                  60
   Val Gly Glu Ser Thr Asp Val Val Ser Lys Glu Met Tyr Thr Phe Ala
   65                  70                  75                  80
   Asp Arg Gly Glu Arg Ser Val Thr Leu Arg Pro Glu Gly Thr Ala Gly
                   85                  90                  95
   Val Met Arg Ala Val Ile Glu His Ser Leu Asp Arg Gly Gln Leu Pro
               100                 105                 110
   Val Lys Leu Asn Tyr Ala Gly Pro Phe Phe Arg Tyr Glu Arg Pro Gln
           115                 120                 125
   Ala Gly Arg Tyr Arg Gln Leu Gln Gln Val Gly Val Glu Ala Ile Gly
       130                 135                 140
   Val Asp Asp Pro Ala Leu Asp Ala Glu Ile Ile Ala Leu Ala Asp Arg
   145                 150                 155                 160
   Ser Tyr Arg Ser Leu Gly Leu Gln Asp Phe Arg Leu Glu Leu Thr Ser
                   165                 170                 175
   Leu Gly Asp Arg His Cys Arg Pro Glu Tyr Arg Gln Lys Leu Gln Asp
               180                 185                 190
   Phe Leu Phe Ala Leu Pro Leu Asp Glu Glu Thr Arg Lys Arg Ala Glu
           195                 200                 205
   Ile Asn Pro Leu Arg Val Leu Asp Asp Lys Arg Pro Glu Val Gln Glu
       210                 215                 220
   Met Thr Ala Asp Ala Pro Leu Met Leu Asp His Leu Asp Ala Glu Cys
   225                 230                 235                 240
   Arg Glu His Phe Glu Thr Val Thr Gly Leu Leu Asp Asp Met Gly Val
                   245                 250                 255
   Pro Tyr Val Ile Asn Pro Arg Met Val Arg Gly Leu Asp Tyr Tyr Thr
               260                 265                 270
   Lys Thr Cys Phe Glu Phe Val His Asp Gly Leu Gly Ala Gln Ser Gly
           275                 280                 285
   Ile Gly Gly Gly Gly Arg Tyr Asp Gly Leu Met Ala Gln Leu Gly Gly
       290                 295                 300
   Gln Asp Leu Ser Gly Ile Gly Tyr Gly Leu Gly Val Asp Arg Thr Met
   305                 310                 315                 320
   Leu Ala Leu Glu Ala Glu Gly Val Thr Val Gly Ala Glu Arg Arg Val
                   325                 330                 335
   Asp Val Tyr Gly Val Pro Leu Gly Lys Asp Ala Lys Lys Ala Leu Ala
               340                 345                 350
   Gly Ile Val Asn Thr Leu Arg Ala Ala Gly Ile Ser Thr Asp Met Ser
           355                 360                 365
   Tyr Gly Asp Arg Gly Leu Lys Gly Ala Met Lys Gly Ala Asp Arg Ser
       370                 375                 380
   Asn Ala Leu Tyr Thr Leu Val Leu Gly Glu Gln Glu Leu Glu Asn Asn
   385                 390                 395                 400
   Thr Ile Ala Val Lys Asp Met Arg Ala His Glu Gln His Asp Val Ala
                   405                 410                 415
   Leu Asp Glu Val Val Ala Phe Leu Gln Gly Lys Leu Ile
               420                 425
<210> 51
   <211> 955
   <212> DNA
   <213> Corynebacterium glutamicum
<220>
   <221> CDS
   <222> (101)..(925)
   <223> RXA01913
<400> 51
   acctgtacga tcacttttta gacgggcggg tagggctact gtgccctaac ctaagcttgt 60
   aaagcattaa ttatccatac ataaggagga tcgccccgta atg gcg aac tac acc   115
                                              Met Ala Asn Tyr Thr
                                               1               5
   gct gcg gat gtt aag aag ctc cgc gaa ctc acc ggt tcc ggc atg ctc   163
   Ala Ala Asp Val Lys Lys Leu Arg Glu Leu Thr Gly Ser Gly Met Leu
                   10                  15                  20
   gat tgc aag aag gct ctg gag gag tcc gct ggc gac ttc gac aag gct   211
   Asp Cys Lys Lys Ala Leu Glu Glu Ser Ala Gly Asp Phe Asp Lys Ala
               25                  30                  35
   gtt gag atc ctg cgc gtc aag ggc gca aag gac gtc gga aag cgt gca   259
   Val Glu Ile Leu Arg Val Lys Gly Ala Lys Asp Val Gly Lys Arg Ala
           40                  45                  50
   gag cgt aac gct acc gaa ggt ctc gtt gca gtt tct ggc aac acc atg   307
   Glu Arg Asn Ala Thr Glu Gly Leu Val Ala Val Ser Gly Asn Thr Met
       55                  60                  65
   gtc gag gtc aac tct gag acc gac ttc gtt gca aag aac tct gac ttc   355
   Val Glu Val Asn Ser Glu Thr Asp Phe Val Ala Lys Asn Ser Asp Phe
   70                  75                  80                  85
   aag gaa ttc gct gca aag gtt gca gac gca gca gca gct gca aag gct   403
   Lys Glu Phe Ala Ala Lys Val Ala Asp Ala Ala Ala Ala Ala Lys Ala
                   90                  95                  100
   aac tcc cag gaa gag ctc gca gca gtt gac gtg gac gga cag acc gca   451
   Asn Ser Gln Glu Glu Leu Ala Ala Val Asp Val Asp Gly Gln Thr Ala
               105                 110                 115
   gac gca gct ctg cag gag ttc tcc gca aag atc ggc gag aag ctt gag    499
   Asp Ala Ala Leu Gln Glu Phe Ser Ala Lys Ile Gly Glu Lys Leu Glu
           120                 125                 130
   ctt cgt cgc gca gta acc ctc gag ggc gac aag acc gct gtt tac ctc    547
   Leu Arg Arg Ala Val Thr Leu Glu Gly Asp Lys Thr Ala Val Tyr Leu
       135                 140                 145
   cac cag cgt tcc gct gac ctg cca cca gca gtt ggc gtt ttg gtt gct    595
   His Gln Arg Ser Ala Asp Leu Pro Pro Ala Val Gly Val Leu Val Ala
   150                 155                 160                 165
   ttc acc ggt gaa ggt gaa gca gct gag gca gct gca cgt cag gct gca    643
   Phe Thr Gly Glu Gly Glu Ala Ala Glu Ala Ala Ala Arg Gln Ala Ala
                   170                 175                 180
   atg cag att gct gct ctg aag gct tct tac ctc acc cgt gag gac gtt    691
   Met Gln Ile Ala Ala Leu Lys Ala Ser Tyr Leu Thr Arg Glu Asp Val
               185                 190                 195
   cct gca gag atc atc gag aag gag cgc tcc atc gct gag cag atc act    739
   Pro Ala Glu Ile Ile Glu Lys Glu Arg Ser Ile Ala Glu Gln Ile Thr
           200                 205                 210
   cgc gaa gag ggc aag cca gag cag gct atc cct aag atc gtt gag ggt    787
   Arg Glu Glu Gly Lys Pro Glu Gln Ala Ile Pro Lys Ile Val Glu Gly
       215                 220                 225
   cgt ttg aat ggc ttc tac aag gag aac gta ctt ctt gag cag tcc tcg    835
   Arg Leu Asn Gly Phe Tyr Lys Glu Asn Val Leu Leu Glu Gln Ser Ser
   230                 235                 240                 245
   gta gct gac agc aag aag acc gtt aag gct ctt ctg gac gag gct ggc    883
   Val Ala Asp Ser Lys Lys Thr Val Lys Ala Leu Leu Asp Glu Ala Gly
                   250                 255                 260
   gtt acc gtc acc tcc ttc gct cgc ttc gag gtc ggc cag gct            925
   Val Thr Val Thr Ser Phe Ala Arg Phe Glu Val Gly Gln Ala
               265                 270                 275
   taaggccact tgaaggttgt gggtgggtgt                                   955
<210> 52
   <211> 275
   <212> PRT
   <213> Corynebacterium glutamicum
<400> 52
   Met Ala Asn Tyr Thr Ala Ala Asp Val Lys Lys Leu Arg Glu Leu Thr
   1               5                   10                  15
   Gly Ser Gly Met Leu Asp Cys Lys Lys Ala Leu Glu Glu Ser Ala Gly
               20                  25                  30
   Asp Phe Asp Lys Ala Val Glu Ile Leu Arg Val Lys Gly Ala Lys Asp
           35                  40                  45
   Val Gly Lys Arg Ala Glu Arg Asn Ala Thr Glu Gly Leu Val Ala Val
       50                  55                  60
   Ser Gly Asn Thr Met Val Glu Val Asn Ser Glu Thr Asp Phe Val Ala
   65                  70                  75                  80
   Lys Asn Ser Asp Phe Lys Glu Phe Ala Ala Lys Val Ala Asp Ala Ala
                   85                  90                  95
   Ala Ala Ala Lys Ala Asn Ser Gln Glu Glu Leu Ala Ala Val Asp Val
               100                 105                 110
   Asp Gly Gln Thr Ala Asp Ala Ala Leu Gln Glu Phe Ser Ala Lys Ile
           115                 120                 125
   Gly Glu Lys Leu Glu Leu Arg Arg Ala Val Thr Leu Glu Gly Asp Lys
       130                 135                 140
   Thr Ala Val Tyr Leu His Gln Arg Ser Ala Asp Leu Pro Pro Ala Val
   145                 150                 155                 160
   Gly Val Leu Val Ala Phe Thr Gly Glu Gly Glu Ala Ala Glu Ala Ala
                   165                 170                 175
   Ala Arg Gln Ala Ala Met Gln Ile Ala Ala Leu Lys Ala Ser Tyr Leu
               180                 185                 190
   Thr Arg Glu Asp Val Pro Ala Glu Ile Ile Glu Lys Glu Arg Ser Ile
           195                 200                 205
   Ala Glu Gln Ile Thr Arg Glu Glu Gly Lys Pro Glu Gln Ala Ile Pro
       210                 215                 220
   Lys Ile Val Glu Gly Arg Leu Asn Gly Phe Tyr Lys Glu Asn Val Leu
   225                 230                 235                 240
   Leu Glu Gln Ser Ser Val Ala Asp Ser Lys Lys Thr Val Lys Ala Leu
                   245                 250                 255
   Leu Asp Glu Ala Gly Val Thr Val Thr Ser Phe Ala Arg Phe Glu Val
               260                 265                 270
   Gly Gln Ala
           275
<210> 53
   <211> 1747
   <212> DNA
   <213> Corynebacterium glutamicum
<220>
   <221> CDS
   <222> (101)..(1717)
   <223> RXA02145
<400> 53
   cactgccaca gctgccaatt accgttgatg aagagggcta cctcatcgcc gctggtaact 60
   tcattgagcc actcggccct gcattctggg agcgtaagtc atg agt cta gct acc   115
                                               Met Ser Leu Ala Thr
                                               1               5
   gtg gga aac aat ctt gat tcc cgt tac acc atg gcg tcg ggt atc cgt   163
   Val Gly Asn Asn Leu Asp Ser Arg Tyr Thr Met Ala Ser Gly Ile Arg
                   10                  15                  20
   cgc cag atc aac aag gtc ttc cca act cac tgg tcc ttc atg ctc ggc   211
   Arg Gln Ile Asn Lys Val Phe Pro Thr His Trp Ser Phe Met Leu Gly
               25                  30                  35
   gag att gcg ctt tac agc ttc atc gtc ttg ctg ctg act ggt gtc tac      259
   Glu Ile Ala Leu Tyr Ser Phe Ile Val Leu Leu Leu Thr Gly Val Tyr
           40                  45                  50
   ctg acc ctg ttc ttc gac cca tca atc acc aag gtc att tat gac ggc      307
   Leu Thr Leu Phe Phe Asp Pro Ser Ile Thr Lys Val Ile Tyr Asp Gly
       55                  60                  65
   ggc tac ctc cca ctg aac ggt gtg gag atg tcc cgt gca tac gca act      355
   Gly Tyr Leu Pro Leu Asn Gly Val Glu Met Ser Arg Ala Tyr Ala Thr
   70                  75                  80                  85
   gcg ttg gat att tcc ttc gag gtt cgc ggt ggt ctg ttc atc cgc cag      403
   Ala Leu Asp Ile Ser Phe Glu Val Arg Gly Gly Leu Phe Ile Arg Gln
                   90                  95                  100
   atg cac cac tgg gca gcc ctg ctg ttc gtt gta tcc atg ctg gtt cac      451
   Met His His Trp Ala Ala Leu Leu Phe Val Val Ser Met Leu Val His
               105                 110                 115
   atg ctc cgt att ttc ttc acc ggt gcg ttc cgt cgc cca cgt gaa gca      499
   Met Leu Arg Ile Phe Phe Thr Gly Ala Phe Arg Arg Pro Arg Glu Ala
           120                 125                 130
   aac tgg atc atc ggt gtt gtt ctg atc atc ctg ggt atg gct gaa ggc      547
   Asn Trp Ile Ile Gly Val Val Leu Ile Ile Leu Gly Met Ala Glu Gly
       135                 140                 145
   ttc atg ggt tac tcc ctg cct gat gac ctg ctc tct ggt gtt ggt ctt      595
   Phe Met Gly Tyr Ser Leu Pro Asp Asp Leu Leu Ser Gly Val Gly Leu
   150                 155                 160                 165
   cga atc atg tcc gcc atc atc gtt ggt ctt ccg atc ata ggt acc tgg      643
   Arg Ile Met Ser Ala Ile Ile Val Gly Leu Pro Ile Ile Gly Thr Trp
                   170                 175                 180
   atg cac tgg ctg atc ttc ggt gga gac ttc cca tcc gat ctg atg ctg      691
   Met His Trp Leu Ile Phe Gly Gly Asp Phe Pro Ser Asp Leu Met Leu
               185                 190                 195
   gac cgc ttc tac atc gca cac gtt cta atc atc cca gct atc ctg ctt      739
   Asp Arg Phe Tyr Ile Ala His Val Leu Ile Ile Pro Ala Ile Leu Leu
           200                 205                 210
   ggc ttg atc gca gct cac ctg gca ctt gtt tgg tac cag aag cac acc      787
   Gly Leu Ile Ala Ala His Leu Ala Leu Val Trp Tyr Gln Lys His Thr
       215                 220                 225
   cag ttc cca ggc gct ggc cgc act gag aac aac gtg atc ggt atc cga      835
   Gln Phe Pro Gly Ala Gly Arg Thr Glu Asn Asn Val Ile Gly Ile Arg
   230                 235                 240                 245
   atc atg cct ctg ttc gca gtt aag gct gtt gct ttc ggc ctc atc gtc      883
   Ile Met Pro Leu Phe Ala Val Lys Ala Val Ala Phe Gly Leu Ile Val
                   250                 255                 260
   ttc ggt ttc ctc gca ctg ctt gct ggt gtc acc acc att aac gca att      931
   Phe Gly Phe Leu Ala Leu Leu Ala Gly Val Thr Thr Ile Asn Ala Ile
               265                 270                 275
   tgg aat ctt gga ccg tac aac cct tca cag gtg tct gct ggt tcc cag     979
   Trp Asn Leu Gly Pro Tyr Asn Pro Ser Gln Val Ser Ala Gly Ser Gln
           280                 285                 290
   cct gac gtt tac atg ctg tgg aca gat ggt gct gct cgt gtc atg ccg     1027
   Pro Asp Val Tyr Met Leu Trp Thr Asp Gly Ala Ala Arg Val Met Pro
       295                 300                 305
   gca tgg gag ctc tac ctc ggt aac tac act att cca gca gtc ttc tgg     1075
   Ala Trp Glu Leu Tyr Leu Gly Asn Tyr Thr Ile Pro Ala Val Phe Trp
   310                 315                 320                 325
   gtt gct gtg atg ctg ggt atc ctc gtg gtt ctg ctt gtg act tac cca     1123
   Val Ala Val Met Leu Gly Ile Leu Val Val Leu Leu Val Thr Tyr Pro
                   330                 335                 340
   ttc att gag cgt aag ttc acc ggc gac gat gca cac cac aac ttg ctg     1171
   Phe Ile Glu Arg Lys Phe Thr Gly Asp Asp Ala His His Asn Leu Leu
               345                 350                 355
   cag cgt cct cgc gat gtt cca gtc cgc acc tca ctc ggt gtc atg gcg     1219
   Gln Arg Pro Arg Asp Val Pro Val Arg Thr Ser Leu Gly Val Met Ala
           360                 365                 370
   ctt gtc ttc tac atc ctg ctt acc gtt tct ggt ggt aac gat gtt tac     1267
   Leu Val Phe Tyr Ile Leu Leu Thr Val Ser Gly Gly Asn Asp Val Tyr
       375                 380                 385
   gca atg cag ttc cat gtt tca ctg aac gcg atg acc tgg atc ggt cgt     1315
   Ala Met Gln Phe His Val Ser Leu Asn Ala Met Thr Trp Ile Gly Arg
   390                 395                 400                 405
   atc ggc ctc atc gtt gga cca gct att gca tac ttc atc act tac cga     1363
   Ile Gly Leu Ile Val Gly Pro Ala Ile Ala Tyr Phe Ile Thr Tyr Arg
                   410                 415                 420
   ctg tgc atc ggc ttg cag cgc tct gac cgc gag gtc ctg gag cac ggc     1411
   Leu Cys Ile Gly Leu Gln Arg Ser Asp Arg Glu Val Leu Glu His Gly
               425                 430                 435
   atc gag acc ggt atc atc aag cag atg cca aat ggt gcc ttc att gaa     1459
   Ile Glu Thr Gly Ile Ile Lys Gln Met Pro Asn Gly Ala Phe Ile Glu
           440                 445                 450
   gtt cac cag cca ctt ggc cca gtt gat gac cat ggt cac cca atc cca     1507
   Val His Gln Pro Leu Gly Pro Val Asp Asp His Gly His Pro Ile Pro
       455                 460                 465
   ctg cca tac gct ggc gct gcg gtt cca aag cag atg aac cag ctt ggt     1555
   Leu Pro Tyr Ala Gly Ala Ala Val Pro Lys Gln Met Asn Gln Leu Gly
   470                 475                 480                 485
   tac gct gag gtt gaa acc cgc ggt gga ttc ttc gga cct gat cca gaa     1603
   Tyr Ala Glu Val Glu Thr Arg Gly Gly Phe Phe Gly Pro Asp Pro Glu
                   490                 495                 500
   gac atc cgt gcg aag gct aag gaa att gag cac gca aac cac att gag     1651
   Asp Ile Arg Ala Lys Ala Lys Glu Ile Glu His Ala Asn His Ile Glu
               505                 510                 515
   gaa gcg aac act ctt cgt gca ctc aac gag gca aac att gag cgt gac     1699
   Glu Ala Asn Thr Leu Arg Ala Leu Asn Glu Ala Asn Ile Glu Arg Asp
           520                 525                 530
   aag aat gag ggc aag aac tagtttctag gacttcatct ctgaaactcc        1747
   Lys Asn Glu Gly Lys Asn
       535
<210> 54
   <211> 539
   <212> PRT
   <213> Corynebacterium glutamicum
<400> 54
   Met Ser Leu Ala Thr Val Gly Asn Asn Leu Asp Ser Arg Tyr Thr Met
    1               5                  10                  15
   Ala Ser Gly Ile Arg Arg Gln Ile Asn Lys Val Phe Pro Thr His Trp
               20                  25                  30
   Ser Phe Met Leu Gly Glu Ile Ala Leu Tyr Ser Phe Ile Val Leu Leu
           35                  40                  45
   Leu Thr Gly Val Tyr Leu Thr Leu Phe Phe Asp Pro Ser Ile Thr Lys
       50                  55                  60
   Val Ile Tyr Asp Gly Gly Tyr Leu Pro Leu Asn Gly Val Glu Met Ser
   65                  70                  75                  80
   Arg Ala Tyr Ala Thr Ala Leu Asp Ile Ser Phe Glu Val Arg Gly Gly
                   85                  90                  95
   Leu Phe Ile Arg Gln Met His His Trp Ala Ala Leu Leu Phe Val Val
               100                 105                 110
   Ser Met Leu Val His Met Leu Arg Ile Phe Phe Thr Gly Ala Phe Arg
           115                 120                 125
   Arg Pro Arg Glu Ala Asn Trp Ile Ile Gly Val Val Leu Ile Ile Leu
       130                 135                 140
   Gly Met Ala Glu Gly Phe Met Gly Tyr Ser Leu Pro Asp Asp Leu Leu
   145                 150                 155                 160
   Ser Gly Val Gly Leu Arg Ile Met Ser Ala Ile Ile Val Gly Leu Pro
                   165                 170                 175
   Ile Ile Gly Thr Trp Met His Trp Leu Ile Phe Gly Gly Asp Phe Pro
               180                 185                 190
   Ser Asp Leu Met Leu Asp Arg Phe Tyr Ile Ala His Val Leu Ile Ile
           195                 200                 205
   Pro Ala Ile Leu Leu Gly Leu Ile Ala Ala His Leu Ala Leu Val Trp
       210                 215                 220
   Tyr Gln Lys His Thr Gln Phe Pro Gly Ala Gly Arg Thr Glu Asn Asn
   225                 230                 235                 240
   Val Ile Gly Ile Arg Ile Met Pro Leu Phe Ala Val Lys Ala Val Ala
                   245                 250                 255
   Phe Gly Leu Ile Val Phe Gly Phe Leu Ala Leu Leu Ala Gly Val Thr
               260                 265                 270
   Thr Ile Asn Ala Ile Trp Asn Leu Gly Pro Tyr Asn Pro Ser Gln Val
   275 280 285
   Ser Ala Gly Ser Gln Pro Asp Val Tyr Met Leu Trp Thr Asp Gly Ala
   290 295 300
   Ala Arg Val Met Pro Ala Trp Glu Leu Tyr Leu Gly Asn Tyr Thr Ile
   305 310 315 320
   Pro Ala Val Phe Trp Val Ala Val Met Leu Gly Ile Leu Val Val Leu
   325 330 335
   Leu Val Thr Tyr Pro Phe Ile Glu Arg Lys Phe Thr Gly Asp Asp Ala
   340 345 350
   His His Asn Leu Leu Gln Arg Pro Arg Asp Val Pro Val Arg Thr Ser
   355 360 365
   Leu Gly Val Met Ala Leu Val Phe Tyr Ile Leu Leu Thr Val Ser Gly
   370 375 380
   Gly Asn Asp Val Tyr Ala Met Gln Phe His Val Ser Leu Asn Ala Met
   385 390 395 400
   Thr Trp Ile Gly Arg Ile Gly Leu Ile Val Gly Pro Ala Ile Ala Tyr
   405 410 415
   Phe Ile Thr Tyr Arg Leu Cys Ile Gly Leu Gln Arg Ser Asp Arg Glu
   420 425 430
   Val Leu Glu His Gly Ile Glu Thr Gly Ile Ile Lys Gln Met Pro Asn
   435 440 445
   Gly Ala Phe Ile Glu Val His Gln Pro Leu Gly Pro Val Asp Asp His
   450 455 460
   Gly His Pro Ile Pro Leu Pro Tyr Ala Gly Ala Ala Val Pro Lys Gln
   465 470 475 480
   Met Asn Gln Leu Gly Tyr Ala Glu Val Glu Thr Arg Gly Gly Phe Phe
   485 490 495
   Gly Pro Asp Pro Glu Asp Ile Arg Ala Lys Ala Lys Glu Ile Glu His
   500 505 510
   Ala Asn His Ile Glu Glu Ala Asn Thr Leu Arg Ala Leu Asn Glu Ala
   515 520 525
   Asn Ile Glu Arg Asp Lys Asn Glu Gly Lys Asn
   530 535
<210> 55
   <211> 448
   <212> DNA
   <213> Corynebacterium glutamicum
<220>
   <221> CDS
   <222> (101)..(418)
   <223> RXA02236
<400> 55
   gcaggctgac atccttggta ttaaccaggt gtaccctcga tttctggata ctttggtatt 60
   ccttttgtca ctaaaaacca cacgataacg gaggaacccc gtg gcc ctt cca cag    115
                                             Val Ala Leu Pro Gln
                                             1               5
   ttg act gat gag cag cgc aag gca gcg ctt gct aag gca gca gag gca    163
   Leu Thr Asp Glu Gln Arg Lys Ala Ala Leu Ala Lys Ala Ala Glu Ala
                   10                  15                  20
   cgc aag gca cgc gca gag ctc aaa gag aac ctg aag cgc ggc aac act    211
   Arg Lys Ala Arg Ala Glu Leu Lys Glu Asn Leu Lys Arg Gly Asn Thr
               25                  30                  35
   aac ctc agg gaa gtt ctg gac aag gct gag tct gac gag atc atc ggc    259
   Asn Leu Arg Glu Val Leu Asp Lys Ala Glu Ser Asp Glu Ile Ile Gly
           40                  45                  50
   aag acc aag gtc tcc gct ctc ctc gag gct ctc cct aag gtt ggc aag    307
   Lys Thr Lys Val Ser Ala Leu Leu Glu Ala Leu Pro Lys Val Gly Lys
       55                  60                  65
   gtc aag gca aag gag att atg gac gag ctg ggc att gct cag acc cgt    355
   Val Lys Ala Lys Glu Ile Met Asp Glu Leu Gly Ile Ala Gln Thr Arg
   70                  75                  80                  85
   cgt ctt cgt gga ctg ggt gac cgt cag cgt cgc gca ctt ctc gag cgt    403
   Arg Leu Arg Gly Leu Gly Asp Arg Gln Arg Arg Ala Leu Leu Glu Arg
                   90                  95                  100
   ttc ggc ttc gag gat taattcttca gtgtcgggcg ataaccaact               448
   Phe Gly Phe Glu Asp
               105
<210> 56
   <211> 106
   <212> PRT
   <213> Corynebacterium glutamicum
<400> 56
   Val Ala Leu Pro Gln Leu Thr Asp Glu Gln Arg Lys Ala Ala Leu Ala
   1               5                   10                  15
   Lys Ala Ala Glu Ala Arg Lys Ala Arg Ala Glu Leu Lys Glu Asn Leu
               20                  25                  30
   Lys Arg Gly Asn Thr Asn Leu Arg Glu Val Leu Asp Lys Ala Glu Ser
           35                  40                  45
   Asp Glu Ile Ile Gly Lys Thr Lys Val Ser Ala Leu Leu Glu Ala Leu
       50                  55                  60
   Pro Lys Val Gly Lys Val Lys Ala Lys Glu Ile Met Asp Glu Leu Gly
   65                  70                  75                  80
   Ile Ala Gln Thr Arg Arg Leu Arg Gly Leu Gly Asp Arg Gln Arg Arg
                   85                  90                  95
   Ala Leu Leu Glu Arg Phe Gly Phe Glu Asp
               100                 105
<210> 57
   <211> 1003
   <212> DNA
   <213> Corynebacterium glutamicum
<220>
   <221> CDS'
   <222> (101)..(973)
   <223> RXA02267
<400> 57
   tgcgctcggc aagtgttttg cttatcgacg tctccccaca taacaatccc aactcgaagc 60
   accaacgatt caagccttat cagttttgta caggaaaata gtg caa aaa tgg ggt   115
                                              Val Gln Lys Trp Gly
                                              1               5
   tta agc ttc gtg gag agg att gtc atc atg aac aac gtg caa cag ttt   163
   Leu Ser Phe Val Glu Arg Ile Val Ile Met Asn Asn Val Gln Gln Phe
                   10                  15                  20
   cat cga ttt ttt gat gat tcc gca gtc tat tat ccc tgc ttc gtc ccg   211
   His Arg Phe Phe Asp Asp Ser Ala Val Tyr Tyr Pro Cys Phe Val Pro
               25                  30                  35
   ctt gac cga gcc atc ggc gaa cac ttt gat cgt cag aac aaa ccg atg   259
   Leu Asp Arg Ala Ile Gly Glu His Phe Asp Arg Gln Asn Lys Pro Met
           40                  45                  50
   tcc aga ttc atc gga acg ctc att ctg ccg tta gcc aaa ctg gaa gaa   307
   Ser Arg Phe Ile Gly Thr Leu Ile Leu Pro Leu Ala Lys Leu Glu Glu
       55                  60                  65
   gcc gcc caa tac acc ggc gat gaa gtc ctt cgc gtg tcg gca gta atc   355
   Ala Ala Gln Tyr Thr Gly Asp Glu Val Leu Arg Val Ser Ala Val Ile
   70                  75                  80                  85
   agt act gat ggg ctc gct gat ctg cga agg gat ttt tac gaa ctc ccc   403
   Ser Thr Asp Gly Leu Ala Asp Leu Arg Arg Asp Phe Tyr Glu Leu Pro
                   90                  95                  100
   aac atc gac atc gcc tcg gtg gaa atc aag ctg gtc ggc gca gcc ctc   451
   Asn Ile Asp Ile Ala Ser Val Glu Ile Lys Leu Val Gly Ala Ala Leu
               105                 110                 115
   acc aac acc gct tgg ttg gga gat gtg gaa aaa ctc atc caa caa cat   499
   Thr Asn Thr Ala Trp Leu Gly Asp Val Glu Lys Leu Ile Gln Gln His
           120                 125                 130
   cgc aac act ttc gta tgg gtt gag att ccg aca gcc ctg gtc acc gca   547
   Arg Asn Thr Phe Val Trp Val Glu Ile Pro Thr Ala Leu Val Thr Ala
       135                 140                 145
   gat att gtc cga aaa ctc cgc cac atg gga gct ggc ctg aaa tac aga   595
   Asp Ile Val Arg Lys Leu Arg His Met Gly Ala Gly Leu Lys Tyr Arg
   150                 155                 160                 165
   act gga ggt gat agg gaa gag ctc ttc ccc tca ccg cag gac ttg gtc   643
   Thr Gly Gly Asp Arg Glu Glu Leu Phe Pro Ser Pro Gln Asp Leu Val
                   170                 175                 180
   act gtg ctg cgc acc gcc atc gat gct gca ttg ccg ttt aaa ctc act   691
   Thr Val Leu Arg Thr Ala Ile Asp Ala Ala Leu Pro Phe Lys Leu Thr
               185                 190                 195
   gca ggc ctg cat cgt gct ctc agg tat cgt gac gag aaa acc ggc cga     739
   Ala Gly Leu His Arg Ala Leu Arg Tyr Arg Asp Glu Lys Thr Gly Arg
           200                 205                 210
   ctt cac ttc gga ttc ctc aac att gca gcc gcc gtg gcg aca ctt cgt     787
   Leu His Phe Gly Phe Leu Asn Ile Ala Ala Ala Val Ala Thr Leu Arg
       215                 220                 225
   gct gga aaa ggc gag gca gag gca ctg aag atc ctt gaa ggc gat gat     835
   Ala Gly Lys Gly Glu Ala Glu Ala Leu Lys Ile Leu Glu Gly Asp Asp
   230                 235                 240                 245
   gcc gct ccg ctt att cac gca cta caa agc ggc gaa aac tgg cgg gat     883
   Ala Ala Pro Leu Ile His Ala Leu Gln Ser Gly Glu Asn Trp Arg Asp
                   250                 255                 260
   tcc ttc cgc agc ttc agt acc tgc aat gtt gtt gaa cca ctc aac act     931
   Ser Phe Arg Ser Phe Ser Thr Cys Asn Val Val Glu Pro Leu Asn Thr
               265                 270                 275
   ctg att gat ctt gat gtg ttg gcg gaa gga gac gta cat ccc             973
   Leu Ile Asp Leu Asp Val Leu Ala Glu Gly Asp Val His Pro
           280                 285                 290
   taaggatcga cgctagttag atcggttttt                                    1003
<210> 58
   <211> 291
   <212> PRT
   <213> Corynebacterium glutamicum
<400> 58
   Val Gln Lys Trp Gly Leu Ser Phe Val Glu Arg Ile Val Ile Met Asn
   1               5                   10                  15
   Asn Val Gln Gln Phe His Arg Phe Phe Asp Asp Ser Ala Val Tyr Tyr
               20                  25                  30
   Pro Cys Phe Val Pro Leu Asp Arg Ala Ile Gly Glu His Phe Asp Arg
           35                  40                  45
   Gln Asn Lys Pro Met Ser Arg Phe Ile Gly Thr Leu Ile Leu Pro Leu
       50                  55                  60
   Ala Lys Leu Glu Glu Ala Ala Gln Tyr Thr Gly Asp Glu Val Leu Arg
   65                  70                  75                  80
   Val Ser Ala Val Ile Ser Thr Asp Gly Leu Ala Asp Leu Arg Arg Asp
                   85                  90                  95
   Phe Tyr Glu Leu Pro Asn Ile Asp Ile Ala Ser Val Glu Ile Lys Leu
               100                 105                 110
   Val Gly Ala Ala Leu Thr Asn Thr Ala Trp Leu Gly Asp Val Glu Lys
           115                 120                 125
   Leu Ile Gln Gln His Arg Asn Thr Phe Val Trp Val Glu Ile Pro Thr
       130                 135                 140
   Ala Leu Val Thr Ala Asp Ile Val Arg Lys Leu Arg His Met Gly Ala
   145                 150                 155                 160
   Gly Leu Lys Tyr Arg Thr Gly Gly Asp Arg Glu Glu Leu Phe Pro Ser
                   165                 170                 175
   Pro Gln Asp Leu Val Thr Val Leu Arg Thr Ala Ile Asp Ala Ala Leu
               180                 185                 190
   Pro Phe Lys Leu Thr Ala Gly Leu His Arg Ala Leu Arg Tyr Arg Asp
           195                 200                 205
   Glu Lys Thr Gly Arg Leu His Phe Gly Phe Leu Asn Ile Ala Ala Ala
       210                 215                 220
   Val Ala Thr Leu Arg Ala Gly Lys Gly Glu Ala Glu Ala Leu Lys Ile
   225                 230                 235                 240
   Leu Glu Gly Asp Asp Ala Ala Pro Leu Ile His Ala Leu Gln Ser Gly
                   245                 250                 255
   Glu Asn Trp Arg Asp Ser Phe Arg Ser Phe Ser Thr Cys Asn Val Val
               260                 265                 270
   Glu Pro Leu Asn Thr Leu Ile Asp Leu Asp Val Leu Ala Glu Gly Asp
           275                 280                 285
   Val His Pro
       290
<210> 59
   <211> 1984
   <212> DNA
   <213> Corynebacterium glutamicum
<220>
   <221> CDS
   <222> (101)..(1954)
   <223> RXA02280
<400> 59
   ggtcgaggtg tcgtagatgt caatgagctt cgcgattgcg tcatcgatcg ttgttgcttc 60
   catgcgcacc acactatctt tctgcacgcc ctgatgccct gtg gat tca aaa ctg   115
                                             Val Asp Ser Lys Leu
                                             1               5
   tgc ttt tat agg cgt atg caa gaa tcc tca cgt gat aat ttc caa gtt   163
   Cys Phe Tyr Arg Arg Met Gln Glu Ser Ser Arg Asp Asn Phe Gln Val
                   10                  15                  20
   gac ctc ggc ggc gtt gtt gat ctt ttg agt cgc cac att tat tcc ggt   211
   Asp Leu Gly Gly Val Val Asp Leu Leu Ser Arg His Ile Tyr Ser Gly
               25                  30                  35
   ccg agg gtg tat gtg cgt gag ttg ctg cag aat gcg gtt gat gct tgt   259
   Pro Arg Val Tyr Val Arg Glu Leu Leu Gln Asn Ala Val Asp Ala Cys
           40                  45                  50
   act gca cgt tct gaa cag ggt gag gag ggc tac gag ccg agt att cgt   307
   Thr Ala Arg Ser Glu Gln Gly Glu Glu Gly Tyr Glu Pro Ser Ile Arg
       55                  60                  65
   att cgg ccg gtg acc aag gat cgt gcc acg ttt tca ctg gtt gat aat   355
   Ile Arg Pro Val Thr Lys Asp Arg Ala Thr Phe Ser Leu Val Asp Asn
   70                  75                  80                  85
   ggt acg ggc ctg acc gcg cag gag gcg cgg gaa ttg ctg gcg acg gtg      403
   Gly Thr Gly Leu Thr Ala Gln Glu Ala Arg Glu Leu Leu Ala Thr Val
                   90                  95                  100
   ggg cgg acg tcg aaa cgc gat gaa ttc ggt ctg cag cgg gaa ggt cgc      451
   Gly Arg Thr Ser Lys Arg Asp Glu Phe Gly Leu Gln Arg Glu Gly Arg
               105                 110                 115
   ctg ggg caa ttt ggc atc ggg ctg ctt agt tgt ttc atg gtg gcg gat      499
   Leu Gly Gln Phe Gly Ile Gly Leu Leu Ser Cys Phe Met Val Ala Asp
           120                 125                 130
   gag atc acc atg gtg tcg cat gcg gag ggt gcg tcg gcg att cgg tgg      547
   Glu Ile Thr Met Val Ser His Ala Glu Gly Ala Ser Ala Ile Arg Trp
       135                 140                 145
   act ggt cat gcg gat ggc acc ttt aac ctg gag att ctt ggg gat gac      595
   Thr Gly His Ala Asp Gly Thr Phe Asn Leu Glu Ile Leu Gly Asp Asp
   150                 155                 160                 165
   gca acg gat gtc att ccg gtg ggc acg act gtg cac ctg act ccg cgc      643
   Ala Thr Asp Val Ile Pro Val Gly Thr Thr Val His Leu Thr Pro Arg
                   170                 175                 180
   cct gat gag cgc acg ttg ctg acg gaa aat tcc gtg gtc acc att gct      691
   Pro Asp Glu Arg Thr Leu Leu Thr Glu Asn Ser Val Val Thr Ile Ala
               185                 190                 195
   agt aat tat ggc cgc tac ctg ccg att cct att gtg gtg cag ggt gag      739
   Ser Asn Tyr Gly Arg Tyr Leu Pro Ile Pro Ile Val Val Gln Gly Glu
           200                 205                 210
   aaa aac acc acc atc act aca tcg ccg gtg ttt gca aag gat act gat      787
   Lys Asn Thr Thr Ile Thr Thr Ser Pro Val Phe Ala Lys Asp Thr Asp
       215                 220                 225
   cag cag cac agg ctg tat gcc ggc cgg gag cgc ctt ggt aaa act cct      835
   Gln Gln His Arg Leu Tyr Ala Gly Arg Glu Arg Leu Gly Lys Thr Pro
   230                 235                 240                 245
   ttt gat gtc atc gat ctc acc ggt cct ggc atc gag ggt gtg gct tat      883
   Phe Asp Val Ile Asp Leu Thr Gly Pro Gly Ile Glu Gly Val Ala Tyr
                   250                 255                 260
   gta ttg ccg gag gcc cag gct ccg cat atg tcc agg cgt cac agt att      931
   Val Leu Pro Glu Ala Gln Ala Pro His Met Ser Arg Arg His Ser Ile
               265                 270                 275
   tat gtc aac cgc atg ttg gtc tct gat ggg cct tcc acg gtg ctg ccc      979
   Tyr Val Asn Arg Met Leu Val Ser Asp Gly Pro Ser Thr Val Leu Pro
           280                 285                 290
   aac tgg gcg ttc ttt gtg gaa tgt gaa atc aat tca acc gat ttg gaa      1027
   Asn Trp Ala Phe Phe Val Glu Cys Glu Ile Asn Ser Thr Asp Leu Glu
       295                 300                 305
   ccc acc gca tcg cgt gaa gcg ctc atg gat gac acc gcg ttc gcg gca      1075
   Pro Thr Ala Ser Arg Glu Ala Leu Met Asp Asp Thr Ala Phe Ala Ala
   310                 315                 320                 325
   acc agg gaa cat atc ggt gag tgc att aaa tcg tgg ctg att aat ctc      1123
   Thr Arg Glu His Ile Gly Glu Cys Ile Lys Ser Trp Leu Ile Asn Leu
                   330                 335                 340
   gcc atg acc aag cct cac cgc gtg cgg gaa ttt act gcg att cat gat      1171
   Ala Met Thr Lys Pro His Arg Val Arg Glu Phe Thr Ala Ile His Asp
               345                 350                 355
   ctt gcc ctg cgc gag ctg tgc caa tcg gac gcg gac ctg gct gaa acc      1219
   Leu Ala Leu Arg Glu Leu Cys Gln Ser Asp Ala Asp Leu Ala Glu Thr
           360                 365                 370
   atg ttg ggt ctt ctc acc ttg gag acc tcc cgt ggt cgc atc tcg atc      1267
   Met Leu Gly Leu Leu Thr Leu Glu Thr Ser Arg Gly Arg Ile Ser Ile
       375                 380                 385
   ggt gag atc acc acg ttg tcc atc acc gag gat gtg tcg ctg cag ctg      1315
   Gly Glu Ile Thr Thr Leu Ser Ile Thr Glu Asp Val Ser Leu Gln Leu
   390                 395                 400                 405
   gct acc acg ttg gat gat ttc agg cag ctc aac acc att gcg cgc ccg      1363
   Ala Thr Thr Leu Asp Asp Phe Arg Gln Leu Asn Thr Ile Ala Arg Pro
                   410                 415                 420
   gac acc ttg att att aat ggc ggc tac att cac gac agc gat ctg gct      1411
   Asp Thr Leu Ile Ile Asn Gly Gly Tyr Ile His Asp Ser Asp Leu Ala
               425                 430                 435
   cgg ctc att ccc gtt cac tac cca ccg ctt acg gta tct act gct gac      1459
   Arg Leu Ile Pro Val His Tyr Pro Pro Leu Thr Val Ser Thr Ala Asp
           440                 445                 450
   ctg cgc gaa tcc atg gat ctg atg gag ctt ccg ccg ctg cag gac att      1507
   Leu Arg Glu Ser Met Asp Leu Met Glu Leu Pro Pro Leu Gln Asp Ile
       455                 460                 465
   gag aaa gcc aag gca ctg gat gcg cag gtc acg gaa tca ttg aag gat      1555
   Glu Lys Ala Lys Ala Leu Asp Ala Gln Val Thr Glu Ser Leu Lys Asp
   470                 475                 480                 485
   ttt cag atc aag ggc gca acg agg gtt ttt gaa ccc gca gat gtt cct      1603
   Phe Gln Ile Lys Gly Ala Thr Arg Val Phe Glu Pro Ala Asp Val Pro
                   490                 495                 500
   gcc gtg gtg atc att gat tcc aag gcg cag gcc tca cgg gat cgc aat      1651
   Ala Val Val Ile Ile Asp Ser Lys Ala Gln Ala Ser Arg Asp Arg Asn
               505                 510                 515
   gaa aca caa agc gca acc act gat cgt tgg gct gac att ttg gca acg      1699
   Glu Thr Gln Ser Ala Thr Thr Asp Arg Trp Ala Asp Ile Leu Ala Thr
           520                 525                 530
   gtg gat aac acg ttg agc cgt caa aca gcc aac att cca cag gat cag      1747
   Val Asp Asn Thr Leu Ser Arg Gln Thr Ala Asn Ile Pro Gln Asp Gln
       535                 540                 545
   gga ctg tcg gcg ttg tgc ttg aat tgg aac aat tcg ctg gtc agg aaa      1795
   Gly Leu Ser Ala Leu Cys Leu Asn Trp Asn Asn Ser Leu Val Arg Lys
   550                 555                 560                 565
   ttg gcg tcc act gat gac acc gcc gtg gtg tcg cgc acg gtg cgt ttg      1843
   Leu Ala Ser Thr Asp Asp Thr Ala Val Val Ser Arg Thr Val Arg Leu
                   570                 575                 580
   ctc tac gtt cag gca ttg ttg tcc agc aag agg cca ctg cgg gtg aag      1891
   Leu Tyr Val Gln Ala Leu Leu Ser Ser Lys Arg Pro Leu Arg Val Lys
               585                 590                 595
   gaa cgc gcg ctg ctt aat gat tcg ctg gca gat ctg gtt tct ttg tct    1939
   Glu Arg Ala Leu Leu Asn Asp Ser Leu Ala Asp Leu Val Ser Leu Ser
           600                 605                 610
   ttg tca tcc gat atc taagacaatc ctccgctaat ctcgagggca               1984
   Leu Ser Ser Asp Ile
       615
<210> 60
   <211> 618
   <212> PRT
   <213> Corynebacterium glutamicum
<400> 60
   Val Asp Ser Lys Leu Cys Phe Tyr Arg Arg Met Gln Glu Ser Ser Arg
    1               5                  10                  15
   Asp Asn Phe Gln Val Asp Leu Gly Gly Val Val Asp Leu Leu Ser Arg
               20                  25                  30
   His Ile Tyr Ser Gly Pro Arg Val Tyr Val Arg Glu Leu Leu Gln Asn
           35                  40                  45
   Ala Val Asp Ala Cys Thr Ala Arg Ser Glu Gln Gly Glu Glu Gly Tyr
       50                  55                  60
   Glu Pro Ser Ile Arg Ile Arg Pro Val Thr Lys Asp Arg Ala Thr Phe
   65                  70                  75                  80
   Ser Leu Val Asp Asn Gly Thr Gly Leu Thr Ala Gln Glu Ala Arg Glu
                   85                  90                  95
   Leu Leu Ala Thr Val Gly Arg Thr Ser Lys Arg Asp Glu Phe Gly Leu
               100                 105                 110
   Gln Arg Glu Gly Arg Leu Gly Gln Phe Gly Ile Gly Leu Leu Ser Cys
           115                 120                 125
   Phe Met Val Ala Asp Glu Ile Thr Met Val Ser His Ala Glu Gly Ala
       130                 135                 140
   Ser Ala Ile Arg Trp Thr Gly His Ala Asp Gly Thr Phe Asn Leu Glu
   145                 150                 155                 160
   Ile Leu Gly Asp Asp Ala Thr Asp Val Ile Pro Val Gly Thr Thr Val
                   165                 170                 175
   His Leu Thr Pro Arg Pro Asp Glu Arg Thr Leu Leu Thr Glu Asn Ser
               180                 185                 190
   Val Val Thr Ile Ala Ser Asn Tyr Gly Arg Tyr Leu Pro Ile Pro Ile
           195                 200                 205
   Val Val Gln Gly Glu Lys Asn Thr Thr Ile Thr Thr Ser Pro Val Phe
       210                 215                 220
   Ala Lys Asp Thr Asp Gln Gln His Arg Leu Tyr Ala Gly Arg Glu Arg
   225                 230                 235                 240
   Leu Gly Lys Thr Pro Phe Asp Val Ile Asp Leu Thr Gly Pro Gly Ile
                   245                 250                 255
   Glu Gly Val Ala Tyr Val Leu Pro Glu Ala Gln Ala Pro His Met Ser
   260 265 270
   Arg Arg His Ser Ile Tyr Val Asn Arg Met Leu Val Ser Asp Gly Pro
   275 280 285
   Ser Thr Val Leu Pro Asn Trp Ala Phe Phe Val Glu Cys Glu Ile Asn
   290 295 300
   Ser Thr Asp Leu Glu Pro Thr Ala Ser Arg Glu Ala Leu Met Asp Asp
   305 310 315 320
   Thr Ala Phe Ala Ala Thr Arg Glu His Ile Gly Glu Cys Ile Lys Ser
   325 330 335
   Trp Leu Ile Asn Leu Ala Met Thr Lys Pro His Arg Val Arg Glu Phe
   340 345 350
   Thr Ala Ile His Asp Leu Ala Leu Arg Glu Leu Cys Gln Ser Asp Ala
   355 360 365
   Asp Leu Ala Glu Thr Met Leu Gly Leu Leu Thr Leu Glu Thr Ser Arg
   370 375 380
   Gly Arg Ile Ser Ile Gly Glu Ile Thr Thr Leu Ser Ile Thr Glu Asp
   385 390 395 400
   Val Ser Leu Gln Leu Ala Thr Thr Leu Asp Asp Phe Arg Gln Leu Asn
   405 410 415
   Thr Ile Ala Arg Pro Asp Thr Leu Ile Ile Asn Gly Gly Tyr Ile His
   420 425 430
   Asp Ser Asp Leu Ala Arg Leu Ile Pro Val His Tyr Pro Pro Leu Thr
   435 440 445
   Val Ser Thr Ala Asp Leu Arg Glu Ser Met Asp Leu Met Glu Leu Pro
   450 455 460
   Pro Leu Gln Asp Ile Glu Lys Ala Lys Ala Leu Asp Ala Gln Val Thr
   465 470 475 480
   Glu Ser Leu Lys Asp Phe Gln Ile Lys Gly Ala Thr Arg Val Phe Glu
   485 490 495
   Pro Ala Asp Val Pro Ala Val Val Ile Ile Asp Ser Lys Ala Gln Ala
   500 505 510
   Ser Arg Asp Arg Asn Glu Thr Gln Ser Ala Thr Thr Asp Arg Trp Ala
   515 520 525
   Asp Ile Leu Ala Thr Val Asp Asn Thr Leu Ser Arg Gln Thr Ala Asn
   530 535 540
   Ile Pro Gln Asp Gln Gly Leu Ser Ala Leu Cys Leu Asn Trp Asn Asn
   545 550 555 560
   Ser Leu Val Arg Lys Leu Ala Ser Thr Asp Asp Thr Ala Val Val Ser
   565 570 575
   Arg Thr Val Arg Leu Leu Tyr Val Gln Ala Leu Leu Ser Ser Lys Arg
   580 585 590
   Pro Leu Arg Val Lys Glu Arg Ala Leu Leu Asn Asp Ser Leu Ala Asp
   595 600 605
   Leu Val Ser Leu Ser Leu Ser Ser Asp Ile
   610 615
<210> 61
   <211> 1792
   <212> DNA
   <213> Corynebacterium glutamicum
<220>
   <221> CDS
   <222> (101)..(1762)
   <223> RXA02388
<400> 61
   ttcgggagag caacggtggg tttagcaccg tggaggattt actgcaggtc aaggggattg 60
   ggccctcaaa gtttgagcag atctctggat tggtgtcccc atg att gag gtg cgt    115
                                               Met Ile Glu Val Arg
                                                1               5
   ttg gtt ccg gtg gcg gct gtg atg tgg atg gct gtc gct gcg ttg att    163
   Leu Val Pro Val Ala Ala Val Met Trp Met Ala Val Ala Ala Leu Ile
                   10                  15                  20
   atc agt ggt tcg tgg gtg ttg tcg gtg ggg att gtt ggc atc gcg atc    211
   Ile Ser Gly Ser Trp Val Leu Ser Val Gly Ile Val Gly Ile Ala Ile
               25                  30                  35
   att gct gct tgt gtg ttt aaa cac tgg ggt caa gct gtg gtg ata gct    259
   Ile Ala Ala Cys Val Phe Lys His Trp Gly Gln Ala Val Val Ile Ala
           40                  45                  50
   gca ctg ggc gtt ggt gcc gta gtg atg gct gcg ttg aga atc agc agc    307
   Ala Leu Gly Val Gly Ala Val Val Met Ala Ala Leu Arg Ile Ser Ser
       55                  60                  65
   gcg aag gca ttt gaa gca ccg caa acc tgg gtg ggt acc gca gaa acc    355
   Ala Lys Ala Phe Glu Ala Pro Gln Thr Trp Val Gly Thr Ala Glu Thr
   70                  75                  80                  85
   atc aag ttt tta gac agc ggt gat caa cta atc ggt ttg aga gta gaa    403
   Ile Lys Phe Leu Asp Ser Gly Asp Gln Leu Ile Gly Leu Arg Val Glu
                   90                  95                  100
   ggc tat cca gcg ccg att cca gtg ttt tac tct ggt agc gac acc att    451
   Gly Tyr Pro Ala Pro Ile Pro Val Phe Tyr Ser Gly Ser Asp Thr Ile
               105                 110                 115
   gag aaa gcc tct ctc att gca gtg tcc ggt cgg att aaa cca gat agt    499
   Glu Lys Ala Ser Leu Ile Ala Val Ser Gly Arg Ile Lys Pro Asp Ser
           120                 125                 130
   ttc cct ggg gtg ggt gat ctg acc att tcc act gaa gac att gat cag    547
   Phe Pro Gly Val Gly Asp Leu Thr Ile Ser Thr Glu Asp Ile Asp Gln
       135                 140                 145
   ttg gaa ccg acc act ggt tat agc gca tgg gtg aac cag gtg cgt gac    595
   Leu Glu Pro Thr Thr Gly Tyr Ser Ala Trp Val Asn Gln Val Arg Asp
   150                 155                 160                 165
   ggg ttt tcc caa gcc gtg gaa gaa acc gtg ggg gag tct tcc cgt gga    643
   Gly Phe Ser Gln Ala Val Glu Glu Thr Val Gly Glu Ser Ser Arg Gly
                   170                 175                 180
   ctg att cca ggc atg gtg ttg ggg gat acg cgg ttg cag ggg tca att    691
   Leu Ile Pro Gly Met Val Leu Gly Asp Thr Arg Leu Gln Gly Ser Ile
               185                 190                 195
   gaa gcc caa acc tat att gat acg ggg ttg tct cac ctg tca gct gtt    739
   Glu Ala Gln Thr Tyr Ile Asp Thr Gly Leu Ser His Leu Ser Ala Val
           200                 205                 210
   agt gga agc aat gta gcc att gtg gtg tcc tct gtg gtg gtg ttg tcg    787
   Ser Gly Ser Asn Val Ala Ile Val Val Ser Ser Val Val Val Leu Ser
       215                 220                 225
   tat ttt ctc acc gct ggg cca cgc atc agg gtg gtg gcg tca ttg ctg    835
   Tyr Phe Leu Thr Ala Gly Pro Arg Ile Arg Val Val Ala Ser Leu Leu
   230                 235                 240                 245
   tcc tta gtt att ttt gtc tcc ctc gtg ggg ttt gaa cca agt gtg ctt    883
   Ser Leu Val Ile Phe Val Ser Leu Val Gly Phe Glu Pro Ser Val Leu
                   250                 255                 260
   cgt gct tcg gtc aca ggc atc gtg ggg ctt ctg gca atc atc aac tct    931
   Arg Ala Ser Val Thr Gly Ile Val Gly Leu Leu Ala Ile Ile Asn Ser
               265                 270                 275
   tct cgg atg gag ccg atg cat ggg ttg agt ctt tcg gtg att tgc tta    979
   Ser Arg Met Glu Pro Met His Gly Leu Ser Leu Ser Val Ile Cys Leu
           280                 285                 290
   ctg ttt tat gat tcc aac ctg gcg gtg cat tac gga ttc tta ctc tcg    1027
   Leu Phe Tyr Asp Ser Asn Leu Ala Val His Tyr Gly Phe Leu Leu Ser
       295                 300                 305
   tgt gca gca act gct ggc att gtg atg ctt caa cca ctg ctg tac cgt    1075
   Cys Ala Ala Thr Ala Gly Ile Val Met Leu Gln Pro Leu Leu Tyr Arg
   310                 315                 320                 325
   gcc atc ggt cca cca ctg gcg gtg tgg aaa gta cca gac atc gtg gtg    1123
   Ala Ile Gly Pro Pro Leu Ala Val Trp Lys Val Pro Asp Ile Val Val
                   330                 335                 340
   cgc gct ttc gcg gtg tcc att gcc gct gat ctg gtg acc atc ccg att    1171
   Arg Ala Phe Ala Val Ser Ile Ala Ala Asp Leu Val Thr Ile Pro Ile
               345                 350                 355
   atc gct ctg atg gct cgc caa ata tcc ctc gtg gca gtg ctg gcc aac    1219
   Ile Ala Leu Met Ala Arg Gln Ile Ser Leu Val Ala Val Leu Ala Asn
           360                 365                 370
   gtg ttg gtt gaa tta gct gtt cca ccc atc acg ttg ctt ggg ttg att    1267
   Val Leu Val Glu Leu Ala Val Pro Pro Ile Thr Leu Leu Gly Leu Ile
       375                 380                 385
   gcc gtg ctg gca agc ctt ctt ccc tgg cca gtg gaa tac cca ctc ttg    1315
   Ala Val Leu Ala Ser Leu Leu Pro Trp Pro Val Glu Tyr Pro Leu Leu
   390                 395                 400                 405
   aaa atc att gag ccc ttc acc tgg tgg att cat cac gtg gcc aag tgg    1363
   Lys Ile Ile Glu Pro Phe Thr Trp Trp Ile His His Val Ala Lys Trp
                   410                 415                 420
   tgc caa caa tta ccc aat tcg acg ctg gaa ata agt gct ggt tgg gca    1411
   Cys Gln Gln Leu Pro Asn Ser Thr Leu Glu Ile Ser Ala Gly Trp Ala
               425                 430                 435
   ggg att gcc tgg gcg tgt atg gca gcg gtg tgg gtg gtg gtg att atc    1459
   Gly Ile Ala Trp Ala Cys Met Ala Ala Val Trp Val Val Val Ile Ile
           440                 445                 450
   tac aaa gga tat gtg cgc acc ctt gca gtg tgt tgt gtc tgc ttc ttt    1507
   Tyr Lys Gly Tyr Val Arg Thr Leu Ala Val Cys Cys Val Cys Phe Phe
       455                 460                 465
   ctt ttc ggc gcg tgg aat aac aga ctg cca gcc caa ata gat ccg aca    1555
   Leu Phe Gly Ala Trp Asn Asn Arg Leu Pro Ala Gln Ile Asp Pro Thr
   470                 475                 480                 485
   gag ctg cgg ttt gtc atc atc gcc gat gat tct gag ctc act gat gtg    1603
   Glu Leu Arg Phe Val Ile Ile Ala Asp Asp Ser Glu Leu Thr Asp Val
                   490                 495                 500
   ccc gaa cat gca gaa ttg atc atc gtg gaa gac ccc cac ggc agc atg    1651
   Pro Glu His Ala Glu Leu Ile Ile Val Glu Asp Pro His Gly Ser Met
               505                 510                 515
   tcc gat cgc ccc atc gtc acc aga gaa gga atc cct gtg ctg tat cca    1699
   Ser Asp Arg Pro Ile Val Thr Arg Glu Gly Ile Pro Val Leu Tyr Pro
           520                 525                 530
   tac cgc gat ggg gag gtc agc ctt cat att gat ggc acc cag cat gca    1747
   Tyr Arg Asp Gly Glu Val Ser Leu His Ile Asp Gly Thr Gln His Ala
       535                 540                 545
   gcg gac ggg aga ttt taacgacact tgtggcacga tggtcacgtg               1792
   Ala Asp Gly Arg Phe
   550
<210> 62
   <211> 554
   <212> PRT
   <213> Corynebacterium glutamicum
<400> 62
   Met Ile Glu Val Arg Leu Val Pro Val Ala Ala Val Met Trp Met Ala
   1 5 10 15
   Val Ala Ala Leu Ile Ile Ser Gly Ser Trp Val Leu Ser Val Gly Ile
   20 25 30
   Val Gly Ile Ala Ile Ile Ala Ala Cys Val Phe Lys His Trp Gly Gln
   35 40 45
   Ala Val Val Ile Ala Ala Leu Gly Val Gly Ala Val Val Met Ala Ala
   50 55 60
   Leu Arg Ile Ser Ser Ala Lys Ala Phe Glu Ala Pro Gln Thr Trp Val
   65 70 75 80
   Gly Thr Ala Glu Thr Ile Lys Phe Leu Asp Ser Gly Asp Gln Leu Ile
   85 90 95
   Gly Leu Arg Val Glu Gly Tyr Pro Ala Pro Ile Pro Val Phe Tyr Ser
               100                 105                 110
   Gly Ser Asp Thr Ile Glu Lys Ala Ser Leu Ile Ala Val Ser Gly Arg
           115                 120                 125
   Ile Lys Pro Asp Ser Phe Pro Gly Val Gly Asp Leu Thr Ile Ser Thr
       130                 135                 140
   Glu Asp Ile Asp Gln Leu Glu Pro Thr Thr Gly Tyr Ser Ala Trp Val
   145                 150                 155                 160
   Asn Gln Val Arg Asp Gly Phe Ser Gln Ala Val Glu Glu Thr Val Gly
                   165                 170                 175
   Glu Ser Ser Arg Gly Leu Ile Pro Gly Met Val Leu Gly Asp Thr Arg
               180                 185                 190
   Leu Gln Gly Ser Ile Glu Ala Gln Thr Tyr Ile Asp Thr Gly Leu Ser
           195                 200                 205
   His Leu Ser Ala Val Ser Gly Ser Asn Val Ala Ile Val Val Ser Ser
       210                 215                 220
   Val Val Val Leu Ser Tyr Phe Leu Thr Ala Gly Pro Arg Ile Arg Val
   225                 230                 235                 240
   Val Ala Ser Leu Leu Ser Leu Val Ile Phe Val Ser Leu Val Gly Phe
                   245                 250                 255
   Glu Pro Ser Val Leu Arg Ala Ser Val Thr Gly Ile Val Gly Leu Leu
               260                 265                 270
   Ala Ile Ile Asn Ser Ser Arg Met Glu Pro Met His Gly Leu Ser Leu
           275                 280                 285
   Ser Val Ile Cys Leu Leu Phe Tyr Asp Ser Asn Leu Ala Val His Tyr
       290                 295                 300
   Gly Phe Leu Leu Ser Cys Ala Ala Thr Ala Gly Ile Val Met Leu Gln
   305                 310                 315                 320
   Pro Leu Leu Tyr Arg Ala Ile Gly Pro Pro Leu Ala Val Trp Lys Val
                   325                 330                 335
   Pro Asp Ile Val Val Arg Ala Phe Ala Val Ser Ile Ala Ala Asp Leu
               340                 345                 350
   Val Thr Ile Pro Ile Ile Ala Leu Met Ala Arg Gln Ile Ser Leu Val
           355                 360                 365
   Ala Val Leu Ala Asn Val Leu Val Glu Leu Ala Val Pro Pro Ile Thr
       370                 375                 380
   Leu Leu Gly Leu Ile Ala Val Leu Ala Ser Leu Leu Pro Trp Pro Val
   385                 390                 395                 400
   Glu Tyr Pro Leu Leu Lys Ile Ile Glu Pro Phe Thr Trp Trp Ile His
                   405                 410                 415
   His Val Ala Lys Trp Cys Gln Gln Leu Pro Asn Ser Thr Leu Glu Ile
               420                 425                 430
   Ser Ala Gly Trp Ala Gly Ile Ala Trp Ala Cys Met Ala Ala Val Trp
           435                 440                 445
   Val Val Val Ile Ile Tyr Lys Gly Tyr Val Arg Thr Leu Ala Val Cys
   450 455 460
   Cys Val Cys Phe Phe Leu Phe Gly Ala Trp Asn Asn Arg Leu Pro Ala
   465 470 475 480
   Gln Ile Asp Pro Thr Glu Leu Arg Phe Val Ile Ile Ala Asp Asp Ser
   485 490 495
   Glu Leu Thr Asp Val Pro Glu His Ala Glu Leu Ile Ile Val Glu Asp
   500 505 510
   Pro His Gly Ser Met Ser Asp Arg Pro Ile Val Thr Arg Glu Gly Ile
   515 520 525
   Pro Val Leu Tyr Pro Tyr Arg Asp Gly Glu Val Ser Leu His Ile Asp
   530 535 540
   Gly Thr Gln His Ala Ala Asp Gly Arg Phe
   545 550
<210> 63
   <211> 2977
   <212> DNA
   <213> Corynebacterium glutamicum
<220>
   <221> CDS
   <222> (101)..(2947)
   <223> RXA02416
<400> 63
   agtcatgtgg ttcagactag cggaaacacc ttgttcgatg ctatgttcga aggtgtattt 60
   tttgaagcga caaaaatcga ttttgaaggg cagttgagca ttg gct gat cgc ctc   115
                                               Leu Ala Asp Arg Leu
                                                1               5
   gta gtg cgc gga gcg cgt gaa cat aac cta aaa ggc gtg gat att gat   163
   Val Val Arg Gly Ala Arg Glu His Asn Leu Lys Gly Val Asp Ile Asp
                   10                  15                  20
   ttg cca cgc gac tcg atg gtg gtg ttc acc ggc ctg tca ggt tcc ggt   211
   Leu Pro Arg Asp Ser Met Val Val Phe Thr Gly Leu Ser Gly Ser Gly
               25                  30                  35
   aaa tca tca ctg gcc ttt gac acc atc ttt gcg gaa ggc cag cgc cgt   259
   Lys Ser Ser Leu Ala Phe Asp Thr Ile Phe Ala Glu Gly Gln Arg Arg
           40                  45                  50
   tac gtg gag tcg ttg tcc agt tac gcc cgc atg ttc ttg ggg cag atg   307
   Tyr Val Glu Ser Leu Ser Ser Tyr Ala Arg Met Phe Leu Gly Gln Met
       55                  60                  65
   gac aag ccg gac gtg gat ttg att gat gga tta tcc cca gcg gtc tcc   355
   Asp Lys Pro Asp Val Asp Leu Ile Asp Gly Leu Ser Pro Ala Val Ser
   70                  75                  80                  85
   att gac caa aaa tcc acc aac cgc aac cct cgg tcc aca gtc ggt acc   403
   Ile Asp Gln Lys Ser Thr Asn Arg Asn Pro Arg Ser Thr Val Gly Thr
                   90                  95                  100
   atc acg gaa gtc tat gac tac ctg cgt ctt ctg tac gcc cgc gct ggt   451
   Ile Thr Glu Val Tyr Asp Tyr Leu Arg Leu Leu Tyr Ala Arg Ala Gly
               105                 110                 115
   acc gca cac tgc cca gtg tgt gat gcc cgc gtg gag cgt caa acc ccc     499
   Thr Ala His Cys Pro Val Cys Asp Ala Arg Val Glu Arg Gln Thr Pro
           120                 125                 130
   cag cag atg gtg gac caa atc ctt ggc atg gag gag gga ctg aag ttc     547
   Gln Gln Met Val Asp Gln Ile Leu Gly Met Glu Glu Gly Leu Lys Phe
       135                 140                 145
   caa atc ctt gcg cct gtg gtg cgt acc cgt aaa ggt gag ttc gtt gat     595
   Gln Ile Leu Ala Pro Val Val Arg Thr Arg Lys Gly Glu Phe Val Asp
   150                 155                 160                 165
   ctt ttc gca gat ctt gca tcc caa ggt tat tcc cgc gtg cgg gtt gat     643
   Leu Phe Ala Asp Leu Ala Ser Gln Gly Tyr Ser Arg Val Arg Val Asp
                   170                 175                 180
   ggg gaa gtg cac cag ctc tcg gat cct cca aag cta gaa aag cag atc     691
   Gly Glu Val His Gln Leu Ser Asp Pro Pro Lys Leu Glu Lys Gln Ile
               185                 190                 195
   aag cac gat att gat gtt gtg gtt gac cgt ctg cag gta aaa gcc agc     739
   Lys His Asp Ile Asp Val Val Val Asp Arg Leu Gln Val Lys Ala Ser
           200                 205                 210
   caa aag cag cgc ctg aca gac tct atg gaa acc gca ctt cgc ctg gcc     787
   Gln Lys Gln Arg Leu Thr Asp Ser Met Glu Thr Ala Leu Arg Leu Ala
       215                 220                 225
   gat ggc gtg gct gtg ctg gag ttc gtt ggc ctg gag gaa gat gat ccg     835
   Asp Gly Val Ala Val Leu Glu Phe Val Gly Leu Glu Glu Asp Asp Pro
   230                 235                 240                 245
   aat agg ctt cgt cga ttc tct gaa aag atg agc tgc cct aac ggt cac     883
   Asn Arg Leu Arg Arg Phe Ser Glu Lys Met Ser Cys Pro Asn Gly His
                   250                 255                 260
   gcg ttg acg gtt gat gag ctg gag cct cgt gct ttt tcc ttc aac tct     931
   Ala Leu Thr Val Asp Glu Leu Glu Pro Arg Ala Phe Ser Phe Asn Ser
               265                 270                 275
   cct tat ggc gcg tgt cct gcc tgt gat ggc ttg ggt gtg cgc acc gaa     979
   Pro Tyr Gly Ala Cys Pro Ala Cys Asp Gly Leu Gly Val Arg Thr Glu
           280                 285                 290
   gtt gat att gat ctg atc atc cca gat cca gat gca cct gca act aaa     1027
   Val Asp Ile Asp Leu Ile Ile Pro Asp Pro Asp Ala Pro Ala Thr Lys
       295                 300                 305
   gcg gtt cag ccc tgg aac tcc agc cca aac cac tct tac ttt gaa aag     1075
   Ala Val Gln Pro Trp Asn Ser Ser Pro Asn His Ser Tyr Phe Glu Lys
   310                 315                 320                 325
   ctc att gaa ggc ctg gcg aaa gcc ctc gga ttt gat ccg gaa act ccg     1123
   Leu Ile Glu Gly Leu Ala Lys Ala Leu Gly Phe Asp Pro Glu Thr Pro
                   330                 335                 340
   tac agt gag ctc acc gca gct caa aag aag gct ctg gtc tat gga tcg     1171
   Tyr Ser Glu Leu Thr Ala Ala Gln Lys Lys Ala Leu Val Tyr Gly Ser
               345                 350                 355
   aag gaa gaa gta agc gtt cga tac aag aac cgc tac gga cgc gtg cgt    1219
   Lys Glu Glu Val Ser Val Arg Tyr Lys Asn Arg Tyr Gly Arg Val Arg
           360                 365                 370
   tct tgg act gcg cct ttt gaa ggt gtc atg ggc tac ttt gat cgc aag    1267
   Ser Trp Thr Ala Pro Phe Glu Gly Val Met Gly Tyr Phe Asp Arg Lys
       375                 380                 385
   ttg gag cag act gat tcc gaa acc caa aaa gac cga ctg ttg ggc tac    1315
   Leu Glu Gln Thr Asp Ser Glu Thr Gln Lys Asp Arg Leu Leu Gly Tyr
   390                 395                 400                 405
   acc cgt gaa gtg ccc tgc cca acc tgt aaa ggc gca cgc ctc aag ccg    1363
   Thr Arg Glu Val Pro Cys Pro Thr Cys Lys Gly Ala Arg Leu Lys Pro
                   410                 415                 420
   gaa atc ttg gcc gtt cgc cta gac tcc gga agc cat gga gcg ttg tcc    1411
   Glu Ile Leu Ala Val Arg Leu Asp Ser Gly Ser His Gly Ala Leu Ser
               425                 430                 435
   att gct gga cta acc gcg ctg tcg gtg cat gaa gca ttc gag ttt ttg    1459
   Ile Ala Gly Leu Thr Ala Leu Ser Val His Glu Ala Phe Glu Phe Leu
           440                 445                 450
   gat aac ctc aca ctg ggc aag cgc gag gaa atg atc gcg gga gct gtg    1507
   Asp Asn Leu Thr Leu Gly Lys Arg Glu Glu Met Ile Ala Gly Ala Val
       455                 460                 465
   ctg aag gaa att cac gcc cgc ctg aaa ttc ctg ctt gac gtg ggc ctt    1555
   Leu Lys Glu Ile His Ala Arg Leu Lys Phe Leu Leu Asp Val Gly Leu
   470                 475                 480                 485
   tcc tac ctc acc ctt gat cgc gcc gca ggc acc ctg tct ggt ggt gaa    1603
   Ser Tyr Leu Thr Leu Asp Arg Ala Ala Gly Thr Leu Ser Gly Gly Glu
                   490                 495                 500
   gcg cag cgt atc cgc ctg gct act caa att ggt tcc ggt ctg gct ggt    1651
   Ala Gln Arg Ile Arg Leu Ala Thr Gln Ile Gly Ser Gly Leu Ala Gly
               505                 510                 515
   gtg ctc tac gtc ttg gat gag cca tcc att ggt ctg cac caa cgt gac    1699
   Val Leu Tyr Val Leu Asp Glu Pro Ser Ile Gly Leu His Gln Arg Asp
           520                 525                 530
   aac cag cgc ttg atc act acc ctt gag cat ctc cga gat atc gga aac    1747
   Asn Gln Arg Leu Ile Thr Thr Leu Glu His Leu Arg Asp Ile Gly Asn
       535                 540                 545
   acg ctc att gtt gtg gaa cac gat gaa gac acc atc agg cgc gca gat    1795
   Thr Leu Ile Val Val Glu His Asp Glu Asp Thr Ile Arg Arg Ala Asp
   550                 555                 560                 565
   tgg ctc gtg gat att ggt cct cga gct ggt gaa ttt ggt ggc gaa gtg    1843
   Trp Leu Val Asp Ile Gly Pro Arg Ala Gly Glu Phe Gly Gly Glu Val
                   570                 575                 580
   gtc tac caa ggt gag ccg aag ggc att ttg gac tgc gaa gaa tcc ctc    1891
   Val Tyr Gln Gly Glu Pro Lys Gly Ile Leu Asp Cys Glu Glu Ser Leu
               585                 590                 595
   aca ggt gct tac ttg tct ggt cgt cga acc ctg ggt gtt cct gat act    1939
   Thr Gly Ala Tyr Leu Ser Gly Arg Arg Thr Leu Gly Val Pro Asp Thr
           600                 605                 610
   cgc cgt gag atc gac aaa gag cga cag ctc aag gtg gtt ggt gct agg     1987
   Arg Arg Glu Ile Asp Lys Glu Arg Gln Leu Lys Val Val Gly Ala Arg
       615                 620                 625
   gaa aat aac ctg cag ggc atc gat gtg aaa atc cca ctg ggt gtg ctg     2035
   Glu Asn Asn Leu Gln Gly Ile Asp Val Lys Ile Pro Leu Gly Val Leu
   630                 635                 640                 645
   tgc tgc atc act ggt gtg tcg gga tct ggt aaa tcc acg ctg gtc aat     2083
   Cys Cys Ile Thr Gly Val Ser Gly Ser Gly Lys Ser Thr Leu Val Asn
                   650                 655                 660
   cag att ttg gcc aag gtt ctg gcc aac aaa ctc aac cgc gca cgc caa     2131
   Gln Ile Leu Ala Lys Val Leu Ala Asn Lys Leu Asn Arg Ala Arg Gln
               665                 670                 675
   gtg cct ggt cgc gca aag cgg gtg gaa ggc ctc gag cac ttg gat aag     2179
   Val Pro Gly Arg Ala Lys Arg Val Glu Gly Leu Glu His Leu Asp Lys
           680                 685                 690
   ttg gtc caa gtg gat cag tcg cca att ggt cgt act cca cgt tca aac     2227
   Leu Val Gln Val Asp Gln Ser Pro Ile Gly Arg Thr Pro Arg Ser Asn
       695                 700                 705
   cca gcg acg tac acg ggt gtg ttt gat aaa gtc cgt aac ctt ttt gcc     2275
   Pro Ala Thr Tyr Thr Gly Val Phe Asp Lys Val Arg Asn Leu Phe Ala
   710                 715                 720                 725
   gag acc act gaa gcg aag gtc cgc ggt tac aag cct ggc cgc ttc tcc     2323
   Glu Thr Thr Glu Ala Lys Val Arg Gly Tyr Lys Pro Gly Arg Phe Ser
                   730                 735                 740
   ttc aat att aag ggt gga cgc tgc gaa gca tgt cag ggc gat ggc acg     2371
   Phe Asn Ile Lys Gly Gly Arg Cys Glu Ala Cys Gln Gly Asp Gly Thr
               745                 750                 755
   ctg aag atc gaa atg aac ttc ctg ccc gac gtg tat gtt ccg tgt gaa     2419
   Leu Lys Ile Glu Met Asn Phe Leu Pro Asp Val Tyr Val Pro Cys Glu
           760                 765                 770
   gtc tgt gat ggt cag cgc tac aac cgc gag acc ctc gag gtg aag tac     2467
   Val Cys Asp Gly Gln Arg Tyr Asn Arg Glu Thr Leu Glu Val Lys Tyr
       775                 780                 785
   aag ggc aaa aac atc gct gaa gta ttg ggc atg ccg atc tct gag gct     2515
   Lys Gly Lys Asn Ile Ala Glu Val Leu Gly Met Pro Ile Ser Glu Ala
   790                 795                 800                 805
   gcg gac ttc ttt gag ccc atc acc tca att cac cga tac cta gca acg     2563
   Ala Asp Phe Phe Glu Pro Ile Thr Ser Ile His Arg Tyr Leu Ala Thr
                   810                 815                 820
   ctg gtt gat gtc ggc ctt ggc tat gtc cgt ttg ggc cag gca gca aca     2611
   Leu Val Asp Val Gly Leu Gly Tyr Val Arg Leu Gly Gln Ala Ala Thr
               825                 830                 835
   acc ttg tct ggt ggt gaa gcc cag cgt gtg aaa ctt gcc gct gag ctg     2659
   Thr Leu Ser Gly Gly Glu Ala Gln Arg Val Lys Leu Ala Ala Glu Leu
           840                 845                 850
   cag aag cgt tcc aac ggt cgc acc gtt tac atc ctc gat gag cca act     2707
   Gln Lys Arg Ser Asn Gly Arg Thr Val Tyr Ile Leu Asp Glu Pro Thr
       855                 860                 865
   act ggt ttg cac ttt gaa gat att cgc aaa ctc atg atg gtg atc gaa    2755
   Thr Gly Leu His Phe Glu Asp Ile Arg Lys Leu Met Met Val Ile Glu
   870                 875                 880                 885
   ggc ctg gtg gac aag ggt aac tcc gtg atc atc atc gag cac aac ctc    2803
   Gly Leu Val Asp Lys Gly Asn Ser Val Ile Ile Ile Glu His Asn Leu
                   890                 895                 900
   gac gtg atc aag gct gcc gac tgg atc gtg gac atg ggt cca gag ggc    2851
   Asp Val Ile Lys Ala Ala Asp Trp Ile Val Asp Met Gly Pro Glu Gly
               905                 910                 915
   gga agc ggc ggt gga acc gtg gtc gct gaa gga acc cca gag caa gtt    2899
   Gly Ser Gly Gly Gly Thr Val Val Ala Glu Gly Thr Pro Glu Gln Val
           920                 925                 930
   gct gaa gtt gcg ggt tcc tac acc ggc caa ttc ctt aaa gag ttg ttg    2947
   Ala Glu Val Ala Gly Ser Tyr Thr Gly Gln Phe Leu Lys Glu Leu Leu
       935                 940                 945
   taggagaaga tgaggggctt tcatgggaag                                   2977
<210> 64
   <211> 949
   <212> PRT
   <213> Corynebacterium glutamicum
<400> 64
   Leu Ala Asp Arg Leu Val Val Arg Gly Ala Arg Glu His Asn Leu Lys
   1 5 10 15
   Gly Val Asp Ile Asp Leu Pro Arg Asp Ser Met Val Val Phe Thr Gly
   20 25 30
   Leu Ser Gly Ser Gly Lys Ser Ser Leu Ala Phe Asp Thr Ile Phe Ala
   35 40 45
   Glu Gly Gln Arg Arg Tyr Val Glu Ser Leu Ser Ser Tyr Ala Arg Met
   50 55 60
   Phe Leu Gly Gln Met Asp Lys Pro Asp Val Asp Leu Ile Asp Gly Leu
   65 70 75 80
   Ser Pro Ala Val Ser Ile Asp Gln Lys Ser Thr Asn Arg Asn Pro Arg
   85 90 95
   Ser Thr Val Gly Thr Ile Thr Glu Val Tyr Asp Tyr Leu Arg Leu Leu
   100 105 110
   Tyr Ala Arg Ala Gly Thr Ala His Cys Pro Val Cys Asp Ala Arg Val
   115 120 125
   Glu Arg Gln Thr Pro Gln Gln Met Val Asp Gln Ile Leu Gly Met Glu
   130 135 140
   Glu Gly Leu Lys Phe Gln Ile Leu Ala Pro Val Val Arg Thr Arg Lys
   145 150 155 160
   Gly Glu Phe Val Asp Leu Phe Ala Asp Leu Ala Ser Gln Gly Tyr Ser
   165 170 175
   Arg Val Arg Val Asp Gly Glu Val His Gln Leu Ser Asp Pro Pro Lys
   180 185 190
   Leu Glu Lys Gln Ile Lys His Asp Ile Asp Val Val Val Asp Arg Leu
   195 200 205
   Gln Val Lys Ala Ser Gln Lys Gln Arg Leu Thr Asp Ser Met Glu Thr
   210 215 220
   Ala Leu Arg Leu Ala Asp Gly Val Ala Val Leu Glu Phe Val Gly Leu
   225 230 235 240
   Glu Glu Asp Asp Pro Asn Arg Leu Arg Arg Phe Ser Glu Lys Met Ser
   245 250 255
   Cys Pro Asn Gly His Ala Leu Thr Val Asp Glu Leu Glu Pro Arg Ala
   260 265 270
   Phe Ser Phe Asn Ser Pro Tyr Gly Ala Cys Pro Ala Cys Asp Gly Leu
   275 280 285
   Gly Val Arg Thr Glu Val Asp Ile Asp Leu Ile Ile Pro Asp Pro Asp
   290 295 300
   Ala Pro Ala Thr Lys Ala Val Gln Pro Trp Asn Ser Ser Pro Asn His
   305 310 315 320
   Ser Tyr Phe Glu Lys Leu Ile Glu Gly Leu Ala Lys Ala Leu Gly Phe
   325 330 335
   Asp Pro Glu Thr Pro Tyr Ser Glu Leu Thr Ala Ala Gln Lys Lys Ala
   340 345 350
   Leu Val Tyr Gly Ser Lys Glu Glu Val Ser Val Arg Tyr Lys Asn Arg
   355 360 365
   Tyr Gly Arg Val Arg Ser Trp Thr Ala Pro Phe Glu Gly Val Met Gly
   370 375 380
   Tyr Phe Asp Arg Lys Leu Glu Gln Thr Asp Ser Glu Thr Gln Lys Asp
   385 390 395 400
   Arg Leu Leu Gly Tyr Thr Arg Glu Val Pro Cys Pro Thr Cys Lys Gly
   405 410 415
   Ala Arg Leu Lys Pro Glu Ile Leu Ala Val Arg Leu Asp Ser Gly Ser
   420 425 430
   His Gly Ala Leu Ser Ile Ala Gly Leu Thr Ala Leu Ser Val His Glu
   435 440 445
   Ala Phe Glu Phe Leu Asp Asn Leu Thr Leu Gly Lys Arg Glu Glu Met
   450 455 460
   Ile Ala Gly Ala Val Leu Lys Glu Ile His Ala Arg Leu Lys Phe Leu
   465 470 475 480
   Leu Asp Val Gly Leu Ser Tyr Leu Thr Leu Asp Arg Ala Ala Gly Thr
   485 490 495
   Leu Ser Gly Gly Glu Ala Gln Arg Ile Arg Leu Ala Thr Gln Ile Gly
   500 505 510
   Ser Gly Leu Ala Gly Val Leu Tyr Val Leu Asp Glu Pro Ser Ile Gly
   515 520 525
   Leu His Gln Arg Asp Asn Gln Arg Leu Ile Thr Thr Leu Glu His Leu
   530 535 540
   Arg Asp Ile Gly Asn Thr Leu Ile Val Val Glu His Asp Glu Asp Thr
   545 550 555 560
   Ile Arg Arg Ala Asp Trp Leu Val Asp Ile Gly Pro Arg Ala Gly Glu
   565 570 575
   Phe Gly Gly Glu Val Val Tyr Gln Gly Glu Pro Lys Gly Ile Leu Asp
   580 585 590
   Cys Glu Glu Ser Leu Thr Gly Ala Tyr Leu Ser Gly Arg Arg Thr Leu
   595 600 605
   Gly Val Pro Asp Thr Arg Arg Glu Ile Asp Lys Glu Arg Gln Leu Lys
   610 615 620
   Val Val Gly Ala Arg Glu Asn Asn Leu Gln Gly Ile Asp Val Lys Ile
   625 630 635 640
   Pro Leu Gly Val Leu Cys Cys Ile Thr Gly Val Ser Gly Ser Gly Lys
   645 650 655
   Ser Thr Leu Val Asn Gln Ile Leu Ala Lys Val Leu Ala Asn Lys Leu
   660 665 670
   Asn Arg Ala Arg Gln Val Pro Gly Arg Ala Lys Arg Val Glu Gly Leu
   675 680 685
   Glu His Leu Asp Lys Leu Val Gln Val Asp Gln Ser Pro Ile Gly Arg
   690 695 700
   Thr Pro Arg Ser Asn Pro Ala Thr Tyr Thr Gly Val Phe Asp Lys Val
   705 710 715 720
   Arg Asn Leu Phe Ala Glu Thr Thr Glu Ala Lys Val Arg Gly Tyr Lys
   725 730 735
   Pro Gly Arg Phe Ser Phe Asn Ile Lys Gly Gly Arg Cys Glu Ala Cys
   740 745 750
   Gln Gly Asp Gly Thr Leu Lys Ile Glu Met Asn Phe Leu Pro Asp Val
   755 760 765
   Tyr Val Pro Cys Glu Val Cys Asp Gly Gln Arg Tyr Asn Arg Glu Thr
   770 775 780
   Leu Glu Val Lys Tyr Lys Gly Lys Asn Ile Ala Glu Val Leu Gly Met
   785 790 795 800
   Pro Ile Ser Glu Ala Ala Asp Phe Phe Glu Pro Ile Thr Ser Ile His
   805 810 815
   Arg Tyr Leu Ala Thr Leu Val Asp Val Gly Leu Gly Tyr Val Arg Leu
   820 825 830
   Gly Gln Ala Ala Thr Thr Leu Ser Gly Gly Glu Ala Gln Arg Val Lys
   835 840 845
   Leu Ala Ala Glu Leu Gln Lys Arg Ser Asn Gly Arg Thr Val Tyr Ile
   850 855 860
   Leu Asp Glu Pro Thr Thr Gly Leu His Phe Glu Asp Ile Arg Lys Leu
   865 870 875 880
   Met Met Val Ile Glu Gly Leu Val Asp Lys Gly Asn Ser Val Ile Ile
   885 890 895
   Ile Glu His Asn Leu Asp Val Ile Lys Ala Ala Asp Trp Ile Val Asp
   900 905 910
   Met Gly Pro Glu Gly Gly Ser Gly Gly Gly Thr Val Val Ala Glu Gly
   915 920 925
   Thr Pro Glu Gln Val Ala Glu Val Ala Gly Ser Tyr Thr Gly Gln Phe
   930 935 940
   Leu Lys Glu Leu Leu
   945
<210> 65
   <211> 697
   <212> DNA
   <213> Corynebacterium glutamicum
<220>
   <221> CDS
   <222> (101)..(667)
   <223> RXA02418
<400> 65
   gacacggact agtgcccgtg tataactctt gaaagtcaga tctcccgttc atcctagatg 60
   atgaacgggt ttttggtctg cataggggca atcacctcaa gtg gtt cgg tac gtc    115
                                               Val Val Arg Tyr Val
                                                1               5
   aaa ttt tcc cgc act gct aac aga gga gtc cac atc agc gct gaa gct   163
   Lys Phe Ser Arg Thr Ala Asn Arg Gly Val His Ile Ser Ala Glu Ala
                   10                  15                  20
   cgc att aat gag cgc atc cga gtt ccc gaa gtc cgc ctt gtc gga cct   211
   Arg Ile Asn Glu Arg Ile Arg Val Pro Glu Val Arg Leu Val Gly Pro
               25                  30                  35
   aac ggt gag caa gta ggc atc gtc cgt atc gaa gat gcc cgc aag ctc   259
   Asn Gly Glu Gln Val Gly Ile Val Arg Ile Glu Asp Ala Arg Lys Leu
           40                  45                  50
   gca ttc gac gca gac cta gac ctg gtc gag gtc gca ccc aac gcc aaa   307
   Ala Phe Asp Ala Asp Leu Asp Leu Val Glu Val Ala Pro Asn Ala Lys
       55                  60                  65
   cct cca gtc tgc aag atc atg gac tac gga aag ttc aag tac gaa gcg   355
   Pro Pro Val Cys Lys Ile Met Asp Tyr Gly Lys Phe Lys Tyr Glu Ala
   70                  75                  80                  85
   gcc caa aag gct cgt gag tca cgc aag aat cag cag cag acc gtg gtc   403
   Ala Gln Lys Ala Arg Glu Ser Arg Lys Asn Gln Gln Gln Thr Val Val
                   90                  95                  100
   aaa gag caa aag ctt cgt ccc aag atc gat gat cat gat tat gag acg   451
   Lys Glu Gln Lys Leu Arg Pro Lys Ile Asp Asp His Asp Tyr Glu Thr
               105                 110                 115
   aag aag aac aat gtg atc cga ttc ctt gaa aag gga tca aag gtc aaa   499
   Lys Lys Asn Asn Val Ile Arg Phe Leu Glu Lys Gly Ser Lys Val Lys
           120                 125                 130
   gtc acg atc atg ttc cgt ggt cgt gag cag gct cgc cca gag ctt ggc   547
   Val Thr Ile Met Phe Arg Gly Arg Glu Gln Ala Arg Pro Glu Leu Gly
       135                 140                 145
   tac agg ctc ctc gag cga ctg gca aac gat gtc gta gat ttt ggc atc   595
   Tyr Arg Leu Leu Glu Arg Leu Ala Asn Asp Val Val Asp Phe Gly Ile
   150                 155                 160                 165
   gtg gaa acc cgc gca aag cag gac gga cga aac atg aca atg gtt ctc   643
   Val Glu Thr Arg Ala Lys Gln Asp Gly Arg Asn Met Thr Met Val Leu
                   170                 175                 180
   ggt ccg gtg cgc aag ggc aag aaa taatcacgaa tagggtttaa ggacaacttt  697
   Gly Pro Val Arg Lys Gly Lys Lys
               185
<210> 66
   <211> 189
   <212> PRT
   <213> Corynebacterium glutamicum
<400> 66
   Val Val Arg Tyr Val Lys Phe Ser Arg Thr Ala Asn Arg Gly Val His
    1               5                  10                  15
   Ile Ser Ala Glu Ala Arg Ile Asn Glu Arg Ile Arg Val Pro Glu Val
               20                  25                  30
   Arg Leu Val Gly Pro Asn Gly Glu Gln Val Gly Ile Val Arg Ile Glu
           35                  40                  45
   Asp Ala Arg Lys Leu Ala Phe Asp Ala Asp Leu Asp Leu Val Glu Val
       50                  55                  60
   Ala Pro Asn Ala Lys Pro Pro Val Cys Lys Ile Met Asp Tyr Gly Lys
   65                  70                  75                  80
   Phe Lys Tyr Glu Ala Ala Gln Lys Ala Arg Glu Ser Arg Lys Asn Gln
                   85                  90                  95
   Gln Gln Thr Val Val Lys Glu Gln Lys Leu Arg Pro Lys Ile Asp Asp
               100                 105                 110
   His Asp Tyr Glu Thr Lys Lys Asn Asn Val Ile Arg Phe Leu Glu Lys
           115                 120                 125
   Gly Ser Lys Val Lys Val Thr Ile Met Phe Arg Gly Arg Glu Gln Ala
       130                 135                 140
   Arg Pro Glu Leu Gly Tyr Arg Leu Leu Glu Arg Leu Ala Asn Asp Val
   145                 150                 155                 160
   Val Asp Phe Gly Ile Val Glu Thr Arg Ala Lys Gln Asp Gly Arg Asn
                   165                 170                 175
   Met Thr Met Val Leu Gly Pro Val Arg Lys Gly Lys Lys
               180                 185
<210> 67
   <211> 2419
   <212> DNA
   <213> Corynebacterium glutamicum
<220>
   <221> CDS
   <222> (101)..(2389)
   <223> RXA02429
<400> 67
   tcttacccac cagtgcaatg taggtcacgt cgtatcacgt ctgagggtga ttgagtaggg 60
   ttaaacagat gaattcattt agctcaccgg aggtataacc gtg gcc ggt ttt gat   115
                                               Val Ala Gly Phe Asp
                                                1               5
   tgg ttt tgg aag gcc ctt ggc ggc aaa tcg ggc aga aac caa aaa cgt   163
   Trp Phe Trp Lys Ala Leu Gly Gly Lys Ser Gly Arg Asn Gln Lys Arg
                   10                  15                  20
   agc gtg gca att gtc aat cag gta gaa aac cat gca gcg gaa tta gac   211
   Ser Val Ala Ile Val Asn Gln Val Glu Asn His Ala Ala Glu Leu Asp
               25                  30                  35
   gcg ctg gat gat gtt gca ttg gcg cag cgt gcc aag gat cta gcc agt   259
   Ala Leu Asp Asp Val Ala Leu Ala Gln Arg Ala Lys Asp Leu Ala Ser
           40                  45                  50
   ggt gga cgc att gac aat cat gcg gaa ttc ctc gcc att ttg ggt gtg   307
   Gly Gly Arg Ile Asp Asn His Ala Glu Phe Leu Ala Ile Leu Gly Val
       55                  60                  65
   gca tcg cag cgg aca ttg ggg ctg aag ccg tat ccg gtg caa tca cag   355
   Ala Ser Gln Arg Thr Leu Gly Leu Lys Pro Tyr Pro Val Gln Ser Gln
   70                  75                  80                  85
   gcg gtg ttg cgt ctc att gaa ggc gat gtg gtg cac atg gct acc ggt   403
   Ala Val Leu Arg Leu Ile Glu Gly Asp Val Val His Met Ala Thr Gly
                   90                  95                  100
   gag ggc aag act ttg gtg ggc gcg atg gcg gcc acc ggt ctg ggg ttg   451
   Glu Gly Lys Thr Leu Val Gly Ala Met Ala Ala Thr Gly Leu Gly Leu
               105                 110                 115
   atg ggc aag cga gtc cat tcg att acc gtc aat gat tat ttg gcg gtg   499
   Met Gly Lys Arg Val His Ser Ile Thr Val Asn Asp Tyr Leu Ala Val
           120                 125                 130
   cgc gat gcc gaa tgg atg cgg cca ttg gtc gaa ttt ttc ggt ctg agc   547
   Arg Asp Ala Glu Trp Met Arg Pro Leu Val Glu Phe Phe Gly Leu Ser
       135                 140                 145
   gtg gcg agc atc agc gag aag atg gat gca ggg gag cgt cga caa gca   595
   Val Ala Ser Ile Ser Glu Lys Met Asp Ala Gly Glu Arg Arg Gln Ala
   150                 155                 160                 165
   tat aaa gcc gca att gtc tac gga cct gtc aat gaa atc ggc ttt gac   643
   Tyr Lys Ala Ala Ile Val Tyr Gly Pro Val Asn Glu Ile Gly Phe Asp
                   170                 175                 180
   gtg ctg cgt gat cag cta att acc cgg cgc gaa gac gcc gtg cag cat   691
   Val Leu Arg Asp Gln Leu Ile Thr Arg Arg Glu Asp Ala Val Gln His
                   185                 190                 195
   ggc gcc gac gtc gcg att atc gat gag gcc gat tcc gtg ctt gtc gac      739
   Gly Ala Asp Val Ala Ile Ile Asp Glu Ala Asp Ser Val Leu Val Asp
           200                 205                 210
   gag gcc ctg gtg cca ctc gtc ctc gcc ggc aac cag ccc ggc cat gcg      787
   Glu Ala Leu Val Pro Leu Val Leu Ala Gly Asn Gln Pro Gly His Ala
       215                 220                 225
   ccg cgc ggc aaa atc acc gat gtg gtg cgc tcg ttg aaa gaa aac gac      835
   Pro Arg Gly Lys Ile Thr Asp Val Val Arg Ser Leu Lys Glu Asn Asp
   230                 235                 240                 245
   gat tac acc atc gac gat gat cgt cgc aac gtc ttc ctc acc gac aag      883
   Asp Tyr Thr Ile Asp Asp Asp Arg Arg Asn Val Phe Leu Thr Asp Lys
                   250                 255                 260
   ggt gcc gcc aaa tta gag cag cag ctg ggc atc agc agc ctc tac gac      931
   Gly Ala Ala Lys Leu Glu Gln Gln Leu Gly Ile Ser Ser Leu Tyr Asp
               265                 270                 275
   gat gag cac gtc ggc tcg acg ctc gtg cag gtc aac ctc gcc ctc cac      979
   Asp Glu His Val Gly Ser Thr Leu Val Gln Val Asn Leu Ala Leu His
           280                 285                 290
   gcg cag gca ctg ctc atc cgc gac atc cac tac atc gtc cgc gac agc      1027
   Ala Gln Ala Leu Leu Ile Arg Asp Ile His Tyr Ile Val Arg Asp Ser
       295                 300                 305
   aag gtc ttg ctt atc gac gcc tcc cgc ggc cgt gtc gcc gac ctg cag      1075
   Lys Val Leu Leu Ile Asp Ala Ser Arg Gly Arg Val Ala Asp Leu Gln
   310                 315                 320                 325
   cgc tgg ccc gac ggc ctg caa gca gca gtg gag gcc aag gaa ggt ctc      1123
   Arg Trp Pro Asp Gly Leu Gln Ala Ala Val Glu Ala Lys Glu Gly Leu
                   330                 335                 340
   gcg gtt tct gaa ggc ggc aag atc ctt gac acc atc aca ctt cag gcg      1171
   Ala Val Ser Glu Gly Gly Lys Ile Leu Asp Thr Ile Thr Leu Gln Ala
               345                 350                 355
   ttg att ggt cgc tac cca atg gca tgc ggc atg aca ggt acc gcc gtg      1219
   Leu Ile Gly Arg Tyr Pro Met Ala Cys Gly Met Thr Gly Thr Ala Val
           360                 365                 370
   gag gca acc gat cag cta cgc acc ttc tat gac ttg cat gtt tct gtc      1267
   Glu Ala Thr Asp Gln Leu Arg Thr Phe Tyr Asp Leu His Val Ser Val
       375                 380                 385
   att gag cgc aat cat ccg ctg aag cgc ttt gat gaa gct gac cgt atc      1315
   Ile Glu Arg Asn His Pro Leu Lys Arg Phe Asp Glu Ala Asp Arg Ile
   390                 395                 400                 405
   tac gcc acc atg gcg gag aaa aac cgc gcc atc atc gat gaa atc gca      1363
   Tyr Ala Thr Met Ala Glu Lys Asn Arg Ala Ile Ile Asp Glu Ile Ala
                   410                 415                 420
   ctc ctt cac agc acg ggg cag cca gtc ctg gtg ggt acc cac gat gtg      1411
   Leu Leu His Ser Thr Gly Gln Pro Val Leu Val Gly Thr His Asp Val
               425                 430                 435
   gca gag tcg gaa gaa ctc gcc act gca ctg cgt gaa ctc aac atc gaa      1459
   Ala Glu Ser Glu Glu Leu Ala Thr Ala Leu Arg Glu Leu Asn Ile Glu
           440                 445                 450
   gta agc gtt ctc aac gcc aag aat gat gcc gaa gaa gcc cag atc atc    1507
   Val Ser Val Leu Asn Ala Lys Asn Asp Ala Glu Glu Ala Gln Ile Ile
       455                 460                 465
   gca gag gct ggc gat att gga cga gtg acc gtt tcc act cag atg gcc    1555
   Ala Glu Ala Gly Asp Ile Gly Arg Val Thr Val Ser Thr Gln Met Ala
   470                 475                 480                 485
   ggc cgc ggt acc gat att cgc ctc ggt ggc gcc gat gaa gcc gac tac    1603
   Gly Arg Gly Thr Asp Ile Arg Leu Gly Gly Ala Asp Glu Ala Asp Tyr
                   490                 495                 500
   gat gaa gtg gtg aaa ctc ggt gga ctc gcc gtt atc ggc acc gcc cgc    1651
   Asp Glu Val Val Lys Leu Gly Gly Leu Ala Val Ile Gly Thr Ala Arg
               505                 510                 515
   cac cgt tct cag cgc ctg gac aac cag ctg cgc gga cgt gcg gga cga    1699
   His Arg Ser Gln Arg Leu Asp Asn Gln Leu Arg Gly Arg Ala Gly Arg
           520                 525                 530
   caa gga gat cca ggc ctg agc ctt ttc ttt gtc tcc ctc gat gat gat    1747
   Gln Gly Asp Pro Gly Leu Ser Leu Phe Phe Val Ser Leu Asp Asp Asp
       535                 540                 545
   gtg gtg gtc tca ggc ggg tca agg gag agc gtg agc gcg caa ccc gat    1795
   Val Val Val Ser Gly Gly Ser Arg Glu Ser Val Ser Ala Gln Pro Asp
   550                 555                 560                 565
   gcc acc ggg ctg att gac tca gat cgc atc cgc gat tgg gtc gga cac    1843
   Ala Thr Gly Leu Ile Asp Ser Asp Arg Ile Arg Asp Trp Val Gly His
                   570                 575                 580
   tgc cag cgc gtc acc gaa gga cag ctg ctg gaa atc cac tcc cag agc    1891
   Cys Gln Arg Val Thr Glu Gly Gln Leu Leu Glu Ile His Ser Gln Ser
               585                 590                 595
   tgg aat tac aac aag ctc ctt gcc gat caa cgc gtg atc att gac gag    1939
   Trp Asn Tyr Asn Lys Leu Leu Ala Asp Gln Arg Val Ile Ile Asp Glu
           600                 605                 610
   cgc cgc gaa cgc ctc ctc gac acc gcc tta gcg tgg gag gaa ctg gca    1987
   Arg Arg Glu Arg Leu Leu Asp Thr Ala Leu Ala Trp Glu Glu Leu Ala
       615                 620                 625
   cag cat gca cca gcg cgg gct gca gag ctt gaa gac ctt gat cag tcc    2035
   Gln His Ala Pro Ala Arg Ala Ala Glu Leu Glu Asp Leu Asp Gln Ser
   630                 635                 640                 645
   gtg agg gaa cag gca gca cga gac atc atg ctg tac cac ctc gat tac    2083
   Val Arg Glu Gln Ala Ala Arg Asp Ile Met Leu Tyr His Leu Asp Tyr
                   650                 655                 660
   aac tgg tca gag cac ctc gcg ttg atg gat gat gtc cgc gaa tcc att    2131
   Asn Trp Ser Glu His Leu Ala Leu Met Asp Asp Val Arg Glu Ser Ile
               665                 670                 675
   cac ctg cgc gcc atc gcc agg gaa acc ccc ctt gat gaa tac cac cgc    2179
   His Leu Arg Ala Ile Ala Arg Glu Thr Pro Leu Asp Glu Tyr His Arg
           680                 685                 690
   atc gct gtg cgt gaa ttc aag gat ttg gca caa cgc gct gtc gat gat    2227
   Ile Ala Val Arg Glu Phe Lys Asp Leu Ala Gln Arg Ala Val Asp Asp
       695                 700                 705
   gcg gtg tcc acg ttc aag tct gtg acc atc gat cac gag ggt gcc cat    2275
   Ala Val Ser Thr Phe Lys Ser Val Thr Ile Asp His Glu Gly Ala His
   710                 715                 720                 725
   ttg gat gat gag ggc ttg gcg cgt cca tca gca acg tgg acc tac atg    2323
   Leu Asp Asp Glu Gly Leu Ala Arg Pro Ser Ala Thr Trp Thr Tyr Met
                   730                 735                 740
   gtc tct gac aac cca ctt gcg ggt agt ggt aac tca gtg atc agt ggc    2371
   Val Ser Asp Asn Pro Leu Ala Gly Ser Gly Asn Ser Val Ile Ser Gly
               745                 750                 755
   ata gga aat atc ttt aga taacctgaga actatgaaat tccagctcac           2419
   Ile Gly Asn Ile Phe Arg
           760
<210> 68
   <211> 763
   <212> PRT
   <213> Corynebacterium glutamicum
<400> 68
   Val Ala Gly Phe Asp Trp Phe Trp Lys Ala Leu Gly Gly Lys Ser Gly
   1               5                   10                  15
   Arg Asn Gln Lys Arg Ser Val Ala Ile Val Asn Gln Val Glu Asn His
               20                  25                  30
   Ala Ala Glu Leu Asp Ala Leu Asp Asp Val Ala Leu Ala Gln Arg Ala
           35                  40                  45
   Lys Asp Leu Ala Ser Gly Gly Arg Ile Asp Asn His Ala Glu Phe Leu
       50                  55                  60
   Ala Ile Leu Gly Val Ala Ser Gln Arg Thr Leu Gly Leu Lys Pro Tyr
   65                  70                  75                  80
   Pro Val Gln Ser Gln Ala Val Leu Arg Leu Ile Glu Gly Asp Val Val
                   85                  90                  95
   His Met Ala Thr Gly Glu Gly Lys Thr Leu Val Gly Ala Met Ala Ala
               100                 105                 110
   Thr Gly Leu Gly Leu Met Gly Lys Arg Val His Ser Ile Thr Val Asn
           115                 120                 125
   Asp Tyr Leu Ala Val Arg Asp Ala Glu Trp Met Arg Pro Leu Val Glu
       130                 135                 140
   Phe Phe Gly Leu Ser Val Ala Ser Ile Ser Glu Lys Met Asp Ala Gly
   145                 150                 155                 160
   Glu Arg Arg Gln Ala Tyr Lys Ala Ala Ile Val Tyr Gly Pro Val Asn
                   165                 170                 175
   Glu Ile Gly Phe Asp Val Leu Arg Asp Gln Leu Ile Thr Arg Arg Glu
               180                 185                 190
   Asp Ala Val Gln His Gly Ala Asp Val Ala Ile Ile Asp Glu Ala Asp
   195 200 205
   Ser Val Leu Val Asp Glu Ala Leu Val Pro Leu Val Leu Ala Gly Asn
   210 215 220
   Gln Pro Gly His Ala Pro Arg Gly Lys Ile Thr Asp Val Val Arg Ser
   225 230 235 240
   Leu Lys Glu Asn Asp Asp Tyr Thr Ile Asp Asp Asp Arg Arg Asn Val
   245 250 255
   Phe Leu Thr Asp Lys Gly Ala Ala Lys Leu Glu Gln Gln Leu Gly Ile
   260 265 270
   Ser Ser Leu Tyr Asp Asp Glu His Val Gly Ser Thr Leu Val Gln Val
   275 280 285
   Asn Leu Ala Leu His Ala Gln Ala Leu Leu Ile Arg Asp Ile His Tyr
   290 295 300
   Ile Val Arg Asp Ser Lys Val Leu Leu Ile Asp Ala Ser Arg Gly Arg
   305 310 315 320
   Val Ala Asp Leu Gln Arg Trp Pro Asp Gly Leu Gln Ala Ala Val Glu
   325 330 335
   Ala Lys Glu Gly Leu Ala Val Ser Glu Gly Gly Lys Ile Leu Asp Thr
   340 345 350
   Ile Thr Leu Gln Ala Leu Ile Gly Arg Tyr Pro Met Ala Cys Gly Met
   355 360 365
   Thr Gly Thr Ala Val Glu Ala Thr Asp Gln Leu Arg Thr Phe Tyr Asp
   370 375 380
   Leu His Val Ser Val Ile Glu Arg Asn His Pro Leu Lys Arg Phe Asp
   385 390 395 400
   Glu Ala Asp Arg Ile Tyr Ala Thr Met Ala Glu Lys Asn Arg Ala Ile
   405 410 415
   Ile Asp Glu Ile Ala Leu Leu His Ser Thr Gly Gln Pro Val Leu Val
   420 425 430
   Gly Thr His Asp Val Ala Glu Ser Glu Glu Leu Ala Thr Ala Leu Arg
   435 440 445
   Glu Leu Asn Ile Glu Val Ser Val Leu Asn Ala Lys Asn Asp Ala Glu
   450 455 460
   Glu Ala Gln Ile Ile Ala Glu Ala Gly Asp Ile Gly Arg Val Thr Val
   465 470 475 480
   Ser Thr Gln Met Ala Gly Arg Gly Thr Asp Ile Arg Leu Gly Gly Ala
   485 490 495
   Asp Glu Ala Asp Tyr Asp Glu Val Val Lys Leu Gly Gly Leu Ala Val
   500 505 510
   Ile Gly Thr Ala Arg His Arg Ser Gln Arg Leu Asp Asn Gln Leu Arg
   515 520 525
   Gly Arg Ala Gly Arg Gln Gly Asp Pro Gly Leu Ser Leu Phe Phe Val
   530 535 540
   Ser Leu Asp Asp Asp Val Val Val Ser Gly Gly Ser Arg Glu Ser Val
   545 550 555 560
   Ser Ala Gln Pro Asp Ala Thr Gly Leu Ile Asp Ser Asp Arg Ile Arg
   565 570 575
   Asp Trp Val Gly His Cys Gln Arg Val Thr Glu Gly Gln Leu Leu Glu
   580 585 590
   Ile His Ser Gln Ser Trp Asn Tyr Asn Lys Leu Leu Ala Asp Gln Arg
   595 600 605
   Val Ile Ile Asp Glu Arg Arg Glu Arg Leu Leu Asp Thr Ala Leu Ala
   610 615 620
   Trp Glu Glu Leu Ala Gln His Ala Pro Ala Arg Ala Ala Glu Leu Glu
   625 630 635 640
   Asp Leu Asp Gln Ser Val Arg Glu Gln Ala Ala Arg Asp Ile Met Leu
   645 650 655
   Tyr His Leu Asp Tyr Asn Trp Ser Glu His Leu Ala Leu Met Asp Asp
   660 665 670
   Val Arg Glu Ser Ile His Leu Arg Ala Ile Ala Arg Glu Thr Pro Leu
   675 680 685
   Asp Glu Tyr His Arg Ile Ala Val Arg Glu Phe Lys Asp Leu Ala Gln
   690 695 700
   Arg Ala Val Asp Asp Ala Val Ser Thr Phe Lys Ser Val Thr Ile Asp
   705 710 715 720
   His Glu Gly Ala His Leu Asp Asp Glu Gly Leu Ala Arg Pro Ser Ala
   725 730 735
   Thr Trp Thr Tyr Met Val Ser Asp Asn Pro Leu Ala Gly Ser Gly Asn
   740 745 750
   Ser Val Ile Ser Gly Ile Gly Asn Ile Phe Arg
   755 760
<210> 69
   <211> 1582
   <212> DNA
   <213> Corynebacterium glutamicum
<220>
   <221> CDS
   <222> (101)..(1552)
   <223> RXA02431
<400> 69
   ggtggctgag cttgtcggca ttgtgctggt tgtcatcgca gttgctttgc gacgcccctc 60
   ctagcggttt cccacaccgc cagtcttctc aaactaatcg ttg acc tgt tcg att   115
                                               Leu Thr Cys Ser Ile
                                                1               5
   aac cta att ttc ggc tgg tca act acc ata aaa agc atg caa cgc tgg   163
   Asn Leu Ile Phe Gly Trp Ser Thr Thr Ile Lys Ser Met Gln Arg Trp
                   10                  15                  20
   gtg ctt cac atc gat atg gat gcc ttc ttc gca tcc tgc gaa caa ctg    211
   Val Leu His Ile Asp Met Asp Ala Phe Phe Ala Ser Cys Glu Gln Leu
               25                  30                  35
   acc cgg ccc act tta aga ggc cgc ccc gtc ttg gtc ggt gga gtc tcc    259
   Thr Arg Pro Thr Leu Arg Gly Arg Pro Val Leu Val Gly Gly Val Ser
           40                  45                  50
   ggt agg gga gtt gtc gcc gga gca tcc tat gaa gcc aga aaa ttt ggc    307
   Gly Arg Gly Val Val Ala Gly Ala Ser Tyr Glu Ala Arg Lys Phe Gly
       55                  60                  65
   gcc cgc tca gcg atg ccc atg cac caa gcc aaa gcc cga gta ggt ttt    355
   Ala Arg Ser Ala Met Pro Met His Gln Ala Lys Ala Arg Val Gly Phe
   70                  75                  80                  85
   ggg gca gtg gtg gtg aca ccc cgt cat atc gtt tac tcc gca gcc tcg    403
   Gly Ala Val Val Val Thr Pro Arg His Ile Val Tyr Ser Ala Ala Ser
                   90                  95                  100
   cgc cgg gtg ttc caa atc gtg gaa aaa cgc gcc gga att gtc gaa cgc    451
   Arg Arg Val Phe Gln Ile Val Glu Lys Arg Ala Gly Ile Val Glu Arg
               105                 110                 115
   ctc agc atc gat gaa ggc ttc atg gaa cca gag gct ctc gtt gga gcc    499
   Leu Ser Ile Asp Glu Gly Phe Met Glu Pro Glu Ala Leu Val Gly Ala
           120                 125                 130
   acc cca gaa gag gtg aaa cag tgg gcg gaa gaa tta cgc gcg gaa att    547
   Thr Pro Glu Glu Val Lys Gln Trp Ala Glu Glu Leu Arg Ala Glu Ile
       135                 140                 145
   aaa gaa gtt act ggc tta ccc tcc tcg gtt ggt gct ggc tcc ggt aag    595
   Lys Glu Val Thr Gly Leu Pro Ser Ser Val Gly Ala Gly Ser Gly Lys
   150                 155                 160                 165
   cag atc gcc aaa att ggt tca ggc gaa gca aag cca gat ggt gtg ttt    643
   Gln Ile Ala Lys Ile Gly Ser Gly Glu Ala Lys Pro Asp Gly Val Phe
                   170                 175                 180
   gtc gtg cca gta gac aag caa cat gac ttg ctt gat cca ctt cct gtg    691
   Val Val Pro Val Asp Lys Gln His Asp Leu Leu Asp Pro Leu Pro Val
               185                 190                 195
   ggc gca ctt tgg gga gtg ggt cct gtg aca ggc tcc aag ctt gcc tca    739
   Gly Ala Leu Trp Gly Val Gly Pro Val Thr Gly Ser Lys Leu Ala Ser
           200                 205                 210
   atg ggg gtg gaa aca att ggt gat cta gca gcg cta acc caa aaa gaa    787
   Met Gly Val Glu Thr Ile Gly Asp Leu Ala Ala Leu Thr Gln Lys Glu
       215                 220                 225
   gta gaa atc agc ctc ggt gca acc atc gga ata tca ctg tgg aac ctt    835
   Val Glu Ile Ser Leu Gly Ala Thr Ile Gly Ile Ser Leu Trp Asn Leu
   230                 235                 240                 245
   gcc cga gga atc gac gac cgc cct gtg gaa ccc cgc gcc gaa gca aaa    883
   Ala Arg Gly Ile Asp Asp Arg Pro Val Glu Pro Arg Ala Glu Ala Lys
                   250                 255                 260
   cag atc tcc caa gag cac acc tat gaa aaa gac ctc ctc acc agg caa    931
   Gln Ile Ser Gln Glu His Thr Tyr Glu Lys Asp Leu Leu Thr Arg Gln
               265                 270                 275
   caa gta gat gct gcc atc att cga tca gcc gaa ggc gca cac cga cgg     979
   Gln Val Asp Ala Ala Ile Ile Arg Ser Ala Glu Gly Ala His Arg Arg
           280                 285                 290
   ctc ctc aaa gac gga cgc ggt gcc aga act gtc agc gtg aaa ctg cgg    1027
   Leu Leu Lys Asp Gly Arg Gly Ala Arg Thr Val Ser Val Lys Leu Arg
       295                 300                 305
   atg gcc gac ttt cgt att gag tct cgt tcc tac acc ttg tcc tat gcc    1075
   Met Ala Asp Phe Arg Ile Glu Ser Arg Ser Tyr Thr Leu Ser Tyr Ala
   310                 315                 320                 325
   acc gat gat tac gca act ctt gag gca aca gca ttc cga ctt gcc cgc    1123
   Thr Asp Asp Tyr Ala Thr Leu Glu Ala Thr Ala Phe Arg Leu Ala Arg
                   330                 335                 340
   tac ccc gga gaa gta ggc ccc atc cgc ctt gtc gga gta agt ttt tct    1171
   Tyr Pro Gly Glu Val Gly Pro Ile Arg Leu Val Gly Val Ser Phe Ser
               345                 350                 355
   ggt ttg gaa gaa tcc cgc caa gac atc ctc ttc ccg gaa ctt gac caa    1219
   Gly Leu Glu Glu Ser Arg Gln Asp Ile Leu Phe Pro Glu Leu Asp Gln
           360                 365                 370
   caa atc atc gta cca cca gca ccc gac acc gat tat gag gta ggc gtg    1267
   Gln Ile Ile Val Pro Pro Ala Pro Asp Thr Asp Tyr Glu Val Gly Val
       375                 380                 385
   caa tcc tct tct agt tcc gaa agt act caa gtt gaa gcg ccg caa gat    1315
   Gln Ser Ser Ser Ser Ser Glu Ser Thr Gln Val Glu Ala Pro Gln Asp
   390                 395                 400                 405
   gtc gcg ttg agt atg tgg tgc gca acg caa gat gtc tac cac cca gaa    1363
   Val Ala Leu Ser Met Trp Cys Ala Thr Gln Asp Val Tyr His Pro Glu
                   410                 415                 420
   tat ggc cac ggt tgg gta caa ggt gcc ggt cac ggt gtt gta tca gta    1411
   Tyr Gly His Gly Trp Val Gln Gly Ala Gly His Gly Val Val Ser Val
               425                 430                 435
   cgt ttt gaa acc cgc agc acc aca aaa ggg cga act aaa agt ttt tcc    1459
   Arg Phe Glu Thr Arg Ser Thr Thr Lys Gly Arg Thr Lys Ser Phe Ser
           440                 445                 450
   atg gat gac ccg gac ctc acc ccg gca gac cct cta gat agt ttg gat    1507
   Met Asp Asp Pro Asp Leu Thr Pro Ala Asp Pro Leu Asp Ser Leu Asp
       455                 460                 465
   tgg gct gac tgg ttt gct gaa aat ggt gaa acg ggg gat gac gaa        1552
   Trp Ala Asp Trp Phe Ala Glu Asn Gly Glu Thr Gly Asp Asp Glu
   470                 475                 480
   tagggtttca tcgggtttcg gggtgctttt                                   1582
<210> 70
   <211> 484
   <212> PRT
   <213> Corynebacterium glutamicum
<400> 70
   Leu Thr Cys Ser Ile Asn Leu Ile Phe Gly Trp Ser Thr Thr Ile Lys
    1               5                  10                  15
   Ser Met Gln Arg Trp Val Leu His Ile Asp Met Asp Ala Phe Phe Ala
               20                  25                  30
   Ser Cys Glu Gln Leu Thr Arg Pro Thr Leu Arg Gly Arg Pro Val Leu
           35                  40                  45
   Val Gly Gly Val Ser Gly Arg Gly Val Val Ala Gly Ala Ser Tyr Glu
       50                  55                  60
   Ala Arg Lys Phe Gly Ala Arg Ser Ala Met Pro Met His Gln Ala Lys
   65                  70                  75                  80
   Ala Arg Val Gly Phe Gly Ala Val Val Val Thr Pro Arg His Ile Val
                   85                  90                  95
   Tyr Ser Ala Ala Ser Arg Arg Val Phe Gln Ile Val Glu Lys Arg Ala
               100                 105                 110
   Gly Ile Val Glu Arg Leu Ser Ile Asp Glu Gly Phe Met Glu Pro Glu
           115                 120                 125
   Ala Leu Val Gly Ala Thr Pro Glu Glu Val Lys Gln Trp Ala Glu Glu
       130                 135                 140
   Leu Arg Ala Glu Ile Lys Glu Val Thr Gly Leu Pro Ser Ser Val Gly
   145                 150                 155                 160
   Ala Gly Ser Gly Lys Gln Ile Ala Lys Ile Gly Ser Gly Glu Ala Lys
                   165                 170                 175
   Pro Asp Gly Val Phe Val Val Pro Val Asp Lys Gln His Asp Leu Leu
               180                 185                 190
   Asp Pro Leu Pro Val Gly Ala Leu Trp Gly Val Gly Pro Val Thr Gly
           195                 200                 205
   Ser Lys Leu Ala Ser Met Gly Val Glu Thr Ile Gly Asp Leu Ala Ala
       210                 215                 220
   Leu Thr Gln Lys Glu Val Glu Ile Ser Leu Gly Ala Thr Ile Gly Ile
   225                 230                 235                 240
   Ser Leu Trp Asn Leu Ala Arg Gly Ile Asp Asp Arg Pro Val Glu Pro
                   245                 250                 255
   Arg Ala Glu Ala Lys Gln Ile Ser Gln Glu His Thr Tyr Glu Lys Asp
               260                 265                 270
   Leu Leu Thr Arg Gln Gln Val Asp Ala Ala Ile Ile Arg Ser Ala Glu
           275                 280                 285
   Gly Ala His Arg Arg Leu Leu Lys Asp Gly Arg Gly Ala Arg Thr Val
       290                 295                 300
   Ser Val Lys Leu Arg Met Ala Asp Phe Arg Ile Glu Ser Arg Ser Tyr
   305                 310                 315                 320
   Thr Leu Ser Tyr Ala Thr Asp Asp Tyr Ala Thr Leu Glu Ala Thr Ala
                   325                 330                 335
   Phe Arg Leu Ala Arg Tyr Pro Gly Glu Val Gly Pro Ile Arg Leu Val
   340 345 350
   Gly Val Ser Phe Ser Gly Leu Glu Glu Ser Arg Gln Asp Ile Leu Phe
   355 360 365
   Pro Glu Leu Asp Gln Gln Ile Ile Val Pro Pro Ala Pro Asp Thr Asp
   370 375 380
   Tyr Glu Val Gly Val Gln Ser Ser Ser Ser Ser Glu Ser Thr Gln Val
   385 390 395 400
   Glu Ala Pro Gln Asp Val Ala Leu Ser Met Trp Cys Ala Thr Gln Asp
   405 410 415
   Val Tyr His Pro Glu Tyr Gly His Gly Trp Val Gln Gly Ala Gly His
   420 425 430
   Gly Val Val Ser Val Arg Phe Glu Thr Arg Ser Thr Thr Lys Gly Arg
   435 440 445
   Thr Lys Ser Phe Ser Met Asp Asp Pro Asp Leu Thr Pro Ala Asp Pro
   450 455 460
   Leu Asp Ser Leu Asp Trp Ala Asp Trp Phe Ala Glu Asn Gly Glu Thr
   465 470 475 480
   Gly Asp Asp Glu
<210> 71
   <211> 1819
   <212> DNA
   <213> Corynebacterium glutamicum
<220>
   <221> CDS
   <222> (101)..(1789)
   <223> RXA02445
<400> 71
   tgtataaagt gacgctctca gtgtagaaac tgagggtctc agtgaagtag actgacaggg 60
   atacctaata cgtttaatag attgtggggc actcgccacc gtg gaa cca ttc gaa    115
                                               Val Glu Pro Phe Glu
                                               1               5
   tta gag aaa gac ctt gag cgt ctt agg aaa aac gga aaa gac gat gaa    163
   Leu Glu Lys Asp Leu Glu Arg Leu Arg Lys Asn Gly Lys Asp Asp Glu
                   10                  15                  20
   acc gta gaa gtg aaa tct tgg ggt cgg tta cct tta agc aaa gga tca    211
   Thr Val Glu Val Lys Ser Trp Gly Arg Leu Pro Leu Ser Lys Gly Ser
               25                  30                  35
   aaa agc ttc tgg gaa tca tta agc gca ttc gca aac acc aac ggt gga    259
   Lys Ser Phe Trp Glu Ser Leu Ser Ala Phe Ala Asn Thr Asn Gly Gly
           40                  45                  50
   tac atc cta ttg ggg cta agc gaa cca gat ttc act cca gtt gaa gga    307
   Tyr Ile Leu Leu Gly Leu Ser Glu Pro Asp Phe Thr Pro Val Glu Gly
       55                  60                  65
   ttt gat tca cag gcg agt atc cag ttc att cgt gca ggt tta aat cca    355
   Phe Asp Ser Gln Ala Ser Ile Gln Phe Ile Arg Ala Gly Leu Asn Pro
    70                 75                  80                  85
   caa gat cgc gac gcc caa aaa gtg gaa cca gtg ccc cat cat gaa att    403
   Gln Asp Arg Asp Ala Gln Lys Val Glu Pro Val Pro His His Glu Ile
                   90                  95                 100
   cat gaa atg act gtt gat ggt gct gaa gtt gtt tta gtt tca gtc tca    451
   His Glu Met Thr Val Asp Gly Ala Glu Val Val Leu Val Ser Val Ser
               105                 110                 115
   ccg ttg tca gtg aac ggg ccc tgt tat tat ctt ccc gtc gga atc act    499
   Pro Leu Ser Val Asn Gly Pro Cys Tyr Tyr Leu Pro Val Gly Ile Thr
           120                 125                 130
   aat ggc agc ttc aaa cgc gtt ggc gat gaa gac cgg aag ctc agt cat    547
   Asn Gly Ser Phe Lys Arg Val Gly Asp Glu Asp Arg Lys Leu Ser His
       135                 140                 145
   ctt gaa att tac gag ctc caa aat agg ttt gtt caa acc aaa aca gat    595
   Leu Glu Ile Tyr Glu Leu Gln Asn Arg Phe Val Gln Thr Lys Thr Asp
   150                 155                 160                 165
   aga aat cca gtt cca gat tca agc atc gac gat ctc aac aat cag ctc    643
   Arg Asn Pro Val Pro Asp Ser Ser Ile Asp Asp Leu Asn Asn Gln Leu
                   170                 175                 180
   gcg gcg tca ttt aag cag cgc cta att gag tca aat agt cgc tcc ctt    691
   Ala Ala Ser Phe Lys Gln Arg Leu Ile Glu Ser Asn Ser Arg Ser Leu
               185                 190                 195
   gga aca gac gat aac tgg tta ctg cgc aaa aat atc act aca tca aag    739
   Gly Thr Asp Asp Asn Trp Leu Leu Arg Lys Asn Ile Thr Thr Ser Lys
           200                 205                 210
   gga gaa ctg acg att gct ggc tta ctg gct ctc gga agc tat cct caa    787
   Gly Glu Leu Thr Ile Ala Gly Leu Leu Ala Leu Gly Ser Tyr Pro Gln
       215                 220                 225
   cag ttt ttc ccc cga gtg atc att gat gtt gcc gta cat cca ggt ctg    835
   Gln Phe Phe Pro Arg Val Ile Ile Asp Val Ala Val His Pro Gly Leu
   230                 235                 240                 245
   cat aag tca cca atc ggt acc tca att cgt ttt gaa gac cga aaa atc    883
   His Lys Ser Pro Ile Gly Thr Ser Ile Arg Phe Glu Asp Arg Lys Ile
                   250                 255                 260
   tgc gag gga aat ctt ctc gag atg gtt caa gag gct atg tct gcc atc    931
   Cys Glu Gly Asn Leu Leu Glu Met Val Gln Glu Ala Met Ser Ala Ile
               265                 270                 275
   aaa cga aac cta cgt gta cgc cgc gtc gtt gaa gga ctc tca ggt aaa    979
   Lys Arg Asn Leu Arg Val Arg Arg Val Val Glu Gly Leu Ser Gly Lys
           280                 285                 290
   gat gtt cta gaa atc cca gaa gaa gtt ttg aga gag gct cta gca aac   1027
   Asp Val Leu Glu Ile Pro Glu Glu Val Leu Arg Glu Ala Leu Ala Asn
       295                 300                 305
   gcc gta ctt cac cgt gat tat tct gag cta gct caa aat gaa gca att   1075
   Ala Val Leu His Arg Asp Tyr Ser Glu Leu Ala Gln Asn Glu Ala Ile
   310                 315                 320                 325
   cat gta gac atc tat aag gat cga gtt gag atc acg agt cca ggt gga    1123
   His Val Asp Ile Tyr Lys Asp Arg Val Glu Ile Thr Ser Pro Gly Gly
                   330                 335                 340
   tta ccc aat ggt aaa cgc cca gag tca ata ctg gac gga tac tct gaa    1171
   Leu Pro Asn Gly Lys Arg Pro Glu Ser Ile Leu Asp Gly Tyr Ser Glu
               345                 350                 355
   cca aga aat cgt gtg ctt tca aga atc cta atg gat att cca tgg aca    1219
   Pro Arg Asn Arg Val Leu Ser Arg Ile Leu Met Asp Ile Pro Trp Thr
           360                 365                 370
   cat gaa gta caa gga gta ctt gct gaa agc aac ggt act ggc gtt ccc    1267
   His Glu Val Gln Gly Val Leu Ala Glu Ser Asn Gly Thr Gly Val Pro
       375                 380                 385
   cga atg ttc aat ttg atg cgt gaa gcg gga ctt ccg gta ccg aat ttt    1315
   Arg Met Phe Asn Leu Met Arg Glu Ala Gly Leu Pro Val Pro Asn Phe
   390                 395                 400                 405
   aag att gat att tct agc gtc act gtc gaa ctc agc cgt cac ggt ctt    1363
   Lys Ile Asp Ile Ser Ser Val Thr Val Glu Leu Ser Arg His Gly Leu
                   410                 415                 420
   cta gat gcc caa aca agt gaa tgg ctt gta gaa aaa ctc gga tca gat    1411
   Leu Asp Ala Gln Thr Ser Glu Trp Leu Val Glu Lys Leu Gly Ser Asp
               425                 430                 435
   ttt tct aac aca caa ggc att gct ctt gtt ctc gca aaa gaa ctt gga    1459
   Phe Ser Asn Thr Gln Gly Ile Ala Leu Val Leu Ala Lys Glu Leu Gly
           440                 445                 450
   gcg gta acg tct cga gat ctc cgc aat caa act ggt cat gat tca gaa    1507
   Ala Val Thr Ser Arg Asp Leu Arg Asn Gln Thr Gly His Asp Ser Glu
       455                 460                 465
   gac atg cgc agc tta ctt gac gct ttg gtt gat cgg ggc gtt cta aac    1555
   Asp Met Arg Ser Leu Leu Asp Ala Leu Val Asp Arg Gly Val Leu Asn
   470                 475                 480                 485
   caa aac tta cag aac caa tat cag ctt gcg aca tcg tct gtg aat gta    1603
   Gln Asn Leu Gln Asn Gln Tyr Gln Leu Ala Thr Ser Ser Val Asn Val
                   490                 495                 500
   act caa agc gaa caa gaa gtc tta gat gca atc aat aaa aca act cct    1651
   Thr Gln Ser Glu Gln Glu Val Leu Asp Ala Ile Asn Lys Thr Thr Pro
               505                 510                 515
   gtc aca att cga gaa att gcc aca aaa aca ggg aaa act gca tcg tct    1699
   Val Thr Ile Arg Glu Ile Ala Thr Lys Thr Gly Lys Thr Ala Ser Ser
           520                 525                 530
   ctt cgg ccg ctg ctt cgt ggc ctt gtt gaa gca ggt ctt gtg gtt gca    1747
   Leu Arg Pro Leu Leu Arg Gly Leu Val Glu Ala Gly Leu Val Val Ala
       535                 540                 545
   act gct cca cca tca agc cgc aac cga gcg tac ttg aag gct            1789
   Thr Ala Pro Pro Ser Ser Arg Asn Arg Ala Tyr Leu Lys Ala
   550                 555                 560
   tgacccacca acgaactcac cggtgtcagc                                   1819
<210> 72
   <211> 563
   <212> PRT
   <213> Corynebacterium glutamicum
<400> 72
   Val Glu Pro Phe Glu Leu Glu Lys Asp Leu Glu Arg Leu Arg Lys Asn
   1 5 10 15
   Gly Lys Asp Asp Glu Thr Val Glu Val Lys Ser Trp Gly Arg Leu Pro
   20 25 30
   Leu Ser Lys Gly Ser Lys Ser Phe Trp Glu Ser Leu Ser Ala Phe Ala
   35 40 45
   Asn Thr Asn Gly Gly Tyr Ile Leu Leu Gly Leu Ser Glu Pro Asp Phe
   50 55 60
   Thr Pro Val Glu Gly Phe Asp Ser Gln Ala Ser Ile Gln Phe Ile Arg
   65 70 75 80
   Ala Gly Leu Asn Pro Gln Asp Arg Asp Ala Gln Lys Val Glu Pro Val
   85 90 95
   Pro His His Glu Ile His Glu Met Thr Val Asp Gly Ala Glu Val Val
   100 105 110
   Leu Val Ser Val Ser Pro Leu Ser Val Asn Gly Pro Cys Tyr Tyr Leu
   115 120 125
   Pro Val Gly Ile Thr Asn Gly Ser Phe Lys Arg Val Gly Asp Glu Asp
   130 135 140
   Arg Lys Leu Ser His Leu Glu Ile Tyr Glu Leu Gln Asn Arg Phe Val
   145 150 155 160
   Gln Thr Lys Thr Asp Arg Asn Pro Val Pro Asp Ser Ser Ile Asp Asp
   165 170 175
   Leu Asn Asn Gln Leu Ala Ala Ser Phe Lys Gln Arg Leu Ile Glu Ser
   180 185 190
   Asn Ser Arg Ser Leu Gly Thr Asp Asp Asn Trp Leu Leu Arg Lys Asn
   195 200 205
   Ile Thr Thr Ser Lys Gly Glu Leu Thr Ile Ala Gly Leu Leu Ala Leu
   210 215 220
   Gly Ser Tyr Pro Gln Gln Phe Phe Pro Arg Val Ile Ile Asp Val Ala
   225 230 235 240
   Val His Pro Gly Leu His Lys Ser Pro Ile Gly Thr Ser Ile Arg Phe
   245 250 255
   Glu Asp Arg Lys Ile Cys Glu Gly Asn Leu Leu Glu Met Val Gln Glu
   260 265 270
   Ala Met Ser Ala Ile Lys Arg Asn Leu Arg Val Arg Arg Val Val Glu
   275 280 285
   Gly Leu Ser Gly Lys Asp Val Leu Glu Ile Pro Glu Glu Val Leu Arg
   290 295 300
   Glu Ala Leu Ala Asn Ala Val Leu His Arg Asp Tyr Ser Glu Leu Ala
   305 310 315 320
   Gln Asn Glu Ala Ile His Val Asp Ile Tyr Lys Asp Arg Val Glu Ile
   325 330 335
   Thr Ser Pro Gly Gly Leu Pro Asn Gly Lys Arg Pro Glu Ser Ile Leu
   340 345 350
   Asp Gly Tyr Ser Glu Pro Arg Asn Arg Val Leu Ser Arg Ile Leu Met
   355 360 365
   Asp Ile Pro Trp Thr His Glu Val Gln Gly Val Leu Ala Glu Ser Asn
   370 375 380
   Gly Thr Gly Val Pro Arg Met Phe Asn Leu Met Arg Glu Ala Gly Leu
   385 390 395 400
   Pro Val Pro Asn Phe Lys Ile Asp Ile Ser Ser Val Thr Val Glu Leu
   405 410 415
   Ser Arg His Gly Leu Leu Asp Ala Gln Thr Ser Glu Trp Leu Val Glu
   420 425 430
   Lys Leu Gly Ser Asp Phe Ser Asn Thr Gln Gly Ile Ala Leu Val Leu
   435 440 445
   Ala Lys Glu Leu Gly Ala Val Thr Ser Arg Asp Leu Arg Asn Gln Thr
   450 455 460
   Gly His Asp Ser Glu Asp Met Arg Ser Leu Leu Asp Ala Leu Val Asp
   465 470 475 480
   Arg Gly Val Leu Asn Gln Asn Leu Gln Asn Gln Tyr Gln Leu Ala Thr
   485 490 495
   Ser Ser Val Asn Val Thr Gln Ser Glu Gln Glu Val Leu Asp Ala Ile
   500 505 510
   Asn Lys Thr Thr Pro Val Thr Ile Arg Glu Ile Ala Thr Lys Thr Gly
   515 520 525
   Lys Thr Ala Ser Ser Leu Arg Pro Leu Leu Arg Gly Leu Val Glu Ala
   530 535 540
   Gly Leu Val Val Ala Thr Ala Pro Pro Ser Ser Arg Asn Arg Ala Tyr
   545 550 555 560
   Leu Lys Ala
<210> 73
   <211> 1009
   <212> DNA
   <213> Corynebacterium glutamicum
<220>
   <221> CDS
   <222> (101)..(979)
   <223> RXA02476
<400> 73
   cgggcggagt tctatcaaca ttacgcaaag gcataagctt tattattcca ctcggtgtga 60
   catatgacct aaagtgccag tcagtacaat catttaggtc atg tca ttt aca gct    115
                                               Met Ser Phe Thr Ala
                                               1               5
   ttt caa aca gcc ctg ctc gtg tgg ttt aga gca aat gcc cgc gat ctt    163
   Phe Gln Thr Ala Leu Leu Val Trp Phe Arg Ala Asn Ala Arg Asp Leu
                   10                  15                  20
   gcg tgg cgt gat ccc aat act tca gca tgg gga att ctc ctt tca gag    211
   Ala Trp Arg Asp Pro Asn Thr Ser Ala Trp Gly Ile Leu Leu Ser Glu
               25                  30                  35
   gtg atg agc caa caa act ccc gtc gcg cga gtc gag ccg att tgg cgt    259
   Val Met Ser Gln Gln Thr Pro Val Ala Arg Val Glu Pro Ile Trp Arg
           40                  45                  50
   gag tgg atg gaa aaa tgg ccc act ccg gaa gat ttc gcg aat gcg agc    307
   Glu Trp Met Glu Lys Trp Pro Thr Pro Glu Asp Phe Ala Asn Ala Ser
       55                  60                  65
   acc gat gag att ttg cgg tcg tgg ggc aag ttg ggc tat cca cgt agg    355
   Thr Asp Glu Ile Leu Arg Ser Trp Gly Lys Leu Gly Tyr Pro Arg Arg
   70                  75                  80                  85
   gcg ctg agg ttg aag gaa tgt gcg gag gtg atc gtc gaa aag cat gcc    403
   Ala Leu Arg Leu Lys Glu Cys Ala Glu Val Ile Val Glu Lys His Ala
                   90                  95                  100
   ggc gag gtg ccg gat acg gtg gag gcg ctg ctc gcg ttg ccg ggg atc    451
   Gly Glu Val Pro Asp Thr Val Glu Ala Leu Leu Ala Leu Pro Gly Ile
               105                 110                 115
   ggt gat tac acg gcg cgc gcg gtc gcg gcg ttt cat ttt ggg cag cgc    499
   Gly Asp Tyr Thr Ala Arg Ala Val Ala Ala Phe His Phe Gly Gln Arg
           120                 125                 130
   gtg ccg gtg gtc gat acg aac gtg cgt cgc gtg tac cag cgc gcg gta    547
   Val Pro Val Val Asp Thr Asn Val Arg Arg Val Tyr Gln Arg Ala Val
       135                 140                 145
   gcc gga cgt tac ctt gcg ggg cct gcg aaa aag caa gag ctt atc gac    595
   Ala Gly Arg Tyr Leu Ala Gly Pro Ala Lys Lys Gln Glu Leu Ile Asp
   150                 155                 160                 165
   gtc tcc ctt ctc ctt ccc aac act cac gcc cca gaa ttc tct gcc gca    643
   Val Ser Leu Leu Leu Pro Asn Thr His Ala Pro Glu Phe Ser Ala Ala
                   170                 175                 180
   ata atg gag ttg ggt gct ctt atc tgc acg gcc act tcc cca aag tgt    691
   Ile Met Glu Leu Gly Ala Leu Ile Cys Thr Ala Thr Ser Pro Lys Cys
               185                 190                 195
   gac acc tgc cca ctg ctt gac cag tgt caa tgg caa aaa ctt ggc tgt    739
   Asp Thr Cys Pro Leu Leu Asp Gln Cys Gln Trp Gln Lys Leu Gly Cys
           200                 205                 210
   ccc tcc ccg agt gaa gag gag ctg gct tca gcg aaa aag cgt gtg cag    787
   Pro Ser Pro Ser Glu Glu Glu Leu Ala Ser Ala Lys Lys Arg Val Gln
       215                 220                 225
   aaa ttt gtg gga acc gac cga caa gtc cgt ggc cta atc atg gac gta    835
   Lys Phe Val Gly Thr Asp Arg Gln Val Arg Gly Leu Ile Met Asp Val
   230                 235                 240                 245
   ctg cgc aat gcc acc gca cct gtg cca cta tcc gcg att gat gtc gtg    883
   Leu Arg Asn Ala Thr Ala Pro Val Pro Leu Ser Ala Ile Asp Val Val
                   250                 255                 260
   tgg cct gac gat gcc caa cgc tcc cgg gcg ctg ttt tcg ctc att gag    931
   Trp Pro Asp Asp Ala Gln Arg Ser Arg Ala Leu Phe Ser Leu Ile Glu
               265                 270                 275
   gac gga ctc gcg gaa caa aat gag gcg ggt tat ttc cac ctg cca cgg    979
   Asp Gly Leu Ala Glu Gln Asn Glu Ala Gly Tyr Phe His Leu Pro Arg
           280                 285                 290
   taaaccactg cgcgcctgca aaaaacagta                                  1009
<210> 74
   <211> 293
   <212> PRT
   <213> Corynebacterium glutamicum
<400> 74
   Met Ser Phe Thr Ala Phe Gln Thr Ala Leu Leu Val Trp Phe Arg Ala
   1               5                   10                  15
   Asn Ala Arg Asp Leu Ala Trp Arg Asp Pro Asn Thr Ser Ala Trp Gly
               20                  25                  30
   Ile Leu Leu Ser Glu Val Met Ser Gln Gln Thr Pro Val Ala Arg Val
           35                  40                  45
   Glu Pro Ile Trp Arg Glu Trp Met Glu Lys Trp Pro Thr Pro Glu Asp
       50                  55                  60
   Phe Ala Asn Ala Ser Thr Asp Glu Ile Leu Arg Ser Trp Gly Lys Leu
   65                  70                  75                  80
   Gly Tyr Pro Arg Arg Ala Leu Arg Leu Lys Glu Cys Ala Glu Val Ile
                   85                  90                  95
   Val Glu Lys His Ala Gly Glu Val Pro Asp Thr Val Glu Ala Leu Leu
               100                 105                 110
   Ala Leu Pro Gly Ile Gly Asp Tyr Thr Ala Arg Ala Val Ala Ala Phe
           115                 120                 125
   His Phe Gly Gln Arg Val Pro Val Val Asp Thr Asn Val Arg Arg Val
       130                 135                 140
   Tyr Gln Arg Ala Val Ala Gly Arg Tyr Leu Ala Gly Pro Ala Lys Lys
   145                 150                 155                 160
   Gln Glu Leu Ile Asp Val Ser Leu Leu Leu Pro Asn Thr His Ala Pro
                   165                 170                 175
   Glu Phe Ser Ala Ala Ile Met Glu Leu Gly Ala Leu Ile Cys Thr Ala
               180                 185                 190
   Thr Ser Pro Lys Cys Asp Thr Cys Pro Leu Leu Asp Gln Cys Gln Trp
           195                 200                 205
   Gln Lys Leu Gly Cys Pro Ser Pro Ser Glu Glu Glu Leu Ala Ser Ala
       210                 215                 220
   Lys Lys Arg Val Gln Lys Phe Val Gly Thr Asp Arg Gln Val Arg Gly
   225                 230                 235                 240
   Leu Ile Met Asp Val Leu Arg Asn Ala Thr Ala Pro Val Pro Leu Ser
                   245                 250                 255
   Ala Ile Asp Val Val Trp Pro Asp Asp Ala Gln Arg Ser Arg Ala Leu
               260                 265                 270
   Phe Ser Leu Ile Glu Asp Gly Leu Ala Glu Gln Asn Glu Ala Gly Tyr
           275                 280                 285
   Phe His Leu Pro Arg
       290
<210> 75
   <211> 3319
   <212> DNA
   <213> Corynebacterium glutamicum
<220>
   <221> CDS
   <222> (101)..(3289)
   <223> RXA02726
<400> 75
   ttgtgtcaac gaagtggagc tagttaattt agctcaagct gggtggtacc gcgtccgttt 60
   tttagggcgt ccccgcaggt agaacgataa ttattgttac ttg cgt gaa gga tgg   115
                                              Leu Arg Glu Gly Trp
                                              1               5
   gac cga aca cac atg tct gaa gcc gtt ggc gga gtt tac cca cag gtt   163
   Asp Arg Thr His Met Ser Glu Ala Val Gly Gly Val Tyr Pro Gln Val
                   10                  15                  20
   gat tta tct ggt ggg tca tcc aga ttt cca gag atg gaa gag aat gta   211
   Asp Leu Ser Gly Gly Ser Ser Arg Phe Pro Glu Met Glu Glu Asn Val
               25                  30                  35
   ctg agc tac tgg aag aag gat gac acc ttc cag gcc agc atc gat cag   259
   Leu Ser Tyr Trp Lys Lys Asp Asp Thr Phe Gln Ala Ser Ile Asp Gln
           40                  45                  50
   cgc gat ggt gct gaa gac tac gtc ttt tac gat ggc cct cct ttt gca   307
   Arg Asp Gly Ala Glu Asp Tyr Val Phe Tyr Asp Gly Pro Pro Phe Ala
       55                  60                  65
   aac ggt ctg cca cac tac ggc cac cta ctg act ggt tac gtc aag gac   355
   Asn Gly Leu Pro His Tyr Gly His Leu Leu Thr Gly Tyr Val Lys Asp
   70                  75                  80                  85
   att gtt cct cgc tac cag acc atg cgt ggc tac cgc gtt cct cgt gtc   403
   Ile Val Pro Arg Tyr Gln Thr Met Arg Gly Tyr Arg Val Pro Arg Val
                   90                  95                  100
   ttc ggt tgg gat acc cac ggt ctg cca gct gaa ctt gag gct gaa aag   451
   Phe Gly Trp Asp Thr His Gly Leu Pro Ala Glu Leu Glu Ala Glu Lys
               105                 110                 115
   cag ctc ggc atc aag gac aag ggc gag atc gag gcc atg ggt ctt gcc   499
   Gln Leu Gly Ile Lys Asp Lys Gly Glu Ile Glu Ala Met Gly Leu Ala
           120                 125                 130
   aag ttc aac gag tac tgt gca acc tcc gtg ttg cag tac acc aag gaa    547
   Lys Phe Asn Glu Tyr Cys Ala Thr Ser Val Leu Gln Tyr Thr Lys Glu
       135                 140                 145
   tgg gaa gag tac gtc acc cgc cag gct cgt tgg gtg gac ttt gaa aac    595
   Trp Glu Glu Tyr Val Thr Arg Gln Ala Arg Trp Val Asp Phe Glu Asn
   150                 155                 160                 165
   ggc tac aag acc atg gac ctt tct ttc atg gag tcc gtg atc tgg gcg    643
   Gly Tyr Lys Thr Met Asp Leu Ser Phe Met Glu Ser Val Ile Trp Ala
                   170                 175                 180
   ttc aag gaa ctc tac gac aag ggc ctg atc tac cag ggt ttc cgc gtt    691
   Phe Lys Glu Leu Tyr Asp Lys Gly Leu Ile Tyr Gln Gly Phe Arg Val
               185                 190                 195
   ctt cct tac tcc tgg gca gag cac acc cca ctg tcc aac cag gaa acc    739
   Leu Pro Tyr Ser Trp Ala Glu His Thr Pro Leu Ser Asn Gln Glu Thr
           200                 205                 210
   cga ctg gat gac tcc tac aag ctg cgc cag gat cca acc ctg acc gtc    787
   Arg Leu Asp Asp Ser Tyr Lys Leu Arg Gln Asp Pro Thr Leu Thr Val
       215                 220                 225
   acg ttc cca gtc acc ggt gtc gtc gaa ggt tct tct gca aac gct ggc    835
   Thr Phe Pro Val Thr Gly Val Val Glu Gly Ser Ser Ala Asn Ala Gly
   230                 235                 240                 245
   ctg gtg gga gcg ttg gct ctt gcg tgg acg act acc ccg tgg acc ctt    883
   Leu Val Gly Ala Leu Ala Leu Ala Trp Thr Thr Thr Pro Trp Thr Leu
                   250                 255                 260
   cca tcc aac ctt gcg ttg gct gtg aac cca gcg gtg acc tac gca ttg    931
   Pro Ser Asn Leu Ala Leu Ala Val Asn Pro Ala Val Thr Tyr Ala Leu
               265                 270                 275
   gtt gag gtt gct gaa gac ggt gag gca gaa ttc gtc ggc aag cgt gtg    979
   Val Glu Val Ala Glu Asp Gly Glu Ala Glu Phe Val Gly Lys Arg Val
           280                 285                 290
   ctt ttg gct aag gac ctc gtt ggt tcc tac gcc aag gaa ctc ggt gct    1027
   Leu Leu Ala Lys Asp Leu Val Gly Ser Tyr Ala Lys Glu Leu Gly Ala
       295                 300                 305
   gag gct gtt atc gtt tct gag cac cca ggc tct gaa ctg gtc gga ctg    1075
   Glu Ala Val Ile Val Ser Glu His Pro Gly Ser Glu Leu Val Gly Leu
   310                 315                 320                 325
   acc tac gag cca atc ttt gga tat ttc cgc gat cac gcg aac gga ttc    1123
   Thr Tyr Glu Pro Ile Phe Gly Tyr Phe Arg Asp His Ala Asn Gly Phe
                   330                 335                 340
   cag atc ctc ggt gca gag tac gtc acc acc gaa gac ggc acc ggt atc    1171
   Gln Ile Leu Gly Ala Glu Tyr Val Thr Thr Glu Asp Gly Thr Gly Ile
               345                 350                 355
   gtc cac cag gca cca gct ttc ggt gaa gac gat atg aac acc tgt aac    1219
   Val His Gln Ala Pro Ala Phe Gly Glu Asp Asp Met Asn Thr Cys Asn
           360                 365                 370
   gct gcc ggc att gag cca gtc atc cca gtg gac atc gac ggc aag ttc    1267
   Ala Ala Gly Ile Glu Pro Val Ile Pro Val Asp Ile Asp Gly Lys Phe
       375                 380                 385
   acc ggt ttg gtt cct gaa tac caa ggt cag ctt gtt ttc gat gcc aac     1315
   Thr Gly Leu Val Pro Glu Tyr Gln Gly Gln Leu Val Phe Asp Ala Asn
   390                 395                 400                 405
   aag gac atc atc aag gac ttg aag gct gca ggt cgc gtg gtt cgc cac     1363
   Lys Asp Ile Ile Lys Asp Leu Lys Ala Ala Gly Arg Val Val Arg His
                   410                 415                 420
   cag acc atc gaa cac tcc tac cca cac tct tgg cgt tcc ggt gag cca     1411
   Gln Thr Ile Glu His Ser Tyr Pro His Ser Trp Arg Ser Gly Glu Pro
               425                 430                 435
   ctg atc tac atg gct ctg cca tct tgg ttt gtg aat gtc acc gaa atc     1459
   Leu Ile Tyr Met Ala Leu Pro Ser Trp Phe Val Asn Val Thr Glu Ile
           440                 445                 450
   cgc gac cgc atg gtt gag gtc aac cag gac atc gag tgg atg cca gcg     1507
   Arg Asp Arg Met Val Glu Val Asn Gln Asp Ile Glu Trp Met Pro Ala
       455                 460                 465
   cac atc cgc gac ggc cag ttc ggc aag tgg cta gaa ggt gcc cgc gac     1555
   His Ile Arg Asp Gly Gln Phe Gly Lys Trp Leu Glu Gly Ala Arg Asp
   470                 475                 480                 485
   tgg aac atc tcc cgt tcc cgt tac tgg ggt tca cca att cca gca tgg     1603
   Trp Asn Ile Ser Arg Ser Arg Tyr Trp Gly Ser Pro Ile Pro Ala Trp
                   490                 495                 500
   gtc tcc gac aac gac gaa tac cca cgc gtt gat gtt tat ggt tcc ctc     1651
   Val Ser Asp Asn Asp Glu Tyr Pro Arg Val Asp Val Tyr Gly Ser Leu
               505                 510                 515
   gat gag ctt gag gct gac ttt ggc gtg cgt cca aag tcc ctg cac cgt     1699
   Asp Glu Leu Glu Ala Asp Phe Gly Val Arg Pro Lys Ser Leu His Arg
           520                 525                 530
   cca gac atc gat gaa cta act cgt cca aac cca gac gat cca acc ggc     1747
   Pro Asp Ile Asp Glu Leu Thr Arg Pro Asn Pro Asp Asp Pro Thr Gly
       535                 540                 545
   aag tcc acc atg cga cgc gtc acc gat gtt ttg gac gtg tgg ttc gac     1795
   Lys Ser Thr Met Arg Arg Val Thr Asp Val Leu Asp Val Trp Phe Asp
   550                 555                 560                 565
   tcc ggt tcc atg ccg ttt gcc cag gtg cac tac cca ttc gag aac aaa     1843
   Ser Gly Ser Met Pro Phe Ala Gln Val His Tyr Pro Phe Glu Asn Lys
                   570                 575                 580
   gaa tgg ttt gat acc cac gca cca gca gac ttc atc gtg gag tac atc     1891
   Glu Trp Phe Asp Thr His Ala Pro Ala Asp Phe Ile Val Glu Tyr Ile
               585                 590                 595
   ggt cag acc cgc ggt tgg ttc tac ctg ctg cac gtg ctg tcc acc gca     1939
   Gly Gln Thr Arg Gly Trp Phe Tyr Leu Leu His Val Leu Ser Thr Ala
           600                 605                 610
   ctg ttt gac cgc cca gct ttc aag aag gtt gtc gca cac ggc atc gtc     1987
   Leu Phe Asp Arg Pro Ala Phe Lys Lys Val Val Ala His Gly Ile Val
       615                 620                 625
   ttg ggt gat gac gga ctg aag atg tcc aag tcc aag ggc aac tac ccg    2035
   Leu Gly Asp Asp Gly Leu Lys Met Ser Lys Ser Lys Gly Asn Tyr Pro
   630                 635                 640                 645
   aac gtc aac gag gtc ttc gac cgc gac ggt tcc gac gcc atg cgt tgg    2083
   Asn Val Asn Glu Val Phe Asp Arg Asp Gly Ser Asp Ala Met Arg Trp
                   650                 655                 660
   ttc ctc atg agt tcc cca atc ctg cgc ggc ggc aac ttg att gtc acc    2131
   Phe Leu Met Ser Ser Pro Ile Leu Arg Gly Gly Asn Leu Ile Val Thr
               665                 670                 675
   gaa aag ggc atc cgc gaa ggt gtg cgc caa gca cag ctt cca atg tgg    2179
   Glu Lys Gly Ile Arg Glu Gly Val Arg Gln Ala Gln Leu Pro Met Trp
           680                 685                 690
   aac gca tac tcc ttc ctg cag ctg tac acc tcc aag aac gca acc tgg    2227
   Asn Ala Tyr Ser Phe Leu Gln Leu Tyr Thr Ser Lys Asn Ala Thr Trp
       695                 700                 705
   tca gtc gac tcc act gac gtg ctg gac cgc tac atc ctg gcg aag ctg    2275
   Ser Val Asp Ser Thr Asp Val Leu Asp Arg Tyr Ile Leu Ala Lys Leu
   710                 715                 720                 725
   cac gat ttg gtg gca gag acc cag gcg gca ctc gac ggc act gac att    2323
   His Asp Leu Val Ala Glu Thr Gln Ala Ala Leu Asp Gly Thr Asp Ile
                   730                 735                 740
   gca aag gct tgc gac ttg gtt cgt aac ttc tgt gat gcg ttg acc aac    2371
   Ala Lys Ala Cys Asp Leu Val Arg Asn Phe Cys Asp Ala Leu Thr Asn
               745                 750                 755
   tgg tac gtg cgt cgt tcc cgc gac cgt ttc tgg gct ggt gat gaa gca    2419
   Trp Tyr Val Arg Arg Ser Arg Asp Arg Phe Trp Ala Gly Asp Glu Ala
           760                 765                 770
   cac cca gag gct ttc aac acc ttg tac acc gtg ctg gaa acc ctc acc    2467
   His Pro Glu Ala Phe Asn Thr Leu Tyr Thr Val Leu Glu Thr Leu Thr
       775                 780                 785
   cgc gtg gca gct cca ctg ctg cca atg acc acc gaa gtg atc tgg cgt    2515
   Arg Val Ala Ala Pro Leu Leu Pro Met Thr Thr Glu Val Ile Trp Arg
   790                 795                 800                 805
   gga ctg acc ggc gag cgt tct gtg cac ctg act gat ttc cca tcc gct    2563
   Gly Leu Thr Gly Glu Arg Ser Val His Leu Thr Asp Phe Pro Ser Ala
                   810                 815                 820
   gag tct ttc cca gca gat gct gat ttg gtt cgc acc atg gat gag atc    2611
   Glu Ser Phe Pro Ala Asp Ala Asp Leu Val Arg Thr Met Asp Glu Ile
               825                 830                 835
   cgt ggc gtg tgc tct gcg gct tcc tct gtt cgt aag gct cac aag ctg    2659
   Arg Gly Val Cys Ser Ala Ala Ser Ser Val Arg Lys Ala His Lys Leu
           840                 845                 850
   cgt aac cgt ctg cca ctt cca ggc ctg act gtt gct ctt cca gac tct    2707
   Arg Asn Arg Leu Pro Leu Pro Gly Leu Thr Val Ala Leu Pro Asp Ser
       855                 860                 865
   gct cgc ctg gca gac ttc gct tcg atc atc cgc gat gag gtc aac gtg    2755
   Ala Arg Leu Ala Asp Phe Ala Ser Ile Ile Arg Asp Glu Val Asn Val
   870                 875                 880                 885
   aag aac gtg gat ctg acc tct gac gtg gat tcc gtg gga acc ttc gag    2803
   Lys Asn Val Asp Leu Thr Ser Asp Val Asp Ser Val Gly Thr Phe Glu
                   890                 895                 900
   gtt gtt gtt aac gct aag gtt gca ggt cct cgc ttg ggc aag gac gtc    2851
   Val Val Val Asn Ala Lys Val Ala Gly Pro Arg Leu Gly Lys Asp Val
               905                 910                 915
   cag cgc gtg atc aag gct gtg aag gct ggc aac tac acc cgc gaa ggc    2899
   Gln Arg Val Ile Lys Ala Val Lys Ala Gly Asn Tyr Thr Arg Glu Gly
           920                 925                 930
   gac gtc gtt gtt gcc gat ggc atc gag ctc aac gag ggt gaa ttc acc    2947
   Asp Val Val Val Ala Asp Gly Ile Glu Leu Asn Glu Gly Glu Phe Thr
       935                 940                 945
   gag cgt ctc gta gca gca aac cct gat tcc acc gcg cag atc gac ggc    2995
   Glu Arg Leu Val Ala Ala Asn Pro Asp Ser Thr Ala Gln Ile Asp Gly
   950                 955                 960                 965
   gtg gat gga ctc gtg gtt ctg gac atg gaa gtc acg gaa gaa ctt gaa    3043
   Val Asp Gly Leu Val Val Leu Asp Met Glu Val Thr Glu Glu Leu Glu
                   970                 975                 980
   gca gaa ggc tgg gca gcg gac gcg atc cgt ggc ctg cag gat gct cga    3091
   Ala Glu Gly Trp Ala Ala Asp Ala Ile Arg Gly Leu Gln Asp Ala Arg
               985                 990                 995
   aag aac tcc ggc ttt gag gtt tct gac cgc att tct gtt gtc gtc agc    3139
   Lys Asn Ser Gly Phe Glu Val Ser Asp Arg Ile Ser Val Val Val Ser
           1000                1005                1010
   gtt cct gag gac aag aag gaa tgg atc acc act cac gct gat cac atc    3187
   Val Pro Glu Asp Lys Lys Glu Trp Ile Thr Thr His Ala Asp His Ile
       1015                1020                1025
   gca gcg gaa gtt ttg gca acc tcc ttt gag atc gtc act gat gcc ctc    3235
   Ala Ala Glu Val Leu Ala Thr Ser Phe Glu Ile Val Thr Asp Ala Leu
   1030                1035                1040                1045
   gac ggc gaa acc cac gac att gtc gct ggt gtg acc gcg aag gtt act    3283
   Asp Gly Glu Thr His Asp Ile Val Ala Gly Val Thr Ala Lys Val Thr
                   1050                1055                1060
   aag aac taagagttgt tttgttgaga aagcccgctg                          3319
   Lys Asn
<210> 76
   <211> 1063
   <212> PRT
   <213> Corynebacterium glutamicum
<400> 76
   Leu Arg Glu Gly Trp Asp Arg Thr His Met Ser Glu Ala Val Gly Gly
   1 5 10 15
   Val Tyr Pro Gln Val Asp Leu Ser Gly Gly Ser Ser Arg Phe Pro Glu
   20 25 30
   Met Glu Glu Asn Val Leu Ser Tyr Trp Lys Lys Asp Asp Thr Phe Gln
               35                  40                  45
   Ala Ser Ile Asp Gln Arg Asp Gly Ala Glu Asp Tyr Val Phe Tyr Asp
       50                  55                  60
   Gly Pro Pro Phe Ala Asn Gly Leu Pro His Tyr Gly His Leu Leu Thr
   65                  70                  75                  80
   Gly Tyr Val Lys Asp Ile Val Pro Arg Tyr Gln Thr Met Arg Gly Tyr
                   85                  90                  95
   Arg Val Pro Arg Val Phe Gly Trp Asp Thr His Gly Leu Pro Ala Glu
               100                 105                 110
   Leu Glu Ala Glu Lys Gln Leu Gly Ile Lys Asp Lys Gly Glu Ile Glu
           115                 120                 125
   Ala Met Gly Leu Ala Lys Phe Asn Glu Tyr Cys Ala Thr Ser Val Leu
       130                 135                 140
   Gln Tyr Thr Lys Glu Trp Glu Glu Tyr Val Thr Arg Gln Ala Arg Trp
   145                 150                 155                 160
   Val Asp Phe Glu Asn Gly Tyr Lys Thr Met Asp Leu Ser Phe Met Glu
                   165                 170                 175
   Ser Val Ile Trp Ala Phe Lys Glu Leu Tyr Asp Lys Gly Leu Ile Tyr
               180                 185                 190
   Gln Gly Phe Arg Val Leu Pro Tyr Ser Trp Ala Glu His Thr Pro Leu
           195                 200                 205
   Ser Asn Gln Glu Thr Arg Leu Asp Asp Ser Tyr Lys Leu Arg Gln Asp
       210                 215                 220
   Pro Thr Leu Thr Val Thr Phe Pro Val Thr Gly Val Val Glu Gly Ser
   225                 230                 235                 240
   Ser Ala Asn Ala Gly Leu Val Gly Ala Leu Ala Leu Ala Trp Thr Thr
                   245                 250                 255
   Thr Pro Trp Thr Leu Pro Ser Asn Leu Ala Leu Ala Val Asn Pro Ala
               260                 265                 270
   Val Thr Tyr Ala Leu Val Glu Val Ala Glu Asp Gly Glu Ala Glu Phe
           275                 280                 285
   Val Gly Lys Arg Val Leu Leu Ala Lys Asp Leu Val Gly Ser Tyr Ala
       290                 295                 300
   Lys Glu Leu Gly Ala Glu Ala Val Ile Val Ser Glu His Pro Gly Ser
   305                 310                 315                 320
   Glu Leu Val Gly Leu Thr Tyr Glu Pro Ile Phe Gly Tyr Phe Arg Asp
                   325                 330                 335
   His Ala Asn Gly Phe Gln Ile Leu Gly Ala Glu Tyr Val Thr Thr Glu
               340                 345                 350
   Asp Gly Thr Gly Ile Val His Gln Ala Pro Ala Phe Gly Glu Asp Asp
           355                 360                 365
   Met Asn Thr Cys Asn Ala Ala Gly Ile Glu Pro Val Ile Pro Val Asp
       370                 375                 380
   Ile Asp Gly Lys Phe Thr Gly Leu Val Pro Glu Tyr Gln Gly Gln Leu
   385 390 395 400
   Val Phe Asp Ala Asn Lys Asp Ile Ile Lys Asp Leu Lys Ala Ala Gly
   405 410 415
   Arg Val Val Arg His Gln Thr Ile Glu His Ser Tyr Pro His Ser Trp
   420 425 430
   Arg Ser Gly Glu Pro Leu Ile Tyr Met Ala Leu Pro Ser Trp Phe Val
   435 440 445
   Asn Val Thr Glu Ile Arg Asp Arg Met Val Glu Val Asn Gln Asp Ile
   450 455 460
   Glu Trp Met Pro Ala His Ile Arg Asp Gly Gln Phe Gly Lys Trp Leu
   465 470 475 480
   Glu Gly Ala Arg Asp Trp Asn Ile Ser Arg Ser Arg Tyr Trp Gly Ser
   485 490 495
   Pro Ile Pro Ala Trp Val Ser Asp Asn Asp Glu Tyr Pro Arg Val Asp
   500 505 510
   Val Tyr Gly Ser Leu Asp Glu Leu Glu Ala Asp Phe Gly Val Arg Pro
   515 520 525
   Lys Ser Leu His Arg Pro Asp Ile Asp Glu Leu Thr Arg Pro Asn Pro
   530 535 540
   Asp Asp Pro Thr Gly Lys Ser Thr Met Arg Arg Val Thr Asp Val Leu
   545 550 555 560
   Asp Val Trp Phe Asp Ser Gly Ser Met Pro Phe Ala Gln Val His Tyr
   565 570 575
   Pro Phe Glu Asn Lys Glu Trp Phe Asp Thr His Ala Pro Ala Asp Phe
   580 585 590
   Ile Val Glu Tyr Ile Gly Gln Thr Arg Gly Trp Phe Tyr Leu Leu His
   595 600 605
   Val Leu Ser Thr Ala Leu Phe Asp Arg Pro Ala Phe Lys Lys Val Val
   610 615 620
   Ala His Gly Ile Val Leu Gly Asp Asp Gly Leu Lys Met Ser Lys Ser
   625 630 635 640
   Lys Gly Asn Tyr Pro Asn Val Asn Glu Val Phe Asp Arg Asp Gly Ser
   645 650 655
   Asp Ala Met Arg Trp Phe Leu Met Ser Ser Pro Ile Leu Arg Gly Gly
   660 665 670
   Asn Leu Ile Val Thr Glu Lys Gly Ile Arg Glu Gly Val Arg Gln Ala
   675 680 685
   Gln Leu Pro Met Trp Asn Ala Tyr Ser Phe Leu Gln Leu Tyr Thr Ser
   690 695 700
   Lys Asn Ala Thr Trp Ser Val Asp Ser Thr Asp Val Leu Asp Arg Tyr
   705 710 715 720
   Ile Leu Ala Lys Leu His Asp Leu Val Ala Glu Thr Gln Ala Ala Leu
   725 730 735
   Asp Gly Thr Asp Ile Ala Lys Ala Cys Asp Leu Val Arg Asn Phe Cys
   740 745 750
   Asp Ala Leu Thr Asn Trp Tyr Val Arg Arg Ser Arg Asp Arg Phe Trp
   755 760 765
   Ala Gly Asp Glu Ala His Pro Glu Ala Phe Asn Thr Leu Tyr Thr Val
   770 775 780
   Leu Glu Thr Leu Thr Arg Val Ala Ala Pro Leu Leu Pro Met Thr Thr
   785 790 795 800
   Glu Val Ile Trp Arg Gly Leu Thr Gly Glu Arg Ser Val His Leu Thr
   805 810 815
   Asp Phe Pro Ser Ala Glu Ser Phe Pro Ala Asp Ala Asp Leu Val Arg
   820 825 830
   Thr Met Asp Glu Ile Arg Gly Val Cys Ser Ala Ala Ser Ser Val Arg
   835 840 845
   Lys Ala His Lys Leu Arg Asn Arg Leu Pro Leu Pro Gly Leu Thr Val
   850 855 860
   Ala Leu Pro Asp Ser Ala Arg Leu Ala Asp Phe Ala Ser Ile Ile Arg
   865 870 875 880
   Asp Glu Val Asn Val Lys Asn Val Asp Leu Thr Ser Asp Val Asp Ser
   885 890 895
   Val Gly Thr Phe Glu Val Val Val Asn Ala Lys Val Ala Gly Pro Arg
   900 905 910
   Leu Gly Lys Asp Val Gln Arg Val Ile Lys Ala Val Lys Ala Gly Asn
   915 920 925
   Tyr Thr Arg Glu Gly Asp Val Val Val Ala Asp Gly Ile Glu Leu Asn
   930 935 940
   Glu Gly Glu Phe Thr Glu Arg Leu Val Ala Ala Asn Pro Asp Ser Thr
   945 950 955 960
   Ala Gln Ile Asp Gly Val Asp Gly Leu Val Val Leu Asp Met Glu Val
   965 970 975
   Thr Glu Glu Leu Glu Ala Glu Gly Trp Ala Ala Asp Ala Ile Arg Gly
   980 985 990
   Leu Gln Asp Ala Arg Lys Asn Ser Gly Phe Glu Val Ser Asp Arg Ile
   995 1000 1005
   Ser Val Val Val Ser Val Pro Glu Asp Lys Lys Glu Trp Ile Thr Thr
   1010 1015 1020
   His Ala Asp His Ile Ala Ala Glu Val Leu Ala Thr Ser Phe Glu Ile
   1025 1030 1035 1040
   Val Thr Asp Ala Leu Asp Gly Glu Thr His Asp Ile Val Ala Gly Val
   1045 1050 1055
   Thr Ala Lys Val Thr Lys Asn
   1060
<210> 77
   <211> 2290
   <212> DNA
   <213> Corynebacterium glutamicum
<220>
   <221> CDS
   <222> (101)..(2260)
   <223> RXA02731
<400> 77
   gttgcatcga gtccgcgggt ttcgcacgct tgatctaaat tcttgagggt tttccggccg 60
   ttgtttgcgc taaacgtagg ggtcaagcgt cgaaaagcgc ttg ctt gca cgc tgt    115
                                              Leu Leu Ala Arg Cys
                                              1               5
   ttt gct gcg ggc cgc aac gtg tcc acc ctg tgg cgt atc cta gaa tgc    163
   Phe Ala Ala Gly Arg Asn Val Ser Thr Leu Trp Arg Ile Leu Glu Cys
                   10                  15                  20
   atg gct ttt gct gct gaa cat cct gtc ctg tcc cac tct gag cac cgc    211
   Met Ala Phe Ala Ala Glu His Pro Val Leu Ser His Ser Glu His Arg
               25                  30                  35
   ccg gtt ggt gaa atc gag cgt agc gat gac aaa ttt gtt gtc gtt agt    259
   Pro Val Gly Glu Ile Glu Arg Ser Asp Asp Lys Phe Val Val Val Ser
           40                  45                  50
   gaa ttt gag cct gcg ggt gac cag cct gcg gct att aaa gag ctc gat    307
   Glu Phe Glu Pro Ala Gly Asp Gln Pro Ala Ala Ile Lys Glu Leu Asp
       55                  60                  65
   gag cgc ttg gat cgc ggt gag cgg gac gtc gtt ttg atg ggt gct act    355
   Glu Arg Leu Asp Arg Gly Glu Arg Asp Val Val Leu Met Gly Ala Thr
   70                  75                  80                  85
   ggt acg ggt aag tcc gcg act gcg gcg tgg ttg atc gaa aag cag cag    403
   Gly Thr Gly Lys Ser Ala Thr Ala Ala Trp Leu Ile Glu Lys Gln Gln
                   90                  95                  100
   cgc ccc gct ttg gtg atg gcg ccg aat aag acg ctg gct gcg cag ttg    451
   Arg Pro Ala Leu Val Met Ala Pro Asn Lys Thr Leu Ala Ala Gln Leu
               105                 110                 115
   gct aat gaa ttg cgg cag ctg ttg ccc aat aac gcg gtg gag tat ttc    499
   Ala Asn Glu Leu Arg Gln Leu Leu Pro Asn Asn Ala Val Glu Tyr Phe
           120                 125                 130
   gtg tct tat tac gat tac tac cag cca gaa gcg tat atc gcg cag act    547
   Val Ser Tyr Tyr Asp Tyr Tyr Gln Pro Glu Ala Tyr Ile Ala Gln Thr
       135                 140                 145
   gat acc tat att gaa aag gac tcc tcg att aat gag gat gtg gag cgt    595
   Asp Thr Tyr Ile Glu Lys Asp Ser Ser Ile Asn Glu Asp Val Glu Arg
   150                 155                 160                 165
   ctg cgt cac tcg gcg acg tcg tct ttg ctg agt agg cga gac gtc gtg    643
   Leu Arg His Ser Ala Thr Ser Ser Leu Leu Ser Arg Arg Asp Val Val
                   170                 175                 180
   gtt gtt agt tcg gtg tcg tgt att tat ggc ttg ggc act cca cag tct    691
   Val Val Ser Ser Val Ser Cys Ile Tyr Gly Leu Gly Thr Pro Gln Ser
                   185                 190                 195
   tat ctt gac cgt tcc gtt gtg ttg aac gtg ggg gag gag atc gac cgc    739
   Tyr Leu Asp Arg Ser Val Val Leu Asn Val Gly Glu Glu Ile Asp Arg
               200                 205                 210
   gat cgc ttt ttg cgc cta ttg gta gat att caa tac gaa cgc aat gat    787
   Asp Arg Phe Leu Arg Leu Leu Val Asp Ile Gln Tyr Glu Arg Asn Asp
       215                 220                 225
   gtg ggc ttt act cgt ggt gct ttc cgc gtg aag ggc gat acc gtg gac    835
   Val Gly Phe Thr Arg Gly Ala Phe Arg Val Lys Gly Asp Thr Val Asp
   230                 235                 240                 245
   atc atc ccg gcc tat gag gaa ttg gcg gtg cgc att gag ttt ttc ggt    883
   Ile Ile Pro Ala Tyr Glu Glu Leu Ala Val Arg Ile Glu Phe Phe Gly
                   250                 255                 260
   gat gaa att gat gcg ttg tac tac atc cat ccc ctg act ggt gac acc    931
   Asp Glu Ile Asp Ala Leu Tyr Tyr Ile His Pro Leu Thr Gly Asp Thr
               265                 270                 275
   atc cgg cag gtg aat gag atc cgt att ttc cca gct acg cac tat gtt    979
   Ile Arg Gln Val Asn Glu Ile Arg Ile Phe Pro Ala Thr His Tyr Val
           280                 285                 290
   gcg gga cct gag cgg atg gaa aag gca gtc gct gat att aag gcg gag    1027
   Ala Gly Pro Glu Arg Met Glu Lys Ala Val Ala Asp Ile Lys Ala Glu
       295                 300                 305
   ttg gaa gtg cgc ctg gct gat ttg gag aac cgt ggc aag tta ttg gaa    1075
   Leu Glu Val Arg Leu Ala Asp Leu Glu Asn Arg Gly Lys Leu Leu Glu
   310                 315                 320                 325
   gcg cag cgt ctt agg atg cgt act gaa tat gac tta gaa atg atc gag    1123
   Ala Gln Arg Leu Arg Met Arg Thr Glu Tyr Asp Leu Glu Met Ile Glu
                   330                 335                 340
   cag gtt ggt ttc tgt tcg ggc att gag aac tat tct cgc cac att gat    1171
   Gln Val Gly Phe Cys Ser Gly Ile Glu Asn Tyr Ser Arg His Ile Asp
               345                 350                 355
   gga cgt ggg gag gga acc gca ccg gcc acg ctg att gac tat ttc cca    1219
   Gly Arg Gly Glu Gly Thr Ala Pro Ala Thr Leu Ile Asp Tyr Phe Pro
           360                 365                 370
   gag gat ttc ctc acc atc atc gat gag tct cac gtg aca gtc ccg cag    1267
   Glu Asp Phe Leu Thr Ile Ile Asp Glu Ser His Val Thr Val Pro Gln
       375                 380                 385
   atc ggc ggc atg ttt gag ggc gat atg tcc cgt aaa cgt aac ctc gta    1315
   Ile Gly Gly Met Phe Glu Gly Asp Met Ser Arg Lys Arg Asn Leu Val
   390                 395                 400                 405
   gaa ttc ggt ttc cgc ctg cca tcc gcg atg gat aac cgc cca ttg acc    1363
   Glu Phe Gly Phe Arg Leu Pro Ser Ala Met Asp Asn Arg Pro Leu Thr
                   410                 415                 420
   tgg gag gag ttc gat gaa cgc cgt ggc caa acg gtg ttc atg tct gca    1411
   Trp Glu Glu Phe Asp Glu Arg Arg Gly Gln Thr Val Phe Met Ser Ala
               425                 430                 435
   act cca ggc aag ttt gag atc gct gct gct gat ggt gag ttt gtg gag    1459
   Thr Pro Gly Lys Phe Glu Ile Ala Ala Ala Asp Gly Glu Phe Val Glu
           440                 445                 450
   cag gtc att cgc cca aca ggt ctg gtg gat cca aag gtc acc gtc aag    1507
   Gln Val Ile Arg Pro Thr Gly Leu Val Asp Pro Lys Val Thr Val Lys
       455                 460                 465
   cca acg aag ggg cag att gat gat ctg atc cat gaa att cgc caa cgc    1555
   Pro Thr Lys Gly Gln Ile Asp Asp Leu Ile His Glu Ile Arg Gln Arg
   470                 475                 480                 485
   acc gat aaa gat gag cgc gtt ttg gtc acc aca ttg acc aag aaa atg    1603
   Thr Asp Lys Asp Glu Arg Val Leu Val Thr Thr Leu Thr Lys Lys Met
                   490                 495                 500
   gct gag gat ctt act gat tac ctg ctg gaa aac ggc atc cgc gtg cgc    1651
   Ala Glu Asp Leu Thr Asp Tyr Leu Leu Glu Asn Gly Ile Arg Val Arg
               505                 510                 515
   tac ctg cac tca gat att gat acc ttg cag cgt gtg gaa ttg ctg cgt    1699
   Tyr Leu His Ser Asp Ile Asp Thr Leu Gln Arg Val Glu Leu Leu Arg
           520                 525                 530
   cag ctt cgc ctg ggc gaa tac gat gtg ttg gta ggt att aac ctg ctg    1747
   Gln Leu Arg Leu Gly Glu Tyr Asp Val Leu Val Gly Ile Asn Leu Leu
       535                 540                 545
   cgt gag ggc ctt gac ctg cca gaa gtc tct ctg gtt gcg att ctc gac    1795
   Arg Glu Gly Leu Asp Leu Pro Glu Val Ser Leu Val Ala Ile Leu Asp
   550                 555                 560                 565
   gcc gac aag gaa ggc ttc ctg cgc tcc acc acc tca ctg att cag acc    1843
   Ala Asp Lys Glu Gly Phe Leu Arg Ser Thr Thr Ser Leu Ile Gln Thr
                   570                 575                 580
   att ggc cgc gcc gcc cga aat gtg tcc ggc gag gtc atc atg tac gcc    1891
   Ile Gly Arg Ala Ala Arg Asn Val Ser Gly Glu Val Ile Met Tyr Ala
               585                 590                 595
   gac aag atc act gat tcg atg cag tat gcc atc gag gaa acc gat cga    1939
   Asp Lys Ile Thr Asp Ser Met Gln Tyr Ala Ile Glu Glu Thr Asp Arg
           600                 605                 610
   cgc cgt gaa aag cag gtc gct tat aac aag gaa cac ggc atc gat ccg    1987
   Arg Arg Glu Lys Gln Val Ala Tyr Asn Lys Glu His Gly Ile Asp Pro
       615                 620                 625
   cag ccg ctt cga aag aaa atc gcg gac atc ctc gac cag gtc tat gac    2035
   Gln Pro Leu Arg Lys Lys Ile Ala Asp Ile Leu Asp Gln Val Tyr Asp
   630                 635                 640                 645
   aat tcc gct gat gga gca gga cct tct gcc tct ggc gat gcg gca gtc    2083
   Asn Ser Ala Asp Gly Ala Gly Pro Ser Ala Ser Gly Asp Ala Ala Val
                   650                 655                 660
   gtg gct aaa cct gac gtg tct agc atg ccc gcc aaa gaa gtg caa aag    2131
   Val Ala Lys Pro Asp Val Ser Ser Met Pro Ala Lys Glu Val Gln Lys
               665                 670                 675
   ctt atc gac gac ctc agc gct cag atg gct gcg gcc gcg cgg gag ctc    2179
   Leu Ile Asp Asp Leu Ser Ala Gln Met Ala Ala Ala Ala Arg Glu Leu
           680                 685                 690
   aag ttc gag ctg gca ggg cgt ctg cga gat gag atc ttc gag ctc aag   2227
   Lys Phe Glu Leu Ala Gly Arg Leu Arg Asp Glu Ile Phe Glu Leu Lys
       695                 700                 705
   aag gaa ctg aga ggt atc aag gat gcc ggc atc taagtcagct tgctcactta 2280
   Lys Glu Leu Arg Gly Ile Lys Asp Ala Gly Ile
   710                 715                 720
   aagcttcgaa                                                        2290
<210> 78
   <211> 720
   <212> PRT
   <213> Corynebacterium glutamicum
<400> 78
   Leu Leu Ala Arg Cys Phe Ala Ala Gly Arg Asn Val Ser Thr Leu Trp
    1               5                  10                  15
   Arg Ile Leu Glu Cys Met Ala Phe Ala Ala Glu His Pro Val Leu Ser
               20                  25                  30
   His Ser Glu His Arg Pro Val Gly Glu Ile Glu Arg Ser Asp Asp Lys
           35                  40                  45
   Phe Val Val Val Ser Glu Phe Glu Pro Ala Gly Asp Gln Pro Ala Ala
       50                  55                  60
   Ile Lys Glu Leu Asp Glu Arg Leu Asp Arg Gly Glu Arg Asp Val Val
   65                  70                  75                  80
   Leu Met Gly Ala Thr Gly Thr Gly Lys Ser Ala Thr Ala Ala Trp Leu
                   85                  90                  95
   Ile Glu Lys Gln Gln Arg Pro Ala Leu Val Met Ala Pro Asn Lys Thr
               100                 105                 110
   Leu Ala Ala Gln Leu Ala Asn Glu Leu Arg Gln Leu Leu Pro Asn Asn
           115                 120                 125
   Ala Val Glu Tyr Phe Val Ser Tyr Tyr Asp Tyr Tyr Gln Pro Glu Ala
       130                 135                 140
   Tyr Ile Ala Gln Thr Asp Thr Tyr Ile Glu Lys Asp Ser Ser Ile Asn
   145                 150                 155                 160
   Glu Asp Val Glu Arg Leu Arg His Ser Ala Thr Ser Ser Leu Leu Ser
                   165                 170                 175
   Arg Arg Asp Val Val Val Val Ser Ser Val Ser Cys Ile Tyr Gly Leu
               180                 185                 190
   Gly Thr Pro Gln Ser Tyr Leu Asp Arg Ser Val Val Leu Asn Val Gly
           195                 200                 205
   Glu Glu Ile Asp Arg Asp Arg Phe Leu Arg Leu Leu Val Asp Ile Gln
       210                 215                 220
   Tyr Glu Arg Asn Asp Val Gly Phe Thr Arg Gly Ala Phe Arg Val Lys
   225                 230                 235                 240
   Gly Asp Thr Val Asp Ile Ile Pro Ala Tyr Glu Glu Leu Ala Val Arg
   245 250 255
   Ile Glu Phe Phe Gly Asp Glu Ile Asp Ala Leu Tyr Tyr Ile His Pro
   260 265 270
   Leu Thr Gly Asp Thr Ile Arg Gln Val Asn Glu Ile Arg Ile Phe Pro
   275 280 285
   Ala Thr His Tyr Val Ala Gly Pro Glu Arg Met Glu Lys Ala Val Ala
   290 295 300
   Asp Ile Lys Ala Glu Leu Glu Val Arg Leu Ala Asp Leu Glu Asn Arg
   305 310 315 320
   Gly Lys Leu Leu Glu Ala Gln Arg Leu Arg Met Arg Thr Glu Tyr Asp
   325 330 335
   Leu Glu Met Ile Glu Gln Val Gly Phe Cys Ser Gly Ile Glu Asn Tyr
   340 345 350
   Ser Arg His Ile Asp Gly Arg Gly Glu Gly Thr Ala Pro Ala Thr Leu
   355 360 365
   Ile Asp Tyr Phe Pro Glu Asp Phe Leu Thr Ile Ile Asp Glu Ser His
   370 375 380
   Val Thr Val Pro Gln Ile Gly Gly Met Phe Glu Gly Asp Met Ser Arg
   385 390 395 400
   Lys Arg Asn Leu Val Glu Phe Gly Phe Arg Leu Pro Ser Ala Met Asp
   405 410 415
   Asn Arg Pro Leu Thr Trp Glu Glu Phe Asp Glu Arg Arg Gly Gln Thr
   420 425 430
   Val Phe Met Ser Ala Thr Pro Gly Lys Phe Glu Ile Ala Ala Ala Asp
   435 440 445
   Gly Glu Phe Val Glu Gln Val Ile Arg Pro Thr Gly Leu Val Asp Pro
   450 455 460
   Lys Val Thr Val Lys Pro Thr Lys Gly Gln Ile Asp Asp Leu Ile His
   465 470 475 480
   Glu Ile Arg Gln Arg Thr Asp Lys Asp Glu Arg Val Leu Val Thr Thr
   485 490 495
   Leu Thr Lys Lys Met Ala Glu Asp Leu Thr Asp Tyr Leu Leu Glu Asn
   500 505 510
   Gly Ile Arg Val Arg Tyr Leu His Ser Asp Ile Asp Thr Leu Gln Arg
   515 520 525
   Val Glu Leu Leu Arg Gln Leu Arg Leu Gly Glu Tyr Asp Val Leu Val
   530 535 540
   Gly Ile Asn Leu Leu Arg Glu Gly Leu Asp Leu Pro Glu Val Ser Leu
   545 550 555 560
   Val Ala Ile Leu Asp Ala Asp Lys Glu Gly Phe Leu Arg Ser Thr Thr
   565 570 575
   Ser Leu Ile Gln Thr Ile Gly Arg Ala Ala Arg Asn Val Ser Gly Glu
   580 585 590
   Val Ile Met Tyr Ala Asp Lys Ile Thr Asp Ser Met Gln Tyr Ala Ile
   595 600 605
   Glu Glu Thr Asp Arg Arg Arg Glu Lys Gln Val Ala Tyr Asn Lys Glu
   610 615 620
   His Gly Ile Asp Pro Gln Pro Leu Arg Lys Lys Ile Ala Asp Ile Leu
   625 630 635 640
   Asp Gln Val Tyr Asp Asn Ser Ala Asp Gly Ala Gly Pro Ser Ala Ser
   645 650 655
   Gly Asp Ala Ala Val Val Ala Lys Pro Asp Val Ser Ser Met Pro Ala
   660 665 670
   Lys Glu Val Gln Lys Leu Ile Asp Asp Leu Ser Ala Gln Met Ala Ala
   675 680 685
   Ala Ala Arg Glu Leu Lys Phe Glu Leu Ala Gly Arg Leu Arg Asp Glu
   690 695 700
   Ile Phe Glu Leu Lys Lys Glu Leu Arg Gly Ile Lys Asp Ala Gly Ile
   705 710 715 720
<210> 79
   <211> 1087
   <212> DNA
   <213> Corynebacterium glutamicum
<220>
   <221> CDS
   <222> (101)..(1057)
   <223> RXA02736
<400> 79
   cagaggatta cccagcgggt acgtggggtc caaagagcgc tgatgaaatg ctttcccgca 60
   acggtcacac ctggcgcagg ccataattta ggggcaaaaa atg atc ttt gaa ctt   115
                                               Met Ile Phe Glu Leu
                                               1               5
   ccg gat acc acc acc cag caa att tcc aag acc cta act cga ctg cgt   163
   Pro Asp Thr Thr Thr Gln Gln Ile Ser Lys Thr Leu Thr Arg Leu Arg
                   10                  15                  20
   gaa tcg ggc acc cag gtc acc acc ggc cga gtg ctc acc ctc atc gtg   211
   Glu Ser Gly Thr Gln Val Thr Thr Gly Arg Val Leu Thr Leu Ile Val
               25                  30                  35
   gtc act gac tcc gaa agc gat gtc gct gca gtt acc gag tcc acc aat   259
   Val Thr Asp Ser Glu Ser Asp Val Ala Ala Val Thr Glu Ser Thr Asn
           40                  45                  50
   gaa gcc tcg cgc gag cac cca tct cgc gtg atc att ttg gtg gtt ggc   307
   Glu Ala Ser Arg Glu His Pro Ser Arg Val Ile Ile Leu Val Val Gly
       55                  60                  65
   gat aaa act gca gaa aac aaa gtt gac gca gaa gtc cgt atc ggt ggc   355
   Asp Lys Thr Ala Glu Asn Lys Val Asp Ala Glu Val Arg Ile Gly Gly
   70                  75                  80                  85
   gac gct ggt gct tcc gag atg atc atc atg cat ctc aac gga cct gtc    403
   Asp Ala Gly Ala Ser Glu Met Ile Ile Met His Leu Asn Gly Pro Val
                   90                  95                  100
   gct gac aag ctc cag tat gtc gtc aca cca ctg ttg ctt cct gac acc    451
   Ala Asp Lys Leu Gln Tyr Val Val Thr Pro Leu Leu Leu Pro Asp Thr
               105                 110                 115
   ccc atc gtt gct tgg tgg cca ggt gaa tca cca aag aat cct tcc cag    499
   Pro Ile Val Ala Trp Trp Pro Gly Glu Ser Pro Lys Asn Pro Ser Gln
           120                 125                 130
   gac cca att gga cgc atc gca caa cga cgc atc act gat gct ttg tac    547
   Asp Pro Ile Gly Arg Ile Ala Gln Arg Arg Ile Thr Asp Ala Leu Tyr
       135                 140                 145
   gac cgt gat gac gca cta gaa gat cgt gtt gag aac tat cac cca ggt    595
   Asp Arg Asp Asp Ala Leu Glu Asp Arg Val Glu Asn Tyr His Pro Gly
   150                 155                 160                 165
   gat acc gac atg acg tgg gcg cgc ctt acc cag tgg cgg gga ctt gtt    643
   Asp Thr Asp Met Thr Trp Ala Arg Leu Thr Gln Trp Arg Gly Leu Val
                   170                 175                 180
   gcc tcc tca ttg gat cac cca cca cac agc gaa atc act tcc gtg agg    691
   Ala Ser Ser Leu Asp His Pro Pro His Ser Glu Ile Thr Ser Val Arg
               185                 190                 195
   ctg acc ggt gca agc ggc agt acc tcg gtg gat ttg gct gca ggc tgg    739
   Leu Thr Gly Ala Ser Gly Ser Thr Ser Val Asp Leu Ala Ala Gly Trp
           200                 205                 210
   ttg gcg cgg agg ctg aaa gtg cct gtg atc cgc gag gtg aca gat gct    787
   Leu Ala Arg Arg Leu Lys Val Pro Val Ile Arg Glu Val Thr Asp Ala
       215                 220                 225
   ccc acc gtg cca acc gat gag ttt ggt act cca ctg ctg gct atc cag    835
   Pro Thr Val Pro Thr Asp Glu Phe Gly Thr Pro Leu Leu Ala Ile Gln
   230                 235                 240                 245
   cgc ctg gag atc gtt cgc acc acc ggc tcg atc atc atc acc atc tat    883
   Arg Leu Glu Ile Val Arg Thr Thr Gly Ser Ile Ile Ile Thr Ile Tyr
                   250                 255                 260
   gac gct cat acc ctt cag gta gag atg ccg gaa tcc ggc aat gcc cca    931
   Asp Ala His Thr Leu Gln Val Glu Met Pro Glu Ser Gly Asn Ala Pro
               265                 270                 275
   tcg ctg gtg gct att ggt cgt cga agt gag tcc gac tgc ttg tct gag    979
   Ser Leu Val Ala Ile Gly Arg Arg Ser Glu Ser Asp Cys Leu Ser Glu
           280                 285                 290
   gag ctt cgc cac atg gat cca gat ttg ggc tac cag cac gca cta tcc    1027
   Glu Leu Arg His Met Asp Pro Asp Leu Gly Tyr Gln His Ala Leu Ser
       295                 300                 305
   ggc ttg tcc agc gtc aag ctg gaa acc gtc taaggagaaa tacaacacta      1077
   Gly Leu Ser Ser Val Lys Leu Glu Thr Val
   310                 315
   tggttgatgt                                                         1087
<210> 80
   <211> 319
   <212> PRT
   <213> Corynebacterium glutamicum
<400> 80
   Met Ile Phe Glu Leu Pro Asp Thr Thr Thr Gln Gln Ile Ser Lys Thr
   1               5                   10                  15
   Leu Thr Arg Leu Arg Glu Ser Gly Thr Gln Val Thr Thr Gly Arg Val
               20                  25                  30
   Leu Thr Leu Ile Val Val Thr Asp Ser Glu Ser Asp Val Ala Ala Val
           35                  40                  45
   Thr Glu Ser Thr Asn Glu Ala Ser Arg Glu His Pro Ser Arg Val Ile
       50                  55                  60
   Ile Leu Val Val Gly Asp Lys Thr Ala Glu Asn Lys Val Asp Ala Glu
   65                  70                  75                  80
   Val Arg Ile Gly Gly Asp Ala Gly Ala Ser Glu Met Ile Ile Met His
                   85                  90                  95
   Leu Asn Gly Pro Val Ala Asp Lys Leu Gln Tyr Val Val Thr Pro Leu
               100                 105                 110
   Leu Leu Pro Asp Thr Pro Ile Val Ala Trp Trp Pro Gly Glu Ser Pro
           115                 120                 125
   Lys Asn Pro Ser Gln Asp Pro Ile Gly Arg Ile Ala Gln Arg Arg Ile
       130                 135                 140
   Thr Asp Ala Leu Tyr Asp Arg Asp Asp Ala Leu Glu Asp Arg Val Glu
   145                 150                 155                 160
   Asn Tyr His Pro Gly Asp Thr Asp Met Thr Trp Ala Arg Leu Thr Gln
                   165                 170                 175
   Trp Arg Gly Leu Val Ala Ser Ser Leu Asp His Pro Pro His Ser Glu
               180                 185                 190
   Ile Thr Ser Val Arg Leu Thr Gly Ala Ser Gly Ser Thr Ser Val Asp
           195                 200                 205
   Leu Ala Ala Gly Trp Leu Ala Arg Arg Leu Lys Val Pro Val Ile Arg
       210                 215                 220
   Glu Val Thr Asp Ala Pro Thr Val Pro Thr Asp Glu Phe Gly Thr Pro
   225                 230                 235                 240
   Leu Leu Ala Ile Gln Arg Leu Glu Ile Val Arg Thr Thr Gly Ser Ile
                   245                 250                 255
   Ile Ile Thr Ile Tyr Asp Ala His Thr Leu Gln Val Glu Met Pro Glu
               260                 265                 270
   Ser Gly Asn Ala Pro Ser Leu Val Ala Ile Gly Arg Arg Ser Glu Ser
           275                 280                 285
   Asp Cys Leu Ser Glu Glu Leu Arg His Met Asp Pro Asp Leu Gly Tyr
       290                 295                 300
   Gln His Ala Leu Ser Gly Leu Ser Ser Val Lys Leu Glu Thr Val
   305 310 315
<210> 81
   <211> 2479
   <212> DNA
   <213> Corynebacterium glutamicum
<220>
   <221> CDS
   <222> (101)..(2449)
   <223> RXA02742
<400> 81
   gtatcggtaa aaggcccaat caaggattct tggttgggtc tttttccatg cttttcatgc 60
   ttcgaacagt ccagaatagc cctgacctag acttagagct atg tcc gaa caa aga   115
                                              Met Ser Glu Gln Arg
                                              1               5
   ctc gat cag ctt gag cga cgg ctt tct gaa ctg gaa cgg gag atc gcc   163
   Leu Asp Gln Leu Glu Arg Arg Leu Ser Glu Leu Glu Arg Glu Ile Ala
                   10                  15                  20
   gcg att cgt cag gag atc cgc cag gaa cgc cta gtg ctt ccg gaa ccg   211
   Ala Ile Arg Gln Glu Ile Arg Gln Glu Arg Leu Val Leu Pro Glu Pro
               25                  30                  35
   gaa cct gtg aaa gtt gat aca gtc atc gcc acc gaa gcg acc gga gtc   259
   Glu Pro Val Lys Val Asp Thr Val Ile Ala Thr Glu Ala Thr Gly Val
           40                  45                  50
   aat gca tcg tcg ggt ccg gag gcg aag atc gct ttg ttc atg gag agg   307
   Asn Ala Ser Ser Gly Pro Glu Ala Lys Ile Ala Leu Phe Met Glu Arg
       55                  60                  65
   ttt agt ggt cgc cac gat gtg tat gcg cgg cgc tgg acc agc aga aaa   355
   Phe Ser Gly Arg His Asp Val Tyr Ala Arg Arg Trp Thr Ser Arg Lys
   70                  75                  80                  85
   acg ggc aaa agt gga tgg tcg ccg gct act cgc cag ggt ttt tac tca   403
   Thr Gly Lys Ser Gly Trp Ser Pro Ala Thr Arg Gln Gly Phe Tyr Ser
                   90                  95                  100
   aaa gac acc aca ccg aag gac tat ctc ccc ttc acc gtt gac acc gtc   451
   Lys Asp Thr Thr Pro Lys Asp Tyr Leu Pro Phe Thr Val Asp Thr Val
               105                 110                 115
   aat gcg cat ctg cgc cgg ggc ggc gac cat atc ggt ctc tat gtg atg   499
   Asn Ala His Leu Arg Arg Gly Gly Asp His Ile Gly Leu Tyr Val Met
           120                 125                 130
   gtc ccc atc gac acg tgc aaa ctt ctc gcc tgc gat ttc gac gat ggc   547
   Val Pro Ile Asp Thr Cys Lys Leu Leu Ala Cys Asp Phe Asp Asp Gly
       135                 140                 145
   acc tgg aag caa gat gcg gcc gct ttc gtg tca gcc tgc acc gac cac   595
   Thr Trp Lys Gln Asp Ala Ala Ala Phe Val Ser Ala Cys Thr Asp His
   150                 155                 160                 165
   gga atc gat gcg ttg gct gaa att tct cga tcc gac gac ggc gcc ccc   643
   Gly Ile Asp Ala Leu Ala Glu Ile Ser Arg Ser Asp Asp Gly Ala Pro
                   170                 175                 180
   gtg tgg ata ttt ttc gat acc cca atc tcc gcg atg ctg gct cgg cgc     691
   Val Trp Ile Phe Phe Asp Thr Pro Ile Ser Ala Met Leu Ala Arg Arg
               185                 190                 195
   cta ggt ttt gcc atg ctc cgc caa gcc atg aac tcc cgc cct gac atg     739
   Leu Gly Phe Ala Met Leu Arg Gln Ala Met Asn Ser Arg Pro Asp Met
           200                 205                 210
   gat atg tct tct tat gat cgc ttc ttc cct gct caa gac acc atc gca     787
   Asp Met Ser Ser Tyr Asp Arg Phe Phe Pro Ala Gln Asp Thr Ile Ala
       215                 220                 225
   acg cgc gca aac gga agc gca cgg ctg gga aat ttg atc gcg ctg ccc     835
   Thr Arg Ala Asn Gly Ser Ala Arg Leu Gly Asn Leu Ile Ala Leu Pro
   230                 235                 240                 245
   ctc aac ggc gac tgt cga gcc cgc aac acc gcc gtc ttc gcc gat tcg     883
   Leu Asn Gly Asp Cys Arg Ala Arg Asn Thr Ala Val Phe Ala Asp Ser
                   250                 255                 260
   gaa acg tgg gtt ccc ttc gaa gat cct ttc gca gcg ctc gcg gcc atc     931
   Glu Thr Trp Val Pro Phe Glu Asp Pro Phe Ala Ala Leu Ala Ala Ile
               265                 270                 275
   acg cca cta gcc acc gaa aaa atc gag cag atc ctt gcc acc acg cag     979
   Thr Pro Leu Ala Thr Glu Lys Ile Glu Gln Ile Leu Ala Thr Thr Gln
           280                 285                 290
   gaa aaa ttt ggc ccc gaa ccc gaa cac atc aaa cgc ccc acc cgc gcc    1027
   Glu Lys Phe Gly Pro Glu Pro Glu His Ile Lys Arg Pro Thr Arg Ala
       295                 300                 305
   gaa ctc aaa cag gtt aaa gcc aac ggc gaa acc atc aaa ctc acc atc    1075
   Glu Leu Lys Gln Val Lys Ala Asn Gly Glu Thr Ile Lys Leu Thr Ile
   310                 315                 320                 325
   acc aac gag ctg agc gtc ccc acc gaa agg tta ccc gcg gcc gtc atc    1123
   Thr Asn Glu Leu Ser Val Pro Thr Glu Arg Leu Pro Ala Ala Val Ile
                   330                 335                 340
   gcg gag att aaa cac cgg gcg gta atc cca aac cct gag ttt tat cgt    1171
   Ala Glu Ile Lys His Arg Ala Val Ile Pro Asn Pro Glu Phe Tyr Arg
               345                 350                 355
   cga caa gcg caa aga ttt tcg acc ttc ggc gtg ccg cgc atc gtc atc    1219
   Arg Gln Ala Gln Arg Phe Ser Thr Phe Gly Val Pro Arg Ile Val Ile
           360                 365                 370
   cgc ttc gcc cag gcc gag cag cgc ttg ctg ctc cca cgc ggg ctt gtc    1267
   Arg Phe Ala Gln Ala Glu Gln Arg Leu Leu Leu Pro Arg Gly Leu Val
       375                 380                 385
   gac gac acc ctc cgg atc ctc acc ctc gcc ggg tac aaa gtc agc gtc    1315
   Asp Asp Thr Leu Arg Ile Leu Thr Leu Ala Gly Tyr Lys Val Ser Val
   390                 395                 400                 405
   atc tgg cct cgg caa act cgg aaa acc atc gac gcg tct ttc gag ggc    1363
   Ile Trp Pro Arg Gln Thr Arg Lys Thr Ile Asp Ala Ser Phe Glu Gly
                   410                 415                 420
   gaa ttg cga tcc atg caa caa gag gga atc gac tcg ctc aaa ggc caa    1411
   Glu Leu Arg Ser Met Gln Gln Glu Gly Ile Asp Ser Leu Lys Gly Gln
   425 430 435
   cgc acc ggc gta ttg gta gca ccg ccg ggc gct gga aaa aca gtg atg 1459
   Arg Thr Gly Val Leu Val Ala Pro Pro Gly Ala Gly Lys Thr Val Met
   440 445 450
   gcc tgt gca ctc atc gcg aac aga aaa atc ccc acc gca gtg ata gtc 1507
   Ala Cys Ala Leu Ile Ala Asn Arg Lys Ile Pro Thr Ala Val Ile Val
   455 460 465
   aac cgt gca gaa ttg att tcc caa tgg cgg gat cgt ctc gcg caa tac 1555
   Asn Arg Ala Glu Leu Ile Ser Gln Trp Arg Asp Arg Leu Ala Gln Tyr
   470 475 480 485
   ctg agc atc gac gca gac tcc atc gga cag atc ggc gcg ggc cga cgc 1603
   Leu Ser Ile Asp Ala Asp Ser Ile Gly Gln Ile Gly Ala Gly Arg Arg
   490 495 500
   aaa acc acc gga att atc gat ctc atc acc gtc caa tcc ttg agc cgt 1651
   Lys Thr Thr Gly Ile Ile Asp Leu Ile Thr Val Gln Ser Leu Ser Arg
   505 510 515
   aaa gat tcc gat ccg aaa att ttg gaa caa tac ggc caa atc atc gtc 1699
   Lys Asp Ser Asp Pro Lys Ile Leu Glu Gln Tyr Gly Gln Ile Ile Val
   520 525 530
   gac gag tgc cac aac atc gca gcc cca ggc gcc gaa gcc gca ttg aac 1747
   Asp Glu Cys His Asn Ile Ala Ala Pro Gly Ala Glu Ala Ala Leu Asn
   535 540 545
   cag gtc aag gcc ccc tac tgg ctg ggt cta acc gcc acg ccg ttt cgt 1795
   Gln Val Lys Ala Pro Tyr Trp Leu Gly Leu Thr Ala Thr Pro Phe Arg
   550 555 560 565
   tca gac cac atg gat gaa atc atc acc atg cag tgc ggt cct gtg cgc 1843
   Ser Asp His Met Asp Glu Ile Ile Thr Met Gln Cys Gly Pro Val Arg
   570 575 580
   cac cgc atg gaa gtg gca aca gac aat gaa cag cgc ttg att cac atc 1891
   His Arg Met Glu Val Ala Thr Asp Asn Glu Gln Arg Leu Ile His Ile
   585 590 595
   cac gaa acc tct ttc gac tct gag gaa acc acc gaa atc cag gat ctc 1939
   His Glu Thr Ser Phe Asp Ser Glu Glu Thr Thr Glu Ile Gln Asp Leu
   600 605 610
   tac aat gag ctc gcg gtc gat tct gcc cga aat gcg caa atc act gcc 1987
   Tyr Asn Glu Leu Ala Val Asp Ser Ala Arg Asn Ala Gln Ile Thr Ala
   615 620 625
   gaa gtg cac aaa gcg ctt gaa gct ggc gac cga tgt cta gtt ttg gtc 2035
   Glu Val His Lys Ala Leu Glu Ala Gly Asp Arg Cys Leu Val Leu Val
   630 635 640 645
   aac cga att gca gcc ctt gaa gca ctg acc agc agt att acc gaa tct 2083
   Asn Arg Ile Ala Ala Leu Glu Ala Leu Thr Ser Ser Ile Thr Glu Ser
   650 655 660
   ggc gat cac act gtc tta gtg atg cat ggc cgc caa acc caa gag gag 2131
   Gly Asp His Thr Val Leu Val Met His Gly Arg Gln Thr Gln Glu Glu
   665 670 675
   cga gtt cac ctt cgt gcg caa ctt gcc tca ttg agt gaa aag cag gat     2179
   Arg Val His Leu Arg Ala Gln Leu Ala Ser Leu Ser Glu Lys Gln Asp
           680                 685                 690
   ccg ttt gta ctg gtc gcg atg aat aaa gtc gcc ggc gaa ggc ctt gac     2227
   Pro Phe Val Leu Val Ala Met Asn Lys Val Ala Gly Glu Gly Leu Asp
       695                 700                 705
   atc ccc agc ctc aac acg ctg ttt ttg gca gcg ccg gtg tcc ttc aag     2275
   Ile Pro Ser Leu Asn Thr Leu Phe Leu Ala Ala Pro Val Ser Phe Lys
   710                 715                 720                 725
   ggg ctg gtg att cag caa atc ggc cga gtt act cgc gca acc ggt gat     2323
   Gly Leu Val Ile Gln Gln Ile Gly Arg Val Thr Arg Ala Thr Gly Asp
                   730                 735                 740
   caa aac gct cct ccg gtg act gcc acg gtc cat gat ttt gtt gat tcc     2371
   Gln Asn Ala Pro Pro Val Thr Ala Thr Val His Asp Phe Val Asp Ser
               745                 750                 755
   aag att ccg aca ctc aaa cgc atg cac ggt cgc cga ttg cgg gct atg     2419
   Lys Ile Pro Thr Leu Lys Arg Met His Gly Arg Arg Leu Arg Ala Met
           760                 765                 770
   caa aag gaa gga ttc gct gtt tcg gag cct tgaggaggac cagaccaaac       2469
   Gln Lys Glu Gly Phe Ala Val Ser Glu Pro
       775                 780
   cagcgtgccc                                                          2479
<210> 82
   <211> 783
   <212> PRT
   <213> Corynebacterium glutamicum
<400> 82
   Met Ser Glu Gln Arg Leu Asp Gln Leu Glu Arg Arg Leu Ser Glu Leu
     1               5                  10                  15
   Glu Arg Glu Ile Ala Ala Ile Arg Gln Glu Ile Arg Gln Glu Arg Leu
                20                  25                  30
   Val Leu Pro Glu Pro Glu Pro Val Lys Val Asp Thr Val Ile Ala Thr
            35                  40                  45
   Glu Ala Thr Gly Val Asn Ala Ser Ser Gly Pro Glu Ala Lys Ile Ala
        50                  55                  60
   Leu Phe Met Glu Arg Phe Ser Gly Arg His Asp Val Tyr Ala Arg Arg
    65                  70                  75                  80
   Trp Thr Ser Arg Lys Thr Gly Lys Ser Gly Trp Ser Pro Ala Thr Arg
                    85                  90                  95
   Gln Gly Phe Tyr Ser Lys Asp Thr Thr Pro Lys Asp Tyr Leu Pro Phe
               100                 105                 110
   Thr Val Asp Thr Val Asn Ala His Leu Arg Arg Gly Gly Asp His Ile
           115                 120                 125
   Gly Leu Tyr Val Met Val Pro Ile Asp Thr Cys Lys Leu Leu Ala Cys
       130                 135                 140
   Asp Phe Asp Asp Gly Thr Trp Lys Gln Asp Ala Ala Ala Phe Val Ser
   145 150 155 160
   Ala Cys Thr Asp His Gly Ile Asp Ala Leu Ala Glu Ile Ser Arg Ser
   165 170 175
   Asp Asp Gly Ala Pro Val Trp Ile Phe Phe Asp Thr Pro Ile Ser Ala
   180 185 190
   Met Leu Ala Arg Arg Leu Gly Phe Ala Met Leu Arg Gln Ala Met Asn
   195 200 205
   Ser Arg Pro Asp Met Asp Met Ser Ser Tyr Asp Arg Phe Phe Pro Ala
   210 215 220
   Gln Asp Thr Ile Ala Thr Arg Ala Asn Gly Ser Ala Arg Leu Gly Asn
   225 230 235 240
   Leu Ile Ala Leu Pro Leu Asn Gly Asp Cys Arg Ala Arg Asn Thr Ala
   245 250 255
   Val Phe Ala Asp Ser Glu Thr Trp Val Pro Phe Glu Asp Pro Phe Ala
   260 265 270
   Ala Leu Ala Ala Ile Thr Pro Leu Ala Thr Glu Lys Ile Glu Gln Ile
   275 280 285
   Leu Ala Thr Thr Gln Glu Lys Phe Gly Pro Glu Pro Glu His Ile Lys
   290 295 300
   Arg Pro Thr Arg Ala Glu Leu Lys Gln Val Lys Ala Asn Gly Glu Thr
   305 310 315 320
   Ile Lys Leu Thr Ile Thr Asn Glu Leu Ser Val Pro Thr Glu Arg Leu
   325 330 335
   Pro Ala Ala Val Ile Ala Glu Ile Lys His Arg Ala Val Ile Pro Asn
   340 345 350
   Pro Glu Phe Tyr Arg Arg Gln Ala Gln Arg Phe Ser Thr Phe Gly Val
   355 360 365
   Pro Arg Ile Val Ile Arg Phe Ala Gln Ala Glu Gln Arg Leu Leu Leu
   370 375 380
   Pro Arg Gly Leu Val Asp Asp Thr Leu Arg Ile Leu Thr Leu Ala Gly
   385 390 395 400
   Tyr Lys Val Ser Val Ile Trp Pro Arg Gln Thr Arg Lys Thr Ile Asp
   405 410 415
   Ala Ser Phe Glu Gly Glu Leu Arg Ser Met Gln Gln Glu Gly Ile Asp
   420 425 430
   Ser Leu Lys Gly Gln Arg Thr Gly Val Leu Val Ala Pro Pro Gly Ala
   435 440 445
   Gly Lys Thr Val Met Ala Cys Ala Leu Ile Ala Asn Arg Lys Ile Pro
   450 455 460
   Thr Ala Val Ile Val Asn Arg Ala Glu Leu Ile Ser Gln Trp Arg Asp
   465 470 475 480
   Arg Leu Ala Gln Tyr Leu Ser Ile Asp Ala Asp Ser Ile Gly Gln Ile
   485 490 495
   Gly Ala Gly Arg Arg Lys Thr Thr Gly Ile Ile Asp Leu Ile Thr Val
   500 505 510
   Gln Ser Leu Ser Arg Lys Asp Ser Asp Pro Lys Ile Leu Glu Gln Tyr
   515 520 525
   Gly Gln Ile Ile Val Asp Glu Cys His Asn Ile Ala Ala Pro Gly Ala
   530 535 540
   Glu Ala Ala Leu Asn Gln Val Lys Ala Pro Tyr Trp Leu Gly Leu Thr
   545 550 555 560
   Ala Thr Pro Phe Arg Ser Asp His Met Asp Glu Ile Ile Thr Met Gln
   565 570 575
   Cys Gly Pro Val Arg His Arg Met Glu Val Ala Thr Asp Asn Glu Gln
   580 585 590
   Arg Leu Ile His Ile His Glu Thr Ser Phe Asp Ser Glu Glu Thr Thr
   595 600 605
   Glu Ile Gln Asp Leu Tyr Asn Glu Leu Ala Val Asp Ser Ala Arg Asn
   610 615 620
   Ala Gln Ile Thr Ala Glu Val His Lys Ala Leu Glu Ala Gly Asp Arg
   625 630 635 640
   Cys Leu Val Leu Val Asn Arg Ile Ala Ala Leu Glu Ala Leu Thr Ser
   645 650 655
   Ser Ile Thr Glu Ser Gly Asp His Thr Val Leu Val Met His Gly Arg
   660 665 670
   Gln Thr Gln Glu Glu Arg Val His Leu Arg Ala Gln Leu Ala Ser Leu
   675 680 685
   Ser Glu Lys Gln Asp Pro Phe Val Leu Val Ala Met Asn Lys Val Ala
   690 695 700
   Gly Glu Gly Leu Asp Ile Pro Ser Leu Asn Thr Leu Phe Leu Ala Ala
   705 710 715 720
   Pro Val Ser Phe Lys Gly Leu Val Ile Gln Gln Ile Gly Arg Val Thr
   725 730 735
   Arg Ala Thr Gly Asp Gln Asn Ala Pro Pro Val Thr Ala Thr Val His
   740 745 750
   Asp Phe Val Asp Ser Lys Ile Pro Thr Leu Lys Arg Met His Gly Arg
   755 760 765
   Arg Leu Arg Ala Met Gln Lys Glu Gly Phe Ala Val Ser Glu Pro
   770 775 780
<210> 83
   <211> 1771
   <212> DNA
   <213> Corynebacterium glutamicum
<220>
   <221> CDS
   <222> (101)..(1741)
   <223> RXA02748
<400> 83
   caggtcgttg gcaggtagct gacgtgacct gagcgggggc aaccggctaa catgtaggga 60
   ttagcaaccc cgcactctag aacctactgg gagttcattc gtg ttt gag tca ctg   115
                                               Val Phe Glu Ser Leu
                                               1               5
   tcc gat cgg ttg aat agc gcg ctt tcc ggc ctg cgc ggc aaa gga aag   163
   Ser Asp Arg Leu Asn Ser Ala Leu Ser Gly Leu Arg Gly Lys Gly Lys
                   10                  15                  20
   ctc acc gag gca gac atc aat gca acc aca cgc gag atc cgt ctc gcg   211
   Leu Thr Glu Ala Asp Ile Asn Ala Thr Thr Arg Glu Ile Arg Leu Ala
               25                  30                  35
   ctg ctg gaa gct gac gtt tca tta acg gtt gtt cgt gcc ttc att aac   259
   Leu Leu Glu Ala Asp Val Ser Leu Thr Val Val Arg Ala Phe Ile Asn
           40                  45                  50
   cga atc aag gaa cgc gcc gct ggt gca gaa gtt tct cag gca ctc aac   307
   Arg Ile Lys Glu Arg Ala Ala Gly Ala Glu Val Ser Gln Ala Leu Asn
       55                  60                  65
   ccc gcg cag caa gtc atc aag atc gtc aac gag gaa ctg gtt cag atc   355
   Pro Ala Gln Gln Val Ile Lys Ile Val Asn Glu Glu Leu Val Gln Ile
   70                  75                  80                  85
   ctc ggt ggc gaa acc cgc cga ctg tca ctg gcc aaa aac cca ccg acc   403
   Leu Gly Gly Glu Thr Arg Arg Leu Ser Leu Ala Lys Asn Pro Pro Thr
                   90                  95                  100
   gtc atc atg ctg gca ggt ctg cag ggt gca ggt aag acc acc ctc gca   451
   Val Ile Met Leu Ala Gly Leu Gln Gly Ala Gly Lys Thr Thr Leu Ala
               105                 110                 115
   ggt aaa ctg tcc aag cac ctg gtc aag cag ggt cac act cct atg ctt   499
   Gly Lys Leu Ser Lys His Leu Val Lys Gln Gly His Thr Pro Met Leu
           120                 125                 130
   gtt gcc tgt gac ctt cag cgt cca ggc gca gtt cag cag ctg caa att   547
   Val Ala Cys Asp Leu Gln Arg Pro Gly Ala Val Gln Gln Leu Gln Ile
       135                 140                 145
   gtg ggt gaa cgc gca ggc gtt acc act ttc gca ccg gat cca ggc acc   595
   Val Gly Glu Arg Ala Gly Val Thr Thr Phe Ala Pro Asp Pro Gly Thr
   150                 155                 160                 165
   agc atc gac tcc ctc gag cac gaa atg ggc acc tcc cac ggt gat cca   643
   Ser Ile Asp Ser Leu Glu His Glu Met Gly Thr Ser His Gly Asp Pro
                   170                 175                 180
   gtc gag gta gcg cgc gca ggt atc gaa gaa gcc aag cgc acc cag cac   691
   Val Glu Val Ala Arg Ala Gly Ile Glu Glu Ala Lys Arg Thr Gln His
               185                 190                 195
   gac atc gtg atc gtg gat acc gca ggt cgc ctc ggt atc gat gaa acc   739
   Asp Ile Val Ile Val Asp Thr Ala Gly Arg Leu Gly Ile Asp Glu Thr
           200                 205                 210
   ctg atg act cag gca cgc aac atc cgc gaa gcc atc aac cct gat gaa   787
   Leu Met Thr Gln Ala Arg Asn Ile Arg Glu Ala Ile Asn Pro Asp Glu
       215                 220                 225
   gtg ctc ttt gtc att gac tcc atg att ggt caa gac gcc gta gac acc     835
   Val Leu Phe Val Ile Asp Ser Met Ile Gly Gln Asp Ala Val Asp Thr
   230                 235                 240                 245
   gcc gaa gca ttc cgc gac ggc gtc gac ttc acc ggt gtt gtc ctg acc     883
   Ala Glu Ala Phe Arg Asp Gly Val Asp Phe Thr Gly Val Val Leu Thr
                   250                 255                 260
   aag ctt gat ggc gac gcc cgc ggt ggt gct gca cta tcc atc cgt gaa     931
   Lys Leu Asp Gly Asp Ala Arg Gly Gly Ala Ala Leu Ser Ile Arg Glu
               265                 270                 275
   gtc acc ggc aag ccc atc atg ttt gcc tcc act ggt gaa aaa ctc gac     979
   Val Thr Gly Lys Pro Ile Met Phe Ala Ser Thr Gly Glu Lys Leu Asp
           280                 285                 290
   gac ttc gac gtc ttc cac cca gag cgc atg gcc agc cga atc ctg ggc     1027
   Asp Phe Asp Val Phe His Pro Glu Arg Met Ala Ser Arg Ile Leu Gly
       295                 300                 305
   atg ggt gac gta ctg tca ctc atc gag cag gcc gaa gca gtc atg gat     1075
   Met Gly Asp Val Leu Ser Leu Ile Glu Gln Ala Glu Ala Val Met Asp
   310                 315                 320                 325
   cag gaa aag gca gag gtc gct gcc cag aag ttg ggc tcc ggc gag ctc     1123
   Gln Glu Lys Ala Glu Val Ala Ala Gln Lys Leu Gly Ser Gly Glu Leu
                   330                 335                 340
   acc ctg gaa gac ttc ctt gac caa atg ctg atg atc cgc cgc atg gga     1171
   Thr Leu Glu Asp Phe Leu Asp Gln Met Leu Met Ile Arg Arg Met Gly
               345                 350                 355
   cca atc ggc aac atc ctc aag atg ctg cct ggt ggc aag cag atg tcc     1219
   Pro Ile Gly Asn Ile Leu Lys Met Leu Pro Gly Gly Lys Gln Met Ser
           360                 365                 370
   caa atg gcg gac atg gtt gat gag aag caa ctc gac cgc atc cag gcg     1267
   Gln Met Ala Asp Met Val Asp Glu Lys Gln Leu Asp Arg Ile Gln Ala
       375                 380                 385
   att atc cgc ggt atg acc ccg gcc gag cgc gat aat cca aag atc ctc     1315
   Ile Ile Arg Gly Met Thr Pro Ala Glu Arg Asp Asn Pro Lys Ile Leu
   390                 395                 400                 405
   aac gct tcc agg cgc aag cgc atc gcc aac ggt tcc ggt gtg acc gtg     1363
   Asn Ala Ser Arg Arg Lys Arg Ile Ala Asn Gly Ser Gly Val Thr Val
                   410                 415                 420
   tcc gaa gta aac aaa ctt gtt gaa cgc ttc ttc gag gct cgc aag atg     1411
   Ser Glu Val Asn Lys Leu Val Glu Arg Phe Phe Glu Ala Arg Lys Met
               425                 430                 435
   atg ggt caa atg gct ggc cag ttt ggc atg ggt cct gga tcc cgc agt     1459
   Met Gly Gln Met Ala Gly Gln Phe Gly Met Gly Pro Gly Ser Arg Ser
           440                 445                 450
   gca acc aag aag caa gcc aag ggc cgc aag ggt aag aac ggc aag cgt     1507
   Ala Thr Lys Lys Gln Ala Lys Gly Arg Lys Gly Lys Asn Gly Lys Arg
       455                 460                 465
   aaa cca gcc aag aag ggc cca acc cag cca aag atg cca atg ggc ggt    1555
   Lys Pro Ala Lys Lys Gly Pro Thr Gln Pro Lys Met Pro Met Gly Gly
   470                 475                 480                 485
   atg cca gga atg cct ggg atg ccg ggt atg ggt gga gcc gga atg cct    1603
   Met Pro Gly Met Pro Gly Met Pro Gly Met Gly Gly Ala Gly Met Pro
                   490                 495                 500
   gac ctt gct gaa cta cag aag cag ctt ggt gga gca ggt ggc ggt atg    1651
   Asp Leu Ala Glu Leu Gln Lys Gln Leu Gly Gly Ala Gly Gly Gly Met
               505                 510                 515
   gga ggc ctt ggt ggc gga ctc ccg ggc atg cca aag ccg cct aaa ggc    1699
   Gly Gly Leu Gly Gly Gly Leu Pro Gly Met Pro Lys Pro Pro Lys Gly
           520                 525                 530
   atg gag aac ata gat ctc aac aac cta gac ttc ggt aag aag            1741
   Met Glu Asn Ile Asp Leu Asn Asn Leu Asp Phe Gly Lys Lys
       535                 540                 545
   taactttgct ttagttggtc ggcgcatcac                                   1771
<210> 84
   <211> 547
   <212> PRT
   <213> Corynebacterium glutamicum
<400> 84
   Val Phe Glu Ser Leu Ser Asp Arg Leu Asn Ser Ala Leu Ser Gly Leu
   1 5 10 15
   Arg Gly Lys Gly Lys Leu Thr Glu Ala Asp Ile Asn Ala Thr Thr Arg
   20 25 30
   Glu Ile Arg Leu Ala Leu Leu Glu Ala Asp Val Ser Leu Thr Val Val
   35 40 45
   Arg Ala Phe Ile Asn Arg Ile Lys Glu Arg Ala Ala Gly Ala Glu Val
   50 55 60
   Ser Gln Ala Leu Asn Pro Ala Gln Gln Val Ile Lys Ile Val Asn Glu
   65 70 75 80
   Glu Leu Val Gln Ile Leu Gly Gly Glu Thr Arg Arg Leu Ser Leu Ala
   85 90 95
   Lys Asn Pro Pro Thr Val Ile Met Leu Ala Gly Leu Gln Gly Ala Gly
   100 105 110
   Lys Thr Thr Leu Ala Gly Lys Leu Ser Lys His Leu Val Lys Gln Gly
   115 120 125
   His Thr Pro Met Leu Val Ala Cys Asp Leu Gln Arg Pro Gly Ala Val
   130 135 140
   Gln Gln Leu Gln Ile Val Gly Glu Arg Ala Gly Val Thr Thr Phe Ala
   145 150 155 160
   Pro Asp Pro Gly Thr Ser Ile Asp Ser Leu Glu His Glu Met Gly Thr
   165 170 175
   Ser His Gly Asp Pro Val Glu Val Ala Arg Ala Gly Ile Glu Glu Ala
   180 185 190
   Lys Arg Thr Gln His Asp Ile Val Ile Val Asp Thr Ala Gly Arg Leu
   195 200 205
   Gly Ile Asp Glu Thr Leu Met Thr Gln Ala Arg Asn Ile Arg Glu Ala
   210 215 220
   Ile Asn Pro Asp Glu Val Leu Phe Val Ile Asp Ser Met Ile Gly Gln
   225 230 235 240
   Asp Ala Val Asp Thr Ala Glu Ala Phe Arg Asp Gly Val Asp Phe Thr
   245 250 255
   Gly Val Val Leu Thr Lys Leu Asp Gly Asp Ala Arg Gly Gly Ala Ala
   260 265 270
   Leu Ser Ile Arg Glu Val Thr Gly Lys Pro Ile Met Phe Ala Ser Thr
   275 280 285
   Gly Glu Lys Leu Asp Asp Phe Asp Val Phe His Pro Glu Arg Met Ala
   290 295 300
   Ser Arg Ile Leu Gly Met Gly Asp Val Leu Ser Leu Ile Glu Gln Ala
   305 310 315 320
   Glu Ala Val Met Asp Gln Glu Lys Ala Glu Val Ala Ala Gln Lys Leu
   325 330 335
   Gly Ser Gly Glu Leu Thr Leu Glu Asp Phe Leu Asp Gln Met Leu Met
   340 345 350
   Ile Arg Arg Met Gly Pro Ile Gly Asn Ile Leu Lys Met Leu Pro Gly
   355 360 365
   Gly Lys Gln Met Ser Gln Met Ala Asp Met Val Asp Glu Lys Gln Leu
   370 375 380
   Asp Arg Ile Gln Ala Ile Ile Arg Gly Met Thr Pro Ala Glu Arg Asp
   385 390 395 400
   Asn Pro Lys Ile Leu Asn Ala Ser Arg Arg Lys Arg Ile Ala Asn Gly
   405 410 415
   Ser Gly Val Thr Val Ser Glu Val Asn Lys Leu Val Glu Arg Phe Phe
   420 425 430
   Glu Ala Arg Lys Met Met Gly Gln Met Ala Gly Gln Phe Gly Met Gly
   435 440 445
   Pro Gly Ser Arg Ser Ala Thr Lys Lys Gln Ala Lys Gly Arg Lys Gly
   450 455 460
   Lys Asn Gly Lys Arg Lys Pro Ala Lys Lys Gly Pro Thr Gln Pro Lys
   465 470 475 480
   Met Pro Met Gly Gly Met Pro Gly Met Pro Gly Met Pro Gly Met Gly
   485 490 495
   Gly Ala Gly Met Pro Asp Leu Ala Glu Leu Gln Lys Gln Leu Gly Gly
   500 505 510
   Ala Gly Gly Gly Met Gly Gly Leu Gly Gly Gly Leu Pro Gly Met Pro
   515 520 525
   Lys Pro Pro Lys Gly Met Glu Asn Ile Asp Leu Asn Asn Leu Asp Phe
   530 535 540
   Gly Lys Lys
   545
<210> 85
   <211> 958
   <212> DNA
   <213> Corynebacterium glutamicum
<220>
   <221> CDS
   <222> (101)..(928)
   <223> RXA03070
<400> 85
   gtggataaaa gggaaaacat aggggtcatg aaatagaaca agcacgaggc ctggtaaata 60
   cgaattcgac caagaaaacg taaacacccc aggagtactc gtg cct gcc ctt cca    115
                                             Val Pro Ala Leu Pro
                                             1               5
   tca tct atc atc gac ccc ctc tgg cgc cag ttc tcc gcc tta atc cca    163
   Ser Ser Ile Ile Asp Pro Leu Trp Arg Gln Phe Ser Ala Leu Ile Pro
                   10                  15                  20
   ccg gtt atc atc acc cac cca cta ggg tgc cac cgt gca cgc att gct    211
   Pro Val Ile Ile Thr His Pro Leu Gly Cys His Arg Ala Arg Ile Ala
               25                  30                  35
   gac cgg atc atc gtc gac aaa ctc atc gca gtg ctt gtc ctc ggt gtc    259
   Asp Arg Ile Ile Val Asp Lys Leu Ile Ala Val Leu Val Leu Gly Val
           40                  45                  50
   tcc tat atc aag att tcc gat tcc acc tgc tca gcc acc acg ata cgc    307
   Ser Tyr Ile Lys Ile Ser Asp Ser Thr Cys Ser Ala Thr Thr Ile Arg
       55                  60                  65
   acc cgc cga gac gag tgg atc act gcc ggg att ttc aag aat tta gaa    355
   Thr Arg Arg Asp Glu Trp Ile Thr Ala Gly Ile Phe Lys Asn Leu Glu
   70                  75                  80                  85
   cag atc tgt ctg gag tcc tac gac cgt ttc atc ggg tta gac cta gaa    403
   Gln Ile Cys Leu Glu Ser Tyr Asp Arg Phe Ile Gly Leu Asp Leu Glu
                   90                  95                  100
   aac tta aat gtt gat ggc tgc att gtt aaa gct ccc tgc ggc gga gag    451
   Asn Leu Asn Val Asp Gly Cys Ile Val Lys Ala Pro Cys Gly Gly Glu
               105                 110                 115
   gta gcc ggc aga ttc ccg gtt gac cgg gaa aaa ggc acc aaa cgc tcg    499
   Val Ala Gly Arg Phe Pro Val Asp Arg Glu Lys Gly Thr Lys Arg Ser
           120                 125                 130
   tta atg gtc gat gga cat gga atc ccg atc ggg tgc gtg gtc gcc gga    547
   Leu Met Val Asp Gly His Gly Ile Pro Ile Gly Cys Val Val Ala Gly
       135                 140                 145
   gcc aat cgg cat gat tta ccg ttg tta gct gca acc ttg gac acg ctc    595
   Ala Asn Arg His Asp Leu Pro Leu Leu Ala Ala Thr Leu Asp Thr Leu
   150                 155                 160                 165
   ggc cgg ttt ggg ggc tct ctt ccc gat cag atc acg gtg cat ctc gat     643
   Gly Arg Phe Gly Gly Ser Leu Pro Asp Gln Ile Thr Val His Leu Asp
                   170                 175                 180
   gct ggg tat gac tcg aag aaa acc cgc agg cta ctc agc gaa ttt ggt     691
   Ala Gly Tyr Asp Ser Lys Lys Thr Arg Arg Leu Leu Ser Glu Phe Gly
               185                 190                 195
   tat agc tgg gtg atc agc att aaa ggt gag ccg ctg cag gct ggg act     739
   Tyr Ser Trp Val Ile Ser Ile Lys Gly Glu Pro Leu Gln Ala Gly Thr
           200                 205                 210
   cgg tgg gtg gtg gag cgt act aac tct tgg cat aac cgg ggt ttt aag     787
   Arg Trp Val Val Glu Arg Thr Asn Ser Trp His Asn Arg Gly Phe Lys
       215                 220                 225
   aaa ctt agt atc tgc acc gaa cgt tgt acc cgg gtt gtg gaa gcg ttt     835
   Lys Leu Ser Ile Cys Thr Glu Arg Cys Thr Arg Val Val Glu Ala Phe
   230                 235                 240                 245
   atc gct tta gcc aac gcg gtg att att ctg cgt cgg ctt atc aaa cag     883
   Ile Ala Leu Ala Asn Ala Val Ile Ile Leu Arg Arg Leu Ile Lys Gln
                   250                 255                 260
   gcc tgg act agt tac cgc tgg gac acc cga ccg ggc cac aga cct         928
   Ala Trp Thr Ser Tyr Arg Trp Asp Thr Arg Pro Gly His Arg Pro
               265                 270                 275
   taatctatcc gcgcaatctc taaggagaaa                                    958
<210> 86
   <211> 276
   <212> PRT
   <213> Corynebacterium glutamicum
<400> 86
   Val Pro Ala Leu Pro Ser Ser Ile Ile Asp Pro Leu Trp Arg Gln Phe
   1               5                   10                  15
   Ser Ala Leu Ile Pro Pro Val Ile Ile Thr His Pro Leu Gly Cys His
               20                  25                  30
   Arg Ala Arg Ile Ala Asp Arg Ile Ile Val Asp Lys Leu Ile Ala Val
           35                  40                  45
   Leu Val Leu Gly Val Ser Tyr Ile Lys Ile Ser Asp Ser Thr Cys Ser
       50                  55                  60
   Ala Thr Thr Ile Arg Thr Arg Arg Asp Glu Trp Ile Thr Ala Gly Ile
   65                  70                  75                  80
   Phe Lys Asn Leu Glu Gln Ile Cys Leu Glu Ser Tyr Asp Arg Phe Ile
                   85                  90                  95
   Gly Leu Asp Leu Glu Asn Leu Asn Val Asp Gly Cys Ile Val Lys Ala
               100                 105                 110
   Pro Cys Gly Gly Glu Val Ala Gly Arg Phe Pro Val Asp Arg Glu Lys
           115                 120                 125
   Gly Thr Lys Arg Ser Leu Met Val Asp Gly His Gly Ile Pro Ile Gly
       130                 135                 140
   Cys Val Val Ala Gly Ala Asn Arg His Asp Leu Pro Leu Leu Ala Ala
   145                 150                 155                 160
   Thr Leu Asp Thr Leu Gly Arg Phe Gly Gly Ser Leu Pro Asp Gln Ile
                   165                 170                 175
   Thr Val His Leu Asp Ala Gly Tyr Asp Ser Lys Lys Thr Arg Arg Leu
               180                 185                 190
   Leu Ser Glu Phe Gly Tyr Ser Trp Val Ile Ser Ile Lys Gly Glu Pro
           195                 200                 205
   Leu Gln Ala Gly Thr Arg Trp Val Val Glu Arg Thr Asn Ser Trp His
       210                 215                 220
   Asn Arg Gly Phe Lys Lys Leu Ser Ile Cys Thr Glu Arg Cys Thr Arg
   225                 230                 235                 240
   Val Val Glu Ala Phe Ile Ala Leu Ala Asn Ala Val Ile Ile Leu Arg
                   245                 250                 255
   Arg Leu Ile Lys Gln Ala Trp Thr Ser Tyr Arg Trp Asp Thr Arg Pro
               260                 265                 270
   Gly His Arg Pro
           275
<210> 87
   <211> 754
   <212> DNA
   <213> Corynebacterium glutamicum
<220>
   <221> CDS
   <222> (101)..(724)
   <223> RXA03098
<400> 87
   gaccgttttg tcgatcgcac cgctgctggc tcgcaccatc aacgagatct tcgaaaacgg 60
   ttccgtcacc accctcttcg agggcgaggc ctaaacaccc atg ccc acc acg gac   115
                                            Met Pro Thr Thr Asp
                                             1               5
   gtc ttc aac cgc gtc cgg ttg gca ttg gaa cct cta gct gat ccc gca   163
   Val Phe Asn Arg Val Arg Leu Ala Leu Glu Pro Leu Ala Asp Pro Ala
                   10                  15                  20
   cgt gcc acc gga atg gca agc tac atg cgg gat cag ttt tct ttt ctc   211
   Arg Ala Thr Gly Met Ala Ser Tyr Met Arg Asp Gln Phe Ser Phe Leu
               25                  30                  35
   ggc atc cca tcc acc ccc aga aaa gaa gcc tgc aaa ccc gtg ctg tcc   259
   Gly Ile Pro Ser Thr Pro Arg Lys Glu Ala Cys Lys Pro Val Leu Ser
           40                  45                  50
   gcg cta aaa gag ttg gac act gac ttt gtc tca gac tgc ttt ggc gca   307
   Ala Leu Lys Glu Leu Asp Thr Asp Phe Val Ser Asp Cys Phe Gly Ala
       55                  60                  65
   gct gaa cgg gaa tac cag tat gtc gcc tgc gat cac atc aat cgc gtc   355
   Ala Glu Arg Glu Tyr Gln Tyr Val Ala Cys Asp His Ile Asn Arg Val
   70                  75                  80                  85
   ggc atc acc gat cta ggt ttt gcc aaa gca tta gtg cag acc aaa tcc    403
   Gly Ile Thr Asp Leu Gly Phe Ala Lys Ala Leu Val Gln Thr Lys Ser
                    90                  95                  100
   tgg tgg gac acc gtc gat tcc cta gca aaa ccg atc ggc gcc aaa cac    451
   Trp Trp Asp Thr Val Asp Ser Leu Ala Lys Pro Ile Gly Ala Lys His
               105                 110                 115
   gat gat gat ctg atg aaa acg tgg gcg ctt gat gag gac ttc tgg gtg    499
   Asp Asp Asp Leu Met Lys Thr Trp Ala Leu Asp Glu Asp Phe Trp Val
           120                 125                 130
   cgc cgc atc gcg atc atc cac caa ctg ggc cgc aag aaa aac acc gac    547
   Arg Arg Ile Ala Ile Ile His Gln Leu Gly Arg Lys Lys Asn Thr Asp
       135                 140                 145
   gct gcc ctg ctg gcc tgg atc atc gag cag aac ctc ggc tcc agc gag    595
   Ala Ala Leu Leu Ala Trp Ile Ile Glu Gln Asn Leu Gly Ser Ser Glu
   150                 155                 160                 165
   ttc ttc atc aac aaa gcg atc ggc tgg gca ctg cgg gat ttc gcc cgc    643
   Phe Phe Ile Asn Lys Ala Ile Gly Trp Ala Leu Arg Asp Phe Ala Arg
                   170                 175                 180
   cac gac ccc agc tgg gtc cgg gct ttt gtc gac gcc acg gac ctt tcc    691
   His Asp Pro Ser Trp Val Arg Ala Phe Val Asp Ala Thr Asp Leu Ser
               185                 190                 195
   cca ctg agc cgg cga gaa gcc ctg aag aat att tagccctcag gcatcatctg 744
   Pro Leu Ser Arg Arg Glu Ala Leu Lys Asn Ile
           200                 205
   agcgagtgcc                                                         754
<210> 88
   <211> 208
   <212> PRT
   <213> Corynebacterium glutamicum
<400> 88
   Met Pro Thr Thr Asp Val Phe Asn Arg Val Arg Leu Ala Leu Glu Pro
   1               5                   10                  15
   Leu Ala Asp Pro Ala Arg Ala Thr Gly Met Ala Ser Tyr Met Arg Asp
               20                  25                  30
   Gln Phe Ser Phe Leu Gly Ile Pro Ser Thr Pro Arg Lys Glu Ala Cys
           35                  40                  45
   Lys Pro Val Leu Ser Ala Leu Lys Glu Leu Asp Thr Asp Phe Val Ser
       50                  55                  60
   Asp Cys Phe Gly Ala Ala Glu Arg Glu Tyr Gln Tyr Val Ala Cys Asp
   65                  70                  75                  80
   His Ile Asn Arg Val Gly Ile Thr Asp Leu Gly Phe Ala Lys Ala Leu
                   85                  90                  95
   Val Gln Thr Lys Ser Trp Trp Asp Thr Val Asp Ser Leu Ala Lys Pro
               100                 105                 110
   Ile Gly Ala Lys His Asp Asp Asp Leu Met Lys Thr Trp Ala Leu Asp
           115                 120                 125
   Glu Asp Phe Trp Val Arg Arg Ile Ala Ile Ile His Gln Leu Gly Arg
       130                 135                 140
   Lys Lys Asn Thr Asp Ala Ala Leu Leu Ala Trp Ile Ile Glu Gln Asn
   145                 150                 155                 160
   Leu Gly Ser Ser Glu Phe Phe Ile Asn Lys Ala Ile Gly Trp Ala Leu
                   165                 170                 175
   Arg Asp Phe Ala Arg His Asp Pro Ser Trp Val Arg Ala Phe Val Asp
               180                 185                 190
   Ala Thr Asp Leu Ser Pro Leu Ser Arg Arg Glu Ala Leu Lys Asn Ile
           195                 200                 205
<210> 89
   <211> 562
   <212> DNA
   <213> Corynebacterium glutamicum
<220>
   <221> CDS
   <222> (101)..(532)
   <223> RXA03206
<400> 89
   gaatccctgc acaacggcgc cattgcggcg ttggtggatc tcatccgcca cggattggtg 60
   ttgcccgctg atcttctcga ttcttaaata aggactgatt gtg aaa gcc gtt tta   115
                                               Val Lys Ala Val Leu
                                               1               5
   acc cgt gtg agt tcc gcc agc gtc agc gtg gat gat gaa att gtt gga   163
   Thr Arg Val Ser Ser Ala Ser Val Ser Val Asp Asp Glu Ile Val Gly
                   10                  15                  20
   gcc atc gat tgc ccc gac acc gga ggc att ttg gcg ctg gtt gga gtc   211
   Ala Ile Asp Cys Pro Asp Thr Gly Gly Ile Leu Ala Leu Val Gly Val
               25                  30                  35
   ggc gct gct gat agc gac gac gcc tgg gaa acc atg gtg cga aaa att   259
   Gly Ala Ala Asp Ser Asp Asp Ala Trp Glu Thr Met Val Arg Lys Ile
           40                  45                  50
   gct gag ctg cgc atc ttg gat ggc gaa caa tcc gtc agt gat gtc aat   307
   Ala Glu Leu Arg Ile Leu Asp Gly Glu Gln Ser Val Ser Asp Val Asn
       55                  60                  65
   gct ccc gta ctg ctt gtt agc caa ttc acc ctg cat ggt cgc acc gca   355
   Ala Pro Val Leu Leu Val Ser Gln Phe Thr Leu His Gly Arg Thr Ala
   70                  75                  80                  85
   aaa ggc cgg cgc cca tcg tgg tct gat gca gca cct ggt gag gtg gct   403
   Lys Gly Arg Arg Pro Ser Trp Ser Asp Ala Ala Pro Gly Glu Val Ala
                   90                  95                  100
   gag ccg gtg att gaa aag att gca caa ggt tta cgt gag cgc gga atc   451
   Glu Pro Val Ile Glu Lys Ile Ala Gln Gly Leu Arg Glu Arg Gly Ile
               105                 110                 115
   acc gtg gaa caa gga cga ttc ggc gca atg atg aag gtc aca tcg gtt   499
   Thr Val Glu Gln Gly Arg Phe Gly Ala Met Met Lys Val Thr Ser Val
           120                 125                 130
   aac gaa ggc ccc ttc acc gtt ttg gtc gag tgc tagccagtca atcctaagag 552
   Asn Glu Gly Pro Phe Thr Val Leu Val Glu Cys
       135                 140
   cttgaaacgc                                                        562
<210> 90
   <211> 144
   <212> PRT
   <213> Corynebacterium glutamicum
<400> 90
   Val Lys Ala Val Leu Thr Arg Val Ser Ser Ala Ser Val Ser Val Asp
   1 5 10 15
   Asp Glu Ile Val Gly Ala Ile Asp Cys Pro Asp Thr Gly Gly Ile Leu
   20 25 30
   Ala Leu Val Gly Val Gly Ala Ala Asp Ser Asp Asp Ala Trp Glu Thr
   35 40 45
   Met Val Arg Lys Ile Ala Glu Leu Arg Ile Leu Asp Gly Glu Gln Ser
   50 55 60
   Val Ser Asp Val Asn Ala Pro Val Leu Leu Val Ser Gln Phe Thr Leu
   65 70 75 80
   His Gly Arg Thr Ala Lys Gly Arg Arg Pro Ser Trp Ser Asp Ala Ala
   85 90 95
   Pro Gly Glu Val Ala Glu Pro Val Ile Glu Lys Ile Ala Gln Gly Leu
   100 105 110
   Arg Glu Arg Gly Ile Thr Val Glu Gln Gly Arg Phe Gly Ala Met Met
   115 120 125
   Lys Val Thr Ser Val Asn Glu Gly Pro Phe Thr Val Leu Val Glu Cys
   130 135 140
<210> 91
   <211> 607
   <212> DNA
   <213> Corynebacterium glutamicum
<220>
   <221> CDS
   <222> (101)..(577)
   <223> RXA03260
<400> 91
   cagatggctg gcagggatca gctgaccacg ttgctcaacc agcgtggtgt caaagtttct 60
   actgggactg tgggatcaat tatgaacgaa tgaggagtgc gtg tca gac gaa tgc    115
                                               Val Ser Asp Glu Cys
                                               1               5
   ggg cct gga aga aca cca cgg tca gtg acc ctt tct gcc cgg acc gag    163
   Gly Pro Gly Arg Thr Pro Arg Ser Val Thr Leu Ser Ala Arg Thr Glu
                   10                  15                  20
   cat att aaa aat cat atg ctc gat agc cac ggg aaa cga gac ttt acc    211
   His Ile Lys Asn His Met Leu Asp Ser His Gly Lys Arg Asp Phe Thr
               25                  30                  35
   gct acc gtg cct ggg acc agg ctc gtt ggt gac att acg tac tta aag    259
   Ala Thr Val Pro Gly Thr Arg Leu Val Gly Asp Ile Thr Tyr Leu Lys
           40                  45                  50
   acg ggt tcc ggg tgg ctg tat gtg gct acc gtg atc gat ttg gct acg    307
   Thr Gly Ser Gly Trp Leu Tyr Val Ala Thr Val Ile Asp Leu Ala Thr
       55                  60                  65
   cgg atg gtg gtg ggg tgg tct atg gat tct aat atg cgc aca ccg ttg    355
   Arg Met Val Val Gly Trp Ser Met Asp Ser Asn Met Arg Thr Pro Leu
   70                  75                  80                  85
   gtg atc aat gcg ctg gct atg gcg cgt gat cat ggg tgt ctt cat cct    403
   Val Ile Asn Ala Leu Ala Met Ala Arg Asp His Gly Cys Leu His Pro
                   90                  95                  100
   gaa ggc gca att ttt cac tcc gat aga gga tcg caa tac acc tcc gag    451
   Glu Gly Ala Ile Phe His Ser Asp Arg Gly Ser Gln Tyr Thr Ser Glu
               105                 110                 115
   cag ttc cag aca tgg tgc gcc ggc aac aag atc acc caa tcc atg gga    499
   Gln Phe Gln Thr Trp Cys Ala Gly Asn Lys Ile Thr Gln Ser Met Gly
           120                 125                 130
   ttg acc ggg gtg tgt tgg gat aac gga agt cgc gga gaa ttt ttt ctc    547
   Leu Thr Gly Val Cys Trp Asp Asn Gly Ser Arg Gly Glu Phe Phe Leu
       135                 140                 145
   aca ttt gaa gac cga aat gta tca cca cta tgattttgag aatcacctgt      597
   Thr Phe Glu Asp Arg Asn Val Ser Pro Leu
   150                 155
   cggaccgaac                                                         607
<210> 92
   <211> 159
   <212> PRT
   <213> Corynebacterium glutamicum
<400> 92
   Val Ser Asp Glu Cys Gly Pro Gly Arg Thr Pro Arg Ser Val Thr Leu
   1               5                   10                  15
   Ser Ala Arg Thr Glu His Ile Lys Asn His Met Leu Asp Ser His Gly
               20                  25                  30
   Lys Arg Asp Phe Thr Ala Thr Val Pro Gly Thr Arg Leu Val Gly Asp
           35                  40                  45
   Ile Thr Tyr Leu Lys Thr Gly Ser Gly Trp Leu Tyr Val Ala Thr Val
       50                  55                  60
   Ile Asp Leu Ala Thr Arg Met Val Val Gly Trp Ser Met Asp Ser Asn
   65                  70                  75                  80
   Met Arg Thr Pro Leu Val Ile Asn Ala Leu Ala Met Ala Arg Asp His
                   85                  90                  95
   Gly Cys Leu His Pro Glu Gly Ala Ile Phe His Ser Asp Arg Gly Ser
               100                 105                 110
   Gln Tyr Thr Ser Glu Gln Phe Gln Thr Trp Cys Ala Gly Asn Lys Ile
           115                 120                 125
   Thr Gln Ser Met Gly Leu Thr Gly Val Cys Trp Asp Asn Gly Ser Arg
       130                 135                 140
   Gly Glu Phe Phe Leu Thr Phe Glu Asp Arg Asn Val Ser Pro Leu
   145                 150                 155
<210> 93
   <211> 1969
   <212> DNA
   <213> Corynebacterium glutamicum
<220>
   <221> CDS
   <222> (101)..(1939)
   <223> RXA03394
<400> 93
   ctgcaaagcg acgcagggag cgtaaggcga gtggcgcggg gaagcgtcga taggcaattt 60
   ttaacccctg ataccccttt ccggccgggc ataaattaag gtg gta cgc atg acg   115
                                               Val Val Arg Met Thr
                                               1               5
   aag aac gtg ctc gta tct gtt gcc tgg ccg tat gcc aac gga ccc cgt   163
   Lys Asn Val Leu Val Ser Val Ala Trp Pro Tyr Ala Asn Gly Pro Arg
                   10                  15                  20
   cac att gga cat gtg gcg ggg ttt ggt gtc ccc tcc gat gtg ttc gca   211
   His Ile Gly His Val Ala Gly Phe Gly Val Pro Ser Asp Val Phe Ala
               25                  30                  35
   agg ttc cag cga atg tct ggc aac aac gtg ctc atg gtg tcc ggc acc   259
   Arg Phe Gln Arg Met Ser Gly Asn Asn Val Leu Met Val Ser Gly Thr
           40                  45                  50
   gat gag cac ggc acg cca ctt ctg gtt caa gca gac aaa gaa ggc gtc   307
   Asp Glu His Gly Thr Pro Leu Leu Val Gln Ala Asp Lys Glu Gly Val
       55                  60                  65
   acc gtt caa gac cta gcg gat aag tac aac cgc cag atc gtc gaa gac   355
   Thr Val Gln Asp Leu Ala Asp Lys Tyr Asn Arg Gln Ile Val Glu Asp
   70                  75                  80                  85
   ctc acc ggc ctg ggc ctg tcc tat gac ctt ttc acc cgc acc acc acc   403
   Leu Thr Gly Leu Gly Leu Ser Tyr Asp Leu Phe Thr Arg Thr Thr Thr
                   90                  95                  100
   tcc aac cac tac gca gta gtg cag gaa ctg ttc cgt ggt ctg tac gac   451
   Ser Asn His Tyr Ala Val Val Gln Glu Leu Phe Arg Gly Leu Tyr Asp
               105                 110                 115
   aac ggt tac atg atc aag gaa acc acc ctc ggt gcg att tcc cca tcc     499
   Asn Gly Tyr Met Ile Lys Glu Thr Thr Leu Gly Ala Ile Ser Pro Ser
           120                 125                 130
   act ggc cgt acc ctg cca gac cgc tac att gaa ggc acc tgc cca atc     547
   Thr Gly Arg Thr Leu Pro Asp Arg Tyr Ile Glu Gly Thr Cys Pro Ile
       135                 140                 145
   tgt ggc acc gac ggc gct cgt ggc gac cag tgc gac aac tgc gga aac     595
   Cys Gly Thr Asp Gly Ala Arg Gly Asp Gln Cys Asp Asn Cys Gly Asn
   150                 155                 160                 165
   cag ctc gat cca gcg gac ctg atc aac ccg gtg tcc aag atc aac ggc     643
   Gln Leu Asp Pro Ala Asp Leu Ile Asn Pro Val Ser Lys Ile Asn Gly
                   170                 175                 180
   gaa acc cca gag ttc gtt gag acc gaa cac ttc ctg ctc gac ctg cca     691
   Glu Thr Pro Glu Phe Val Glu Thr Glu His Phe Leu Leu Asp Leu Pro
               185                 190                 195
   gca ctg gct gaa gca cta acc gag tgg ctg aag gga cgc gaa gac tgg     739
   Ala Leu Ala Glu Ala Leu Thr Glu Trp Leu Lys Gly Arg Glu Asp Trp
           200                 205                 210
   cgt cca aac gtg ttg aag ttc tcg ctc aac ctg ctg gac gat atc cgc     787
   Arg Pro Asn Val Leu Lys Phe Ser Leu Asn Leu Leu Asp Asp Ile Arg
       215                 220                 225
   cca cgc gca atg tcg cgc gat atc gac tgg ggc atc cca atc cca gtt     835
   Pro Arg Ala Met Ser Arg Asp Ile Asp Trp Gly Ile Pro Ile Pro Val
   230                 235                 240                 245
   gaa gga tgg caa gac aac aac gcc aag aag ctc tac gtc tgg ttc gac     883
   Glu Gly Trp Gln Asp Asn Asn Ala Lys Lys Leu Tyr Val Trp Phe Asp
                   250                 255                 260
   gct gtc gtg ggc tac ttg tcc gca tcc atc gaa tgg gcc tac cgc tcc     931
   Ala Val Val Gly Tyr Leu Ser Ala Ser Ile Glu Trp Ala Tyr Arg Ser
               265                 270                 275
   ggc gac cca gaa gca tgg cgc acc ttc tgg aat gat cca gaa acc aag     979
   Gly Asp Pro Glu Ala Trp Arg Thr Phe Trp Asn Asp Pro Glu Thr Lys
           280                 285                 290
   tcc tac tac ttc atg ggc aaa gac aac atc acc ttc cac tcc cag atc     1027
   Ser Tyr Tyr Phe Met Gly Lys Asp Asn Ile Thr Phe His Ser Gln Ile
       295                 300                 305
   tgg cca gcg gag ctt ctc ggc tac gca ggc aag ggc tcc cgc ggt gga     1075
   Trp Pro Ala Glu Leu Leu Gly Tyr Ala Gly Lys Gly Ser Arg Gly Gly
   310                 315                 320                 325
   gaa atc ggt gac ctg ggt gtt ctg aac ctg cct act gag gtt gtt tcc     1123
   Glu Ile Gly Asp Leu Gly Val Leu Asn Leu Pro Thr Glu Val Val Ser
                   330                 335                 340
   tct gag ttc ctg act atg tct gga tcc aag ttc tcc tca tcc aag ggc     1171
   Ser Glu Phe Leu Thr Met Ser Gly Ser Lys Phe Ser Ser Ser Lys Gly
               345                 350                 355
   gtt gtc atc tac gtg aag gac ttc ctc aag gag ttc ggc cca gat gcg    1219
   Val Val Ile Tyr Val Lys Asp Phe Leu Lys Glu Phe Gly Pro Asp Ala
           360                 365                 370
   ctg cga tac ttc atc gct gtc gca ggc cca gaa aac aac gac acc gac    1267
   Leu Arg Tyr Phe Ile Ala Val Ala Gly Pro Glu Asn Asn Asp Thr Asp
       375                 380                 385
   ttc acc tgg gat gaa ttt gtc cgc cgc gta aat aac gag ctg gca aac    1315
   Phe Thr Trp Asp Glu Phe Val Arg Arg Val Asn Asn Glu Leu Ala Asn
   390                 395                 400                 405
   ggc tgg ggc aac ctg gtc aac cgc act gta tcc atg gcg cac aag aac    1363
   Gly Trp Gly Asn Leu Val Asn Arg Thr Val Ser Met Ala His Lys Asn
                   410                 415                 420
   ttc ggt gaa gta cca gta cct ggc gca ctg gaa gaa tct gac aag aag    1411
   Phe Gly Glu Val Pro Val Pro Gly Ala Leu Glu Glu Ser Asp Lys Lys
               425                 430                 435
   atc ctt gat ctt gct acc gct gcc ttt gaa tcc gtt gct gcg aac ctg    1459
   Ile Leu Asp Leu Ala Thr Ala Ala Phe Glu Ser Val Ala Ala Asn Leu
           440                 445                 450
   gat cag tcc aag ttc aag gcc ggt atc tct gaa atc atg cac gtt gtc    1507
   Asp Gln Ser Lys Phe Lys Ala Gly Ile Ser Glu Ile Met His Val Val
       455                 460                 465
   ggt gag gcc aac gcc tac atc gca gag caa gaa cca tgg aag ctt gcc    1555
   Gly Glu Ala Asn Ala Tyr Ile Ala Glu Gln Glu Pro Trp Lys Leu Ala
   470                 475                 480                 485
   aag gat gac acc aag cgc gag cgt ctt gcc acc gtg ctg tgg act gcg    1603
   Lys Asp Asp Thr Lys Arg Glu Arg Leu Ala Thr Val Leu Trp Thr Ala
                   490                 495                 500
   ctg cag gtt gtt tct gac tgc aac acc atg ctg acc cca tac ctg cca    1651
   Leu Gln Val Val Ser Asp Cys Asn Thr Met Leu Thr Pro Tyr Leu Pro
               505                 510                 515
   cac acc gcc caa aag gtg cat gag acc ttg ggc cgt gat gga atc tgg    1699
   His Thr Ala Gln Lys Val His Glu Thr Leu Gly Arg Asp Gly Ile Trp
           520                 525                 530
   gct gca aca cca cag atc gtg gaa gtc acc aac gaa tca cca cgc cag    1747
   Ala Ala Thr Pro Gln Ile Val Glu Val Thr Asn Glu Ser Pro Arg Gln
       535                 540                 545
   cca atc ggc gtg ggg cta cca gat cca gag cac acc tac cca gta atc    1795
   Pro Ile Gly Val Gly Leu Pro Asp Pro Glu His Thr Tyr Pro Val Ile
   550                 555                 560                 565
   atg ggc gac tac aag acc cag ctg gct aag tgg cag cgc atc gac gtt    1843
   Met Gly Asp Tyr Lys Thr Gln Leu Ala Lys Trp Gln Arg Ile Asp Val
                   570                 575                 580
   gtg cca ggc acc acc ttg gag aag cca gca ccg ctg att gct aag ctc    1891
   Val Pro Gly Thr Thr Leu Glu Lys Pro Ala Pro Leu Ile Ala Lys Leu
               585                 590                 595
   gat cca gaa ctt ggt gaa acc ggc cca gaa tgg gca cca gtg cag aac    1939
   Asp Pro Glu Leu Gly Glu Thr Gly Pro Glu Trp Ala Pro Val Gln Asn
           600                 605                 610
   taaagcatct ttagcatgaa ccgagcaggt 1969
<210> 94
   <211> 613
   <212> PRT
   <213> Corynebacterium glutamicum
<400> 94
   Val Val Arg Met Thr Lys Asn Val Leu Val Ser Val Ala Trp Pro Tyr
   1 5 10 15
   Ala Asn Gly Pro Arg His Ile Gly His Val Ala Gly Phe Gly Val Pro
   20 25 30
   Ser Asp Val Phe Ala Arg Phe Gln Arg Met Ser Gly Asn Asn Val Leu
   35 40 45
   Met Val Ser Gly Thr Asp Glu His Gly Thr Pro Leu Leu Val Gln Ala
   50 55 60
   Asp Lys Glu Gly Val Thr Val Gln Asp Leu Ala Asp Lys Tyr Asn Arg
   65 70 75 80
   Gln Ile Val Glu Asp Leu Thr Gly Leu Gly Leu Ser Tyr Asp Leu Phe
   85 90 95
   Thr Arg Thr Thr Thr Ser Asn His Tyr Ala Val Val Gln Glu Leu Phe
   100 105 110
   Arg Gly Leu Tyr Asp Asn Gly Tyr Met Ile Lys Glu Thr Thr Leu Gly
   115 120 125
   Ala Ile Ser Pro Ser Thr Gly Arg Thr Leu Pro Asp Arg Tyr Ile Glu
   130 135 140
   Gly Thr Cys Pro Ile Cys Gly Thr Asp Gly Ala Arg Gly Asp Gln Cys
   145 150 155 160
   Asp Asn Cys Gly Asn Gln Leu Asp Pro Ala Asp Leu Ile Asn Pro Val
   165 170 175
   Ser Lys Ile Asn Gly Glu Thr Pro Glu Phe Val Glu Thr Glu His Phe
   180 185 190
   Leu Leu Asp Leu Pro Ala Leu Ala Glu Ala Leu Thr Glu Trp Leu Lys
   195 200 205
   Gly Arg Glu Asp Trp Arg Pro Asn Val Leu Lys Phe Ser Leu Asn Leu
   210 215 220
   Leu Asp Asp Ile Arg Pro Arg Ala Met Ser Arg Asp Ile Asp Trp Gly
   225 230 235 240
   Ile Pro Ile Pro Val Glu Gly Trp Gln Asp Asn Asn Ala Lys Lys Leu
   245 250 255
   Tyr Val Trp Phe Asp Ala Val Val Gly Tyr Leu Ser Ala Ser Ile Glu
   260 265 270
   Trp Ala Tyr Arg Ser Gly Asp Pro Glu Ala Trp Arg Thr Phe Trp Asn
   275 280 285
   Asp Pro Glu Thr Lys Ser Tyr Tyr Phe Met Gly Lys Asp Asn Ile Thr
       290                 295                 300
   Phe His Ser Gln Ile Trp Pro Ala Glu Leu Leu Gly Tyr Ala Gly Lys
   305                 310                 315                 320
   Gly Ser Arg Gly Gly Glu Ile Gly Asp Leu Gly Val Leu Asn Leu Pro
                   325                 330                 335
   Thr Glu Val Val Ser Ser Glu Phe Leu Thr Met Ser Gly Ser Lys Phe
               340                 345                 350
   Ser Ser Ser Lys Gly Val Val Ile Tyr Val Lys Asp Phe Leu Lys Glu
           355                 360                 365
   Phe Gly Pro Asp Ala Leu Arg Tyr Phe Ile Ala Val Ala Gly Pro Glu
       370                 375                 380
   Asn Asn Asp Thr Asp Phe Thr Trp Asp Glu Phe Val Arg Arg Val Asn
   385                 390                 395                 400
   Asn Glu Leu Ala Asn Gly Trp Gly Asn Leu Val Asn Arg Thr Val Ser
                   405                 410                 415
   Met Ala His Lys Asn Phe Gly Glu Val Pro Val Pro Gly Ala Leu Glu
               420                 425                 430
   Glu Ser Asp Lys Lys Ile Leu Asp Leu Ala Thr Ala Ala Phe Glu Ser
           435                 440                 445
   Val Ala Ala Asn Leu Asp Gln Ser Lys Phe Lys Ala Gly Ile Ser Glu
       450                 455                 460
   Ile Met His Val Val Gly Glu Ala Asn Ala Tyr Ile Ala Glu Gln Glu
   465                 470                 475                 480
   Pro Trp Lys Leu Ala Lys Asp Asp Thr Lys Arg Glu Arg Leu Ala Thr
                   485                 490                 495
   Val Leu Trp Thr Ala Leu Gln Val Val Ser Asp Cys Asn Thr Met Leu
               500                 505                 510
   Thr Pro Tyr Leu Pro His Thr Ala Gln Lys Val His Glu Thr Leu Gly
           515                 520                 525
   Arg Asp Gly Ile Trp Ala Ala Thr Pro Gln Ile Val Glu Val Thr Asn
       530                 535                 540
   Glu Ser Pro Arg Gln Pro Ile Gly Val Gly Leu Pro Asp Pro Glu His
   545                 550                 555                 560
   Thr Tyr Pro Val Ile Met Gly Asp Tyr Lys Thr Gln Leu Ala Lys Trp
                   565                 570                 575
   Gln Arg Ile Asp Val Val Pro Gly Thr Thr Leu Glu Lys Pro Ala Pro
               580                 585                 590
   Leu Ile Ala Lys Leu Asp Pro Glu Leu Gly Glu Thr Gly Pro Glu Trp
           595                 600                 605
   Ala Pro Val Gln Asn
       610
<210> 95
   <211> 3016
   <212> DNA
   <213> Corynebacterium glutamicum
<220>
   <221> CDS
   <222> (101)..(2986)
   <223> RXA03674
<400> 95
   tccccgcagc ccaccaccgt gggctgcggg gtgtggcgtt tttgccacaa agtggaccgt 60
   attcgcaaat actttgttaa gacgcgttaa tctttaacct atg tct gaa tca ggt   115
                                               Met Ser Glu Ser Gly
                                                1               5
   gcg cta agt tct act gac tct cta tcc ccg ggt gtc acc att gaa gtc   163
   Ala Leu Ser Ser Thr Asp Ser Leu Ser Pro Gly Val Thr Ile Glu Val
                   10                  15                  20
   cga gat gaa att tgg ctg gtt act cac gtt act cgc tcc aca gat ggt   211
   Arg Asp Glu Ile Trp Leu Val Thr His Val Thr Arg Ser Thr Asp Gly
               25                  30                  35
   ttt agg gtt aaa gct cgt ggt ctc tct gat tat gtg cgg gac cac gaa   259
   Phe Arg Val Lys Ala Arg Gly Leu Ser Asp Tyr Val Arg Asp His Glu
           40                  45                  50
   gct acg ttc ttc acc gca ctt gat aaa gat ttg aag gtc att gac cct   307
   Ala Thr Phe Phe Thr Ala Leu Asp Lys Asp Leu Lys Val Ile Asp Pro
       55                  60                  65
   acc cag gtc acc gtc agt ctt gat gat tcc tcc aat tac cgt cgc acc   355
   Thr Gln Val Thr Val Ser Leu Asp Asp Ser Ser Asn Tyr Arg Arg Thr
   70                  75                  80                  85
   cgc ctg tgg ttg gag gcc acc atg cgt aaa act ccg gta ccg ctc tat   403
   Arg Leu Trp Leu Glu Ala Thr Met Arg Lys Thr Pro Val Pro Leu Tyr
                   90                  95                  100
   caa gag tca ctt tcc gtg gca gat caa atg ctc gcc gat cca ctg gag   451
   Gln Glu Ser Leu Ser Val Ala Asp Gln Met Leu Ala Asp Pro Leu Glu
               105                 110                 115
   tac caa tta gca gcc gtg cgc aaa acc ctc tct agt gct aac ttg cgc   499
   Tyr Gln Leu Ala Ala Val Arg Lys Thr Leu Ser Ser Ala Asn Leu Arg
           120                 125                 130
   ccc cgc gtg ctt att gct gat gcc gtg gga ctt ggc aaa acc cta gaa   547
   Pro Arg Val Leu Ile Ala Asp Ala Val Gly Leu Gly Lys Thr Leu Glu
       135                 140                 145
   atg ggc atg atc ttg gcg gaa ctt atc cgc cgt ggc cgt ggt gag cgc   595
   Met Gly Met Ile Leu Ala Glu Leu Ile Arg Arg Gly Arg Gly Glu Arg
   150                 155                 160                 165
   att ttg gta gtc acc ccg cgc cac att atg gag cag ttc cag cag gaa   643
   Ile Leu Val Val Thr Pro Arg His Ile Met Glu Gln Phe Gln Gln Glu
                   170                 175                 180
   atg tgg acc cgt ttt gcc atc ccg ctc gtt cgt cta gat tcc gtg ggc   691
   Met Trp Thr Arg Phe Ala Ile Pro Leu Val Arg Leu Asp Ser Val Gly
               185                 190                 195
   atc cag caa gtg cgc caa aaa ttg cca gca tca cgc aac cct ttt act    739
   Ile Gln Gln Val Arg Gln Lys Leu Pro Ala Ser Arg Asn Pro Phe Thr
           200                 205                 210
   tat ttc ccg cgc gtg att gtc tct atg gat act ttg aaa tct ccg aag    787
   Tyr Phe Pro Arg Val Ile Val Ser Met Asp Thr Leu Lys Ser Pro Lys
       215                 220                 225
   tac cgc gcg caa cta gaa aag gtg cac tgg gat gcg gtg gtt ata gat    835
   Tyr Arg Ala Gln Leu Glu Lys Val His Trp Asp Ala Val Val Ile Asp
   230                 235                 240                 245
   gaa atc cac aat gca acc aat gct ggc acc caa aat aat gag cta gcc    883
   Glu Ile His Asn Ala Thr Asn Ala Gly Thr Gln Asn Asn Glu Leu Ala
                   250                 255                 260
   cgc aca ctt gcg cct act gcc gag gct ctt att ttg gcc tct gcc acc    931
   Arg Thr Leu Ala Pro Thr Ala Glu Ala Leu Ile Leu Ala Ser Ala Thr
               265                 270                 275
   ccg cac aat ggt gat cca gaa tcc ttt aag gag atc ttg cgt ttg ctt    979
   Pro His Asn Gly Asp Pro Glu Ser Phe Lys Glu Ile Leu Arg Leu Leu
           280                 285                 290
   gat ccc acc gct gtg atg cct gat ggc acc att gat gcc gaa gct gca    1027
   Asp Pro Thr Ala Val Met Pro Asp Gly Thr Ile Asp Ala Glu Ala Ala
       295                 300                 305
   cag cgt ctg atc att cgt cgc cat cgc aat agc cct gag gtt tca ggt    1075
   Gln Arg Leu Ile Ile Arg Arg His Arg Asn Ser Pro Glu Val Ser Gly
   310                 315                 320                 325
   ttt gtg ggc gaa aaa tgg gct cca cgc aat gag cct cag aac ttc ctg    1123
   Phe Val Gly Glu Lys Trp Ala Pro Arg Asn Glu Pro Gln Asn Phe Leu
                   330                 335                 340
   gtc gct gcg tca aaa gaa gaa aac ggc gtt gct gca gaa ctc aac cat    1171
   Val Ala Ala Ser Lys Glu Glu Asn Gly Val Ala Ala Glu Leu Asn His
               345                 350                 355
   gtg tgg att tca cca ggt gcg agc aat ccg atc aag gat cgc ctc ttc    1219
   Val Trp Ile Ser Pro Gly Ala Ser Asn Pro Ile Lys Asp Arg Leu Phe
           360                 365                 370
   ccc tgg aca ttg gtg aag gct ttt ctc tcc tcc cct gca gcc ttg ggc    1267
   Pro Trp Thr Leu Val Lys Ala Phe Leu Ser Ser Pro Ala Ala Leu Gly
       375                 380                 385
   gaa aca gtg tcc aat cgc ctc aaa aag gcc tct gca cca gaa gaa aaa    1315
   Glu Thr Val Ser Asn Arg Leu Lys Lys Ala Ser Ala Pro Glu Glu Lys
   390                 395                 400                 405
   cgc gcc cta gaa acc ctt tca caa ctt aat tct gcg atc acc ccg cag    1363
   Arg Ala Leu Glu Thr Leu Ser Gln Leu Asn Ser Ala Ile Thr Pro Gln
                   410                 415                 420
   acc tca cag aag tac caa tct cta ctg agc tac ctc ggt gac atc gga    1411
   Thr Ser Gln Lys Tyr Gln Ser Leu Leu Ser Tyr Leu Gly Asp Ile Gly
               425                 430                 435
   gtg aag aag aac tcc gat acc cgc gtg gtg att ttc tct gag cgt gtc    1459
   Val Lys Lys Asn Ser Asp Thr Arg Val Val Ile Phe Ser Glu Arg Val
           440                 445                 450
   gct act ttg cac tgg ctg cag gaa aac ctc atc cgt gat ctc aag atg    1507
   Ala Thr Leu His Trp Leu Gln Glu Asn Leu Ile Arg Asp Leu Lys Met
       455                 460                 465
   cca ccc aac tct att gct gtt atg cac ggc ggt ctc ccc gac cag gag    1555
   Pro Pro Asn Ser Ile Ala Val Met His Gly Gly Leu Pro Asp Gln Glu
   470                 475                 480                 485
   caa atg cgc ctg gtg gat gag ttt aaa aag acg gat tct ccc atc cgc    1603
   Gln Met Arg Leu Val Asp Glu Phe Lys Lys Thr Asp Ser Pro Ile Arg
                   490                 495                 500
   atc atg atc acc ggc gat gtt gcc tca gaa ggt gtg aac ctg cat act    1651
   Ile Met Ile Thr Gly Asp Val Ala Ser Glu Gly Val Asn Leu His Thr
               505                 510                 515
   ctc tgc cac aac ttg gtg cac tat gac atc ccg tgg tca ctg atc cgc    1699
   Leu Cys His Asn Leu Val His Tyr Asp Ile Pro Trp Ser Leu Ile Arg
           520                 525                 530
   att cag cag cgc aat ggc cgt att gat cgt tat ggt caa acc cac aac    1747
   Ile Gln Gln Arg Asn Gly Arg Ile Asp Arg Tyr Gly Gln Thr His Asn
       535                 540                 545
   cct tcc atc gtt acc ttc ttg ctc gat ccc gcc gag gat tcc aaa gta    1795
   Pro Ser Ile Val Thr Phe Leu Leu Asp Pro Ala Glu Asp Ser Lys Val
   550                 555                 560                 565
   ggt gaa gtc cat gtg ctg gag agg ctc atg gag cgc gaa cat gag gcg    1843
   Gly Glu Val His Val Leu Glu Arg Leu Met Glu Arg Glu His Glu Ala
                   570                 575                 580
   cac tct ttg ctc ggt gat gcc gca tct ctc atg ggc aag cac tct gag    1891
   His Ser Leu Leu Gly Asp Ala Ala Ser Leu Met Gly Lys His Ser Glu
               585                 590                 595
   cgt ttg gaa gaa gaa acc atc cgc gaa gtc ctg cgc ggt gcc caa aac    1939
   Arg Leu Glu Glu Glu Thr Ile Arg Glu Val Leu Arg Gly Ala Gln Asn
           600                 605                 610
   ttt aat gat gca gtg gct gat cca gcg gaa gtc cta gaa aac cca gca    1987
   Phe Asn Asp Ala Val Ala Asp Pro Ala Glu Val Leu Glu Asn Pro Ala
       615                 620                 625
   ggc cta gat gat att gat tgg ttg cta gcc caa atc gcc caa gcc gat    2035
   Gly Leu Asp Asp Ile Asp Trp Leu Leu Ala Gln Ile Ala Gln Ala Asp
   630                 635                 640                 645
   gcc aag gca gaa aca gaa gca gaa gca gaa aca gaa aac caa aca gca    2083
   Ala Lys Ala Glu Thr Glu Ala Glu Ala Glu Thr Glu Asn Gln Thr Ala
                   650                 655                 660
   cca gat gca gct tcc aat agc acg cag cat gca caa cgc cgg ttg tat    2131
   Pro Asp Ala Ala Ser Asn Ser Thr Gln His Ala Gln Arg Arg Leu Tyr
               665                 670                 675
   gca cag gaa agc tct ttc ctc tat gac tgc ctc ctc gaa ggt ttc aat    2179
   Ala Gln Glu Ser Ser Phe Leu Tyr Asp Cys Leu Leu Glu Gly Phe Asn
           680                 685                 690
   aac gta ccg gag gat tcc atc aac cgc ggt ggc gtg ggg ttc aaa aaa    2227
   Asn Val Pro Glu Asp Ser Ile Asn Arg Gly Gly Val Gly Phe Lys Lys
       695                 700                 705
   cac gat aat gac atc gtg gag ctc acc ccc acc gat gat ctg cgc cgt     2275
   His Asp Asn Asp Ile Val Glu Leu Thr Pro Thr Asp Asp Leu Arg Arg
   710                 715                 720                 725
   cgt cta gat ttc ctc ccg cag gat tat gtg gct gcc cgg aaa gtt aag     2323
   Arg Leu Asp Phe Leu Pro Gln Asp Tyr Val Ala Ala Arg Lys Val Lys
                   730                 735                 740
   gaa gat ctc cta cta gct tcc aca ctg atg cgt ggc caa gaa cgc ctc     2371
   Glu Asp Leu Leu Leu Ala Ser Thr Leu Met Arg Gly Gln Glu Arg Leu
               745                 750                 755
   aac gct gcg cgc act ggt gaa gat ggc agt acc tgg cca agt gcc cac     2419
   Asn Ala Ala Arg Thr Gly Glu Asp Gly Ser Thr Trp Pro Ser Ala His
           760                 765                 770
   tat cta ggc ccc ctg cac cca gtc act tcg tgg gca gct gac cgc gcg     2467
   Tyr Leu Gly Pro Leu His Pro Val Thr Ser Trp Ala Ala Asp Arg Ala
       775                 780                 785
   ctg gca acc atg cca cgt tcg gaa att ccg gcg gct agt ggc aaa gtc     2515
   Leu Ala Thr Met Pro Arg Ser Glu Ile Pro Ala Ala Ser Gly Lys Val
   790                 795                 800                 805
   aca gag cca acg gtg ctg ctt atg tcc aca ttg agc aat cgg cgt ggc     2563
   Thr Glu Pro Thr Val Leu Leu Met Ser Thr Leu Ser Asn Arg Arg Gly
                   810                 815                 820
   caa att gtg tct cgt tct ttt gtg gct tct tct ggc ccc ttt gat act     2611
   Gln Ile Val Ser Arg Ser Phe Val Ala Ser Ser Gly Pro Phe Asp Thr
               825                 830                 835
   gag gtg ctg tcc gat ccc atc caa tgg tta cat tcc ata ggc ctc gat     2659
   Glu Val Leu Ser Asp Pro Ile Gln Trp Leu His Ser Ile Gly Leu Asp
           840                 845                 850
   gaa acc gcc att aac cca ggt acc gct gca ctc ccc gac gat att gag     2707
   Glu Thr Ala Ile Asn Pro Gly Thr Ala Ala Leu Pro Asp Asp Ile Glu
       855                 860                 865
   cag ctt att tcc ctt gct gtt cag gcc gcc cgc ggc gag atc cgt cca     2755
   Gln Leu Ile Ser Leu Ala Val Gln Ala Ala Arg Gly Glu Ile Arg Pro
   870                 875                 880                 885
   tta atg atc gcc gcc cgc gct cag gct caa act cgc gtt gag cat tgg     2803
   Leu Met Ile Ala Ala Arg Ala Gln Ala Gln Thr Arg Val Glu His Trp
                   890                 895                 900
   gct aag cga gcc gaa gcc tgg aat aac aaa cga agt ggc gca gcg tcc     2851
   Ala Lys Arg Ala Glu Ala Trp Asn Asn Lys Arg Ser Gly Ala Ala Ser
               905                 910                 915
   acg tcc cgt acc gcg cga act gca aaa ttg att gag gag cag cag aaa     2899
   Thr Ser Arg Thr Ala Arg Thr Ala Lys Leu Ile Glu Glu Gln Gln Lys
           920                 925                 930
   ttg agt aat gct ctc gag cca gac cgt gaa ctt att agg cct ttg gcc     2947
   Leu Ser Asn Ala Leu Glu Pro Asp Arg Glu Leu Ile Arg Pro Leu Ala
       935                 940                 945
   gtc att ctt ccg cag ccc gca act ttg aac acc gag gtt taacacaatg      2996
   Val Ile Leu Pro Gln Pro Ala Thr Leu Asn Thr Glu Val
   950                 955                 960
   agtgcatttg attcgatcct 3016
<210> 96
   <211> 962
   <212> PRT
   <213> Corynebacterium glutamicum
<400> 96
   Met Ser Glu Ser Gly Ala Leu Ser Ser Thr Asp Ser Leu Ser Pro Gly
   1 5 10 15
   Val Thr Ile Glu Val Arg Asp Glu Ile Trp Leu Val Thr His Val Thr
   20 25 30
   Arg Ser Thr Asp Gly Phe Arg Val Lys Ala Arg Gly Leu Ser Asp Tyr
   35 40 45
   Val Arg Asp His Glu Ala Thr Phe Phe Thr Ala Leu Asp Lys Asp Leu
   50 55 60
   Lys Val Ile Asp Pro Thr Gln Val Thr Val Ser Leu Asp Asp Ser Ser
   65 70 75 80
   Asn Tyr Arg Arg Thr Arg Leu Trp Leu Glu Ala Thr Met Arg Lys Thr
   85 90 95
   Pro Val Pro Leu Tyr Gln Glu Ser Leu Ser Val Ala Asp Gln Met Leu
   100 105 110
   Ala Asp Pro Leu Glu Tyr Gln Leu Ala Ala Val Arg Lys Thr Leu Ser
   115 120 125
   Ser Ala Asn Leu Arg Pro Arg Val Leu Ile Ala Asp Ala Val Gly Leu
   130 135 140
   Gly Lys Thr Leu Glu Met Gly Met Ile Leu Ala Glu Leu Ile Arg Arg
   145 150 155 160
   Gly Arg Gly Glu Arg Ile Leu Val Val Thr Pro Arg His Ile Met Glu
   165 170 175
   Gln Phe Gln Gln Glu Met Trp Thr Arg Phe Ala Ile Pro Leu Val Arg
   180 185 190
   Leu Asp Ser Val Gly Ile Gln Gln Val Arg Gln Lys Leu Pro Ala Ser
   195 200 205
   Arg Asn Pro Phe Thr Tyr Phe Pro Arg Val Ile Val Ser Met Asp Thr
   210 215 220
   Leu Lys Ser Pro Lys Tyr Arg Ala Gln Leu Glu Lys Val His Trp Asp
   225 230 235 240
   Ala Val Val Ile Asp Glu Ile His Asn Ala Thr Asn Ala Gly Thr Gln
   245 250 255
   Asn Asn Glu Leu Ala Arg Thr Leu Ala Pro Thr Ala Glu Ala Leu Ile
   260 265 270
   Leu Ala Ser Ala Thr Pro His Asn Gly Asp Pro Glu Ser Phe Lys Glu
   275 280 285
   Ile Leu Arg Leu Leu Asp Pro Thr Ala Val Met Pro Asp Gly Thr Ile
       290                 295                 300
   Asp Ala Glu Ala Ala Gln Arg Leu Ile Ile Arg Arg His Arg Asn Ser
   305                 310                 315                 320
   Pro Glu Val Ser Gly Phe Val Gly Glu Lys Trp Ala Pro Arg Asn Glu
                   325                 330                 335
   Pro Gln Asn Phe Leu Val Ala Ala Ser Lys Glu Glu Asn Gly Val Ala
               340                 345                 350
   Ala Glu Leu Asn His Val Trp Ile Ser Pro Gly Ala Ser Asn Pro Ile
           355                 360                 365
   Lys Asp Arg Leu Phe Pro Trp Thr Leu Val Lys Ala Phe Leu Ser Ser
       370                 375                 380
   Pro Ala Ala Leu Gly Glu Thr Val Ser Asn Arg Leu Lys Lys Ala Ser
   385                 390                 395                 400
   Ala Pro Glu Glu Lys Arg Ala Leu Glu Thr Leu Ser Gln Leu Asn Ser
                   405                 410                 415
   Ala Ile Thr Pro Gln Thr Ser Gln Lys Tyr Gln Ser Leu Leu Ser Tyr
               420                 425                 430
   Leu Gly Asp Ile Gly Val Lys Lys Asn Ser Asp Thr Arg Val Val Ile
           435                 440                 445
   Phe Ser Glu Arg Val Ala Thr Leu His Trp Leu Gln Glu Asn Leu Ile
       450                 455                 460
   Arg Asp Leu Lys Met Pro Pro Asn Ser Ile Ala Val Met His Gly Gly
   465                 470                 475                 480
   Leu Pro Asp Gln Glu Gln Met Arg Leu Val Asp Glu Phe Lys Lys Thr
                   485                 490                 495
   Asp Ser Pro Ile Arg Ile Met Ile Thr Gly Asp Val Ala Ser Glu Gly
               500                 505                 510
   Val Asn Leu His Thr Leu Cys His Asn Leu Val His Tyr Asp Ile Pro
           515                 520                 525
   Trp Ser Leu Ile Arg Ile Gln Gln Arg Asn Gly Arg Ile Asp Arg Tyr
       530                 535                 540
   Gly Gln Thr His Asn Pro Ser Ile Val Thr Phe Leu Leu Asp Pro Ala
   545                 550                 555                 560
   Glu Asp Ser Lys Val Gly Glu Val His Val Leu Glu Arg Leu Met Glu
                   565                 570                 575
   Arg Glu His Glu Ala His Ser Leu Leu Gly Asp Ala Ala Ser Leu Met
               580                 585                 590
   Gly Lys His Ser Glu Arg Leu Glu Glu Glu Thr Ile Arg Glu Val Leu
           595                 600                 605
   Arg Gly Ala Gln Asn Phe Asn Asp Ala Val Ala Asp Pro Ala Glu Val
       610                 615                 620
   Leu Glu Asn Pro Ala Gly Leu Asp Asp Ile Asp Trp Leu Leu Ala Gln
   625 630 635 640
   Ile Ala Gln Ala Asp Ala Lys Ala Glu Thr Glu Ala Glu Ala Glu Thr
   645 650 655
   Glu Asn Gln Thr Ala Pro Asp Ala Ala Ser Asn Ser Thr Gln His Ala
   660 665 670
   Gln Arg Arg Leu Tyr Ala Gln Glu Ser Ser Phe Leu Tyr Asp Cys Leu
   675 680 685
   Leu Glu Gly Phe Asn Asn Val Pro Glu Asp Ser Ile Asn Arg Gly Gly
   690 695 700
   Val Gly Phe Lys Lys His Asp Asn Asp Ile Val Glu Leu Thr Pro Thr
   705 710 715 720
   Asp Asp Leu Arg Arg Arg Leu Asp Phe Leu Pro Gln Asp Tyr Val Ala
   725 730 735
   Ala Arg Lys Val Lys Glu Asp Leu Leu Leu Ala Ser Thr Leu Met Arg
   740 745 750
   Gly Gln Glu Arg Leu Asn Ala Ala Arg Thr Gly Glu Asp Gly Ser Thr
   755 760 765
   Trp Pro Ser Ala His Tyr Leu Gly Pro Leu His Pro Val Thr Ser Trp
   770 775 780
   Ala Ala Asp Arg Ala Leu Ala Thr Met Pro Arg Ser Glu Ile Pro Ala
   785 790 795 800
   Ala Ser Gly Lys Val Thr Glu Pro Thr Val Leu Leu Met Ser Thr Leu
   805 810 815
   Ser Asn Arg Arg Gly Gln Ile Val Ser Arg Ser Phe Val Ala Ser Ser
   820 825 830
   Gly Pro Phe Asp Thr Glu Val Leu Ser Asp Pro Ile Gln Trp Leu His
   835 840 845
   Ser Ile Gly Leu Asp Glu Thr Ala Ile Asn Pro Gly Thr Ala Ala Leu
   850 855 860
   Pro Asp Asp Ile Glu Gln Leu Ile Ser Leu Ala Val Gln Ala Ala Arg
   865 870 875 880
   Gly Glu Ile Arg Pro Leu Met Ile Ala Ala Arg Ala Gln Ala Gln Thr
   885 890 895
   Arg Val Glu His Trp Ala Lys Arg Ala Glu Ala Trp Asn Asn Lys Arg
   900 905 910
   Ser Gly Ala Ala Ser Thr Ser Arg Thr Ala Arg Thr Ala Lys Leu Ile
   915 920 925
   Glu Glu Gln Gln Lys Leu Ser Asn Ala Leu Glu Pro Asp Arg Glu Leu
   930 935 940
   Ile Arg Pro Leu Ala Val Ile Leu Pro Gln Pro Ala Thr Leu Asn Thr
   945 950 955 960
   Glu Val
<210> 97
   <211> 1624
   <212> DNA
   <213> Corynebacterium glutamicum
<220>
   <221> CDS
   <222> (101)..(1594)
   <223> RXA03793
<400> 97
   atggaaagaa gctaggcgga aagggcgtta agtacttgcc atttaatcct cagcatcact 60
   cggatcagtc ggagatgtcg atgaaaatgc accaggagcc gtg gag agc agc atg   115
                                               Val Glu Ser Ser Met
                                                1               5
   gta gaa aac aac gta gca aaa aag acg gtc gct aaa aag acc gca cgc   163
   Val Glu Asn Asn Val Ala Lys Lys Thr Val Ala Lys Lys Thr Ala Arg
                   10                  15                  20
   aag acc gca cgc aaa gca gcc ccg cgc gtg gca acc cca ttg gga gtc   211
   Lys Thr Ala Arg Lys Ala Ala Pro Arg Val Ala Thr Pro Leu Gly Val
               25                  30                  35
   gca tct gag tct ccc att tcg gcc acc cct gcg cgc agc atc gat gga   259
   Ala Ser Glu Ser Pro Ile Ser Ala Thr Pro Ala Arg Ser Ile Asp Gly
           40                  45                  50
   acc tca acc cct gtt gaa gct gct gac acc ata gag acc acc gcc cct   307
   Thr Ser Thr Pro Val Glu Ala Ala Asp Thr Ile Glu Thr Thr Ala Pro
       55                  60                  65
   gca gcg aag gct cct gcg gcc aag gct ccc gct aaa aag gtt gcc aag   355
   Ala Ala Lys Ala Pro Ala Ala Lys Ala Pro Ala Lys Lys Val Ala Lys
   70                  75                  80                  85
   aag aca gct cgc aag gca cct gcg aaa aag act gtc gcc aag aaa gcc   403
   Lys Thr Ala Arg Lys Ala Pro Ala Lys Lys Thr Val Ala Lys Lys Ala
                   90                  95                  100
   aca acc gcc aag gct gca cct gca act gcc aag gac gaa aac gca cct   451
   Thr Thr Ala Lys Ala Ala Pro Ala Thr Ala Lys Asp Glu Asn Ala Pro
               105                 110                 115
   gtt gat gac gac gag gag aac ctc gct cag gat gaa cag gac ttc gac   499
   Val Asp Asp Asp Glu Glu Asn Leu Ala Gln Asp Glu Gln Asp Phe Asp
           120                 125                 130
   ggc gat gac ttc gta gac ggc atc gaa gac gaa gaa gat gaa gac ggc   547
   Gly Asp Asp Phe Val Asp Gly Ile Glu Asp Glu Glu Asp Glu Asp Gly
       135                 140                 145
   gtc gaa gcc ctc ggt gaa gaa agc gaa gac gac gaa gag gac ggc tca   595
   Val Glu Ala Leu Gly Glu Glu Ser Glu Asp Asp Glu Glu Asp Gly Ser
   150                 155                 160                 165
   tcc gtt tgg gat gaa gac gaa tcc gca acc ctg cgt cag gca cgt aaa   643
   Ser Val Trp Asp Glu Asp Glu Ser Ala Thr Leu Arg Gln Ala Arg Lys
                   170                 175                 180
   gat gcc gag ctc acc gct tcc gcc gac tct gtt cgc gct tac ctg aag   691
   Asp Ala Glu Leu Thr Ala Ser Ala Asp Ser Val Arg Ala Tyr Leu Lys
               185                 190                 195
   caa atc ggt aaa gtt gcc ctg ctg aac gct gaa cag gaa gtc tcc ctg     739
   Gln Ile Gly Lys Val Ala Leu Leu Asn Ala Glu Gln Glu Val Ser Leu
           200                 205                 210
   gca aag cgc atc gaa gca ggc ctt tac gcc acc cac cgc atg gag gaa     787
   Ala Lys Arg Ile Glu Ala Gly Leu Tyr Ala Thr His Arg Met Glu Glu
       215                 220                 225
   atg gaa gaa gct ttc gca gcc ggt gac aag gac gcg aaa ctc acc cca     835
   Met Glu Glu Ala Phe Ala Ala Gly Asp Lys Asp Ala Lys Leu Thr Pro
   230                 235                 240                 245
   gcc gtc aag cgt gac ctc cgc gcc atc gct cgt gac ggc cgc aag gcg     883
   Ala Val Lys Arg Asp Leu Arg Ala Ile Ala Arg Asp Gly Arg Lys Ala
                   250                 255                 260
   aaa aac cac ctc ctg gaa gcc aac ctt cgt ctg gtt gtc tcc ctg gca     931
   Lys Asn His Leu Leu Glu Ala Asn Leu Arg Leu Val Val Ser Leu Ala
               265                 270                 275
   aag cgc tac acc ggc cgt ggc atg gca ttc ctg gac ctc atc cag gaa     979
   Lys Arg Tyr Thr Gly Arg Gly Met Ala Phe Leu Asp Leu Ile Gln Glu
           280                 285                 290
   ggc aac ctc ggt ctg att cgt gcc gta gag aag ttc gac tac tcc aag     1027
   Gly Asn Leu Gly Leu Ile Arg Ala Val Glu Lys Phe Asp Tyr Ser Lys
       295                 300                 305
   ggc tac aag ttc tcc acc tac gca acc tgg tgg atc cgt cag gca atc     1075
   Gly Tyr Lys Phe Ser Thr Tyr Ala Thr Trp Trp Ile Arg Gln Ala Ile
   310                 315                 320                 325
   acc cgc gcc atg gcc gac caa gca cga acc atc cgt atc cca gtc cac     1123
   Thr Arg Ala Met Ala Asp Gln Ala Arg Thr Ile Arg Ile Pro Val His
                   330                 335                 340
   atg gtt gaa gtg atc aac aaa ctt ggt cgc atc caa cgt gaa ctc ctt     1171
   Met Val Glu Val Ile Asn Lys Leu Gly Arg Ile Gln Arg Glu Leu Leu
               345                 350                 355
   cag gaa ctc ggc cgc gaa cca acc cca cag gaa ctg tcc aaa gaa atg     1219
   Gln Glu Leu Gly Arg Glu Pro Thr Pro Gln Glu Leu Ser Lys Glu Met
           360                 365                 370
   gac atc tcc gag gaa aag gta ctg gaa atc cag cag tac gcc cgc gaa     1267
   Asp Ile Ser Glu Glu Lys Val Leu Glu Ile Gln Gln Tyr Ala Arg Glu
       375                 380                 385
   cca atc tcc ctg gac caa acc atc ggc gac gaa ggc gac agc cag ctc     1315
   Pro Ile Ser Leu Asp Gln Thr Ile Gly Asp Glu Gly Asp Ser Gln Leu
   390                 395                 400                 405
   ggc gac ttc atc gaa gac tcc gaa gcc gtc gtc gca gtc gac gcc gtc     1363
   Gly Asp Phe Ile Glu Asp Ser Glu Ala Val Val Ala Val Asp Ala Val
                   410                 415                 420
   tca ttc acc ctg ctg caa gac cag cta cag gac gtc cta gag acc ctc     1411
   Ser Phe Thr Leu Leu Gln Asp Gln Leu Gln Asp Val Leu Glu Thr Leu
               425                 430                 435
   tcc gaa cgt gaa gcc ggc gtg gtt aaa ctc cgc ttc gga ctc acc gac     1459
   Ser Glu Arg Glu Ala Gly Val Val Lys Leu Arg Phe Gly Leu Thr Asp
           440                 445                 450
   gga atg cca cgc act tta gac gaa atc ggc caa gtt tac ggt gtc acc    1507
   Gly Met Pro Arg Thr Leu Asp Glu Ile Gly Gln Val Tyr Gly Val Thr
       455                 460                 465
   cgt gag cgc atc cgc cag att gag tcc aag acc atg tct aag ctg cgc    1555
   Arg Glu Arg Ile Arg Gln Ile Glu Ser Lys Thr Met Ser Lys Leu Arg
   470                 475                 480                 485
   cac cca tca cgc tcc cag gtc ctt cgc gac tac ctg gac taaaacccca     1604
   His Pro Ser Arg Ser Gln Val Leu Arg Asp Tyr Leu Asp
                   490                 495
   gtcgggctca agaccgggcc                                              1624
<210> 98
   <211> 498
   <212> PRT
   <213> Corynebacterium glutamicum
<400> 98
   Val Glu Ser Ser Met Val Glu Asn Asn Val Ala Lys Lys Thr Val Ala
   1 5 10 15
   Lys Lys Thr Ala Arg Lys Thr Ala Arg Lys Ala Ala Pro Arg Val Ala
   20 25 30
   Thr Pro Leu Gly Val Ala Ser Glu Ser Pro Ile Ser Ala Thr Pro Ala
   35 40 45
   Arg Ser Ile Asp Gly Thr Ser Thr Pro Val Glu Ala Ala Asp Thr Ile
   50 55 60
   Glu Thr Thr Ala Pro Ala Ala Lys Ala Pro Ala Ala Lys Ala Pro Ala
   65 70 75 80
   Lys Lys Val Ala Lys Lys Thr Ala Arg Lys Ala Pro Ala Lys Lys Thr
   85 90 95
   Val Ala Lys Lys Ala Thr Thr Ala Lys Ala Ala Pro Ala Thr Ala Lys
   100 105 110
   Asp Glu Asn Ala Pro Val Asp Asp Asp Glu Glu Asn Leu Ala Gln Asp
   115 120 125
   Glu Gln Asp Phe Asp Gly Asp Asp Phe Val Asp Gly Ile Glu Asp Glu
   130 135 140
   Glu Asp Glu Asp Gly Val Glu Ala Leu Gly Glu Glu Ser Glu Asp Asp
   145 150 155 160
   Glu Glu Asp Gly Ser Ser Val Trp Asp Glu Asp Glu Ser Ala Thr Leu
   165 170 175
   Arg Gln Ala Arg Lys Asp Ala Glu Leu Thr Ala Ser Ala Asp Ser Val
   180 185 190
   Arg Ala Tyr Leu Lys Gln Ile Gly Lys Val Ala Leu Leu Asn Ala Glu
   195 200 205
   Gln Glu Val Ser Leu Ala Lys Arg Ile Glu Ala Gly Leu Tyr Ala Thr
       210                 215                 220
   His Arg Met Glu Glu Met Glu Glu Ala Phe Ala Ala Gly Asp Lys Asp
   225                 230                 235                 240
   Ala Lys Leu Thr Pro Ala Val Lys Arg Asp Leu Arg Ala Ile Ala Arg
                   245                 250                 255
   Asp Gly Arg Lys Ala Lys Asn His Leu Leu Glu Ala Asn Leu Arg Leu
               260                 265                 270
   Val Val Ser Leu Ala Lys Arg Tyr Thr Gly Arg Gly Met Ala Phe Leu
           275                 280                 285
   Asp Leu Ile Gln Glu Gly Asn Leu Gly Leu Ile Arg Ala Val Glu Lys
       290                 295                 300
   Phe Asp Tyr Ser Lys Gly Tyr Lys Phe Ser Thr Tyr Ala Thr Trp Trp
   305                 310                 315                 320
   Ile Arg Gln Ala Ile Thr Arg Ala Met Ala Asp Gln Ala Arg Thr Ile
                   325                 330                 335
   Arg Ile Pro Val His Met Val Glu Val Ile Asn Lys Leu Gly Arg Ile
               340                 345                 350
   Gln Arg Glu Leu Leu Gln Glu Leu Gly Arg Glu Pro Thr Pro Gln Glu
           355                 360                 365
   Leu Ser Lys Glu Met Asp Ile Ser Glu Glu Lys Val Leu Glu Ile Gln
       370                 375                 380
   Gln Tyr Ala Arg Glu Pro Ile Ser Leu Asp Gln Thr Ile Gly Asp Glu
   385                 390                 395                 400
   Gly Asp Ser Gln Leu Gly Asp Phe Ile Glu Asp Ser Glu Ala Val Val
                   405                 410                 415
   Ala Val Asp Ala Val Ser Phe Thr Leu Leu Gln Asp Gln Leu Gln Asp
               420                 425                 430
   Val Leu Glu Thr Leu Ser Glu Arg Glu Ala Gly Val Val Lys Leu Arg
           435                 440                 445
   Phe Gly Leu Thr Asp Gly Met Pro Arg Thr Leu Asp Glu Ile Gly Gln
       450                 455                 460
   Val Tyr Gly Val Thr Arg Glu Arg Ile Arg Gln Ile Glu Ser Lys Thr
   465                 470                 475                 480
   Met Ser Lys Leu Arg His Pro Ser Arg Ser Gln Val Leu Arg Asp Tyr
                   485                 490                 495
   Leu Asp
<210> 99
   <211> 1234
   <212> DNA
   <213> Corynebacterium glutamicum
<220>
   <221> CDS
   <222> (101)..(1204)
   <223> RXA06048
<400> 99
   agccgctgct gcagcgtgtc agacattttg gccgtcacct tatcaagttt gtggtgctat 60
   cttagagcgc tatatccagc agggtgccca gtttgggtgg atg ttg ttg ctt act   115
                                               Met Leu Leu Leu Thr
                                                1               5
   gaa ggc caa gcg ctt aac cct gat ggt cag gga tat cgt cag cgg ttt   163
   Glu Gly Gln Ala Leu Asn Pro Asp Gly Gln Gly Tyr Arg Gln Arg Phe
                   10                  15                  20
   atg aat ggg ttt atc tat tgg cat cct tct act ggt gcg cac gcg gtt   211
   Met Asn Gly Phe Ile Tyr Trp His Pro Ser Thr Gly Ala His Ala Val
               25                  30                  35
   aat aat tac agt gca caa gtc tgg gag cgt aac ggt tgg gag tct ggg   259
   Asn Asn Tyr Ser Ala Gln Val Trp Glu Arg Asn Gly Trp Glu Ser Gly
           40                  45                  50
   tgg atg ggg tat ccc act ggt ggt gaa gtc cct gtg tct ggg tct aat   307
   Trp Met Gly Tyr Pro Thr Gly Gly Glu Val Pro Val Ser Gly Ser Asn
       55                  60                  65
   ccg att gat ggt gag ttg agt ggg tgg gtg caa acc ttc caa ggt ggg   355
   Pro Ile Asp Gly Glu Leu Ser Gly Trp Val Gln Thr Phe Gln Gly Gly
   70                  75                  80                  85
   cga gtg tat cgc agt ccg gta ttg gac ggt ttc cag gtg gcc agt att   403
   Arg Val Tyr Arg Ser Pro Val Leu Asp Gly Phe Gln Val Ala Ser Ile
                   90                  95                  100
   aat ggg ctg atc ttg gat aaa tgg ctt gaa ttg ggt ggt cct gat agt   451
   Asn Gly Leu Ile Leu Asp Lys Trp Leu Glu Leu Gly Gly Pro Asp Ser
               105                 110                 115
   gac ctt ggt ttt ccc att gcg gat gag gct gtg aca gct gac ggt gtg   499
   Asp Leu Gly Phe Pro Ile Ala Asp Glu Ala Val Thr Ala Asp Gly Val
           120                 125                 130
   ggc aga ttt tct gtt ttc cag aac gga gtt gtc tac tgg cat ccg caa   547
   Gly Arg Phe Ser Val Phe Gln Asn Gly Val Val Tyr Trp His Pro Gln
       135                 140                 145
   cac gga gct cac cct ata tta ggg aat ata tac agc atc tgg aga gaa   595
   His Gly Ala His Pro Ile Leu Gly Asn Ile Tyr Ser Ile Trp Arg Glu
   150                 155                 160                 165
   gaa gga gct gag agt ggg gaa ttc ggt tac cct atc ggc gat cca gaa   643
   Glu Gly Ala Glu Ser Gly Glu Phe Gly Tyr Pro Ile Gly Asp Pro Glu
                   170                 175                 180
   aag tat aca gaa aac atg gct aat cag gta ttc gaa aaa ggc gaa ctt   691
   Lys Tyr Thr Glu Asn Met Ala Asn Gln Val Phe Glu Lys Gly Glu Leu
               185                 190                 195
   gca gct aac cta tac ccc aat cct ctt gag gct ttt att gag ttt tta   739
   Ala Ala Asn Leu Tyr Pro Asn Pro Leu Glu Ala Phe Ile Glu Phe Leu
           200                 205                 210
   ccc ttt gct aat ctt gag gaa gca ata gag tat ttt gag aac gga ttg     787
   Pro Phe Ala Asn Leu Glu Glu Ala Ile Glu Tyr Phe Glu Asn Gly Leu
       215                 220                 225
   tca aat tct cgt gta gag gcg aat tca ctt aac gcc aag aaa gat tcg     835
   Ser Asn Ser Arg Val Glu Ala Asn Ser Leu Asn Ala Lys Lys Asp Ser
   230                 235                 240                 245
   att caa tgt caa tcg caa tcc gct aac att cat gtg aga acg aag agt     883
   Ile Gln Cys Gln Ser Gln Ser Ala Asn Ile His Val Arg Thr Lys Ser
                   250                 255                 260
   gac gga gtc ggg att agg gtt cca aag att ggg ttt aag gct agg atg     931
   Asp Gly Val Gly Ile Arg Val Pro Lys Ile Gly Phe Lys Ala Arg Met
               265                 270                 275
   gat tgc gac ctt cct gga act gtc tca gat gta gtg ggg tat gga tgg     979
   Asp Cys Asp Leu Pro Gly Thr Val Ser Asp Val Val Gly Tyr Gly Trp
           280                 285                 290
   att tac tac gac tat tgg gga cga tgg gct caa gca gca tat gca caa     1027
   Ile Tyr Tyr Asp Tyr Trp Gly Arg Trp Ala Gln Ala Ala Tyr Ala Gln
       295                 300                 305
   caa ttc ttc ggt aat agg aat tct gtt gtg caa acc aat tta gag gcg     1075
   Gln Phe Phe Gly Asn Arg Asn Ser Val Val Gln Thr Asn Leu Glu Ala
   310                 315                 320                 325
   ggt tgc agc ggg gag aag aat aca tta ttt tgg ggt act tca tat ttt     1123
   Gly Cys Ser Gly Glu Lys Asn Thr Leu Phe Trp Gly Thr Ser Tyr Phe
                   330                 335                 340
   cag gtg act tat gaa ggt cag ccg tat ttc ggt cag tca gca act aac     1171
   Gln Val Thr Tyr Glu Gly Gln Pro Tyr Phe Gly Gln Ser Ala Thr Asn
               345                 350                 355
   tac gct tat ctt ccg tgt acg ata gac cgt agt taacataagg aatggaatag 1224
   Tyr Ala Tyr Leu Pro Cys Thr Ile Asp Arg Ser
           360                 365
   gagaattgcg                                                          1234
<210> 100
   <211> 368
   <212> PRT
   <213> Corynebacterium glutamicum
<400> 100
   Met Leu Leu Leu Thr Glu Gly Gln Ala Leu Asn Pro Asp Gly Gln Gly
    1               5                  10                  15
   Tyr Arg Gln Arg Phe Met Asn Gly Phe Ile Tyr Trp His Pro Ser Thr
               20                  25                  30
   Gly Ala His Ala Val Asn Asn Tyr Ser Ala Gln Val Trp Glu Arg Asn
           35                  40                  45
   Gly Trp Glu Ser Gly Trp Met Gly Tyr Pro Thr Gly Gly Glu Val Pro
       50                  55                  60
   Val Ser Gly Ser Asn Pro Ile Asp Gly Glu Leu Ser Gly Trp Val Gln
   65 70 75 80
   Thr Phe Gln Gly Gly Arg Val Tyr Arg Ser Pro Val Leu Asp Gly Phe
   85 90 95
   Gln Val Ala Ser Ile Asn Gly Leu Ile Leu Asp Lys Trp Leu Glu Leu
   100 105 110
   Gly Gly Pro Asp Ser Asp Leu Gly Phe Pro Ile Ala Asp Glu Ala Val
   115 120 125
   Thr Ala Asp Gly Val Gly Arg Phe Ser Val Phe Gln Asn Gly Val Val
   130 135 140
   Tyr Trp His Pro Gln His Gly Ala His Pro Ile Leu Gly Asn Ile Tyr
   145 150 155 160
   Ser Ile Trp Arg Glu Glu Gly Ala Glu Ser Gly Glu Phe Gly Tyr Pro
   165 170 175
   Ile Gly Asp Pro Glu Lys Tyr Thr Glu Asn Met Ala Asn Gln Val Phe
   180 185 190
   Glu Lys Gly Glu Leu Ala Ala Asn Leu Tyr Pro Asn Pro Leu Glu Ala
   195 200 205
   Phe Ile Glu Phe Leu Pro Phe Ala Asn Leu Glu Glu Ala Ile Glu Tyr
   210 215 220
   Phe Glu Asn Gly Leu Ser Asn Ser Arg Val Glu Ala Asn Ser Leu Asn
   225 230 235 240
   Ala Lys Lys Asp Ser Ile Gln Cys Gln Ser Gln Ser Ala Asn Ile His
   245 250 255
   Val Arg Thr Lys Ser Asp Gly Val Gly Ile Arg Val Pro Lys Ile Gly
   260 265 270
   Phe Lys Ala Arg Met Asp Cys Asp Leu Pro Gly Thr Val Ser Asp Val
   275 280 285
   Val Gly Tyr Gly Trp Ile Tyr Tyr Asp Tyr Trp Gly Arg Trp Ala Gln
   290 295 300
   Ala Ala Tyr Ala Gln Gln Phe Phe Gly Asn Arg Asn Ser Val Val Gln
   305 310 315 320
   Thr Asn Leu Glu Ala Gly Cys Ser Gly Glu Lys Asn Thr Leu Phe Trp
   325 330 335
   Gly Thr Ser Tyr Phe Gln Val Thr Tyr Glu Gly Gln Pro Tyr Phe Gly
   340 345 350
   Gln Ser Ala Thr Asn Tyr Ala Tyr Leu Pro Cys Thr Ile Asp Arg Ser
   355 360 365
<210> 101
   <211> 4560
   <212> DNA
   <213> Corynebacterium glutamicum
<220>
   <221> CDS
   <222> (1)..(4560)
   <223> RXA07005
<400> 101
   atg gct aaa agc att ctt tcc cga ttc cga cct caa gta gcg gag tgg    48
   Met Ala Lys Ser Ile Leu Ser Arg Phe Arg Pro Gln Val Ala Glu Trp
     1               5                  10                  15
   ttc cgg gat gtc ttt gca tct ccg acc cct gtt cag gag gga acg tgg    96
   Phe Arg Asp Val Phe Ala Ser Pro Thr Pro Val Gln Glu Gly Thr Trp
                20                  25                  30
   gag gcg gta tct aag ggg aag aat gcc ctc gtg gtg gcg ccg acc ggt   144
   Glu Ala Val Ser Lys Gly Lys Asn Ala Leu Val Val Ala Pro Thr Gly
            35                  40                  45
   agt ggt aaa acc ttg gct gcg ttt ttg tgg gcg tta gat tcc ctc act   192
   Ser Gly Lys Thr Leu Ala Ala Phe Leu Trp Ala Leu Asp Ser Leu Thr
        50                  55                  60
   gaa caa aca ggt caa cag gtt tta gac acg gga aca ccg gtg cct gtt   240
   Glu Gln Thr Gly Gln Gln Val Leu Asp Thr Gly Thr Pro Val Pro Val
    65                  70                  75                  80
   cgt ggt ggg aaa gtg aaa gtg ctc tac att tcc cca ctc aaa gcg ctt   288
   Arg Gly Gly Lys Val Lys Val Leu Tyr Ile Ser Pro Leu Lys Ala Leu
                    85                  90                  95
   ggc gtg gat gta gaa aat aat ctg cgt gca ccg ttg acc ggt att gcg   336
   Gly Val Asp Val Glu Asn Asn Leu Arg Ala Pro Leu Thr Gly Ile Ala
               100                 105                 110
   agg act gcc tct cgg atg ggt ttg gat gtg ccc aat atc act gtg gcg   384
   Arg Thr Ala Ser Arg Met Gly Leu Asp Val Pro Asn Ile Thr Val Ala
           115                 120                 125
   gtt cgt tcg ggt gat acg cca tcg gcg gag cgg gcc cgg cag gtg cgt   432
   Val Arg Ser Gly Asp Thr Pro Ser Ala Glu Arg Ala Arg Gln Val Arg
       130                 135                 140
   aag cct cca gac att ttg atc acc act ccg gag tcg gcg tat ttg atg   480
   Lys Pro Pro Asp Ile Leu Ile Thr Thr Pro Glu Ser Ala Tyr Leu Met
   145                 150                 155                 160
   ttg acc tca aaa gcg ggg gcg acc ctt tcg gat gtt gat gtg gtg atc   528
   Leu Thr Ser Lys Ala Gly Ala Thr Leu Ser Asp Val Asp Val Val Ile
                   165                 170                 175
   atc gat gaa atc cac gcc atg gcc gga acc aaa cgg gga gtg cat ctg   576
   Ile Asp Glu Ile His Ala Met Ala Gly Thr Lys Arg Gly Val His Leu
               180                 185                 190
   gcg ttg acg ctg gag cgt ttg gaa aag ctc gtg ggg cgg cct gtg cag   624
   Ala Leu Thr Leu Glu Arg Leu Glu Lys Leu Val Gly Arg Pro Val Gln
           195                 200                 205
   cga gtt ggt ttg tct gca acg gtg cgt cct ttg gaa acg gtg gcg ggt   672
   Arg Val Gly Leu Ser Ala Thr Val Arg Pro Leu Glu Thr Val Ala Gly
       210                 215                 220
   ttc ttg ggc ggt ggc aga ccc gtt gag att gtg gct cca cct gcg gag   720
   Phe Leu Gly Gly Gly Arg Pro Val Glu Ile Val Ala Pro Pro Ala Glu
   225                 230                 235                 240
   aaa aag tgg gat ctc act gtc act gtg ccg gtg gaa gac atg tcg gat     768
   Lys Lys Trp Asp Leu Thr Val Thr Val Pro Val Glu Asp Met Ser Asp
                   245                 250                 255
   ttg ccg gtt cag gag ccg gga tca act att ggt gaa cta gtc atg gat     816
   Leu Pro Val Gln Glu Pro Gly Ser Thr Ile Gly Glu Leu Val Met Asp
               260                 265                 270
   gat ccg ttg ggg att act ggc gaa tca gcg ctg cct act caa ggc tcg     864
   Asp Pro Leu Gly Ile Thr Gly Glu Ser Ala Leu Pro Thr Gln Gly Ser
           275                 280                 285
   att tgg cca cac att gag cag cag gtg tac aac cag gtg atg tcg gcg     912
   Ile Trp Pro His Ile Glu Gln Gln Val Tyr Asn Gln Val Met Ser Ala
       290                 295                 300
   aaa tcg acc atc gtg ttt gta aat tcc agg cgt tcc gcg gag cgt tta     960
   Lys Ser Thr Ile Val Phe Val Asn Ser Arg Arg Ser Ala Glu Arg Leu
   305                 310                 315                 320
   acc agt cgg ttg aat gaa atc tgg gcg atg gaa cac gat ccg gaa tcg    1008
   Thr Ser Arg Leu Asn Glu Ile Trp Ala Met Glu His Asp Pro Glu Ser
                   325                 330                 335
   ctg tcg ccg cag ctg cga aga gat ccg gcg cag att atg tcg tca gcg    1056
   Leu Ser Pro Gln Leu Arg Arg Asp Pro Ala Gln Ile Met Ser Ser Ala
               340                 345                 350
   gat gtg gca gga aaa gca cca cag gtg atc gca cgt gcg cac cac gga    1104
   Asp Val Ala Gly Lys Ala Pro Gln Val Ile Ala Arg Ala His His Gly
           355                 360                 365
   tcc gta tcc aaa gat gaa cgt gcc acc acc gaa acc atg ctg aag gaa    1152
   Ser Val Ser Lys Asp Glu Arg Ala Thr Thr Glu Thr Met Leu Lys Glu
       370                 375                 380
   ggt cgg ttg cgc gca gtt att tcc acc tcc tcg ctg gag ttg ggc att    1200
   Gly Arg Leu Arg Ala Val Ile Ser Thr Ser Ser Leu Glu Leu Gly Ile
   385                 390                 395                 400
   gat atg ggt gcc gtg gac ctg gtg att cag gtg gaa tcg cca ccg tcc    1248
   Asp Met Gly Ala Val Asp Leu Val Ile Gln Val Glu Ser Pro Pro Ser
                   405                 410                 415
   gtg gca agt ggc ctg cag cgc gtg ggg cgt gcg ggg cac acg gtg ggg    1296
   Val Ala Ser Gly Leu Gln Arg Val Gly Arg Ala Gly His Thr Val Gly
               420                 425                 430
   gcg acg tcg ata ggc tcc ttt tat ccc aag cac cgc tcc gac ttg gtg    1344
   Ala Thr Ser Ile Gly Ser Phe Tyr Pro Lys His Arg Ser Asp Leu Val
           435                 440                 445
   caa acc gcg gtg acc gtg cag cgg atg aag gaa ggg ctg atc gaa gag    1392
   Gln Thr Ala Val Thr Val Gln Arg Met Lys Glu Gly Leu Ile Glu Glu
       450                 455                 460
   atc cac gtg ccc aaa aac gcg ctt gat gta ctg gca cag cag acg gtg    1440
   Ile His Val Pro Lys Asn Ala Leu Asp Val Leu Ala Gln Gln Thr Val
   465                 470                 475                 480
   gcg gct gtc tcg att aaa gat gtg cag gtc gat gag tgg tac gag act    1488
   Ala Ala Val Ser Ile Lys Asp Val Gln Val Asp Glu Trp Tyr Glu Thr
                   485                 490                 495
   att cgc aag gcg tat ccg tac cgg gat ttg gcg cgc gaa gtc ttc gat    1536
   Ile Arg Lys Ala Tyr Pro Tyr Arg Asp Leu Ala Arg Glu Val Phe Asp
               500                 505                 510
   tcc gtc atc gac ctg gtc agc ggt gtg tat ccc tcc aca gat ttt gcc    1584
   Ser Val Ile Asp Leu Val Ser Gly Val Tyr Pro Ser Thr Asp Phe Ala
           515                 520                 525
   gag ctg aag cca cgt gtg gtg tac gac cgg gtt tca ggc gtg ctg gag    1632
   Glu Leu Lys Pro Arg Val Val Tyr Asp Arg Val Ser Gly Val Leu Glu
       530                 535                 540
   ggc cgg cca gga tcc caa cgc gta gca gtg acc agt ggc gga aca att    1680
   Gly Arg Pro Gly Ser Gln Arg Val Ala Val Thr Ser Gly Gly Thr Ile
   545                 550                 555                 560
   ccc gat cga gga atg ttc gga gtc ttc ctc gtc ggc gat ggt ccc cgg    1728
   Pro Asp Arg Gly Met Phe Gly Val Phe Leu Val Gly Asp Gly Pro Arg
                   565                 570                 575
   cgc gtc ggc gag ctc gat gag gaa atg gtc tac gaa tcc cgc gtg ggc    1776
   Arg Val Gly Glu Leu Asp Glu Glu Met Val Tyr Glu Ser Arg Val Gly
               580                 585                 590
   gat gtg ttt acg ctc ggg gcg tcg agt tgg cgg att gaa gag atc acc    1824
   Asp Val Phe Thr Leu Gly Ala Ser Ser Trp Arg Ile Glu Glu Ile Thr
           595                 600                 605
   cgc gac cag gta ctg gtc act ccc gcg ccg ggt cac acg ggt cgg ctg    1872
   Arg Asp Gln Val Leu Val Thr Pro Ala Pro Gly His Thr Gly Arg Leu
       610                 615                 620
   cct ttt tgg acg ggc gat gcc gca ggc cgg ccc gct gag ctg ggt aaa    1920
   Pro Phe Trp Thr Gly Asp Ala Ala Gly Arg Pro Ala Glu Leu Gly Lys
   625                 630                 635                 640
   gct tta ggc gct ttt cga cgc tcg acc ctc acc gat cca tcc agc tcc    1968
   Ala Leu Gly Ala Phe Arg Arg Ser Thr Leu Thr Asp Pro Ser Ser Ser
                   645                 650                 655
   ggc ttg gaa ggc tgg gcg cac gac aac ctg atc gcc ttt tta cag gag    2016
   Gly Leu Glu Gly Trp Ala His Asp Asn Leu Ile Ala Phe Leu Gln Glu
               660                 665                 670
   cag gaa gaa tcc acc ggt gtg ttg ccg gat gag aag acg ttg gtg ttg    2064
   Gln Glu Glu Ser Thr Gly Val Leu Pro Asp Glu Lys Thr Leu Val Leu
           675                 680                 685
   gag cgt ttc aaa gat gaa cta ggc gac tgg cgc att gtc ctg cac act    2112
   Glu Arg Phe Lys Asp Glu Leu Gly Asp Trp Arg Ile Val Leu His Thr
       690                 695                 700
   cct tat gga cga gga gta aac gca gca tgg gct ttg gcc gtc ggg gcg    2160
   Pro Tyr Gly Arg Gly Val Asn Ala Ala Trp Ala Leu Ala Val Gly Ala
   705                 710                 715                 720
   aaa atc gct gaa gag acc ggc atg gat gcg caa gcc gtg gca ggt gat    2208
   Lys Ile Ala Glu Glu Thr Gly Met Asp Ala Gln Ala Val Ala Gly Asp
                   725                 730                 735
   gat ggc att gtg ctt cgg ttg ccg gaa ggg gat gaa gat ccc agc gca    2256
   Asp Gly Ile Val Leu Arg Leu Pro Glu Gly Asp Glu Asp Pro Ser Ala
               740                 745                 750
   gcg ttg ttt atg ttt gag gcg gaa gag atc gaa acg cta gtg aca gag    2304
   Ala Leu Phe Met Phe Glu Ala Glu Glu Ile Glu Thr Leu Val Thr Glu
           755                 760                 765
   cag gtg ggt aac tct gcg ctg ttt gcc agc agg ttc cgt gaa tgc gcc    2352
   Gln Val Gly Asn Ser Ala Leu Phe Ala Ser Arg Phe Arg Glu Cys Ala
       770                 775                 780
   gcg agg gcc cta ttg ctg ccg aga cga aac ccc ggc aag cgc gca ccg    2400
   Ala Arg Ala Leu Leu Leu Pro Arg Arg Asn Pro Gly Lys Arg Ala Pro
   785                 790                 795                 800
   ctg tgg cag caa cga caa cga gca gca cag ctt ctt gat gtg gcc aga    2448
   Leu Trp Gln Gln Arg Gln Arg Ala Ala Gln Leu Leu Asp Val Ala Arg
                   805                 810                 815
   aag tac ccg agt ttc ccg atc att ttg gaa aca gtg cgc gaa tgt ctt    2496
   Lys Tyr Pro Ser Phe Pro Ile Ile Leu Glu Thr Val Arg Glu Cys Leu
               820                 825                 830
   caa gat gtt tac gat ctg ccc gct ctg aag aat ctc att gag gat cta    2544
   Gln Asp Val Tyr Asp Leu Pro Ala Leu Lys Asn Leu Ile Glu Asp Leu
           835                 840                 845
   cag ctg cgg aag gta aga atc gcg gaa gtc acc acc cag cag ccc agt    2592
   Gln Leu Arg Lys Val Arg Ile Ala Glu Val Thr Thr Gln Gln Pro Ser
       850                 855                 860
   cct ttt gcc tcc gca ttg ctg ttc aat tac acc ggt gca ttc atg tac    2640
   Pro Phe Ala Ser Ala Leu Leu Phe Asn Tyr Thr Gly Ala Phe Met Tyr
   865                 870                 875                 880
   gaa ggc gac agc ccg ctc gca gag aaa cgt gcc gca gcg ttg gcc ctg    2688
   Glu Gly Asp Ser Pro Leu Ala Glu Lys Arg Ala Ala Ala Leu Ala Leu
                   885                 890                 895
   gat ccg gca ctg ttg gcg aaa ttg ctg ggt gag gtg gag ctt cga caa    2736
   Asp Pro Ala Leu Leu Ala Lys Leu Leu Gly Glu Val Glu Leu Arg Gln
               900                 905                 910
   tta ctg gat ccc gac atc atc gca gaa gtg cac caa caa ttg cgc agg    2784
   Leu Leu Asp Pro Asp Ile Ile Ala Glu Val His Gln Gln Leu Arg Arg
           915                 920                 925
   caa ggc gat cgt gcg gcg aga aac aat gaa gaa ctc gca gat tct ttg    2832
   Gln Gly Asp Arg Ala Ala Arg Asn Asn Glu Glu Leu Ala Asp Ser Leu
       930                 935                 940
   agg att tta gga ccg att cct ttg gat gaa ttg ggc gaa cac atc acc    2880
   Arg Ile Leu Gly Pro Ile Pro Leu Asp Glu Leu Gly Glu His Ile Thr
   945                 950                 955                 960
   ttt gaa aac cca gac ctg gag gat cga gca atg act gtt cgg atc aac    2928
   Phe Glu Asn Pro Asp Leu Glu Asp Arg Ala Met Thr Val Arg Ile Asn
                   965                 970                 975
   ggt cgg gaa cat tta gcg cag gtc ttg gat gca cct ttg ctt cga gat    2976
   Gly Arg Glu His Leu Ala Gln Val Leu Asp Ala Pro Leu Leu Arg Asp
               980                 985                 990
   gcc tta ggt gtt ccc gta ccg cct ggt gtg cct gcg cag gta gaa acc    3024
   Ala Leu Gly Val Pro Val Pro Pro Gly Val Pro Ala Gln Val Glu Thr
           995                1000                1005
   att acg gat gcg ttg gaa cag tta gtc aac agg tgg gtt cgt acc aga    3072
   Ile Thr Asp Ala Leu Glu Gln Leu Val Asn Arg Trp Val Arg Thr Arg
       1010                1015                1020
   ggg cca ttt act gcg aat gat ttg gca gaa gcc ttt gga ctg ggc atc    3120
   Gly Pro Phe Thr Ala Asn Asp Leu Ala Glu Ala Phe Gly Leu Gly Ile
   1025                1030                1035                1040
   gcc acg gcg atc acc gcc ctt caa agc gca cct gtg att gaa ggc cgc    3168
   Ala Thr Ala Ile Thr Ala Leu Gln Ser Ala Pro Val Ile Glu Gly Arg
                   1045                1050                1055
   tac cga caa ggc gtg gac gtg cag gaa tac tgt gcg aca gaa gtg ttg    3216
   Tyr Arg Gln Gly Val Asp Val Gln Glu Tyr Cys Ala Thr Glu Val Leu
               1060                1065                1070
   tcg atc ata agg cga cgc agc ctc gca gca gcg agg aaa caa acc agg    3264
   Ser Ile Ile Arg Arg Arg Ser Leu Ala Ala Ala Arg Lys Gln Thr Arg
           1075                1080                1085
   ccg gta tcg caa tca gcc ttt gcg cga ttc ctg ctt gat tgg caa cag    3312
   Pro Val Ser Gln Ser Ala Phe Ala Arg Phe Leu Leu Asp Trp Gln Gln
       1090                1095                1100
   atc gca ccg gtg ggc gcc aca cct gaa ctc cga ggc gtt gat ggc acc    3360
   Ile Ala Pro Val Gly Ala Thr Pro Glu Leu Arg Gly Val Asp Gly Thr
   1105                1110                1115                1120
   tac aca gtc att gaa caa ctc gcc ggt gta cgt ctt ccc gcc agt gcg    3408
   Tyr Thr Val Ile Glu Gln Leu Ala Gly Val Arg Leu Pro Ala Ser Ala
                   1125                1130                1135
   tgg gaa gat ctc gtg ttg ccg cgc cgg gtt gcc gac tat tca ccg atc    3456
   Trp Glu Asp Leu Val Leu Pro Arg Arg Val Ala Asp Tyr Ser Pro Ile
               1140                1145                1150
   cat ctc gat gag ctg acc tcc aat ggg gaa gtc ctc atc gtg gga gcg    3504
   His Leu Asp Glu Leu Thr Ser Asn Gly Glu Val Leu Ile Val Gly Ala
           1155                1160                1165
   ggc caa gcc gga agc cgc gat ccg tgg att agc ttg ctg ccc gtg gat    3552
   Gly Gln Ala Gly Ser Arg Asp Pro Trp Ile Ser Leu Leu Pro Val Asp
       1170                1175                1180
   tat gcg gcg cag ttg gtg ggg gag gcg tcg aca agc atg agc cca ttg    3600
   Tyr Ala Ala Gln Leu Val Gly Glu Ala Ser Thr Ser Met Ser Pro Leu
   1185                1190                1195                1200
   cag gac gcc gtg ctt gac cag ctg cgt gcg gga ggc gcc ttc ctg ttt    3648
   Gln Asp Ala Val Leu Asp Gln Leu Arg Ala Gly Gly Ala Phe Leu Phe
                   1205                1210                1215
   tct gac att ctc gaa gag aat ttc ggc tac acc aca gcc cag ctg caa    3696
   Ser Asp Ile Leu Glu Glu Asn Phe Gly Tyr Thr Thr Ala Gln Leu Gln
               1220                1225                1230
   gaa gcg atg tgg ggg ctg gtg gaa gca ggc ctg gtc agc cct gat agc    3744
   Glu Ala Met Trp Gly Leu Val Glu Ala Gly Leu Val Ser Pro Asp Ser
           1235                1240                1245
   ttc gcg ccg atc cgc gcg cgc cta gcg tcg gga acc acg gcg cat cgg 3792
   Phe Ala Pro Ile Arg Ala Arg Leu Ala Ser Gly Thr Thr Ala His Arg
   1250 1255 1260
   gcg aaa cgt cga cca gcg aga tcc cgg ctg cgc acc cgc acc agc ttc 3840
   Ala Lys Arg Arg Pro Ala Arg Ser Arg Leu Arg Thr Arg Thr Ser Phe
   1265 1270 1275 1280
   gcg agc gac gtg ccc cca gac atg cgc gga cga tgg acg ctg tcc gtg 3888
   Ala Ser Asp Val Pro Pro Asp Met Arg Gly Arg Trp Thr Leu Ser Val
   1285 1290 1295
   caa ccc gcc gac gcc acc agc cgc tcc gtc gca cac ggc gaa ggc tgg 3936
   Gln Pro Ala Asp Ala Thr Ser Arg Ser Val Ala His Gly Glu Gly Trp
   1300 1305 1310
   ctc gac cgc tac ggc gtg ctc acc cgc ggg agc gtc gtc gcc gaa gac 3984
   Leu Asp Arg Tyr Gly Val Leu Thr Arg Gly Ser Val Val Ala Glu Asp
   1315 1320 1325
   atc gtc gga ggc ttc gcc ctg gcc tac aaa gtg ctc tcc ggc ttc gaa 4032
   Ile Val Gly Gly Phe Ala Leu Ala Tyr Lys Val Leu Ser Gly Phe Glu
   1330 1335 1340
   gaa agc ggc aaa gcg atg cgc ggc tac ttc atc gaa ggg ctc ggc gcc 4080
   Glu Ser Gly Lys Ala Met Arg Gly Tyr Phe Ile Glu Gly Leu Gly Ala
   1345 1350 1355 1360
   gcg caa ttc tcc acg ccc gcc atc atc gac cgc ctc cgc ggc cac gac 4128
   Ala Gln Phe Ser Thr Pro Ala Ile Ile Asp Arg Leu Arg Gly His Asp
   1365 1370 1375
   gat tcc ccc gac gtc gaa ggc tgg ccc tcc ggc gcc acc gac cca gac 4176
   Asp Ser Pro Asp Val Glu Gly Trp Pro Ser Gly Ala Thr Asp Pro Asp
   1380 1385 1390
   gtc tac ctc ata gcc gcc gcc gac ccc gca aac ccc tac ggc gcc gca 4224
   Val Tyr Leu Ile Ala Ala Ala Asp Pro Ala Asn Pro Tyr Gly Ala Ala
   1395 1400 1405
   ctt ccc tgg cct gag cag ggg ccc agc cgc gcc gcc gga gct atg gtc 4272
   Leu Pro Trp Pro Glu Gln Gly Pro Ser Arg Ala Ala Gly Ala Met Val
   1410 1415 1420
   gtg ctt tgc gac gga ctc ctc ctc gcc cac ctc acc cgc ggc ggg cgc 4320
   Val Leu Cys Asp Gly Leu Leu Leu Ala His Leu Thr Arg Gly Gly Arg
   1425 1430 1435 1440
   acc ctc acc gtg ttc tcc gac aat atc ccc aaa atc gcg aca gcc cta 4368
   Thr Leu Thr Val Phe Ser Asp Asn Ile Pro Lys Ile Ala Thr Ala Leu
   1445 1450 1455
   atc aca tac gaa agg ctc acg gta gaa aaa atc aac ggc gac aac gtc 4416
   Ile Thr Tyr Glu Arg Leu Thr Val Glu Lys Ile Asn Gly Asp Asn Val
   1460 1465 1470
   ttc gac tcc cca ctc ctg gaa caa ttc cgc aaa cac ggc gcc acc atc 4464
   Phe Asp Ser Pro Leu Leu Glu Gln Phe Arg Lys His Gly Ala Thr Ile
   1475 1480 1485
   acc ccg aag gga atg cga ttt cga cca cca gtg gca cgg gaa acc ccc 4512
   Thr Pro Lys Gly Met Arg Phe Arg Pro Pro Val Ala Arg Glu Thr Pro
   1490 1495 1500
   tca gat acg ctt ccc acc agg act ttt cgt gga ggc ttc gga cgg cgc    4560
   Ser Asp Thr Leu Pro Thr Arg Thr Phe Arg Gly Gly Phe Gly Arg Arg
   1505                1510                1515                1520
<210> 102
   <211> 1520
   <212> PRT
   <213> Corynebacterium glutamicum
<400> 102
   Met Ala Lys Ser Ile Leu Ser Arg Phe Arg Pro Gln Val Ala Glu Trp
   1               5                   10                  15
   Phe Arg Asp Val Phe Ala Ser Pro Thr Pro Val Gln Glu Gly Thr Trp
               20                  25                  30
   Glu Ala Val Ser Lys Gly Lys Asn Ala Leu Val Val Ala Pro Thr Gly
           35                  40                  45
   Ser Gly Lys Thr Leu Ala Ala Phe Leu Trp Ala Leu Asp Ser Leu Thr
       50                  55                  60
   Glu Gln Thr Gly Gln Gln Val Leu Asp Thr Gly Thr Pro Val Pro Val
   65                  70                  75                  80
   Arg Gly Gly Lys Val Lys Val Leu Tyr Ile Ser Pro Leu Lys Ala Leu
                   85                  90                  95
   Gly Val Asp Val Glu Asn Asn Leu Arg Ala Pro Leu Thr Gly Ile Ala
               100                 105                 110
   Arg Thr Ala Ser Arg Met Gly Leu Asp Val Pro Asn Ile Thr Val Ala
           115                 120                 125
   Val Arg Ser Gly Asp Thr Pro Ser Ala Glu Arg Ala Arg Gln Val Arg
       130                 135                 140
   Lys Pro Pro Asp Ile Leu Ile Thr Thr Pro Glu Ser Ala Tyr Leu Met
   145                 150                 155                 160
   Leu Thr Ser Lys Ala Gly Ala Thr Leu Ser Asp Val Asp Val Val Ile
                   165                 170                 175
   Ile Asp Glu Ile His Ala Met Ala Gly Thr Lys Arg Gly Val His Leu
               180                 185                 190
   Ala Leu Thr Leu Glu Arg Leu Glu Lys Leu Val Gly Arg Pro Val Gln
           195                 200                 205
   Arg Val Gly Leu Ser Ala Thr Val Arg Pro Leu Glu Thr Val Ala Gly
       210                 215                 220
   Phe Leu Gly Gly Gly Arg Pro Val Glu Ile Val Ala Pro Pro Ala Glu
   225                 230                 235                 240
   Lys Lys Trp Asp Leu Thr Val Thr Val Pro Val Glu Asp Met Ser Asp
                   245                 250                 255
   Leu Pro Val Gln Glu Pro Gly Ser Thr Ile Gly Glu Leu Val Met Asp
               260                 265                 270
   Asp Pro Leu Gly Ile Thr Gly Glu Ser Ala Leu Pro Thr Gln Gly Ser
   275 280 285
   Ile Trp Pro His Ile Glu Gln Gln Val Tyr Asn Gln Val Met Ser Ala
   290 295 300
   Lys Ser Thr Ile Val Phe Val Asn Ser Arg Arg Ser Ala Glu Arg Leu
   305 310 315 320
   Thr Ser Arg Leu Asn Glu Ile Trp Ala Met Glu His Asp Pro Glu Ser
   325 330 335
   Leu Ser Pro Gln Leu Arg Arg Asp Pro Ala Gln Ile Met Ser Ser Ala
   340 345 350
   Asp Val Ala Gly Lys Ala Pro Gln Val Ile Ala Arg Ala His His Gly
   355 360 365
   Ser Val Ser Lys Asp Glu Arg Ala Thr Thr Glu Thr Met Leu Lys Glu
   370 375 380
   Gly Arg Leu Arg Ala Val Ile Ser Thr Ser Ser Leu Glu Leu Gly Ile
   385 390 395 400
   Asp Met Gly Ala Val Asp Leu Val Ile Gln Val Glu Ser Pro Pro Ser
   405 410 415
   Val Ala Ser Gly Leu Gln Arg Val Gly Arg Ala Gly His Thr Val Gly
   420 425 430
   Ala Thr Ser Ile Gly Ser Phe Tyr Pro Lys His Arg Ser Asp Leu Val
   435 440 445
   Gln Thr Ala Val Thr Val Gln Arg Met Lys Glu Gly Leu Ile Glu Glu
   450 455 460
   Ile His Val Pro Lys Asn Ala Leu Asp Val Leu Ala Gln Gln Thr Val
   465 470 475 480
   Ala Ala Val Ser Ile Lys Asp Val Gln Val Asp Glu Trp Tyr Glu Thr
   485 490 495
   Ile Arg Lys Ala Tyr Pro Tyr Arg Asp Leu Ala Arg Glu Val Phe Asp
   500 505 510
   Ser Val Ile Asp Leu Val Ser Gly Val Tyr Pro Ser Thr Asp Phe Ala
   515 520 525
   Glu Leu Lys Pro Arg Val Val Tyr Asp Arg Val Ser Gly Val Leu Glu
   530 535 540
   Gly Arg Pro Gly Ser Gln Arg Val Ala Val Thr Ser Gly Gly Thr Ile
   545 550 555 560
   Pro Asp Arg Gly Met Phe Gly Val Phe Leu Val Gly Asp Gly Pro Arg
   565 570 575
   Arg Val Gly Glu Leu Asp Glu Glu Met Val Tyr Glu Ser Arg Val Gly
   580 585 590
   Asp Val Phe Thr Leu Gly Ala Ser Ser Trp Arg Ile Glu Glu Ile Thr
   595 600 605
   Arg Asp Gln Val Leu Val Thr Pro Ala Pro Gly His Thr Gly Arg Leu
   610 615 620
   Pro Phe Trp Thr Gly Asp Ala Ala Gly Arg Pro Ala Glu Leu Gly Lys
   625 630 635 640
   Ala Leu Gly Ala Phe Arg Arg Ser Thr Leu Thr Asp Pro Ser Ser Ser
   645 650 655
   Gly Leu Glu Gly Trp Ala His Asp Asn Leu Ile Ala Phe Leu Gln Glu
   660 665 670
   Gln Glu Glu Ser Thr Gly Val Leu Pro Asp Glu Lys Thr Leu Val Leu
   675 680 685
   Glu Arg Phe Lys Asp Glu Leu Gly Asp Trp Arg Ile Val Leu His Thr
   690 695 700
   Pro Tyr Gly Arg Gly Val Asn Ala Ala Trp Ala Leu Ala Val Gly Ala
   705 710 715 720
   Lys Ile Ala Glu Glu Thr Gly Met Asp Ala Gln Ala Val Ala Gly Asp
   725 730 735
   Asp Gly Ile Val Leu Arg Leu Pro Glu Gly Asp Glu Asp Pro Ser Ala
   740 745 750
   Ala Leu Phe Met Phe Glu Ala Glu Glu Ile Glu Thr Leu Val Thr Glu
   755 760 765
   Gln Val Gly Asn Ser Ala Leu Phe Ala Ser Arg Phe Arg Glu Cys Ala
   770 775 780
   Ala Arg Ala Leu Leu Leu Pro Arg Arg Asn Pro Gly Lys Arg Ala Pro
   785 790 795 800
   Leu Trp Gln Gln Arg Gln Arg Ala Ala Gln Leu Leu Asp Val Ala Arg
   805 810 815
   Lys Tyr Pro Ser Phe Pro Ile Ile Leu Glu Thr Val Arg Glu Cys Leu
   820 825 830
   Gln Asp Val Tyr Asp Leu Pro Ala Leu Lys Asn Leu Ile Glu Asp Leu
   835 840 845
   Gln Leu Arg Lys Val Arg Ile Ala Glu Val Thr Thr Gln Gln Pro Ser
   850 855 860
   Pro Phe Ala Ser Ala Leu Leu Phe Asn Tyr Thr Gly Ala Phe Met Tyr
   865 870 875 880
   Glu Gly Asp Ser Pro Leu Ala Glu Lys Arg Ala Ala Ala Leu Ala Leu
   885 890 895
   Asp Pro Ala Leu Leu Ala Lys Leu Leu Gly Glu Val Glu Leu Arg Gln
   900 905 910
   Leu Leu Asp Pro Asp Ile Ile Ala Glu Val His Gln Gln Leu Arg Arg
   915 920 925
   Gln Gly Asp Arg Ala Ala Arg Asn Asn Glu Glu Leu Ala Asp Ser Leu
   930 935 940
   Arg Ile Leu Gly Pro Ile Pro Leu Asp Glu Leu Gly Glu His Ile Thr
   945 950 955 960
   Phe Glu Asn Pro Asp Leu Glu Asp Arg Ala Met Thr Val Arg Ile Asn
   965 970 975
   Gly Arg Glu His Leu Ala Gln Val Leu Asp Ala Pro Leu Leu Arg Asp
   980 985 990
   Ala Leu Gly Val Pro Val Pro Pro Gly Val Pro Ala Gln Val Glu Thr
   995 1000 1005
   Ile Thr Asp Ala Leu Glu Gln Leu Val Asn Arg Trp Val Arg Thr Arg
   1010 1015 1020
   Gly Pro Phe Thr Ala Asn Asp Leu Ala Glu Ala Phe Gly Leu Gly Ile
   1025 1030 1035 1040
   Ala Thr Ala Ile Thr Ala Leu Gln Ser Ala Pro Val Ile Glu Gly Arg
   1045 1050 1055
   Tyr Arg Gln Gly Val Asp Val Gln Glu Tyr Cys Ala Thr Glu Val Leu
   1060 1065 1070
   Ser Ile Ile Arg Arg Arg Ser Leu Ala Ala Ala Arg Lys Gln Thr Arg
   1075 1080 1085
   Pro Val Ser Gln Ser Ala Phe Ala Arg Phe Leu Leu Asp Trp Gln Gln
   1090 1095 1100
   Ile Ala Pro Val Gly Ala Thr Pro Glu Leu Arg Gly Val Asp Gly Thr
   1105 1110 1115 1120
   Tyr Thr Val Ile Glu Gln Leu Ala Gly Val Arg Leu Pro Ala Ser Ala
   1125 1130 1135
   Trp Glu Asp Leu Val Leu Pro Arg Arg Val Ala Asp Tyr Ser Pro Ile
   1140 1145 1150
   His Leu Asp Glu Leu Thr Ser Asn Gly Glu Val Leu Ile Val Gly Ala
   1155 1160 1165
   Gly Gln Ala Gly Ser Arg Asp Pro Trp Ile Ser Leu Leu Pro Val Asp
   1170 1175 1180
   Tyr Ala Ala Gln Leu Val Gly Glu Ala Ser Thr Ser Met Ser Pro Leu
   1185 1190 1195 1200
   Gln Asp Ala Val Leu Asp Gln Leu Arg Ala Gly Gly Ala Phe Leu Phe
   1205 1210 1215
   Ser Asp Ile Leu Glu Glu Asn Phe Gly Tyr Thr Thr Ala Gln Leu Gln
   1220 1225 1230
   Glu Ala Met Trp Gly Leu Val Glu Ala Gly Leu Val Ser Pro Asp Ser
   1235 1240 1245
   Phe Ala Pro Ile Arg Ala Arg Leu Ala Ser Gly Thr Thr Ala His Arg
   1250 1255 1260
   Ala Lys Arg Arg Pro Ala Arg Ser Arg Leu Arg Thr Arg Thr Ser Phe
   1265 1270 1275 1280
   Ala Ser Asp Val Pro Pro Asp Met Arg Gly Arg Trp Thr Leu Ser Val
   1285 1290 1295
   Gln Pro Ala Asp Ala Thr Ser Arg Ser Val Ala His Gly Glu Gly Trp
   1300 1305 1310
   Leu Asp Arg Tyr Gly Val Leu Thr Arg Gly Ser Val Val Ala Glu Asp
   1315 1320 1325
   Ile Val Gly Gly Phe Ala Leu Ala Tyr Lys Val Leu Ser Gly Phe Glu
   1330 1335 1340
   Glu Ser Gly Lys Ala Met Arg Gly Tyr Phe Ile Glu Gly Leu Gly Ala
   1345 1350 1355 1360
   Ala Gln Phe Ser Thr Pro Ala Ile Ile Asp Arg Leu Arg Gly His Asp
   1365 1370 1375
   Asp Ser Pro Asp Val Glu Gly Trp Pro Ser Gly Ala Thr Asp Pro Asp
   1380 1385 1390
   Val Tyr Leu Ile Ala Ala Ala Asp Pro Ala Asn Pro Tyr Gly Ala Ala
   1395 1400 1405
   Leu Pro Trp Pro Glu Gln Gly Pro Ser Arg Ala Ala Gly Ala Met Val
   1410 1415 1420
   Val Leu Cys Asp Gly Leu Leu Leu Ala His Leu Thr Arg Gly Gly Arg
   1425 1430 1435 1440
   Thr Leu Thr Val Phe Ser Asp Asn Ile Pro Lys Ile Ala Thr Ala Leu
   1445 1450 1455
   Ile Thr Tyr Glu Arg Leu Thr Val Glu Lys Ile Asn Gly Asp Asn Val
   1460 1465 1470
   Phe Asp Ser Pro Leu Leu Glu Gln Phe Arg Lys His Gly Ala Thr Ile
   1475 1480 1485
   Thr Pro Lys Gly Met Arg Phe Arg Pro Pro Val Ala Arg Glu Thr Pro
   1490 1495 1500
   Ser Asp Thr Leu Pro Thr Arg Thr Phe Arg Gly Gly Phe Gly Arg Arg
   1505 1510 1515 1520
<210> 103
   <211> 1251
   <212> DNA
   <213> Corynebacterium glutamicum
<220>
   <221> CDS
   <222> (1)..(1251)
   <223> RXA07006
<400> 103
   gtg tcg tct gag aaa gct tca tca aaa tca acc cct gag gca ccg tgg    48
   Val Ser Ser Glu Lys Ala Ser Ser Lys Ser Thr Pro Glu Ala Pro Trp
    1               5                  10                  15
   cca gtc cgg gaa gta aat act caa gtc aag cag tgg att gaa cgg ctt    96
   Pro Val Arg Glu Val Asn Thr Gln Val Lys Gln Trp Ile Glu Arg Leu
               20                  25                  30
   ggc cat ttg tgg gtg gag ggc cag ctc gct cag att aat gtg aag ccc   144
   Gly His Leu Trp Val Glu Gly Gln Leu Ala Gln Ile Asn Val Lys Pro
   35 40 45
   aat tgg aag ctg tcg tat ttg acg ctt cgt gat gtg gag caa gaa atg 192
   Asn Trp Lys Leu Ser Tyr Leu Thr Leu Arg Asp Val Glu Gln Glu Met
   50 55 60
   tct gtg cag ctg acc tgc ccg acg gac att atc cgc aat cgc ccc aca 240
   Ser Val Gln Leu Thr Cys Pro Thr Asp Ile Ile Arg Asn Arg Pro Thr
   65 70 75 80
   ccg ctc aag gat ggc gac cgc gtg att gtg tac ggc aag ccc gcg ttt 288
   Pro Leu Lys Asp Gly Asp Arg Val Ile Val Tyr Gly Lys Pro Ala Phe
   85 90 95
   tat gca ggc cgc ggc act ttt tcg ctg tgg gtg act gat atc cgt ccc 336
   Tyr Ala Gly Arg Gly Thr Phe Ser Leu Trp Val Thr Asp Ile Arg Pro
   100 105 110
   gtg ggt att ggt gag ttg ctg gcg cgc att gag gag ctg cgt aaa agg 384
   Val Gly Ile Gly Glu Leu Leu Ala Arg Ile Glu Glu Leu Arg Lys Arg
   115 120 125
   ctt gcc gcg gag ggt ctt ttt gat cca gct cgg aag aag cga ctg cca 432
   Leu Ala Ala Glu Gly Leu Phe Asp Pro Ala Arg Lys Lys Arg Leu Pro
   130 135 140
   ttt ctg ccc aac cgc gtt ggt ttg atc acg gga cgt ggt tca gcg gct 480
   Phe Leu Pro Asn Arg Val Gly Leu Ile Thr Gly Arg Gly Ser Ala Ala
   145 150 155 160
   gag cgc gat gtg ctg agc gtg gct aag gat cgc tgg ccg gaa gtg cag 528
   Glu Arg Asp Val Leu Ser Val Ala Lys Asp Arg Trp Pro Glu Val Gln
   165 170 175
   ttt gag gtg atc aac acg gca gtt cag ggc gct tca gct gtt cct gaa 576
   Phe Glu Val Ile Asn Thr Ala Val Gln Gly Ala Ser Ala Val Pro Glu
   180 185 190
   atc atc gaa gcg ttg cgg gtt tta gat cag gac cct cgc gtg gat gtc 624
   Ile Ile Glu Ala Leu Arg Val Leu Asp Gln Asp Pro Arg Val Asp Val
   195 200 205
   atc atc att gcc cgc ggc ggc ggt tct gtg gag gat ctg ctc ccc ttc 672
   Ile Ile Ile Ala Arg Gly Gly Gly Ser Val Glu Asp Leu Leu Pro Phe
   210 215 220
   tct gag gag gcc ttg cag cgc gca gtc gcg gca gcg cag acg ccc gtg 720
   Ser Glu Glu Ala Leu Gln Arg Ala Val Ala Ala Ala Gln Thr Pro Val
   225 230 235 240
   gtg tcc gcg att ggc cac gaa cca gat acg ccg gtg ttg gac aat gtc 768
   Val Ser Ala Ile Gly His Glu Pro Asp Thr Pro Val Leu Asp Asn Val
   245 250 255
   gcc gac ctt cgc gcg gcg acc ccg acc gat gca gca aag cgc gtg gtg 816
   Ala Asp Leu Arg Ala Ala Thr Pro Thr Asp Ala Ala Lys Arg Val Val
   260 265 270
   cct gat gtg gca gaa gaa cgc atg ttg atc aat cag ctt cgc agt cgt 864
   Pro Asp Val Ala Glu Glu Arg Met Leu Ile Asn Gln Leu Arg Ser Arg
   275 280 285
   agt gcc gcg gcg ttg cgc ggt tgg gtg cag cgc gag cag cag gcg ttg 912
   Ser Ala Ala Ala Leu Arg Gly Trp Val Gln Arg Glu Gln Gln Ala Leu
       290                 295                 300
   gca gcg att cgc acc agg ccg gtg ctg gct gat ccg atg acc ccg att       960
   Ala Ala Ile Arg Thr Arg Pro Val Leu Ala Asp Pro Met Thr Pro Ile
   305                 310                 315                 320
   aac cgc cga cgt gat gag att gcc cag gct gtg ggc ttg att agg cgc      1008
   Asn Arg Arg Arg Asp Glu Ile Ala Gln Ala Val Gly Leu Ile Arg Arg
                   325                 330                 335
   gat gtc acc cat ctc gtc cgc acc gag caa gca ctg gtg gcg tcg ttg      1056
   Asp Val Thr His Leu Val Arg Thr Glu Gln Ala Leu Val Ala Ser Leu
               340                 345                 350
   cgc gca cag gtt tcc gcg ctc ggc ccg tcc gca acc ttg gcg cgc ggt      1104
   Arg Ala Gln Val Ser Ala Leu Gly Pro Ser Ala Thr Leu Ala Arg Gly
           355                 360                 365
   tat tcc gtg gtg cag gtt att cct cgc gac ggc agc gcc ccg gaa gtg      1152
   Tyr Ser Val Val Gln Val Ile Pro Arg Asp Gly Ser Ala Pro Glu Val
       370                 375                 380
   gtc acc acc atc gag caa tca ccg ccc ggc agc cag ctg cgc atc cgc      1200
   Val Thr Thr Ile Glu Gln Ser Pro Pro Gly Ser Gln Leu Arg Ile Arg
   385                 390                 395                 400
   gtt gcc gac ggc tcc atc act gcg gca tcc atg ggc acc cag caa gca      1248
   Val Ala Asp Gly Ser Ile Thr Ala Ala Ser Met Gly Thr Gln Gln Ala
                   405                 410                 415
   aac                                                                  1251
   Asn
<210> 104
   <211> 417
   <212> PRT
   <213> Corynebacterium glutamicum
<400> 104
   Val Ser Ser Glu Lys Ala Ser Ser Lys Ser Thr Pro Glu Ala Pro Trp
   1               5                   10                  15
   Pro Val Arg Glu Val Asn Thr Gln Val Lys Gln Trp Ile Glu Arg Leu
               20                  25                  30
   Gly His Leu Trp Val Glu Gly Gln Leu Ala Gln Ile Asn Val Lys Pro
           35                  40                  45
   Asn Trp Lys Leu Ser Tyr Leu Thr Leu Arg Asp Val Glu Gln Glu Met
       50                  55                  60
   Ser Val Gln Leu Thr Cys Pro Thr Asp Ile Ile Arg Asn Arg Pro Thr
   65                  70                  75                  80
   Pro Leu Lys Asp Gly Asp Arg Val Ile Val Tyr Gly Lys Pro Ala Phe
                   85                  90                  95
   Tyr Ala Gly Arg Gly Thr Phe Ser Leu Trp Val Thr Asp Ile Arg Pro
               100                 105                 110
   Val Gly Ile Gly Glu Leu Leu Ala Arg Ile Glu Glu Leu Arg Lys Arg
   115 120 125
   Leu Ala Ala Glu Gly Leu Phe Asp Pro Ala Arg Lys Lys Arg Leu Pro
   130 135 140
   Phe Leu Pro Asn Arg Val Gly Leu Ile Thr Gly Arg Gly Ser Ala Ala
   145 150 155 160
   Glu Arg Asp Val Leu Ser Val Ala Lys Asp Arg Trp Pro Glu Val Gln
   165 170 175
   Phe Glu Val Ile Asn Thr Ala Val Gln Gly Ala Ser Ala Val Pro Glu
   180 185 190
   Ile Ile Glu Ala Leu Arg Val Leu Asp Gln Asp Pro Arg Val Asp Val
   195 200 205
   Ile Ile Ile Ala Arg Gly Gly Gly Ser Val Glu Asp Leu Leu Pro Phe
   210 215 220
   Ser Glu Glu Ala Leu Gln Arg Ala Val Ala Ala Ala Gln Thr Pro Val
   225 230 235 240
   Val Ser Ala Ile Gly His Glu Pro Asp Thr Pro Val Leu Asp Asn Val
   245 250 255
   Ala Asp Leu Arg Ala Ala Thr Pro Thr Asp Ala Ala Lys Arg Val Val
   260 265 270
   Pro Asp Val Ala Glu Glu Arg Met Leu Ile Asn Gln Leu Arg Ser Arg
   275 280 285
   Ser Ala Ala Ala Leu Arg Gly Trp Val Gln Arg Glu Gln Gln Ala Leu
   290 295 300
   Ala Ala Ile Arg Thr Arg Pro Val Leu Ala Asp Pro Met Thr Pro Ile
   305 310 315 320
   Asn Arg Arg Arg Asp Glu Ile Ala Gln Ala Val Gly Leu Ile Arg Arg
   325 330 335
   Asp Val Thr His Leu Val Arg Thr Glu Gln Ala Leu Val Ala Ser Leu
   340 345 350
   Arg Ala Gln Val Ser Ala Leu Gly Pro Ser Ala Thr Leu Ala Arg Gly
   355 360 365
   Tyr Ser Val Val Gln Val Ile Pro Arg Asp Gly Ser Ala Pro Glu Val
   370 375 380
   Val Thr Thr Ile Glu Gln Ser Pro Pro Gly Ser Gln Leu Arg Ile Arg
   385 390 395 400
   Val Ala Asp Gly Ser Ile Thr Ala Ala Ser Met Gly Thr Gln Gln Ala
   405 410 415
   Asn